(19) Europäisches Patentamt
European Patent Office
Office européen des brevets

(11) EP 4 393 923 A1

(12) # EUROPEAN PATENT APPLICATION
published in accordance with Art. 153(4) EPC

(43) Date of publication:
**03.07.2024 Bulletin 2024/27**

(21) Application number: **21968480.0**

(22) Date of filing: **21.12.2021**

(51) International Patent Classification (IPC):
***C07D 471/14*** *(2006.01)* ***A61K 31/437*** *(2006.01)*
***A61P 35/00*** *(2006.01)* ***A61P 35/02*** *(2006.01)*
***A61P 37/06*** *(2006.01)* ***A61P 17/06*** *(2006.01)*
***A61P 19/02*** *(2006.01)* ***A61P 1/00*** *(2006.01)*
***A61P 25/28*** *(2006.01)* ***A61P 7/00*** *(2006.01)*
***A61P 29/00*** *(2006.01)*

(52) Cooperative Patent Classification (CPC):
**A61K 31/437; A61P 1/00; A61P 7/00; A61P 17/06; A61P 19/02; A61P 25/28; A61P 29/00; A61P 35/00; A61P 35/02; A61P 37/06; C07D 471/14**

(86) International application number:
**PCT/CN2021/140062**

(87) International publication number:
**WO 2023/115327 (29.06.2023 Gazette 2023/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Yiyou Biotech (Shanghai) Co., Ltd.
Fengxian District, Shanghai 201413 (CN)**

(72) Inventors:
- **LI, Zhiyu**
**Nanjing, Jiangsu 210000 (CN)**
- **BIAN, Jinlei**
**Nanjing, Jiangsu 210000 (CN)**
- **JING, Tian**
**Nanjing, Jiangsu 210000 (CN)**
- **XU, Pengfei**
**Nanjing, Jiangsu 210000 (CN)**
- **SHEN, Pei**
**Nanjing, Jiangsu 210000 (CN)**
- **WANG, Jubo**
**Nanjing, Jiangsu 210000 (CN)**
- **QIU, Zhixia**
**Nanjing, Jiangsu 210000 (CN)**
- **XU, Xi**
**Nanjing, Jiangsu 210000 (CN)**

(74) Representative: **Murgitroyd & Company
165-169 Scotland Street
Glasgow G5 8PL (GB)**

(54) **PYRIDO RING COMPOUND, PREPARATION METHOD THEREFOR, INTERMEDIATE, COMPOSITION, AND APPLICATION**

(57) The present invention discloses a pyridocyclic compound as well as its preparation method, intermediates, compositions and applications. The pyridocyclic compound has the structure shown in Formula I below, which exhibits JAK kinase inhibitory activity and can be used to treat diseases associated with JAK kinase, such as autoimmune diseases or cancer.

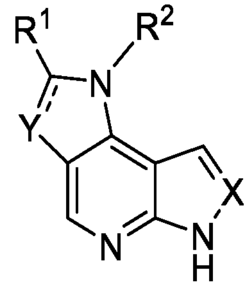

I



Processed by Luminess, 75001 PARIS (FR)

## Description

TECHNICAL FIELD

The present invention relates to a pyridocyclic compound and its preparation method, intermediates, compositions and uses thereof.

BACKGROUND

Janus kinases (JAK) belong to a family of tyrosine kinases involved in inflammation, autoimmune diseases, proliferative diseases, graft rejection, diseases associated with impaired cartilage renewal, congenital cartilage malformations and/or diseases associated with excessive IL6 secretion.

JAK kinases are cytoplasmic tyrosine kinases that transduce cytokine signals from membrane receptors to STAT transcription factors. The prior art has described four JAK kinase family members: JAK1, JAK2, JAK3, and TYK2. When cytokines bind to their receptors, JAK family members autophosphorylate and/or transphosphorylate each other, followed by phosphorylation of STATs, which then migrate into the nucleus to regulate transcription. JAK-STAT intracellular signaling applies to interferons, most interleukins, and a variety of cytokines and endocrine factors, such as EPO, TPO, GH, OSM, LIF, CNTF, GM-CSF, and PRL.

Combinatorial studies using genetic models and small molecule JAK inhibitors have revealed the therapeutic potential of several JAKs. JAK3 was confirmed as an immunosuppressive target by mouse and human genetics. JAK3 inhibitors were successfully used in clinical development, initially for organ transplant rejection but were subsequently used for other immunoinflammatory indications such as rheumatoid arthritis (RA), psoriasis, and Crohn's disease. TYK2 is a potential target for immunoinflammatory diseases and has been confirmed by human genetics and gene knockout studies in mice. JAK2 is currently an effective target in the treatment of myeloproliferative diseases, with two drugs for the treatment of myelofibrosis already on the market. JAK1 is a novel target in the field of immunoinflammatory diseases as the heterodimerization of JAK1 with other JAKs is known to transduce cytokine-driven pro-inflammatory signaling. Thus, the inhibition of JAK1 and/or other JAKs is expected to be therapeutically beneficial for a range of inflammatory conditions and other diseases driven by JAK-mediated signaling.

Tofacitinib was developed by Pfizer Inc. and successfully launched in the United States on November 7, 2012 for the treatment of rheumatoid arthritis under the trade name Xelj anz.

Ruxolitinib, jointly developed by incyte and Novartis, was launched in the United States in 2011 for the treatment of myelofibrosis under the trade name Jakafi.

Baricitinib, jointly developed by Incyte and Eli Lilly, was launched in the United States in 2018 for the treatment of rheumatoid arthritis under the trade name Olumiant.

Upadacitinib, a selective JAK1 inhibitor developed by AbbVie and marketed in 2019 for the treatment of rheumatoid arthritis, has been reported in the literature to have activity of JAK1 $IC_{50}$ = 43 nM and JAK2 $IC_{50}$ = 200 nM.

Fedratinib is a selective JAK2 inhibitor developed by Sanofi and launched in 2019 for the treatment of myelofibrosis, has been reported in the literature to have an activity of JAK2 $IC_{50}$ = 3 nM.

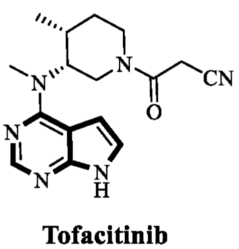
**Tofacitinib**

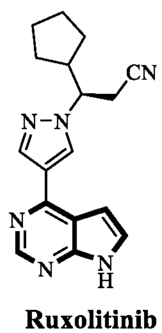
**Ruxolitinib**

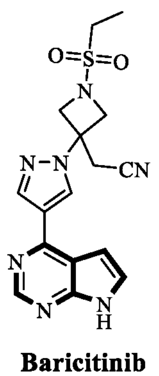
**Baricitinib**

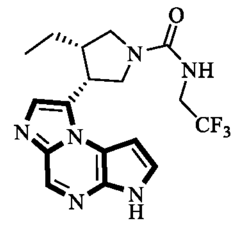

Upadacitinib

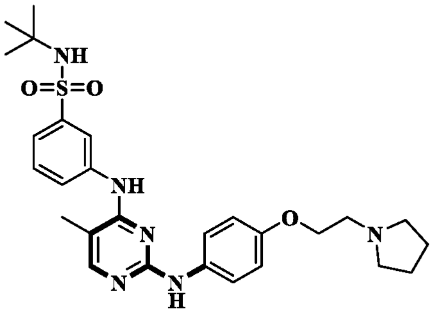

Fedratinib

.

CONTENT OF THE PRESENT INVENTION

The present invention provides a pyridocyclic compound and its preparation method, intermediates, compositions and uses.

On one hand, the present invention provides a compound shown in formula I.

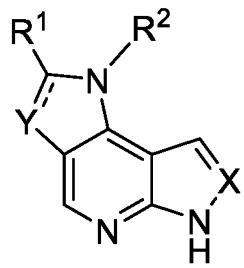

I

or a tautomer, stereoisomer, racemate or isotopic derivative thereof, or a pharmaceutically acceptable salt of any of the foregoing (referring to the foregoing compound as shown in Formula I, its tautomer, stereoisomer, racemate or isotopic derivative), or a crystalline or solvated form of any of the foregoing (referring to the foregoing compound as shown in Formula I, its tautomer, stereoisomer, racemate, isotopic derivative or pharmaceutically acceptable salt as shown in Formula I).

wherein X is CH or N.

Y is NH or N; when Y is N, the --- connected to Y is a double bond; when Y is NH, the --- connected to Y is a single bond.

$R^1$ and $R^2$ are defined as (i), (ii) or (iii) as follows.

(i) $R^1$ is (e.g.[1]

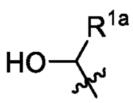

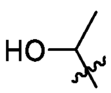

,

e.g.

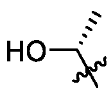

or

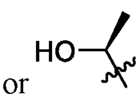

), $R^2$ is

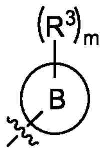

(e.g.

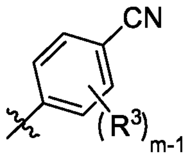

,

e.g.

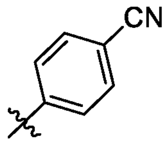

)

or 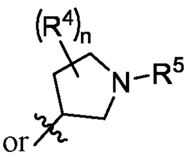

(e.g.

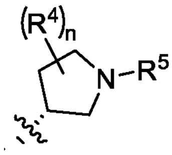

or

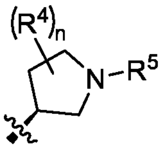

).

Ring B is a benzene ring or a 5-6-membered heteroaromatic ring.

$R^{1a}$ is a $C_{1-3}$ alkyl group (e.g., methyl group).

Each $R^3$ is independently a halogen (e.g., fluorine), a cyano group, a $C_{1-4}$ alkyl group (e.g., methyl group), a $C_{1-4}$ haloalkyl group (e.g., $C_{1-4}$ fluoroalkyl group, e.g., $-CF_3$ ), a $-O-C_{1-4}$ alkyl group (e.g., $-O-CH_3$ ), or a $-O-C_{1-4}$ haloalkyl group (e.g. a $-O-C_{1-4}$ fluoroalkyl group, e.g. $-OCF_3$ ).

Each $R^4$ is independently a halogen (e.g., fluorine), a hydroxyl group, a $C_{1-4}$ alkyl group (e.g., methyl group), a $C_{1-4}$ haloalkyl group (e.g., $C_{1-4}$ fluoroalkyl group, e.g., $-CF_3$ ), an $-O-C_{1-4}$ alkyl group (e.g., $-O-CH_3$ ), a $-O-C_{1-4}$ haloalkyl group (e.g. $-O-C_{1-4}$ fluoroalkyl group, e.g. $-OCF_3$ ) or a $C_{1-4}$ hydroxyalkyl group (e.g. $-CH_2OH$).

Alternatively, two $R^4$ located on the same carbon atom or on different carbon atoms are interconnected to form $-CH_2-$ or $-(CH_2)_2-$ (e.g., to form

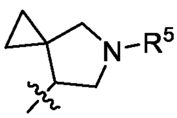

,

for example

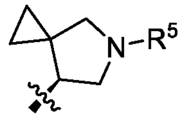
or
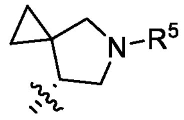

).

$R^5$ is $-S(O)_2R^{5a}$, $-C(O)R^{5b}$, $-C(O)NR^{5c}R^{5d}$, $-C(O)OR^{5e}$, $-C(O)NHR^{5k}$ or $-L^1-R^{5f}$;

$R^{5a}$ is a $C_{2-6}$ alkyl group (e.g. ethyl, n-propyl or n-butyl group), a $C_{2-6}$ haloalkyl group, $-L^1-R^{5f}$ , an unsubstituted or substituted 6-10-membered aryl group (said 6-10-membered aryl group e.g. phenyl or naphthyl group) or an unsubstituted or substituted 5 -10-membered heteroaryl group (said 5-10-membered heteroaryl group e.g. 5-6-membered heteroaryl group), said substituted 6-10-membered aryl group and substituted 5-10-membered heteroaryl group are defined as structures where 1, 2, 3 or 4 hydrogen atoms in the 6-10-membered aryl group and the 5-10-membered heteroaryl group are independently substituted by $R^{5g}$.

$R^{5b}$ , $R^{5c}$ , $R^{5d}$ , $R^{5e}$ and $R^{5k}$ are each independently a $C_{1-6}$ alkyl group, a $C_{1-6}$ haloalkyl group, $-L^1-R^{5f}$ , an unsubstituted or substituted 6-10-membered aryl group (said 6-10-membered aryl group e.g. phenyl or naphthyl group) or an unsubstituted or substituted5-10 membered heteroaryl group (said 5-10-membered heteroaryl group e.g. 5-6-membered heteroaryl group), said substituted 6-10-membered aryl group and substituted 5-10-membered heteroaryl group are defined as structures where 1, 2, 3 or 4 hydrogen atoms in the 6-10-membered-aryl group and the 5-10-membered-heteroaryl group are independently substituted by $R^{5h}$.

Each $R^{5g}$ and $R^{5h}$ is independently a halogen (e.g., fluorine), a cyano group, a $C_{1-4}$ alkyl group (e.g., methyl group), a $C_{1-4}$ haloalkyl group (e.g., a $C_{1-4}$ fluoroalkyl group, e.g., - $CF_3$ ), a $-O-C_{1-4}$ alkyl group (e.g., $-O-CH_3$ ), a $-O-C_{1-4}$ haloalkyl group (e.g. $-OCF_3$ ) or a phenyl group.

Each $L^1$ is independently $-[C(R^aR^b)]_{1-5}-$ (e.g. $-CH_2-$, $-(CH_2)_2-$, $-(CH_2)_3-$, $-(CH_2)_4-$, - $(CH_2)_5-$ or

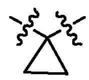

), $-[C(R^aR^b)]_{1-2}-C(O)-[C(R^aR^b)]_{1-2}-$, $-[C(R^aR^b)]_{1-2}-C(O)NH-[C(R^aR^b)]_{1-2}-$(e.g. $-C(R^aR^b)-C(O)NH-C(R^aR^b)-R^{5f}$), $-[C(R^aR^b)]_{1-2}-NHC(O)-[C(R^aR^b)]_{1-2}-$, $-[C(R^aR^b)]_{1-2}-S(O)_2-[C(R^aR^b)]_{1-2}-$, $-[C(R^aR^b))]_{1-2}-NHS(O)_2-[C(R^a{}_R{}^b)]_{1-2}-$ or $-[C(R^aR^b)]_{1-2}-S(O)_2NH-[C(R^aR^b)]_{1-2}-$.

Each $R^{5f}$ is independently H, F, $CRF_2$, $CH_2F$, $CF_3$ or CN.

Each $R^a$ is independently H, a halogen (e.g., fluorine), or a $C_{1-3}$ alkyl group(e.g., methyl or ethyl group).

Each $R^b$ is independently H, a halogen (e.g., fluorine), or a $C_{1-3}$ alkyl group (e.g., methyl or ethyl group).

Alternatively, $R^a$ and $R^b$ along with the carbon atom joining them, form a cyclopropyl group.

m is 0, 1, 2, 3 or 4.

n is 0, 1, 2, 3 or 4.

(ii) $R^1$ is

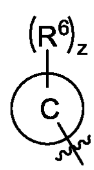

and $R^2$ is

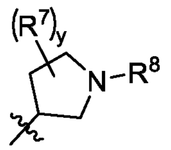

(e.g.

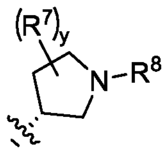 or 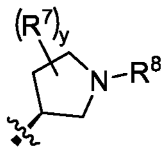

) or

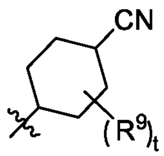

(e.g. *trans* or *cis*).

Ring C is a benzene ring or a 5-6-membered heteroaromatic ring (e.g., imidazole, thiazole, furan, thiophene, or pyridine, e.g.,

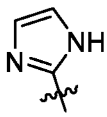 , 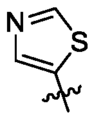 , 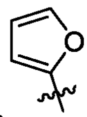 , 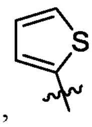 , or 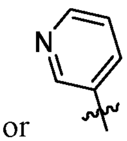

).

Each $R^6$ is independently a halogen (e.g., fluorine or chlorine, e.g., fluorine), a hydroxyl group, an amino group, a cyano group, a $C_{1-4}$ alkyl group (e.g., methyl group), a $C_{1-4}$ haloalkyl group (e.g., $C_{1-4}$ fluoroalkyl group, e.g., $CF_3$ ), an -O-$C_{1-4}$ alkyl group (e.g., -O-$CH_3$), an O-$C_{1-4}$ haloalkyl group (e.g. -O-$C_{1-4}$ fluoroalkyl group, e.g. -O-$CF_3$ ), a -S-$C_{1-4}$ alkyl group (e.g. -S-$CH_3$), a -$S(O)_2$-$C_{1-4}$ alkyl group (e.g. -$S(O)_2$-$CH_3$) or a $C_{1-4}$ hydroxyalkyl group (e.g. -$CH_2$-OH).

Each $R^7$ is independently $R^4$ (i.e., $R^7$ and $R^4$ in the compound as shown in Formula **I** in any of the embodiments of the present invention have the same definition).

Alternatively, two $R^7$ located on the same carbon atom or on different carbon atoms are interconnected to form -$CH_2$ - or -$(CH_2)_2$-.

$R^8$ is $R^5$ (i.e., $R^8$ and $R^5$ in the compound as shown in Formula **I** in any of the embodiments of the present invention have the same definition), -$S(O)_2R^{8a}$, -C(O)$R^{8b}$, - C(O)N$R^{8c}R^{8d}$, -C(O)O$R^{8e}$ or -C(O)NH$R^{8k}$.

$R^{8a}$, $R^{8b}$, $R^{8c}$, $R^{8d}$, $R^{8e}$ and $R^{8k}$ are each independently a methyl group, -$CF_3$, a $C_{2-6}$ alkenyl group (e.g. vinyl group) or a $C_{3-6}$ cycloalkyl group (e.g. cyclopropyl, cyclobutyl or cyclopentyl group).

Each $R^9$ is independently $R^4$.

Alternatively, two $R^9$ located on the same carbon atom or on different carbon atoms are interconnected to form -$CH_2$ - or -$(CH_2)_2$-.

z is 0, 1, 2, 3 or 4.

y is 0, 1, 2, 3 or 4.

t is 0, 1, 2, 3 or 4.

(iii) $R^1$ is H, $CF_3$ or

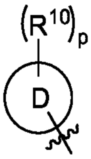

and $R^2$ is

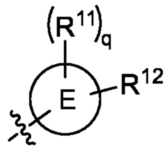

.

Ring D is a $C_{3-6}$ cycloalkyl group (e.g., cyclopropyl or cyclobutyl group), a benzene or a 5-6-membered heteroaromatic ring (e.g., furan or thiophene, e.g.,

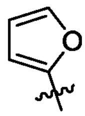 or 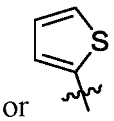

).

Ring E is a benzene ring or a 5-6-membered heteroaromatic ring (e.g., furan or thiophene, e.g.,

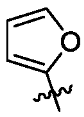 or 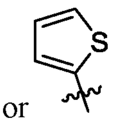

).

Each $R^{10}$ and $R^{11}$ is independently a halogen (e.g., fluorine or chlorine, e.g., fluorine), a hydroxyl group, an amino group, a cyano group, a $C_{1-4}$ alkyl group(e.g., methyl group), a $C_{1-4}$ haloalkyl group (e.g., $C_{1-4}$ fluoroalkyl group, e.g., $CF_3$ ), a $-O-C_{1-4}$ alkyl group (e.g., $-O-CH_3$ ), a $-O-C_{1-4}$ haloalkyl group (e.g. $-O-C_{1-4}$ fluoroalkyl group, e.g. $-O-CF_3$ ), a $-S-C_{1-4}$ alkyl group (e.g. $-S-CH_3$ ), a $-S(O)_2-C_{1-4}$ alkyl group (e.g. $-S(O)_2-CH_3$ ) or a $C_{1-4}$ hydroxyalkyl group (e.g. $-CH_2-OH$).

$R^{12}$ is a cyano group, $-L^3-R^{12f}$ or

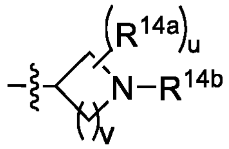

.

Each $R^{14a}$ is independently a halogen (e.g., fluorine), a cyano group, a $C_{1-4}$ alkyl group (e.g., methyl group), a cyano-substituted $C_{1-4}$ alkyl group (e.g., $-CH_2CN$), a $C_{1-4}$ haloalkyl group (e.g., $C_{1-4}$ fluoroalkyl group, e.g., $CF_3$ ), a $-O-C_{1-4}$ alkyl group (e.g., $-O-CH_3$ ), a $-O-C_{1-4}$ haloalkyl group (e.g. $-O-C_{1-4}$ fluoroalkyl group, e.g. $-O-CF_3$ ), or a $C_{1-4}$ hydroxyalkyl group (e.g. $-CH_2OH$).

$R^{14b}$ is $R^5$ (i.e., $R^{14b}$ and $R^5$ in the compound in any of the embodiments of the present invention as shown in Formula **I** have the same definition) or $-S(O)_2-C_{1-4}$ alkyl (e.g., $-S(O)_2$ - $CH_2CH_3$, $-S(O)_2-CH_2CH_2CH_3$ or $-S(O)_2$ $-CH_2CH_2CH_2CH_3$).

$L^3$ is $-[C(R^eR^f)]_{1-5}-$ (e.g. $-CH_2-$, $-(CH_2)_2-$, $-(CH_2)_3-$, $-(CH_2)_4-$ or $-(CH_2)_5-$), -C(O)-, - C(O)NH-, -NHC(O)-, $-S(O)_2$ -, $-NHS(O)_2$ -, $-S(O)_2NH-$, $-[C(R^eR^f)]_{1-2}-C(O)-[C(R^eR^f)]_{1-2}-$, - $[C(R^eR^f)]_{1-2}-C(O)NH-[C(R^eR^f)]_{1-2}-$, $-[C(R^eR^f)]_{1-2}-NHC(O)-[C(R^eR^f)]_{1-2}-$, $-[C(R^eR^f)]_{1-2}-S(O)_2-[C(R^eR^f)]_{1-2}-$, $-[C(R^eR^f)]_{1-2}-NHS(O)_2-[C(R^eR^f)]_{1-2}-$ or

-[C($R^eR^f$)]$_{1-2}$-S(O)$_2$NH-[C($R^eR^f$)]$_{1-2}$-.

$R^{12f}$ is H, F, $CHF_2$, $CH_2F$, $CF_3$ or CN.

Each $R^e$ and $R^f$ is independently H, a halogen (e.g., fluorine), a $C_{1-3}$ alkyl group (e.g., methyl or ethyl group), or a $C_{3-6}$ cycloalkyl group (e.g., cyclopropyl, cyclobutyl, or cyclopentyl group).

Alternatively, $R^e$ and $R^f$ along with their connecting carbon atom, form a cyclopropyl group.

p is 0, 1, 2, 3 or 4.
q is 0, 1, 2, 3 or 4.
v is 0, 1, 2 or 3.
u is 0, 1 or 2.

The heteroatom in the above heteroaryl group is independently N, O or S, and the number of heteroatoms in the above heteroaryl group is independently 1, 2, 3 or 4.

In some embodiments, said compound shown in formula **I** has the structure shown in formula **I-1** as follows.

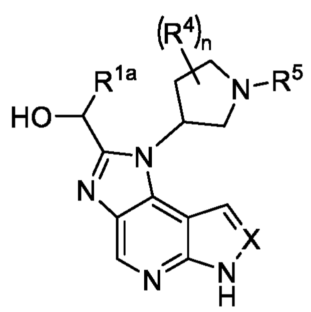

**I-1**

wherein $R^{1a}$, $R^4$, $R^5$, X and n are defined as described in the compound shown in Formula **I as** defined in any of the embodiments of the present invention.

In some embodiments, a compound as shown in Formula **I** or **I-1** has a structure as shown in Formula **I-1C, I-1D, I-1E** or **I-1F as** follows.

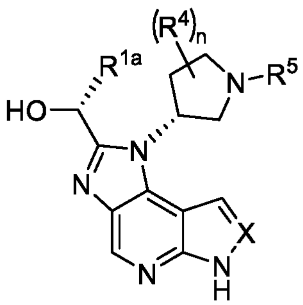

**I-1C**
,

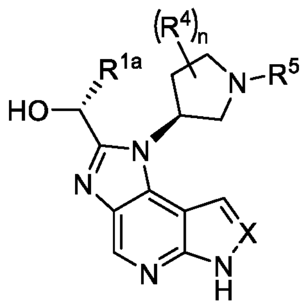

**I-1D**
,

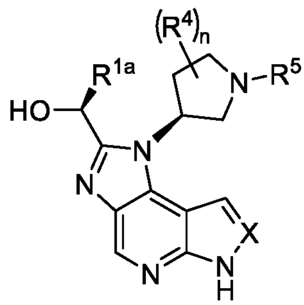

**I-1E**
,

and

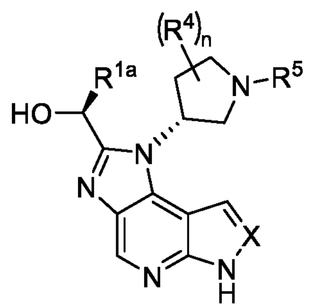

**I-1F**

wherein $R^{1a}$, $R^4$, $R^5$, X and n are defined as described in the compound shown in Formula **I as** defined in any of the embodiments of the present invention.

In some embodiments, said compound shown in formula **I** or **I-1** has the structure shown in formula **I-1a, I-1b, I-1c** or **I-1d** as follows, preferably having the structure shown in **I-1d.**

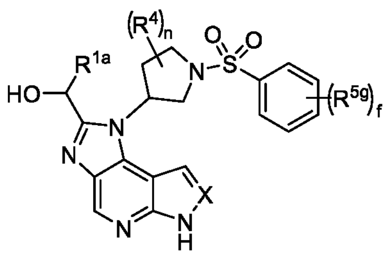

**I-1a**

,

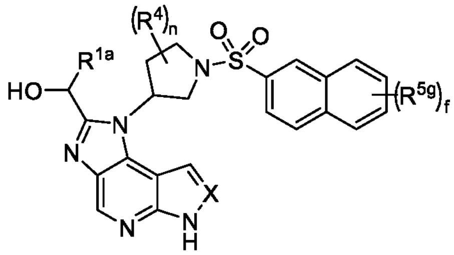

**I-1b**

,

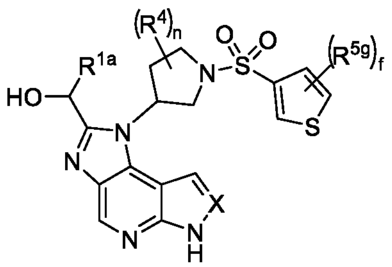

**I-1c**

,

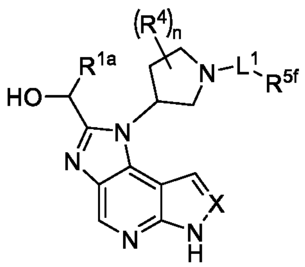

**I-1d**

.

wherein $R^{1a}$, $R^4$, $R^{5g}$, X, n, $L^1$ and $R^{5f}$ are defined as described in the compound shown in Formula **I as** defined in any of the embodiments of the present invention.

Each f is independently 0, 1, 2, 3 or 4, e.g. 0 or 1.

In some embodiments, said compound shown in formula **I** has the structure shown in formula **I-2, I-2A** or **I-2B as** follows.

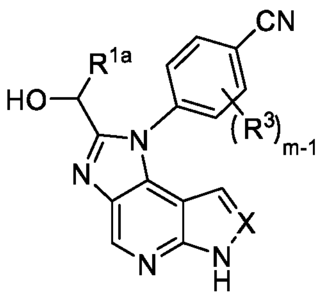

**I-2**

,

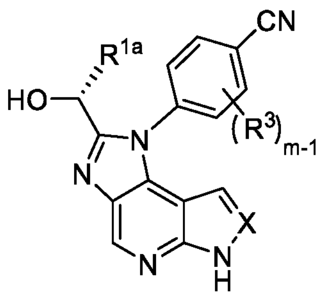

**I-2A**

,

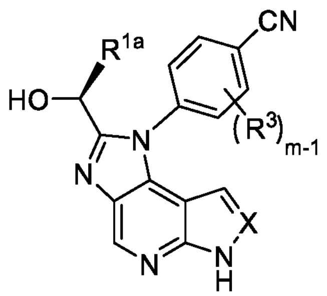

**I-2B**

wherein $R^{1a}$, $R^3$, X and m are defined as described in the compound shown in Formula **I as** defined in any of the embodiments of the present invention.

In some embodiments, as in the compounds shown in Formula **I, I-1, I-1C, I-1D, I-1E, I-IF, I-1a, I-1b, I-1c**, **I-1d**, **I-2, I-2A,** or **I-2B,** X is CH.

In some embodiments, as in the compounds shown in Formula **I, I-1, I-1C**, **I-1D, I-1E, I-1F, I-1a, I-1b, I-1c, I-1d, I-2, I-2A,** or **I-2B,** X is N.

In some embodiments, as in the compounds shown in Formula **I, I-1, I-1C**, **I-1D, I-1E, I-1F, I-1a, I-1b, I-1c, I-1d, I-2, I-2A,** or **I-2B,** $R^{1a}$ is a methyl group.

In some embodiments, , as in the compounds shown in Formula **I, I-1, I-1a, I-1b, I-1c, I-1d,** or **I-2,**

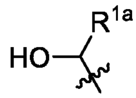

is

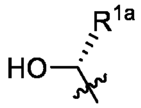 or 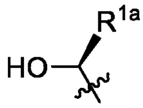 ,

preferably

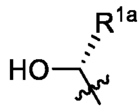 .

(i) In some embodiments, as in the compounds shown in Formula **I, I-2, I-2A** or **I-2B,** m is 1.

In some embodiments, as in the compounds shown in Formula **I, I-1, I-1a, I-1b, I-1c,** or **I-1d,**

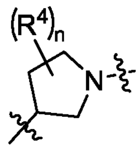

is

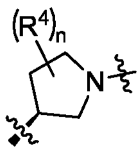 or 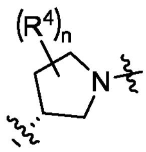 .

In some embodiments,

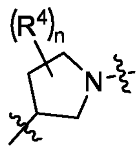

is

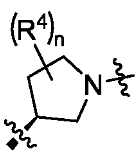 .

In some embodiments,

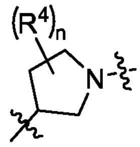

is

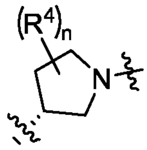

.

In some embodiments, as in the compounds shown in Formula **I, I-1, I-1C, I-1D, I-1E, I-1F, I-1a, I-1b, I-1c** or **I-1d,** each $R^4$ is independently a halogen (e.g., fluorine) or a $C_{1-4}$ hydroxyalkyl group (e.g., $-CH_2OH$), or, alternatively, two $R^4$ located at the same carbon atom or at different carbon atoms are interconnected to form $-CH_2-$ or $-(CH_2)_2-$.

In some embodiments, as in the compounds shown in Formula **I, I-1, I-1C, I-1D, I-1E, I-1F**, **I-1a, I-1b, I-1c**, **I-1d**, **I-2, I-2A,** or **I-2B,** n is 0, 1, or 2.

In some embodiments, as in the compounds shown in Formula **I, I-1, I-1C, I-1D, I-1E, I-1F, I-1a, I-1b, I-1c,** or **I-1d,**

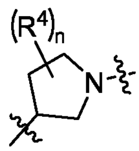

is

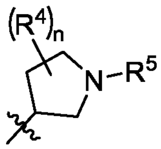

is

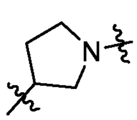

,

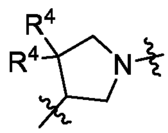

(e.g.,

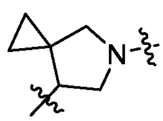

),

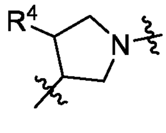

(e.g.,

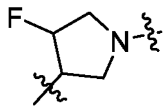

),

or 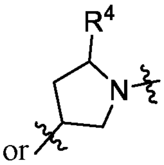

(e.g.,

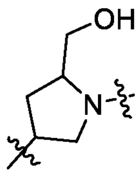

).

In some embodiments, as in the compounds shown in Formula **I, I-1, I-1C, I-1D, I-1E, I-1F, I-1a, I-1b, I-1c,** or **I-1d,**

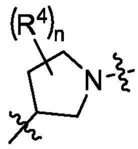

is

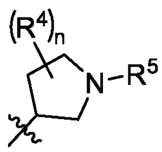

is

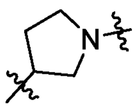 , 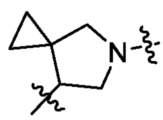 ,

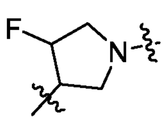 , or 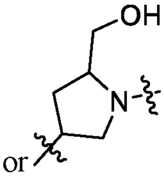 .

In some embodiments, the ring B is a benzene ring in the compound as shown in Formula **I.**

In some embodiments, m is 1 in the compound as shown in Formula **I.**

In some embodiments, as in the compounds shown in Formula **I, I-1, I-1C, I-1D, I-1E** or **I-1F,** $R^{5a}$ is a $C_{2-6}$ alkyl

group (e.g., ethyl, n-propyl or n-butyl group), an unsubstituted or substituted 6-10-membered aryl group (said 6-10-membered aryl group e.g., phenyl or naphthyl group) or an unsubstituted or substituted 5-10-membered heteroaryl group (said 5-10-membered heteroaryl group e.g. 5-6-membered heteroaryl group).

In some embodiments, as in the compounds shown in Formula **I, I-1, I-1C, I-1D, I-1E,** or **I-1F,** the "unsubstituted or substituted 6-10-membered aryl group" in the definition of $R^{5a}$ may be "unsubstituted or substituted phenyl group", or "unsubstituted or substituted naphthyl group", wherein said unsubstituted or substituted phenyl group may be, for example, an unsubstituted phenyl group, or a phenyl group substituted with 1, 2, 3 and 4 (e.g., 1) of the functional groups independently selected from the following: halogens (e.g., fluorine), $C_{1-4}$ alkyl groups (e.g., methyl), -O-$C_{1-4}$ haloalkyl groups (e.g., -$OCF_3$ ) and phenyl groups, , such as

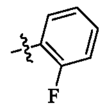 , , 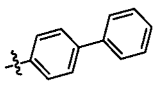 or 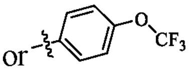 .

Wherein, said unsubstituted or substituted naphthyl group may be, for example, an unsubstituted naphthyl group, such as

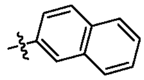 .

In some embodiments, the "unsubstituted or substituted 6-10-membered-aryl group" in $R^{5a}$ is preferably a -$OCF_3$ substituted phenyl group (e.g.

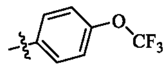

).

In some embodiments, as in the compounds shown in Formula **I, I-1, I-1C, I-1D, I-1E** or **I-1F,** "unsubstituted or substituted 5-10-membered-heteroaryl group" in the definition of $R^{5a}$ may be "unsubstituted or substituted 5-6-membered heteroaryl group", such as an unsubstituted or substituted thiophenyl group, preferably

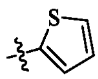 .

In some embodiments, as in the compound shown in Formula **I, I-1, I-1C, I-1D, I-1E, I-1F, I-1a, I-1b,** or **I-1c**, each $R^{5g}$ is independently a halogen (e.g., fluorine), a $C_{1-4}$ alkyl group (e.g., methyl group), a -O-$C_{1-4}$ haloalkyl group (e.g. -$OCF_3$) or a phenyl group, preferably - $OCF_3$.

In some embodiments, as in the compounds shown in Formula **I, I-1, I-1C, I-1D, I-1E** or **I-1F,** -$S(O)_2R^{5a}$ in the definition of $R^5$ is -$S(O)_2(CH_2)_2$-$CF_3$, -$S(O)_2(CH_2)_2$-$CH_3$, - $S(O)_2CH_2$-$CH_3$, -$S(O)_2(CH_2)_3$-$CH_3$, -$S(O)_2(CH_2)_2$-$CF_3$ ,

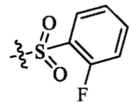 , 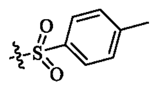 ,

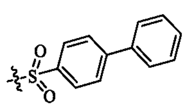 , 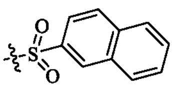 , 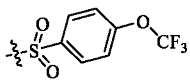 or 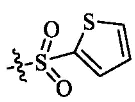

preferably -$S(O)_2(CH_2)_2$-$CF_3$, - $S(O)_2(CH_2)_2$-$CH_3$, -$S(O)_2CH_2$-$CH_3$, -$S(O)_2(CH_2)_3$-$CH_3$, -$S(O)_2(CH_2)_2$-$CF_3$ ,

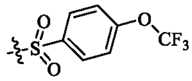

or

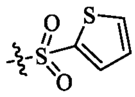

.

In some embodiments, as in the compounds shown in Formula **I, I-1, I-1C, I-1D, I-1E,** or **I-1F,** $R^{5b}$ is -$L^1$-$R^{5f}$, or an unsubstituted or substituted phenyl group.

In some embodiments, as in the compounds shown in Formula **I, I-1, I-1C, I-1D, I-1E,** or **I-1F,** -$L^1$-$R^{5f}$ in the definition of $R^{5b}$ is -C($R^aR^b$)-$R^{5f}$, for example

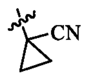

.

In some embodiments, as in the compounds shown in Formula **I, I-1, I-1C, I-1D, I-1E** or **I-1F, the** "unsubstituted or substituted 6-10-membered aryl group" in the definition of $R^{5b}$ may be "unsubstituted or substituted phenyl group", or "unsubstituted or substituted naphthyl group"; wherein unsubstituted or substituted phenyl group may be, for example, an unsubstituted phenyl group, or a phenyl group substituted with 1, 2, 3 and 4 (e.g., 1) of the functional groups independently selected from the following: halogens (e.g., fluorine) and cyano-substituted phenyl groups such as

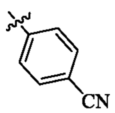 or 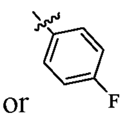

In some embodiments, as in the compounds shown in Formula **I, I-1, I-1C, I-1D, I-1E,** or **I-1F,** the -C(O)$R^{5b}$ in the definition of $R^5$ is

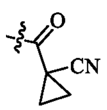 , 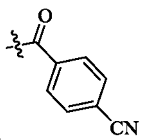 , or 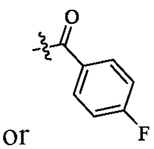 .

In some embodiments, as in the compounds shown in Formula **I, I-1, I-1C, I-1D, I-1E,** or **I-1F,** $R^{5e}$ is a $C_{1-6}$ alkyl group, such as tert-butyl group.

In some embodiments, as in the compounds shown in Formula **I, I-1, I-1C, I-1D, I-1E,** or **I-1F,** -C(O)O$R^{5e}$ in the definition of $R^5$ is -C(O)OC$(CH_3)_2$-$CH_3$.

In some embodiments, as in the compounds shown in Formula **I, I-1, I-1C, I-1D, I-1E,** or **I-1F,** $R^{5c}$ , $R^{5d}$ , and $R^{5k}$ are each independently -$L^1$-$R^{5f}$.

In some embodiments, as in the compounds shown in Formula **I, I-1, I-1C, I-1D, I-1E** or **I-1F,** -$L^1$-$R^{5f}$ in the definitions of $R^{5c}$ , $R^{5d}$ and $R^{5k}$ are -C($R^aR^b$)-$R^{5f}$ , e.g. -$CH_2$-$R^{5f}$, e.g. -$CH_2$-$CF_3$.

In some embodiments, as in the compounds shown in Formula **I, I-1, I-1C, I-1D, I-1E,** or **I-1F,** -C(O)NH$R^{5k}$ in the definition of $R^5$ is -C(O)NHC($R^aR^b$)-$R^{5f}$ , e.g. -C(O)NH$CH_2$-$R^{5f}$ , e.g. -C(O)NH$CH_2$-$CF_3$ .

In some embodiments, as in the compounds shown in Formula **I, I-1, I-1C, I-1D, I-1E** or **I-1F,** -$L^1$-$R^{5f}$ in the definition of $R^5$ is -$[C(R^aR^b)]_{1-5}$-$R^{5f}$ or -$[C(R^aR^b)]_{1-2}$-C(O)NH-$[C(R^aR^b)]_{1-2}$-, preferably -C($R^aR^b$)-$R^{5f}$, -$[C(R^aR^b)]_2$-$R^{5f}$, -$[C(R^aR^b)]_3$-$R^{5f}$, -$[C(R^aR^b)]_4$-$R^{5f}$ or - $[C(R^aR^b)]_5$-$R^{5f}$, further preferably -$[C(R^aR^b)]_2$-$R^{5f}$ or -$[C(R^aR^b)]_3$-$R^{5f}$.

In some embodiments, as in the compounds shown in Formula **I, I-1, I-1C, I-1D, I-1E,** or **I-1F,** -$L^1$-$R^{5f}$ in the definition of $R^5$ is -C($R^aR^b$)-$R^{5f}$, -$[C(R^aR^b)]_2$-$R^{5f}$ , -$[C(R^aR^b)]_3$-$R^{5f}$, - $[C(R^aR^b)]_4$-$R^{5f}$, -$[C(R^aR^b)]_5$-$R^{5f}$ or -C($R^aR^b$)-C(O)NH-C($R^aR^b$)-$R^{5f}$, preferably -$[C(R^aR^b)]_2$-$R^{5f}$ or -$[C(R^aR^b)]_3$-$R^{5f}$.

In some embodiments, as in the compounds shown in Formula **I, I-1, I-1C, I-1D, I-1E, or I-1F,** each $R^{5f}$ is independently F, $CF_3$ , or CN. In some embodiments, each $R^{5f}$ is independently F. In some embodiments, each $R^{5f}$ is

independently $CF_3$. In some embodiments, each $R^{5f}$ is independently CN.

In some embodiments, as in the compounds shown in Formula **I, I-1, I-1C, I-1D, I-1E,** or **I-1F,** $-L^1-R^{5f}$ in the definition of $R^5$ is $-C(R^aR^b)-CN$, $-[C(R^aR^b)]_2-CN$, $-[C(R^aR^b)]_3-CN$, $-[C(R^aR^b)]_4-CN$, $-[C(R^aR^b)]-CF_3$, $-[C(R^aR^b)]_2-CF_3$, $-[C(R^aR^b)]_3-CF_3$, $-C(R^aR^b)C(O)NH-C(R^aR^b)CF_3$ or $-[C(R^aR^b)]_5-F$, preferably $-C(R^aR^b)-CN$, $-[C(R^aR^b)]_2-CN$, $-[C(R^aR^b)]_3-CN$, $- [C(R^aR^b)]_4-CN$, $-[C(R^aR^b)]-CF_3$, $-[C(R^aR^b)]_2-CF_3$, $-[C(R^aR^b)]_3-CF_3$, or $-[C(R^aR^b)]_5-F$.

In some embodiments, as in the compounds shown in Formula **I, I-1, I-1C**, **I-1D, I-1E** or **I-1F,** each $R^a$ is independently H, fluorine, a methyl or ethyl group; each $R^b$ is independently H, fluorine, a methyl or ethyl group; or, $R^a$ and $R^b$ along with the carbon atom connecting them, form a cyclopropyl group together.

In some embodiments, as in the compounds shown in Formula **I, I-1, I-1C**, **I-1D, I-1E** or **I-1F,** each $R^a$ is independently H, fluorine, a methyl group or an ethyl group.

In some embodiments, as in the compounds shown in **Formula I, I-1, I-1C, I-1D, I-1E** or **I-1F,** each $R^a$ is independently H or fluorine.

In some embodiments, as in the compounds shown in Formula **I, I-1, I-1C**, **I-1D, I-1E** or **I-1F,** each $R^b$ is independently H, fluorine, a methyl group or an ethyl group.

In some embodiments, as in the compounds such as those shown in Formula **I, I-1, I-1C, I-1D, I-1E** or **I-1F,** each $R^b$ is independently H, fluorine, a methyl group or an ethyl group.

In some embodiments, as in the compounds shown in Formula **I, I-1, I-1C**, **I-1D, I-1E** or **I-1F,** $-L^1-R^{5f}$ in the definition of $R^5$ is $-CH_2-CN$, $-(CH_2)_2-CN$, $-(CH_2)_3-CN$, $-(CH_2)_4-CN$, $-(CH_2)_2-CF_3$, $-CH_2(CF_2)_2-CF_3$, $-(CF_2)_3-CF_3$, $-(CH_2)_2CF_2-CF_3$, $-(CH_2)_3-CF_3$ , $- CH(CH_2CH_3)C(O)NH-CH_2CF_3$, $-CH_2C(O)NHCH_2-CF_3$, $-CH(CH_3)C(O)NHCH_2-CF_3$ or $- (CH_2)_5-F$, preferably $-(CH_2)_2-CN$, $-(CH_2)_3-CN$, $-(CH_2)_2-CF_3$, $-CH_2(CF_2)_2-CF_3$, $-(CF_2)_3-CF_3$, $- (CH_2)_2CF_2-CF_3$ or $-(CH_2)_3-CF_3$ .

In some embodiments, as in the compounds shown in Formula **I, I-1, I-1C**, **I-1D, I-1E,** or **I-1F,** $R^5$ is $-CH_2-CN$, $-(CH_2)_2-CN$, $-(CH_2)_3-CN$, $-(CH_2)_4-CN$, $-(CH_2)_2-CF_3$ , $-CH_2(CF_2)_2-CF_3$, $-(CF_2)_3-CF_3$, $-(CH_2)CF_2-CF_3$, $-(CH_2)_3-CF_3$, $-CH(CH_2CH_3)C(O)NH-CH_2CF_3$, $- CH_2C(O)NHCH_2-CF_3$, $-CH(CH_3)C(O)NHCH_2-CF_3$, $-(CH_2)_5-F$,

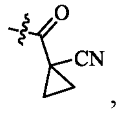,

$-S(O)_2(CH_2)_2-CF_3$ , $- C(O)NHCH_2-CF_3$, $-S(O)_2(CH_2)_2-CH_3$, $-S(O)_2CH_2-CH_3$, $-S(O)_2(CH_2)_3-CH_3$, $-S(O)_2(CH_2)_2-CF_3$ ,

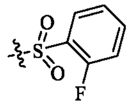, 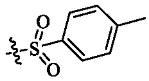, 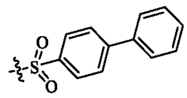, 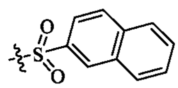, 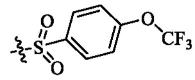, 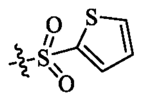, $-C(O)OC(CH_3)_2-CH_3$,

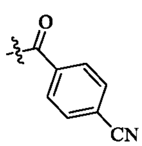 or 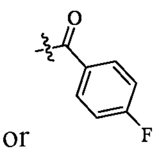;

preferably $-CH_2-CN$, $-(CH_2)_2-CN$, $-(CH_2)_3-CN$, $-(CH_2)_4-CN$, $-(CH_2)_2-CF_3$, $-CH_2(CF_2)_2-CF_3$, $-(CF_2)_3-CF_3$, $-(CH_2)_2CF_2-CF_3$, $-(CH_2)_3-CF_3$ , $-CH(CH_2CH_3)C(O)NH-CH_2CF_3$, $-CH_2C(O)NHCH_2-CF_3$, $-CH(CH_3)C(O)NHCH_2-CF_3$, $-(CH_2)_5-F$, $-S(O)_2(CH_2)_2-CF_3$, $-S(O)_2CH_2-CH_3$, $-S(O)_2(CH_2)_3-CH_3$, $-S(O)_2(CH_2)_2-CF_3$,

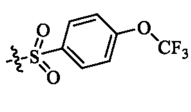 or 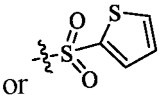.

In some embodiments, said compound shown in formula **I** has the structure shown in formula **I-3, I-3A** or **I-3B as** follows.

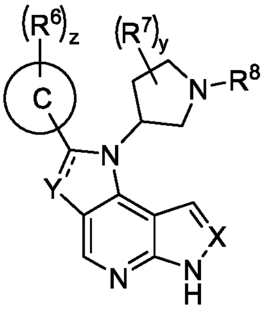
**I-3**
,
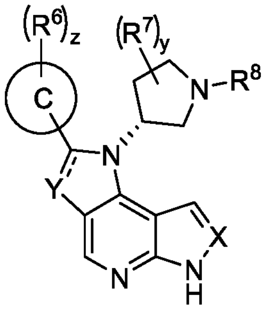
**I-3A**
,
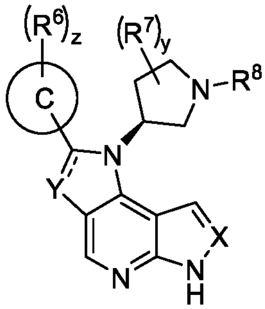
**I-3B**

wherein X, Y, $\overline{---}$ , ring C, $R^6$ , $R^7$ , $R^8$ , z and y are defined as described in the compound shown in Formula **I as** defined in any of the embodiments of the present invention.

In some embodiments, said compound shown in formula **I** has the structure shown in formula **I-4** as follows.

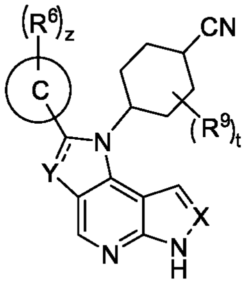
**I-4**

wherein X, Y, $\overline{---}$ , ring C, $R^6$ , $R^9$ , z and t are defined as described in the compound shown in Formula **I as** defined in any of the embodiments of the present invention.

In some embodiments, as in the compounds shown in Formula **I, I-3, I-3A, I-3B,** or **I-4,** $\overline{---}$ is a double bond and Y is N.

In some embodiments, as in the compounds shown in Formula **I, I-3, I-3A, I-3B,** or **I-4,** $\overline{---}$ is a single bond and Y is NH.

In some embodiments, as in the compounds shown in Formula **I, I-3, I-3A, I-3B,** or **I-4,** the 5-6-membered heteroaromatic ring in the definition of ring C is an imidazole (e.g.,

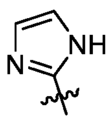

), thiazole (e.g.,

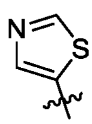

), furan (e.g.,

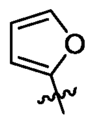

), thiophene (e.g.,

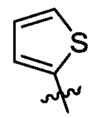

), or pyridine (e.g.,

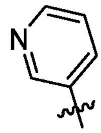

).

In some embodiments, as in the compounds shown in Formula **I, I-3, I-3A, I-3B, I-4, I-4A, I-4B, I-4C** or **I-4D**, when $R^2$ is .

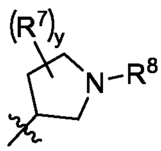

,

ring C is a benzene ring, an imidazole (e.g.,

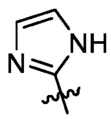

), a furan (e.g.,

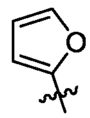

) or a pyridine (e.g.,

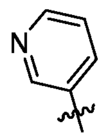

)

In some embodiments, as in the compounds shown in Formula **I, I-3, I-3A, I-3B, I-4, I-4A, I-4B, I-4C** or **I-4D**, when $R^2$ is .

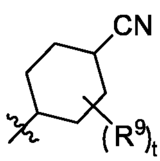

,

ring C is a benzene ring, a thiazole (e.g.,

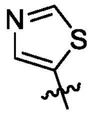

), a furan (e.g.,

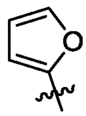

) or a thiophene (e.g.,

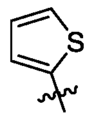

)

In some embodiments, as in the compounds shown in Formula **I, I-3, I-3A, I-3B, I-4, I-4A, I-4B, I-4C,** or **I-4D,**

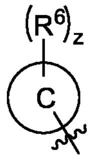

is

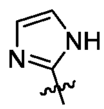 , 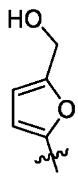 , 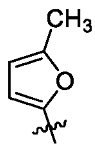 , 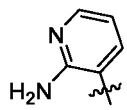 , 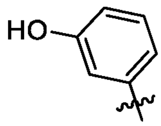 ,

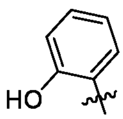 , 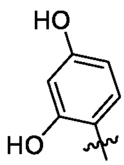 , 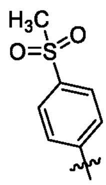 , 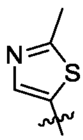 , 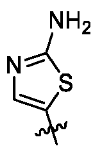 , 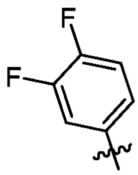 , 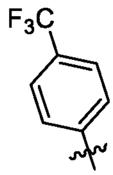 , 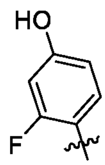 ,

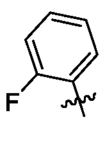 , 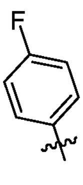 , 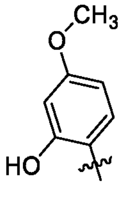 , 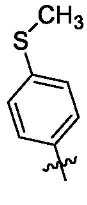 , or 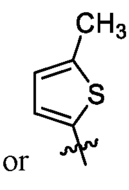 .

In some embodiments, said compound shown in formula I has the structure shown in formula **I-3C, I-3D, I-3E, I-3F** or **I-3G as** follows.

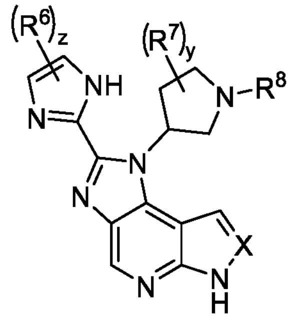
I-3C
,
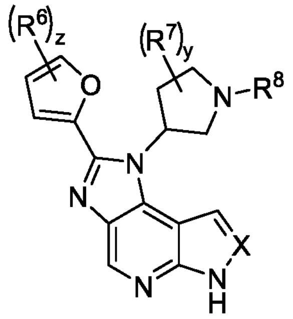
I-3D
,
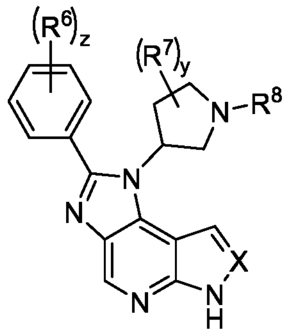
I-3E
,

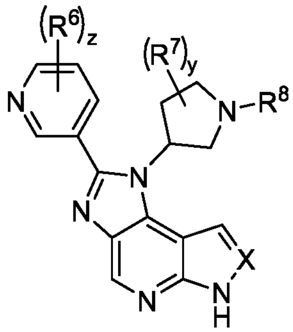
I-3F
,
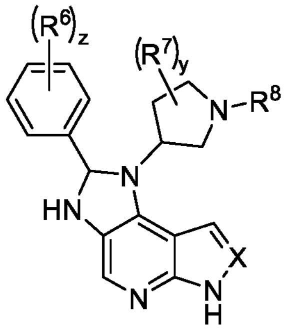
I-3G
.

wherein X, $R^6$, $R^7$, $R^8$, z and y are defined as described in any of the embodiments of the present invention.

In some embodiments, as in the compounds shown in Formula **I, I-3, I-3C, I-3D, I-3E, I-3F,** or **I-3G,**

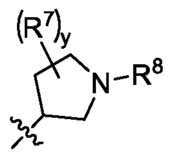

is

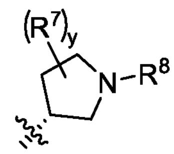
or
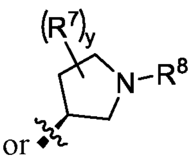

In some embodiments, as in the compounds shown in Formula **I,** I-3, **I-3A, I-3B, I-3C, I-3D, I-3E, I-3F** or **I-3G,** $R^7$ is $R^4$, which means that $R^7$ and $R^4$ in the compound as shown in Formula **I** in any of the embodiments of the present invention have the same definition.

In some embodiments, as in the compounds shown in Formula **I, I-3, I-3A, I-3B, I-3C, I-3D, I-3E, I-3F** or **I-3G,** $R^8$ is $R^5$, which means that $R^8$ and R5 in the compound as shown in Formula **I** in any of the embodiments of the present invention have the same definition.

In some embodiments, as in the compound shown in Formula **I, I-3, I-3A, I-3B, I-3C, I-3D, I-3E, I-3F,** or **I-3G,** y is 0.

In some embodiments, as in the compounds shown in Formula **I, I-3, I-3A, I-3B, I-3C, I-3D, I-3E, I-3F** or **I-3G,** $R^8$ is $R^5$, $-S(O)_2R^{8a}$ or $-C(O)R^{8b}$, wherein $R^5$ is defined in the same way as $R^5$ as defined in the compound shown in Formula **I** in any of the embodiments of the present invention.

In some embodiments, as in the compounds shown in Formula **I, I-3, I-3A, I-3B, I-3C, I-3D, I-3E, I-3F,** or **I-3G,** $R^8$ is a $-S(O)_2C(R^aR^b)-C_{1-6}$ alkyl group, $-S(O)_2-CF_3$, $-S(O)_2C(R^aR^b)-R^{5f}$, $-S(O)_2[C(R^aR^b)_2]-R^{5f}$, $-S(O)_2[C(R^aR^b)_3]-R^{5f}$, $-C(R^aR^b)-R^{5f}$, $-[C(R^aR^b)_2]-R^{5f}$, $-[C(R^aR^b)_3]-R^{5f}$,

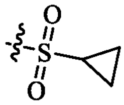, 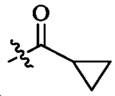,

-C(O)CH=$CH_2$ or -C(O)NHC($R^aR^b$)-$R^{5f}$; wherein $R^{5f}$ is defined in the same way as $R^{5f}$ as defined in the compound shown in Formula **I** in any of the embodiments of the present invention, e.g. $R^{5f}$ is $CF_3$ or CN.

In some embodiments, as in the compounds shown in Formula **I, I-3, I-3A, I-3B, I-3C, I-3D, I-3E, I-3F** or **I-3G,** $R^8$ is -$S(O)_2C(R^aR^b)$-$CH_3$, -$S(O)_2$-$CF_3$, -C(O)NHC($R^aR^b$)-$CF_3$, - C(O)CH=$CH_2$,

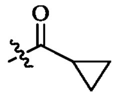, 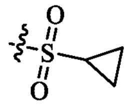,

-C($R^aR^b$)-CN, -[C$(R^aR^b)_2$]-CN or -[C$(R^aR^b)_3$]-$CF_3$ , preferably -$S(O)_2C(R^aR^b)$-$CH_3$, -$S(O)_2$-$CF_3$,

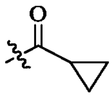, 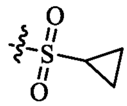,

-[C$(R^aR^b)_2$ ]-CN or - [C$(R^aR^b)_3$]-$CF_3$ .

In some embodiments, as in the compounds shown in Formula **I, I-3, I-3A, I-3B, I-3C, I-3D, I-3E, I-3F,** or **I-3G,** $R^8$ is -$S(O)_2(CH_2)_2$-$CH_3$ , -$S(O)_2$-$CF_3$, -C(O)NH$CH_2$-$CF_3$ , - C(O)CH=$CH_2$ ,

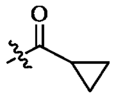, 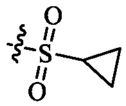,

-$CH_2$-CN, -($CH_2$) -CN or -$(CH_2)_3$-$CF_3$ , preferably - $S(O)_2(CH_2)_2$-$CH_3$ , -$S(O)_2$-CF ,

3 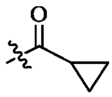, 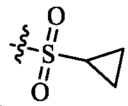,

-$(CH_2)_2$-CN or -$(CH_2)_3$-$CF_3$ .

In some embodiments, said compound shown in Formula **I** has the structure shown in Formula **I-4E, I-4F, I-4J, I-4L** or **I-4M as** follows.

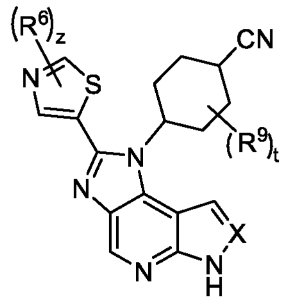
**I-4E** ,

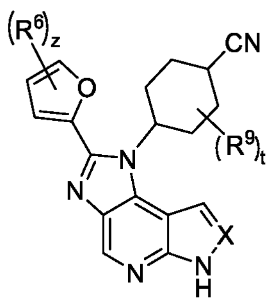
**I-4F** ,

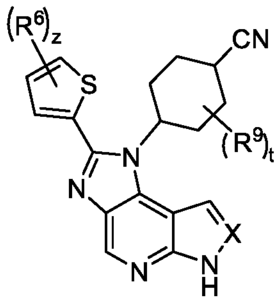
**I-4J** , ,

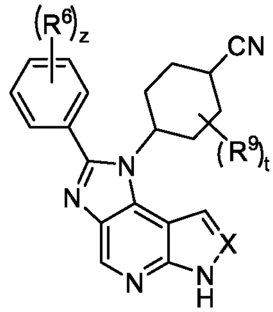
**I-4L**

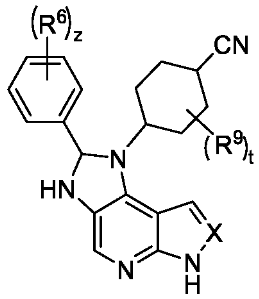

I-4M

wherein X, $R^6$, $R^9$, z and t are defined as described in the compound shown in Formula **I as** defined in any of the embodiments of the present invention.

In some embodiments, compounds such as those shown in Formula **I, I-4, I-4E, I-4F, I-4J**, **I-4L,** or **I-4M,**

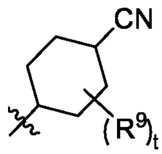

are in the *cis* or *trans* configuration, e.g., *trans.*

In some embodiments, as in the compounds shown in Formula **I, I-4, I-4E, I-4F, I-4J, I-4L,** or **I-4M,** t is 0.

In some embodiments, as in the compounds shown in Formula **I, I-3, I-3A, I-3B, I-3C, I-3D, I-3E, I-3F, I-3G, I-4, I-4E, I-4F, I-4J, I-4L,** or **I-4M,** X is CH.

In some embodiments, as in the compounds shown in Formula **I, I-3, I-3A, I-3B, I-3C, I-3D, I-3E, I-3F, I-3G, I-4, I-4E, I-4F, I-4J, I-4L,** or **I-4M,** X is N.

In some embodiments, as in the compounds shown in Formula **I, I-3, I-3A, I-3B, I-3C, I-3D, I-3E, I-3F, I-3G, I-4, I-4E, I-4F, I-4J, I-4L,** or **I-4M,** z is 0, 1, or 2.

In some embodiments, as in the compounds shown in **Formula I, I-3, I-3A, I-3B, I-3C, I-3D, I-3E, I-3F, I-3G, I-4, I-4E, I-4F, I-4J, I-4L,** or **I-4M,** each $R^6$ is independently a halogen (e.g., fluorine), a hydroxyl group, an amino group, a cyano group, a $C_{1-4}$ alkyl group (e.g., methyl group), a $C_{1-4}$ haloalkyl group (e.g. $C_{1-4}$ fluoroalkyl group, e.g., $CF_3$ ), a $-O-C_{1-4}$ alkyl group (e.g., $-O-CH_3$ ), a $-S-C_{1-4}$ alkyl group (e.g., $-S-CH_3$ ), a $-S(O)_2-C_{1-4}$ alkyl group (e.g. $-S(O)_2-CH_3$ ), or a $C_{1-4}$ hydroxyalkyl group (e.g. $-CH_2-OH$).

In some embodiments, as in the compounds shown in Formula **I, I-3, I-3A, I-3B, I-3C, I-3D, I-3E, I-3F, I-3G, I-4, I-4E, I-4F, I-4J, I-4L** or **I-4M,** each $R^6$ is independently fluorine, a hydroxyl group, an amino group, a cyano group, a methyl group, $CF_3$ , $-O-CH_3$ , - $S-CH_3$ , $-S(O)_2-CH_3$ or $-CH_2-OH$.

In some embodiments, z is 0 in the compound as shown in Formulas **I-3C.**

In some embodiments, as in the compounds shown in Formula **I-3D** or **I-4F,**

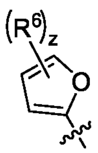

is independently

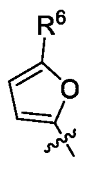
;

wherein $R^6$ is defined as described in the compounds shown in Formula **I** as defined in any of the embodiments of the present invention, such as a $C_{1-4}$ alkyl group (e.g., methyl group) or a $C_{1-4}$ hydroxyalkyl group (e.g., $-CH_2-OH$). For

example, in some embodiments,

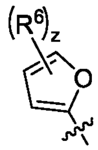

can be

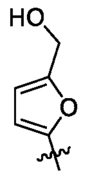

;

for example, in some embodiments,

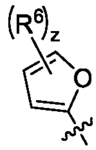

can be

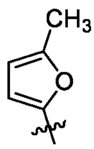

.

In some embodiments, as in the compounds shown in Formula **I-3E, I-3G, I-4L** or **I-4M,**

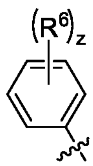

is independently

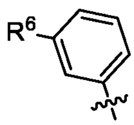

(e.g.

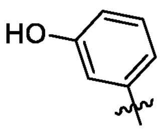

),

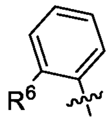

(e.g.

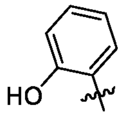

or

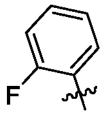

),

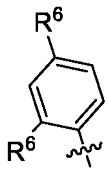

(e.g.

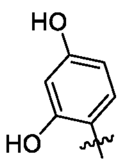
,
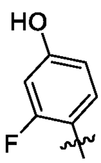
,
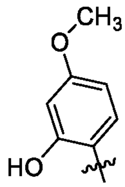

),

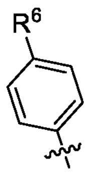

(e.g.

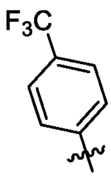
,
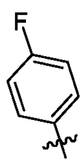
,

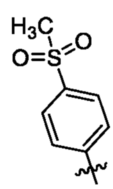 or 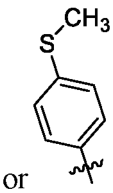

),

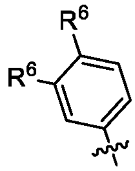

(e.g.

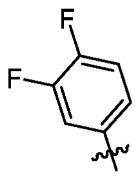

); wherein each $R^6$ is defined independently as described in the compounds shown in Formula **I** as defined in any of the embodiments of the present invention, e.g. halogen (e.g. fluorine), hydroxyl group, $C_{1-4}$ haloalkyl group (e.g. $C_{1-4}$fluoroalkyl group, e.g. $CF_3$ ), -O-$C_{1-4}$ alkyl group (e.g. -O-$CH_3$ ), -S-$C_{1-4}$ alkyl group (e.g. -S-$CH_3$ ) or -$S(O)_2$ -$C_{1-4}$ alkyl group (e.g. -$S(O)_2$-$CH_3$ ).

In some embodiments, as in the compound shown in Formula **I-3F,**

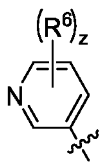

is

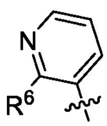 ,

wherein $R^6$ is defined as described in the compounds shown in Formula **I** as defined in any of the embodiments of the present invention, e.g., amino group.

In some embodiments, as in the compound shown in Formula **I-4E,**

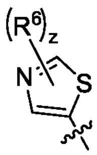

is

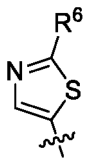

,

wherein $R^6$ is defined as described in the compounds shown in Formula **I** as defined in any of the embodiments of the present invention, such as an amino group or a $C_{1-4}$ alkyl group (e.g., methyl group). For example, in some embodiments,

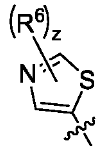

can be

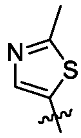

.

For example, in some embodiments,

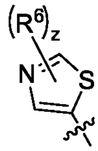

can be

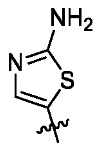

.

In some embodiments, as in the compound shown in Formula **I-4J,**

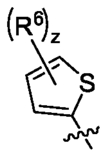

is

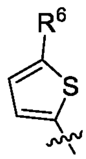

,

wherein $R^6$ is defined as described in the compounds shown in Formula **I** as defined in any of the embodiments of the

present invention, such as a $C_{1-4}$ alkyl group (e.g., methyl group). For example, in some embodiments,

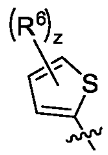

can be

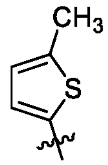

.

In some embodiments, said compound shown in formula **I** has the structure shown in formula **I-5** as follows.

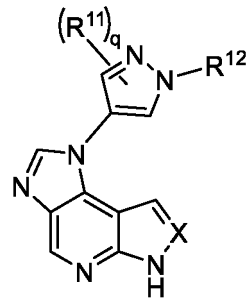

**I-5**

wherein X, $R^{11}$, $R^{12}$ and q are defined as described in the compound shown in Formula **I as** defined in any of the embodiments of the present invention.

In some embodiments, said compound shown in formula **I** has the structure shown in formula **I-6** as follows.

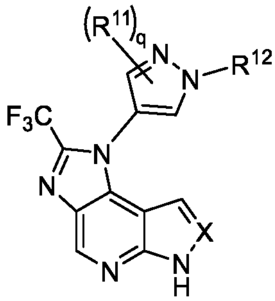

**I-6**

wherein X, $R^{11}$, $R^{12}$ and q are defined as described in the compound shown in Formula **I** as defined in any of the embodiments of the present invention.

In some embodiments, as in the compound shown in Formula **I-5** or **I-6,** $R^{12}$ is independently $-C(R^eR^f)-CN$, $-[C(R^eR^f)_2]-CN$ or $-[C(R^eR^f)_3]-CN$, such as $-CH_2-CN$, $-(CH_2)_2-CN$, $-(CH_2)_3-CN$, or

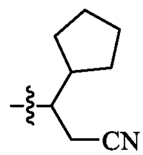

.

In some embodiments, as in the compound shown in Formula **I-5,** $R^{12}$ is preferably - $[C(R^eR^f)_{3]}$-CN, for example $-(CH_2)_3$-CN.

In some embodiments, as in the compound shown in Formula **I-6,** $R^{12}$ is preferably - $CH_2$ -CN or

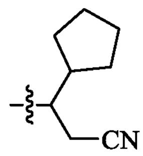

.

In some embodiments, said compound shown in formula **I** has the structure shown in formula **I-7** as follows.

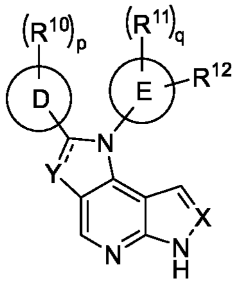

**I-7**

wherein X, Y, - - - , ring D, ring E, $R^{10}$ , $R^{11}$ , $R^{12}$ , p and q are defined as described in the compound shown in Formula **I as** defined in any of the embodiments of the present invention.

In some embodiments, as in the compound shown in Formula **I** or **I-7,** - - - is a double bond and Y is N.

In some embodiments, as in the compound shown in formula **I** or **I-7** as described, - - - is a single bond and Y is NH.

In some embodiments, as in the compound shown in Formula **I** or **I-7,** "the 5-6-membered heteroaromatic ring" in the definition of ring D is a furan (e.g.,

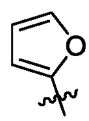

) or a thiophene (e.g.,

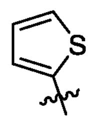

).

In some embodiments, as in the compound shown in Formula **I** or **I-7,** ring D is a cyclopropyl group, a cyclobutyl group, a benzene ring,

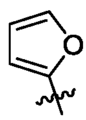

or

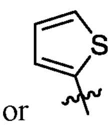

.

In some embodiments, as in the compound shown in Formula **I** or **I-7,**

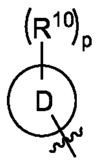

is independently

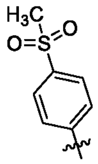 , 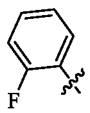 , 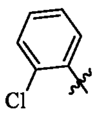 , 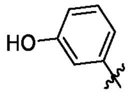 , 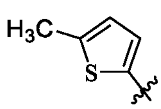 , 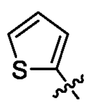 , 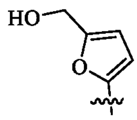 ,

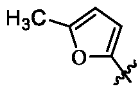 , 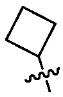 or 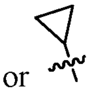 .

In some embodiments, said compound shown in Formula **I** has the structure shown in Formula **I-7A, I-7B**, **I-7C, I-7D, I-7E** or **I-7F as** follows.

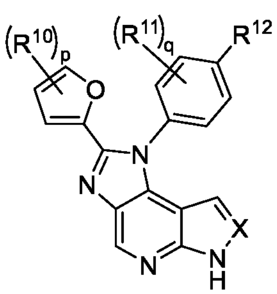
I-7A ,

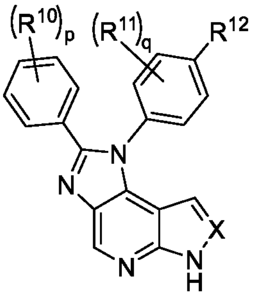
I-7B ,

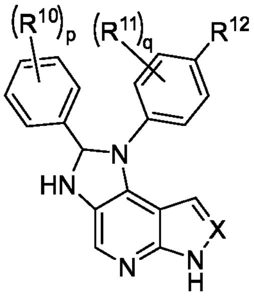
I-7C ,

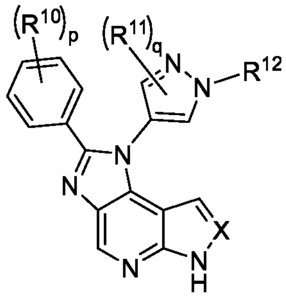
I-7D ,

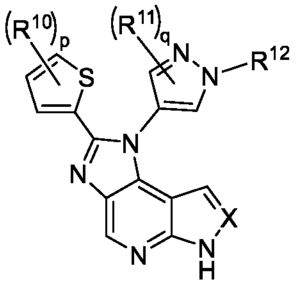
I-7E ,

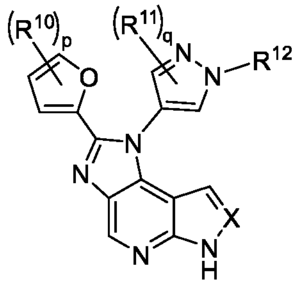
I-7F .

wherein X, $R^{10}$ , $R^{11}$ , $R^{12}$ , p and q are defined as described in any of the embodiments of the present invention.

In some embodiments, as in the compounds in Formula **I, I-5, I-6, I-7, I-7A, I-7B**, **I-7C, I-7D, I-7E** or **I-7F,** $R^{12}$ is a cyano group, $-C(R^eR^f)-R^{12f}$, $-[C(R^eR^f)_2]-R^{12f}$, $-[C(R^eR^f)_3]-R^{12f}$ or

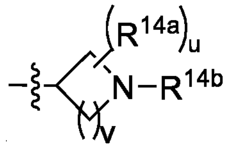

.

In some embodiments, as in the compounds shown in Formula **I, I-5, I-6, I-7, I-7A, I-7B**, **I-7C, I-7D, I-7E,** or **I-7F,** v is 0.

In some embodiments, as in the compounds shown in Formula **I, I-5, I-6, I-7, I-7A, I-7B**, **I-7C, I-7D, I-7E,** or **I-7F,**

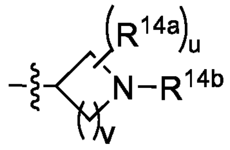

is

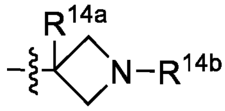

,

for example

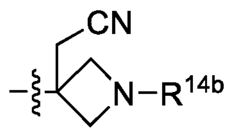

.

In some embodiments, as in the compounds shown in Formula **I, I-5, I-6, I-7, I-7A, I-7B**, **I-7C, I-7D, I-7E,** or **I-7F,** $R^{14a}$ is a cyano-substituted $C_{1-4}$ alkyl group, such as $-CH_2CN$.

In some embodiments, as in the compounds shown in Formula **I, I-5, I-6, I-7, I-7A, I-7B**, **I-7C, I-7D, I-7E,** or **I-7F,** $R^{12}$ is -CN, $-C(R^eR^f)-CN$, $-[C(R^eR^f)_2]-CN$, $-[C(R^eR^f)_3]-CN$ or

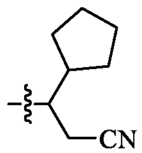

.

In some embodiments, as in the compounds shown in Formula **I, I-5, I-6, I-7, I-7A, I-7B**, **I-7C, I-7D, I-7E** or **I-7F,** each $R^e$ is independently H or a $C_{1-3}$ alkyl group..

In some embodiments, as in the compounds shown in Formula **I, I-5, I-6, I-7, I-7A, I-7B**, **I-7C, I-7D, I-7E** or **I-7F,** each $R^f$ is independently H or a $C_{1-3}$ alkyl group.

In some embodiments, as in the compounds shown in Formula **I, I-5, I-6, I-7, I-7A, I-7B**, **I-7C, I-7D, I-7E** or **I-7F,** $R^{12}$ is -CN, $-CH_2-CN$, $-(CH_2)_2-CN$, $-(CH_2)_3-CN$,

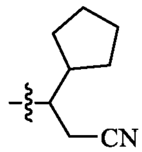

,

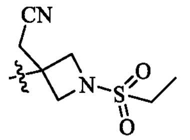, 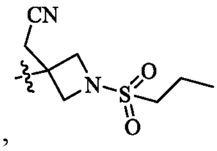 or 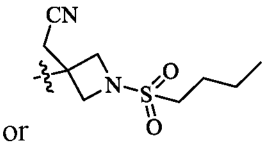 ,

preferably $-CH_2-CN$, $-(CH_2)_2-CN$, $-(CH_2)_3-CN$, further preferably $-(CH_2)_2-CN$.

In some embodiments, as in the compounds shown in formula **I-7A**, **I-7B** or **I-7C,**$R^{12}$ is a cyano group.

In some embodiments, as in the compounds shown in Formula **I-7E** or **I-7F,** $R^{12}$ is $-C(R^eR^f)-R^{12f}$, $-[C(R^eR^f)2]-R^{12f}$, $-[C(R^eR^f)_3]-R^{12f}$ or

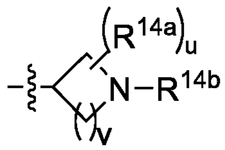 ,

preferably $-C(R^eR^f)-R^{12f}$, $-[C(R^eR^f)_2]-R^{12f}$ or $-[C(R^eR^f)_3]-R^{12f}$ , further preferably $-[C(R^eR^f)_2]-R^{12f}$ .

In some embodiments, said compound shown in Formula **I, I-7E** or **I-7F** has the structure shown in Formula **I-7G** or **I-7H** as follows.

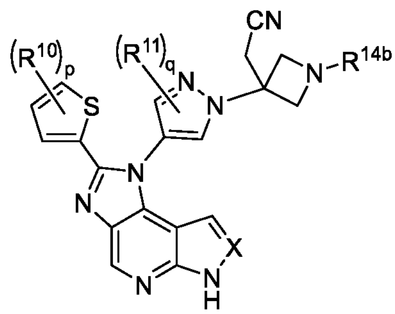

**I-7G** ,

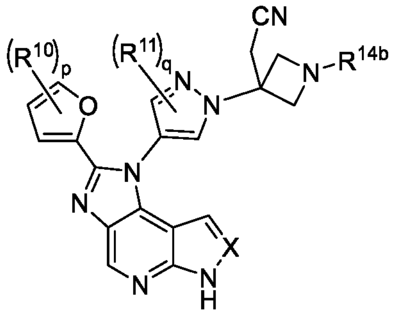

**I-7H** .

wherein X, $R^{10}$ , $R^{11}$ , $R^{14b}$ , p and q are defined as described in any of the embodiments of the present invention.

In some embodiments, as in the compounds shown in Formula I,**I-5, I-6, I-7,** I-7A, **I-7B, I-7C, I-7D, I-7E**, **I-7F, I-7G** or **I-7H,** $R^{14b}$ is $R^5$ , which means that $R^{14b}$ and $R^5$ in the compound as shown in Formula **I** in any of the embodiments of the present invention have the same definition

In some embodiments, as in the compounds shown in Formula **I, I-5, I-6, I-7, I-7A, I-7B**, **I-7C, I-7D, I-7E**, **I-7F, I-7G** or **I-7H,** $R^{14b}$ is a $-S(O)_2-C_{1-4}$ alkyl group, such as $-S(O)_2-CH_2CH_3$, $-S(O)_2-CH_2CH_2CH_3$ or $-S(O)_2-CH_2CH_2CH_2CH_3$.

In some embodiments, as in the compounds shown in Formula **I, I-5, I-6, I-7, I-7A, I-7B**, **I-7C, I-7D, I-7E**, **I-7F, I-7G**, or **I-7H,** X is N.

In some embodiments, as in the compounds shown in Formula **I, I-5, I-6, I-7, I-7A, I-7B**, **I-7C, I-7D, I-7E**, **I-7F, I-7G**, or **I-7H,** X is CH.

In some embodiments, as in the compounds shown in Formula **I, I-5, I-6, I-7, I-7A, I-7B**, **I-7C, I-7D, I-7E**, **I-7F, I-7G**, or **I-7H,** q is 0.

In some embodiments, as in the compounds shown in Formula **I, I-5, I-6, I-7, I-7A, I-7B**, **I-7C, I-7D, I-7E**, **I-7F, I-7G**, or **I-7H,** p is 0, 1, or 2.

In some embodiments, as in the compounds shown in Formula **I, 1-5, 1-6, 1-7, I-7A, I-7B**, **I-7C, I-7D, I-7E**, **I-7F, I-7G**, or **I-7H,** each $R^{10}$ is independently a halogen (e.g., fluorine or chlorine), a hydroxyl group, an amino group, a cyano group, a $C_{1-4}$ alkyl group (e.g., methyl group), a $C_{1-4}$ haloalkyl group (e.g., $C_{1-4}$ fluoroalkyl group, e.g., $CF_3$ ), a $-O-C_{1-4}$ alkyl group (e.g. $-O-CH_3$ ), a $-S(O)_2-C_{1-4}$ alkyl group (e.g. $-S(O)_2-CH_3$ ), or a $C_{1-4}$ hydroxyalkyl group (e.g. $-CH_2-OH$).

In some embodiments, as in the compounds shown in Formula **I, I-5, I-6, I-7, I-7A, I-7B**, **I-7C, I-7D, I-7E**, **I-7F, I-7G** or **I-7H,** each $R^{10}$ is independently fluorine, chlorine, a hydroxy group, a methyl group, $-S(O)_2-CH_3$ or $-CH_2-OH$.

In some embodiments, as in the compounds shown in Formula **I-7A, I-7F,** or **I-7H,**

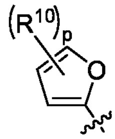

is independently

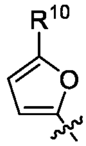
,

wherein each $R^{10}$ is defined as described in any of the embodiments of the present invention, such as a $C_{1-4}$ alkyl group (e.g., methyl group) or a $C_{1-4}$ hydroxyalkyl group (e.g., $-CH_2OH$). For example, in some embodiments,

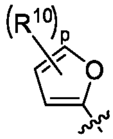

can be

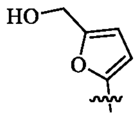
.

For example, in some embodiments,

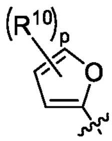

may be

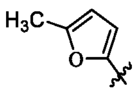
.

In some embodiments, as in the compounds shown in Formula **I-7E** or **I-7G**,

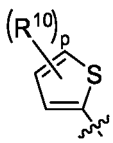

is independently

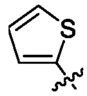 or 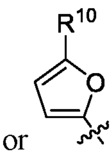 ,

wherein each $R^{10}$ is defined as described in any of the embodiments of the present invention, e.g., a $C_{1-4}$ alkyl group(e.g., methyl group).

In some embodiments, as in the compounds shown in Formula **I-7B**, **I-7C** or **I-7D,**

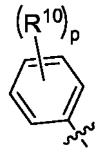

is

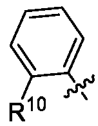

(e.g.,

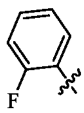 or 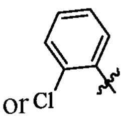

),

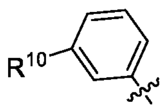

(e.g.,

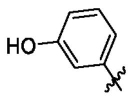

) or

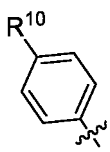

(e.g.,

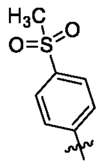

), wherein each $R^{10}$ is defined as described in any of the embodiments of the present invention, such as a hydroxyl group, a halogen (e.g., fluorine or chlorine) or a $-S(O)_2-C_{1-4}$ alkyl group (e.g. $-S(O)_2-CH_3$ ).

In some embodiments, said compound as shown in Formula **I** has the following structure.

| Number | Chemical name | Structure |
|---|---|---|
| **LXS01** | Tert-butyl 3-(2-((R)-1-hydroxyethyl)imidazo[4,5-d]pyrrolo[2,3-b]pyridin-1(6H)-yl)pyrrolidine-1 -carboxylate | 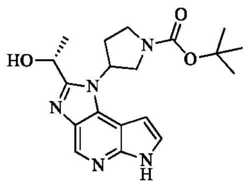 |
| **LXS02** | 4-(3-(2-((R)-1-Hydroxyethyl)imidazo[4,5-d]pyrrolo[2,3-b] pyridin-1(6H)-yl)pyrrolidin-1-carbonyl)benzonitrile | 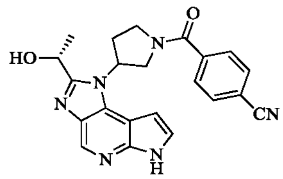 |
| **LXS03** | (4-Fluorophenyl)(3-(2-((R)-1- Hydroxyethyl)imidazo[4,5-d] pyrrolo[2,3-b]pyridin-1(6H)-yl)pyrrolidin-1-yl)methanone | 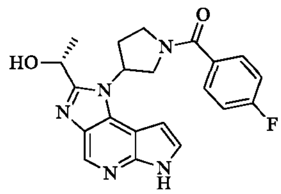 |
| **LXS04** | (1R)-1-(1-(1-(ethylsulfonyl)pyrrolidin-3-yl)-1,6-dihydroimidazo[4,5-d]pyrrolo[2,3-b]pyridin-2-yl)ethan-1-ol | 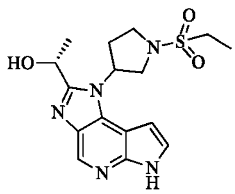 |
| **LXS05** | (1R)-1-(1-(1-(propylsulfonyl)pyrrolidin-3-yl)-1,6-dihydroimidazo[4,5-d]pyrrolo[2,3-b]pyridin-2-yl)ethan-1-ol | 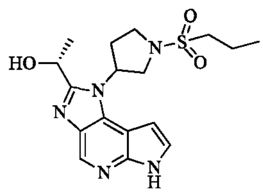 |
| **LXS06** | (1R)-1-(1-(1-(butylsulfonyl)pyrrolidin-3-yl)-1,6-dihydroimidazo[4,5-d]pyrrolo[2,3-b]pyridin-2-yl)ethan-1-ol | 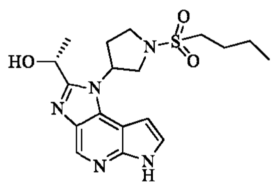 |

(continued)

| Number | Chemical name | Structure |
|---|---|---|
| **LXS07** | (1R)-1-(1-(1-((2-fluorophenyl)sulfonyl)pyrrolidin-3-yl)-1,6-dihydroimidazo[4,5-d]pyrrolo[2,3-b]pyridin-2-yl)ethan-1-ol | 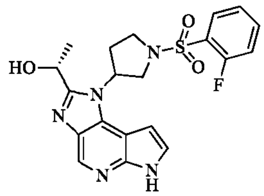 |
| **LXS08** | (1R)-1-(1-(1-tosylpyrrolidin-3-yl)-1,6-dihydroimidazo[4,5-d]pyrrolo[2,3-b]pyridin-2-yl)ethan-1-ol | 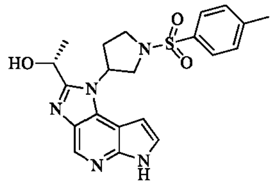 |
| **LXS09** | (1R)-1-(1-(1-([1,1'-biphenyl]-4-ylsulfonyl)pyrrolidin-3-yl)-1,6-dihydroimidazo[4,5-d]pyrrolo[2,3-b]pyridin-2-yl)ethan-1-ol | 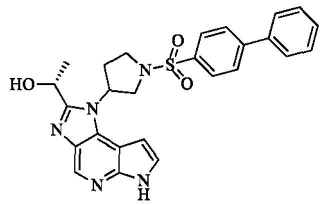 |
| **LXS10** | (1R)-1-(1-(1-(naphthalen-2-ylsulfonyl)pyrrolidin-3-yl)-1,6-dihydroimidazo[4,5-d]pyrrolo[2,3-b]pyridin-2-yl)ethan-1-ol | 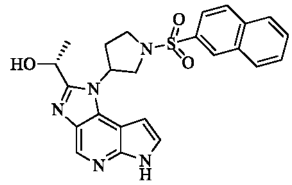 |
| **LXS11** | (1R)-1-(1-(1-((4-(trifluoromethoxy)phenyl)sulfonyl) pyrrolidin-3-yl)-1,6-dihydroimidazo[4,5-d]pyrrolo[2,3-b]pyridin-2-yl) ethan-1-ol | 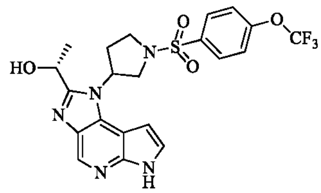 |
| **LXS12** | (1R)-1-(1-(1-(thiophen-2-ylsulfonyl)pyrrolidin-3-yl)-1,6-dihydroimidazo[4,5-d]pyrrolo[2,3-b]pyridin-2-yl)ethan-1-ol | 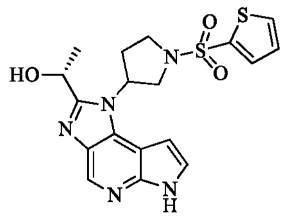 |
| **LXS13** | 2-(1-(Ethylsulfonyl)-3-(4-(2-(5-(hydroxymethyl)furan-2-yl) imidazo[4,5-d]pyrrolo[2,3-b]pyridin-1(6H)-yl)-1H-pyrazol-1-yl) azetidin-3-yl)acetonitrile | 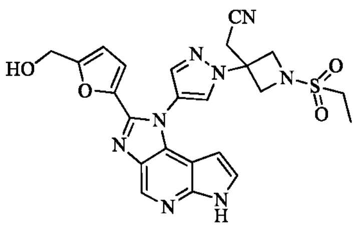 |

(continued)

| Number | Chemical name | Structure |
|---|---|---|
| **LXS14** | 2-(1-(Propylsulfonyl)-3-(4-(2-(5-(hydroxymethyl)furan-2-yl) imidazo[4,5-d]pyrrolo[2,3-b]pyridin-1(6H)-yl)-1H-pyrazol-1-yl) azetidin-3-yl)acetonitrile | 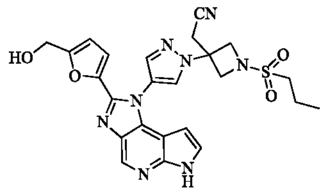 |
| **LXS15** | 2-(1-(Butylsulfonyl)-3-(4-(2-(5-(hydroxymethyl)furan-2-yl) imidazo[4,5-d]pyrrolo[2,3-b]pyridin-1(6H)-yl)-1H-pyrazol-1-yl) azetidin-3-yl)acetonitrile | 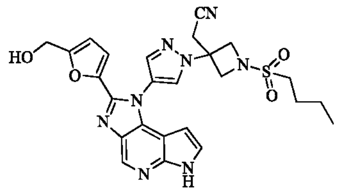 |
| **LXS16** | (5-(1-(1-(thiophen-2-ylsulfonyl)pyrrolidin-3-yl)-1,6-dihydroimidazo[4,5-d]pyrrolo[2,3-b]pyridin-2-yl)furan-2-yl) methanol | 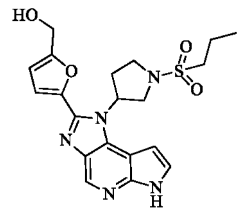 |
| **LXS17** | Cyclopropyl(3-(2-(5-(hydroxymethyl)furan-2-yl)imidazo[4,5-d] pyrrolo[2,3-b]pyridin-1(6H)-yl)pyrrolidin-1-yl) methanone | 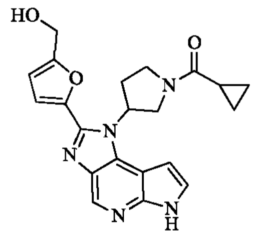 |
| **LXS18** | 2-(1H-imidazol-2-yl)-1-(1-(propylsulfonyl)pyrrolidin-3-yl)-1,6-dihydroimidazo[4,5-d]pyrrolo[2,3-b]pyridine | 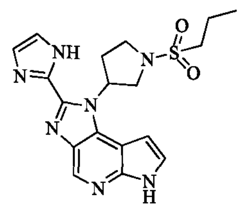 |
| **LXS19** | 2-(1H-imidazol-2-yl)-1-(1-((trifluoromethyl)sulfonyl)pyrrolidi n-3-yl)-1,6-dihydroimidazo[4,5-d]pyrrolo[2,3-b]pyridine | 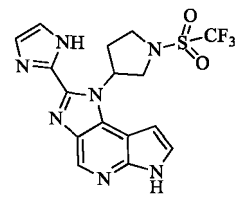 |
| **LXS20** | (R)-3-(2-((R)-1-Hydroxyethyl)imidazo[4,5-d]pyrrolo[2,3-b] pyridin-1(6H)-yl)-N-(2,2,2-trifluoroethyl)pyrrolidine-1-carboxamide | 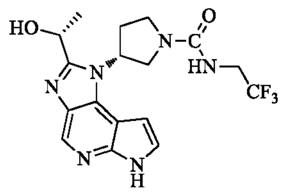 |

(continued)

| Number | Chemical name | Structure |
|---|---|---|
| **LXS21** | (R)-1-(1-((R)-1-(Propylsulfonyl)pyrrolidin-3-yl)-1,6-dihydroimidazo[4,5-d]pyrrolo[2,3-b]pyridin-2-yl)ethanol | 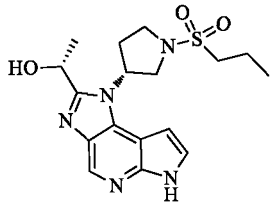 |
| **LXS22** | trans-4-(2-(5-(Hydroxymethyl)furan-2-yl)imidazo[4,5-d] pyrrolo[2,3-b]pyridin-1(6H)-yl)cyclohexanecarbonitrile | 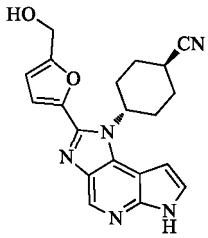 |
| **LXS23** | (R)-3-(2-(5-(Hydroxymethyl)furan-2-yl)imidazo[4,5-d]pyrrolo [2,3-b]pyridin-1(6H)-yl)-N-( 2,2,2-trifluoroethyl)pyrrolidine-1-carboxamide | 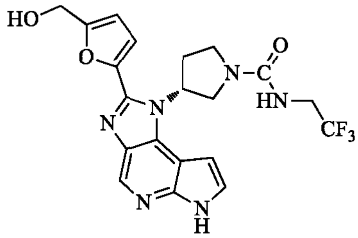 |
| **LXS24** | (R)-3-(2-(1H-imidazol-2-yl)imidazo[4,5-d]pyrrolo[2,3-b] pyridin-1(6H)-yl)-N-(2,2,2-trifluoroethyl)pyrrolidine-1-carboxamide | 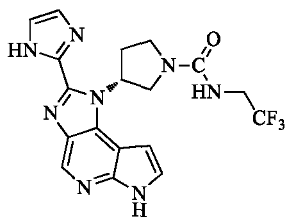 |
| **LXS25** | (R)-1-(3-(2-(1H-imidazol-2-yl)imidazo[4,5-d]pyrrolo[2,3-b] pyridin-1(6H)-yl)pyrrolidin-1-yl)prop-2-en-1-one | 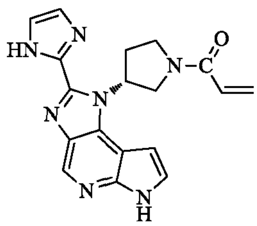 |
| **LXS26** | trans-4-(2-(2-Methylthiazol-5-yl)imidazo[4,5-d]pyrrolo[2,3-b] pyridin-1(6H)-yl)cyclohexanecarbonitrile | 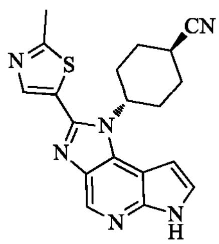 |

(continued)

| Number | Chemical name | Structure |
|---|---|---|
| **LXS27** | trans-4-(2-(2-Aminothiazol-5-yl)imidazo[4,5-d]pyrrolo[2,3-b]pyridin-1(6H)-yl)cyclohexanecarbonitrile | 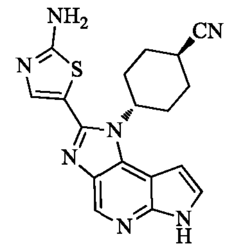 |
| **LXS28** | trans-4-(2-(2-Methylthiophen-5-yl)imidazo[4,5-d]pyrrolo[2,3-b]pyridin-1(6H)-yl)cyclohexanecarbonitrile | 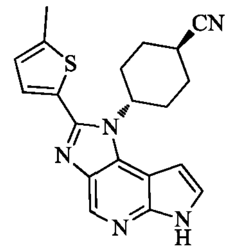 |
| **LXS29** | trans-4-(2-(2-Methylfuran-5-yl)imidazo[4,5-d]pyrrolo[2,3-b]pyridin-1(6H)-yl)cyclohexanecarbonitrile | 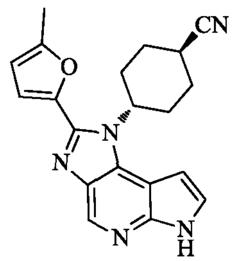 |
| **LXS30** | 2-(1-(1-(propylsulfonyl)pyrrolidin-3-yl)-1,6-dihydroimidazo[4,5-d]pyrrolo[2,3-b]pyridin-2-yl)phenol | 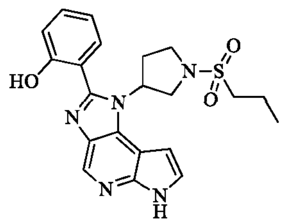 |
| **LXS31** | 4-(1-(1-(Propylsulfonyl)pyrrolidin-3-yl)-1,6-dihydroimidazo[4,5-d]pyrrolo[2,3-b]pyridin-2-yl)benzene-1,3-diol | 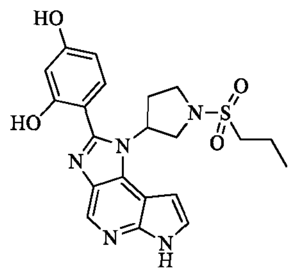 |
| **LXS32** | 3-(1-(1-(propylsulfonyl)pyrrolidin-3-yl)-1,6-dihydroimidazo[4,5-d]pyrrolo[2,3-b]pyridin-2-yl)phenol | 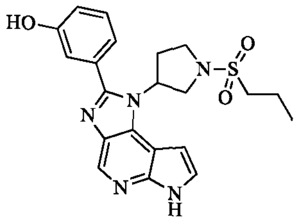 |

(continued)

| Number | Chemical name | Structure |
|---|---|---|
| **LXS33** | 1-(3-(2-(2,4-Dihydroxyphenyl)imidazo[4,5-d]pyrrolo[2,3-b]pyridin-1(6H)-yl)pyrrolidin-din-1-yl)prop-2-en-1-one | 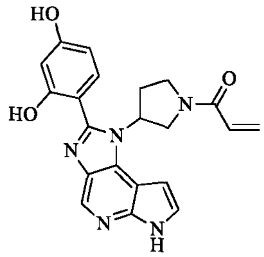 |
| **LXS34** | 3-(2-(3-Hydroxyphenyl)imidazo[4,5-d]pyrrolo[2,3-b]pyridin-1(6H)-yl)-N-(2,2,2-trifluoroethyl)pyrrolidine-1-carboxamide | 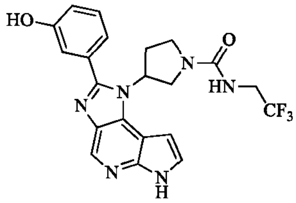 |
| **LXS35** | trans-4-(2-(3- Hydroxyphenyl)imidazo[4,5-d]pyrrolo[2,3-b]pyridin-1(6H)-yl)cyclohexanecarbonitrile | 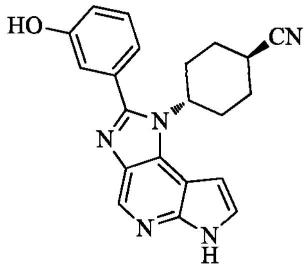 |
| **LXS36** | trans-4-(2-(2,4-Dihydroxyphenyl)imidazo[4,5-d]pyrrolo[2,3-b]pyridin-1(6H)-yl)cyclohexanecarbonitrile | 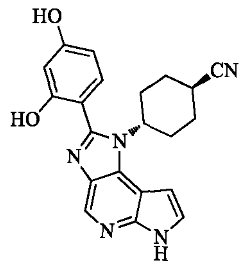 |
| **LXS37** | trans-4-(2-(4-(Trifluoromethyl)phenyl)imidazo[4, 5-d]pyrrolo[2,3-b]pyridin-1(6H)-yl)cyclohexanecarbonitrile | 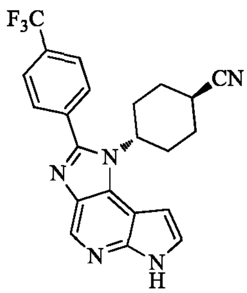 |
| **LXS38** | trans-4-(2-(2-Hydroxyphenyl)imidazo[4,5-d]pyrrolo[2,3-b]pyridin-1(6H)-yl)cyclohexanecarbonitrile | 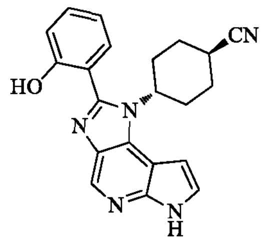 |

(continued)

| Number | Chemical name | Structure |
|---|---|---|
| **LXS39** | trans-4-(2-(2-Fluoro-4-hydroxyphenyl)imidazo[4,5-d]pyrrolo[2,3-b]pyridin-1(6H)-yl)cyclohexanecarbonitrile | 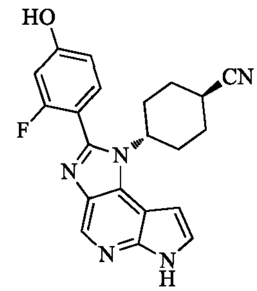 |
| **LXS40** | trans-4-(2-(2-Fluorophenyl)imidazo[4,5-d]pyrrolo[2,3-b]pyridin-1(6H)-yl)cyclohexanecarbonitrile | 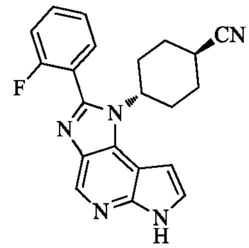 |
| **LXS41** | trans-4-(2-(2-Hydroxy-4-methoxyphenyl)imidazo[4,5-d]pyrrolo[2,3-b]pyridin-1(6H)-yl)cyclohexanecarbonitrile | 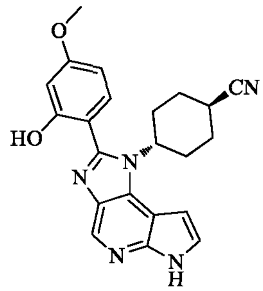 |
| **LXS42** | trans-4-(2-(4-(Methylthio)phenyl)imidazo[4,5-d]pyrrolo[2,3-b]pyridin-1(6H)-yl)cyclohexanecarbonitrile | 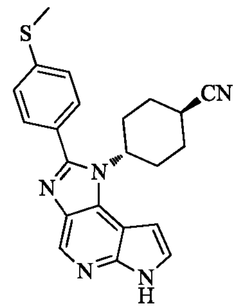 |
| **LXS43** | trans-4-(2-(4-Fluorophenyl)imidazo[4,5-d]pyrrolo[2,3-b]pyridin-1(6H)-yl)cyclohexanecarbonitrile | 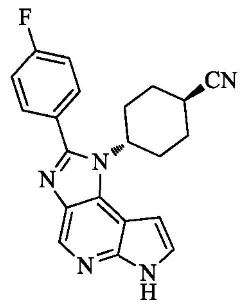 |

(continued)

| Number | Chemical name | Structure |
|---|---|---|
| **LXS44** | trans-4-(2-(4-(Methylsulfonyl)phenyl)-2,3-dihydroimidazo [4,5-d]pyrrolo[2,3-b]pyridin-1(6H)-yl)cyclohexanecarbonitrile | 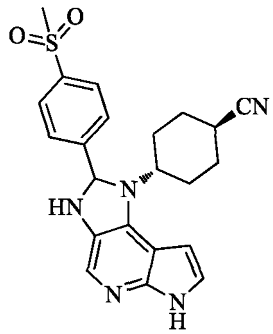 |
| **LXS45** | (5-(1-(1-(cyclopropylsulfonyl)pyrrolidin-3-yl)-1,6-dihydroimidazo[4,5-d]pyrrolo[2,3-b]pyridin-2-yl)furan-2-yl) methanol | 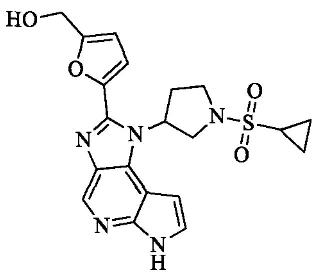 |
| **LXS46** | 3-(4-(2-(5-(Hydroxymethyl)furan-2-yl)imidazo[4,5-d]pyrrolo [2,3-b]pyridin-1(6H)-yl)-1H-pyrazol-1-yl)propanenitrile | 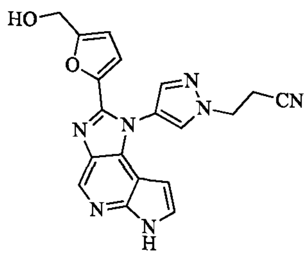 |
| **LXS47** | 3-(1-(1-(propylsulfonyl)pyrrolidin-3-yl)-1,6-dihydroimidazo [4,5-d]pyrrolo[2,3-b]pyridin-2-yl)pyridin-2-amine | 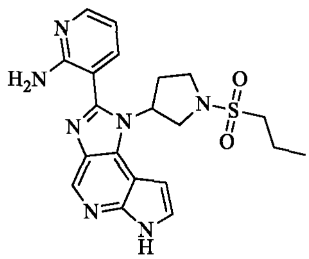 |
| **LXS48** | 2-(3-(2-((R)-1-Hydroxyethyl)imidazo[4,5-d]pyrrolo[2,3-b] pyridin-1(6H)-yl)pyrrolidin-1-yl)acetonitrile | 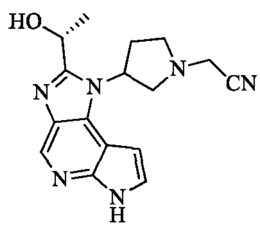 |
| **LXS49** | 3-(3-(2-((R)-1-Hydroxyethyl)imidazo[4,5-d]pyrrolo[2,3-b] pyridin-1(6H)-yl)pyrrolidin-1-yl)propanenitrile | 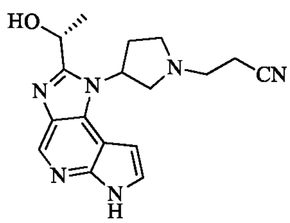 |

(continued)

| Number | Chemical name | Structure |
|---|---|---|
| **LXS50** | 3-(4-(2-(5-methylfuran-2-yl)imidazo[4,5-d]pyrrolo[2,3-b]pyridin-1(6H)-yl)-1H-pyrazol-1-yl)propanenitrile | 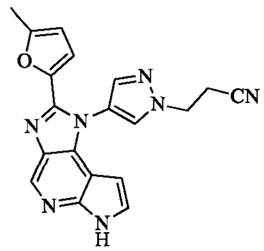 |
| **LXS51** | 3-(3-(2-(5-(Hydroxymethyl)furan-2-yl)imidazo[4,5-d]pyrrolo[2,3-b]pyridin-1(6H)-yl)pyrrolidin-1-yl)propanenitrile | 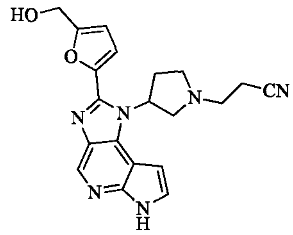 |
| **LXS52** | 2-(3-(2-(5-(hydroxymethyl)furan-2-yl)imidazo[4,5-d]pyrrolo[2,3-b]pyridin-1(6H)-yl)pyrrolidin-1-yl)acetonitrile | 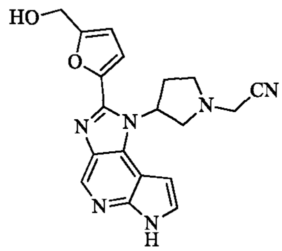 |
| **LXS53** | 2-(3-(2-(3-Hydroxyphenyl)imidazo[4,5-d]pyrrolo[2,3-b]pyridin-1(6H)-yl)pyrrolidin-1-yl)acetonitrile | 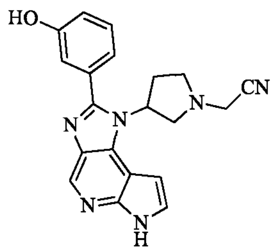 |
| **LXS54** | (R)-4-(2-(1-Hydroxyethyl)imidazo[4,5-d]pyrrolo[2,3-b]pyridin-1(6H)-yl)benzonitrile | 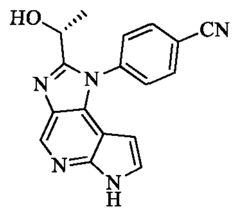 |
| **LXS55** | 4-(2-(5-(Hydroxymethyl)furan-2-yl)imidazo[4,5-d]pyrrolo[2,3-b]pyridin-1(6H)-yl)benzonitrile | 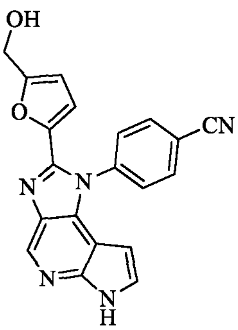 |

(continued)

| Number | Chemical name | Structure |
|---|---|---|
| **LXS56** | 4-(2-(3- Hydroxyphenyl)imidazo[4,5-d]pyrrolo[2,3-b]pyridin-1(6H)-yl)benzonitrile | 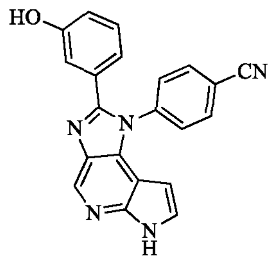 |
| **LXS57** | 4-(2-(4-(Methylsulfonyl)phenyl)-2,3-dihydroimidazo[4,5-d]pyrrolo[2,3-b]pyridin-1(6H)-yl)benzonitrile | 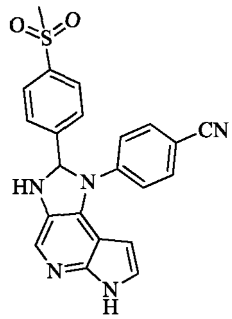 |
| **LXS58** | 3-((R)-3-(2-((R)-1-Hydroxyethyl)imidazo[4,5-d]pyrrolo[2,3-b]pyridin-1(6H)-yl)pyrrolidin-1-yl)propanenitrile | 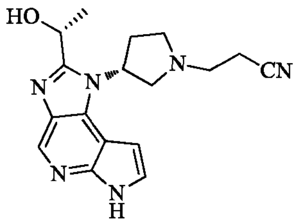 |
| **LXS59** | 3-((S)-3-(2-((R)-1-hydroxyethyl)imidazo[4,5-d]pyrrolo[2,3-b]pyridin-1(6H)-yl)pyrrolidin-1-yl)propanenitrile | 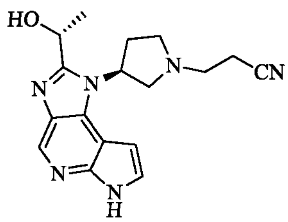 |
| **LXS60** | 3-(3-(2-(4-(Methylsulfonyl)phenyl)-2,3-dihydroimidazo[4,5-d]pyrrolo[2,3-b]pyridin-1(6H)-yl)pyrrolidin-1-yl)propanenitrile | 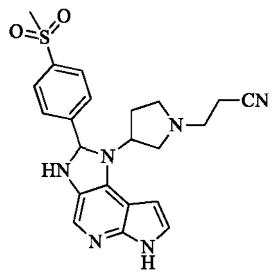 |
| **LXS61** | 1-(3-(2-((R)-1-Hydroxyethyl)imidazo[4,5-d]pyrrolo[2,3-b]pyridin-1(6H)-yl)pyrrolidin-1-carbonyl)cyclopropanecarbonitrile | 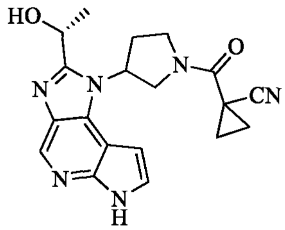 |

(continued)

| Number | Chemical name | Structure |
|---|---|---|
| **LXS62** | 2-(1-(Ethylsulfonyl)-3-(4-(2-(5-methylthien-2-yl)imidazo[4,5-d]pyrrolo[2,3-b]pyridin-1(6H)-yl)-1H-pyrazol-1-yl)azetidin-3-yl)acetonitrile | 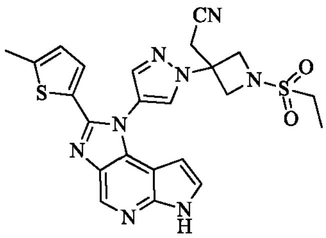 |
| **LXS63** | 2-(1-(Ethylsulfonyl)-3-(4-(2-(thiophen-2-yl)imidazo[4,5-d]pyrrolo[2,3-b]pyridin-1(6H)-yl)-1H-pyrazol-1-yl)azetidin-3-yl)acetonitrile | 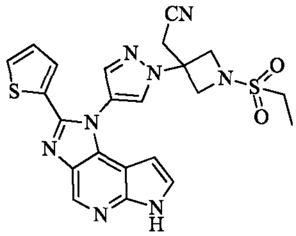 |
| **LXS64** | 3-(4-(2-(Trifluoromethyl)imidazo[4,5-d]pyrrolo[2,3-b]pyridin-1(6H)-yl)-1H-pyrazol-1-yl)propanenitrile | 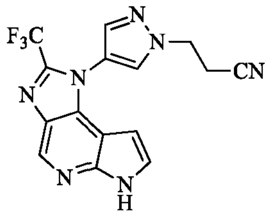 |
| **LXS65** | 4-((S)-3-(2-((R)-1-Hydroxyethyl)imidazo[4,5-d]pyrrolo[2,3-b]pyridin-1(6H)-yl)pyrrolidin-1-yl)butanenitrile | 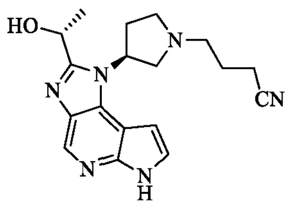 |
| **LXS66** | 3-Cyclopentyl-3-(4-(2-(trifluoromethyl)imidazo[4,5-d]pyrrolo[2,3-b]pyridin-1(6H)-yl)-1H-pyrazol-1-yl)propanenitrile | 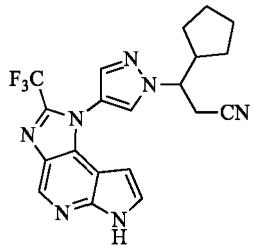 |
| **LXS67** | 3-(4-(2-(4-(Methylsulfonyl)phenyl)-2,3-dihydroimidazo[4,5-d]pyrrolo[2,3-b]pyridin-1(6H)-yl)-1H-pyrazol-1-yl)propanenitrile | 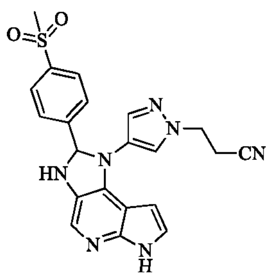 |

(continued)

| Number | Chemical name | Structure |
|---|---|---|
| **LXS68** | 5-((S)-3-(2-((R)-1-Hydroxyethyl)imidazo[4,5-d]pyrrolo[2,3-b]pyridin-1(6H)-yl)pyrrolidin-1-yl)pentanonitrile | 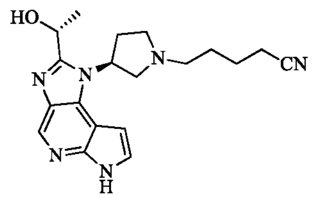 |
| **LXS69** | 4-((S)-7-(2-((R)-1-Hydroxyethyl)imidazo[4,5-d]pyrrolo[2,3-b]pyridin-1(6H)-yl)-5-azaspiro[2.4]hept-5-yl)butanenitrile | 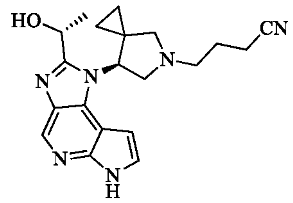 |
| **LXS70** | (R)-1-(1-((S)-5-(4,4,4-Trifluorobutyl)-5-azaspiro[2.4]hept-7-yl)-1,6-dihydroimidazo[4,5-d]pyrrolo[2,3-b]pyridin-2-yl)ethanol | 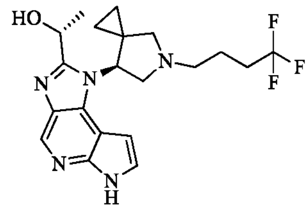 |
| **LXS71** | (R)-1-(1-((S)-1-(4,4,4-Trifluorobutyl)pyrrolidin-3-yl)-1,6-dihydroimidazo[4,5-d]pyrrolo[2,3-b]pyridin-2-yl)ethanol | 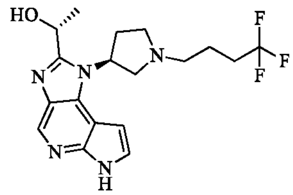 |
| **LXS72** | 5-((S)-7-(2-((R)-1-Hydroxyethyl)imidazo[4,5-d]pyrrolo[2,3-b]pyridin-1(6H)-yl)-5-azaspiro[2.4]hept-5-yl)pentanonitrile | 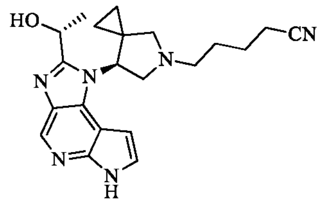 |
| **LXS73** | (R)-1-(1-((S)-1-(5-Fluoropentyl)pyrrolidin-3-yl)-1,6-dihydroimidazo[4,5-d]pyrrolo[2,3-b]pyridin-2-yl)ethanol | 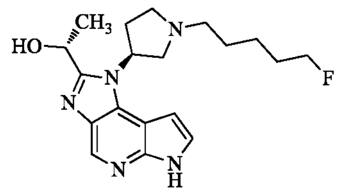 |
| **LXS74** | 2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)acetamide | 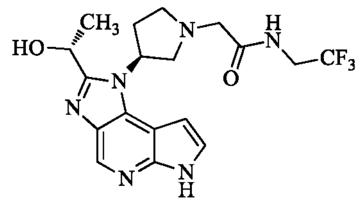 |

(continued)

| Number | Chemical name | Structure |
|---|---|---|
| **LXS75** | 2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)propanamide | 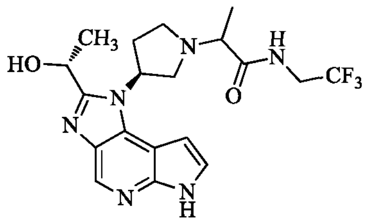 |
| **LXS76** | 2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)butanamide | 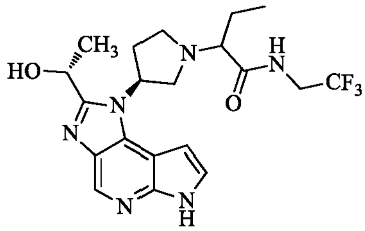 |
| **LXS77** | 2-(1-(ethylsulfonyl)-3-(4-(2-(5-methylfuran-2-yl)imidazo[4,5-d]pyrrolo[2,3-b]pyridin-1(6H)-yl)-1H-pyrazol-1-yl)azetidin-3-yl)acetonitrile | 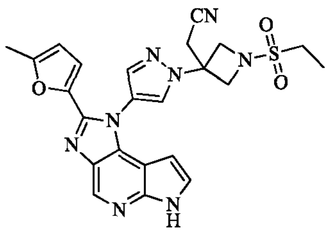 |
| **LXS78** | 2-(4-(2-(Trifluoromethyl)imidazo[4,5-d]pyrrolo[2,3-b]pyridin-1(6H)-yl)-1H-pyrazol-1-yl)acetonitrile | 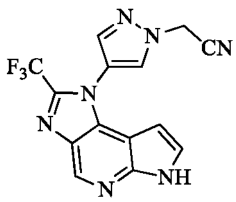 |
| **LXS79** | 3-(4-(Imidazo[4,5-d]pyrrolo[2,3- b]pyridin-1(6H)-yl)-1H-pyrazol-1-yl)propanenitrile | 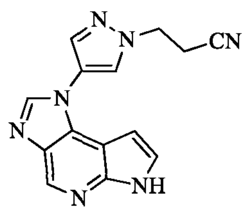 |
| **LXS80** | 3-(4-(2-(2-Chlorophenyl)imidazo[4, 5-d]pyrrolo[2,3-b]pyridin-1(6H)-yl)-1H-pyrazol-1-yl)propanenitrile | 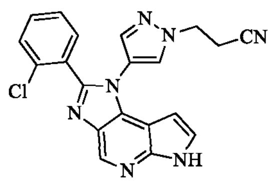 |
| **LXS81** | 2-(4-(2-(5-(Hydroxymethyl)furan-2-yl)imidazo[4,5-d]pyrrolo[2,3-b]pyridin-1(6H)-yl)-1H-pyrazol-1-yl)acetonitrile | 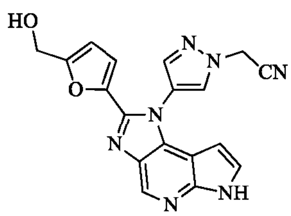 |

(continued)

| Number | Chemical name | Structure |
|---|---|---|
| **LXS82** | 3-(4-(2-(3-Hydroxyphenyl)imidazo[4,5-d]pyrrolo[2,3-b]pyridin-1(6H)-yl)-1H-pyrazol-1-yl)propanenitrile | 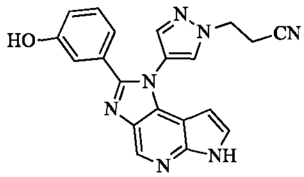 |
| **LXS83** | 3-(4-(2-(2-Fluorophenyl)imidazo[4,5-d]pyrrolo[2,3-b]pyridin-1(6H)-yl)-1H-pyrazol-1-yl)propanenitrile | 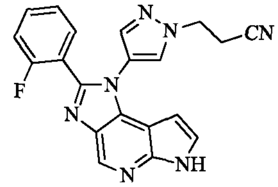 |
| **LXS84** | 3-(4-(2-(5-methylthiophen-2-yl)imidazo[4,5-d]pyrrolo[2,3-b]pyridin-1(6H)-yl)-1H-pyrazol-1-yl)propanenitrile | 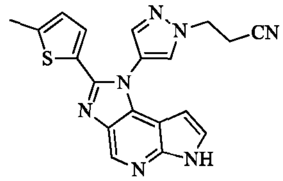 |
| **LXS85** | 4-((S)-3-(2-((R)-1-Hydroxyethyl)imidazo[4,5-d]pyrazolo[3,4-b]pyridin-1(6H)-yl)pyrrolidin-1-yl)butanenitrile | 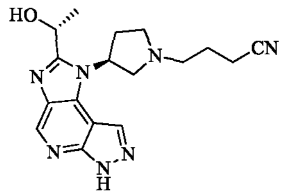 |
| **LXS86** | (1R)-1-(1-((3R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol | 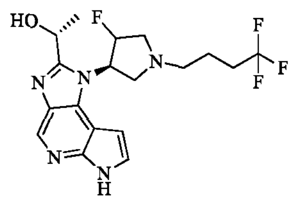 |
| **LXS87** | (R)-1-(1-((3R,4R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol | 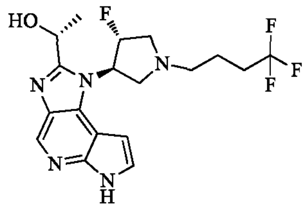 |
| **LXS88** | (R)-1-(1-((3R,4S)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol | 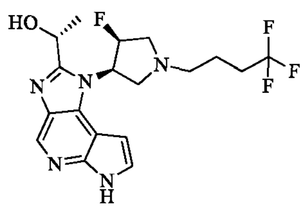 |

(continued)

| Number | Chemical name | Structure |
|---|---|---|
| **LXS89** | (R)-1-(1-((3S,5S)-5-(hydroxymethyl)-1-(4,4,4-trifluorobutyl) pyrrolidin-3-yl)-1,6-dihydroimidazo[4,5-d]pyrrolo[2,3-b] pyridin-2-yl)ethan-1-ol | 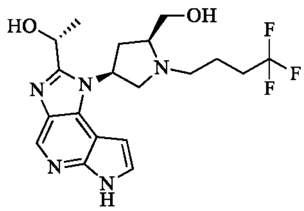 |
| **LXS90** | (R)-1-(1-((S)-1-(4,4,4-Trifluorobutyl)pyrrolidin-3-yl)-1,6-dihydroimidazo[4, 5-d]pyrazolo[3,4-b]pyridin-2-yl)ethanol | 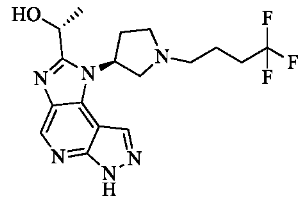 |
| **LXS91** | (R)-1-(1-((3S,5R)-5-(hydroxymethyl)-1-(4,4,4-trifluorobutyl) pyrrolidin-3-yl)-1,6-dihydroimidazo[4,5-d]pyrrolo[2,3-b] pyridin-2-yl)ethan-1-ol | 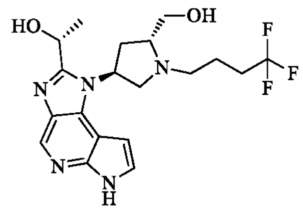 |
| **LXS92** | (R)-1-(1-((3S,5R)-5-(hydroxymethyl)-1-(4,4,4-trifluorobutyl) pyrrolidin-3-yl)-1,6-dihydroimidazo[4,5-d]pyrrolo[2,3-b] pyridin-2-yl)ethan-1-ol | 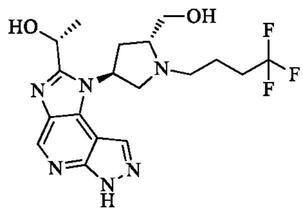 |
| **LXS93** | 4-((4R)-3-Fluoro-4-(2-((R)-1-hydroxyethyl)imidazo[4,5-d] pyrazolo[3,4-b]pyridin-1(6H)-yl) pyrrolidin-1-yl)butanenitrile | 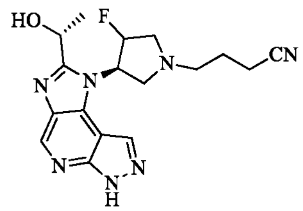 |
| **LXS94** | 4-((2S,4S)-4-(2-((R)-1-Hydroxyethyl)imidazo[4,5-d]pyrazolo [3,4-b]pyridin-1(6H)-yl)-2-(hydroxymethyl)pyrrolidin-1- yl) butanenitrile | 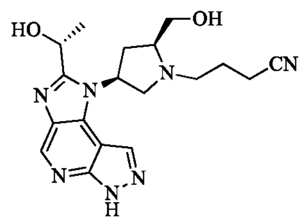 |

(continued)

| Number | Chemical name | Structure |
|---|---|---|
| **LXS95** | trans-4-(2-(3,4-Difluorophenyl)imidazo[4,5-d]pyrrolo[2,3-b]pyridin-1(6H)-yl)cyclohexanecarbonitrile | 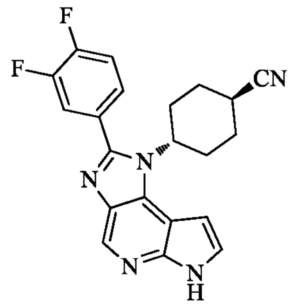 |
| **LXS96** | (R)-1-(1-((S)-1-(3,3,3-Trifluoropropyl)pyrrolidin-3-yl)-1,6-dihydroimidazo[4,5-d]pyrrolo[2,3-b]pyridin-2-yl)ethanol | 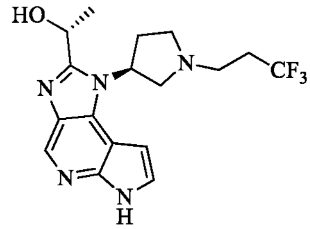 |
| **LXS97** | (R)-1-(1-((S)-1-(3,3,4,4,4-Pentafluorobutyl)pyrrolidin-3-yl)-1,6-dihydroimidazo[4,5-d]pyrrolo[2,3-b]pyridin-2-yl)ethanol | 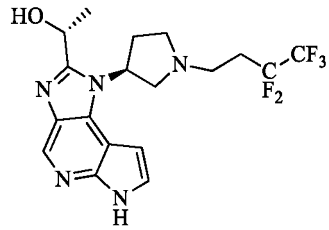 |
| **LXS98** | (S)-(5-(1-(1-(4,4,4-trifluorobutyl)pyrrolidin-3-yl)-1,6-dihydroimidazo[4,5-d]pyrrolo[2,3-b]pyridin-2-yl)furan-2-yl) methanol | 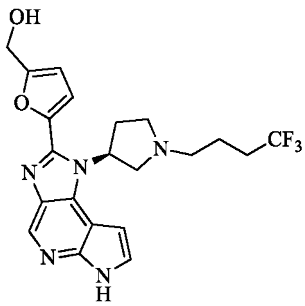 |
| **LXS99** | (S)-3-(1-(1-(4,4,4-trifluorobutyl)pyrrolidin-3-yl)-1,6-dihydroimidazo[4,5-d]pyrrolo[2,3-b]pyridin-2-yl)phenol | 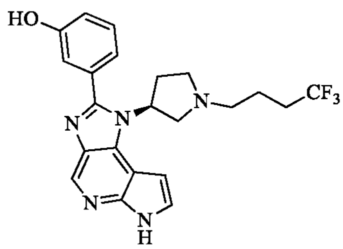 |
| **LXS100** | (R)-1-(1-((S)-1-(2,2,3,3,4,4,4-heptafluorobutyl)pyrrolidin-3-yl)-1,6-dihydroimidazo[4,5-d]pyrrolo[2,3-b]pyridin-2-yl) ethanol | 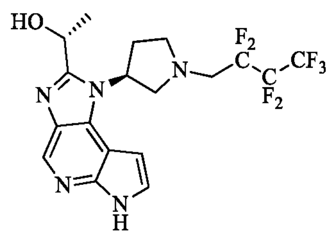 |

(continued)

| Number | Chemical name | Structure |
|---|---|---|
| **LXS101** | (R)-1-(1-((S)-1-(Perfluorobutyl)pyrrolidin-3-yl)-1,6-dihydroimidazo[4,5-d]pyrrolo[2,3-b]pyridin-2-yl) ethanol | 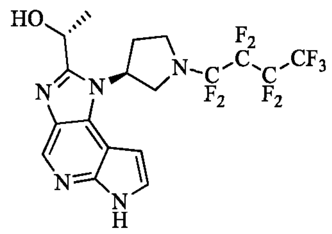 |
| **LXS102** | (R)-1-(1-((S)-1-((3,3,3-Trifluoropropyl)sulfonyl)pyrrolidin -3-yl)-1,6-dihydroimidazo[4,5-d]pyrrolo[2,3-b]pyridin-2-yl) ethanol | 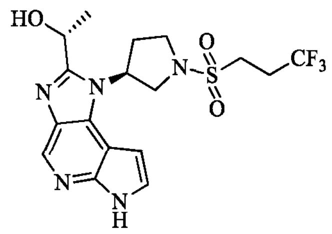 |
| **LXS103** | 4-(4-(Imidazo[4,5-d]pyrrolo[2,3-b]pyridin-1(6H)-yl)-1H-pyrazol-1-yl)butanenitrile | 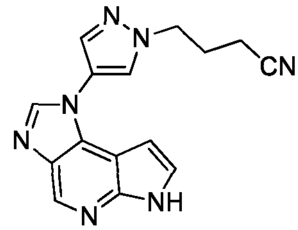 |
| **LXS104** | 4-(4-(2-(5-methylfuran-2-yl)imidazo[4,5-d]pyrrolo[2,3-b] pyridin-1(6H)-yl)-1H-pyrazol-1-yl)butanenitrile | 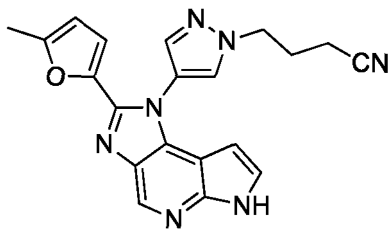 |
| **LXS105** | 4-(4-(2-(5-Methylthiophen-2-yl)imidazo[4,5-d]pyrrolo[2,3-b] pyridin-1(6H)-yl)-1H-pyrazol-1-yl butanenitrile | 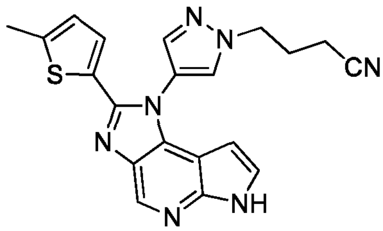 |
| **LXS106** | 3-(4-(2-(Thiophen-2-yl)imidazo[4,5-d]pyrrolo[2,3-b]pyridin-1 (6H)-yl)-1H-pyrazol-1-yl)propanenitrile | 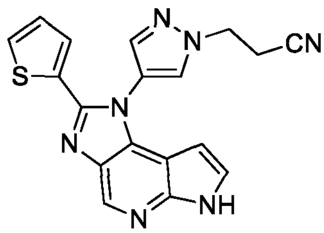 |

(continued)

| Number | Chemical name | Structure |
|---|---|---|
| **LXS107** | 4-(4-(2-Cyclobutylimidazo[4,5-d]pyrrolo[2,3-b]pyridin-1(6H)-yl)-1H-pyrazol-1-yl)butanenitrile | 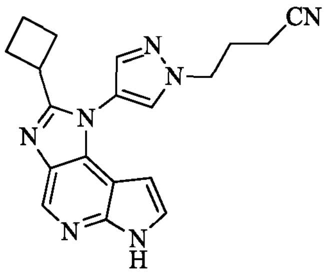 |
| **LXS108** | 4-(4-(2-Cyclopropylimidazo[4,5-d]pyrrolo[2,3-b]pyridin-1(6H)-yl)-1H-pyrazol-1-yl)butanenitrile | 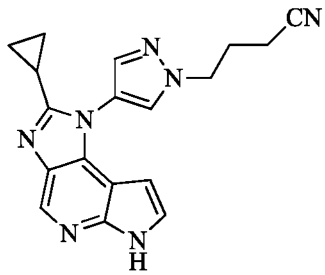 |

On the other hand, the present invention also provides a method for the preparation of a compound as shown in formula **I**, which includes the following steps: reacting a compound as shown in formula **II** with a base (e.g. sodium hydroxide) in an organic solvent (e.g. a mixture of tetrahydrofuran and methanol) to obtain said compound as shown in formula **I.**

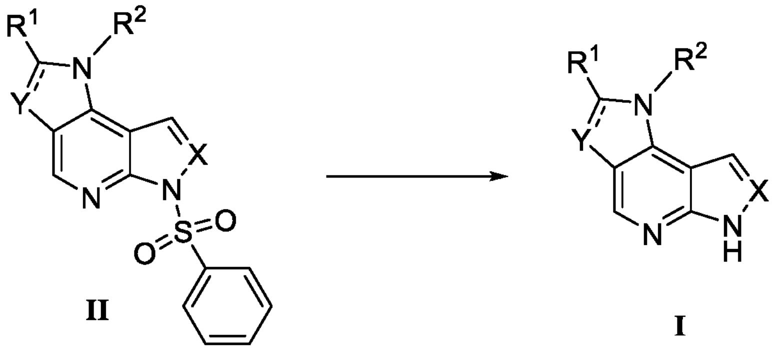

II

I

;.

wherein X, Y, - - - , $R^1$ and $R^2$ are defined as described in any of the embodiments of the present invention.

On the other hand, the present invention also provides a compound as shown in Formula **II.**

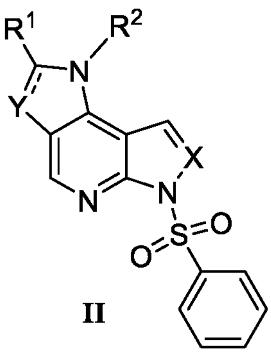

II

;.

wherein X, Y, $\overline{- - -}$, $R^1$ and $R^2$ are defined as described in any of the embodiments of the present invention.

On the other hand, the present invention also provides a pharmaceutical composition comprising:

said compound shown in Formula **I**, or a tautomer, stereoisomer, racemate or isotopic derivative thereof, or a pharmaceutically acceptable salt of any of the foregoing, or a crystalline or solvated form of any of the foregoing; and (ii) a pharmaceutically acceptable vehicle.

On the other hand, the present invention also provides a compound as described in Formula **I**, or a tautomer, stereoisomer, racemate or isotopic derivative thereof, or a pharmaceutically acceptable salt of any of the foregoing, or a crystalline or solvated form of any of the foregoing, or an application of said pharmaceutical composition as a drug.

On the other hand, the present invention also provides a compound as described in Formula **I**, or a tautomer, stereoisomer, racemate or isotopic derivative thereof, or a pharmaceutically acceptable salt of any of the foregoing, or a crystalline or solvated form of any of the foregoing, or an application of said pharmaceutical composition which acts as an inhibitor of Janus kinase (e.g., JAK1 and/or JAK2).

On the other hand, the present invention also provides a method of inhibiting Janus kinase (e.g. JAK1 and/or JAK2) *in vivo, in vitro* or *ex vivo,* which includes the contact of said compound shown in Formula **I**, or a tautomer, stereoisomer, racemate or isotopic derivative thereof, or a pharmaceutically acceptable salt of any of the foregoing, or a crystalline or solvated form of any of the foregoing, or said pharmaceutical composition with said Janus kinase.

On the other hand, the present invention also provides a compound as described in Formula **I**, or a tautomer, stereoisomer, racemate or isotopic derivative thereof, or a pharmaceutically acceptable salt of any of the foregoing, or a crystalline or solvated form of any of the foregoing, or a pharmaceutical composition as described in the preparation of drugs for the treatment of diseases associated with Janus kinases (e.g. JAK1 and/or JAK2) .

On the other hand, the present invention also provides a compound as shown in Formula **I**, or a tautomer, stereoisomer, racemate or isotopic derivative thereof, or a pharmaceutically acceptable salt of any of the foregoing, or a crystalline or solvated form of any of the foregoing, or an application of said pharmaceutical composition in the preparation of a drug for the treatment of an autoimmune disease or cancer.

On the other hand, the present invention also provides a method of treating a disease associated with a Janus kinase (e.g., JAK1 and/or JAK2) which includes administering a therapeutically effective amount of said compound shown in Formula **I**, or a tautomer, stereoisomer, racemate or isotopic derivative thereof, or a pharmaceutically acceptable salt of any of the foregoing, or a crystalline or solvated form of any of the foregoing, or a pharmaceutical composition as described to a subject in need of such treatment.

On the other hand, the present invention also provides a method of treating an autoimmune disease or cancer which includes administering a therapeutically effective amount of said compound shown in Formula **I**, or a tautomer, stereoisomer, racemate or isotopic derivative thereof, or a pharmaceutically acceptable salt of any of the foregoing, or a crystalline or solvated form of any of the foregoing, or a pharmaceutical composition as described to a subject in need of such treatment.

The diseases associated with Janus kinases (e.g., JAK1 and/or JAK2) described in the present invention can be autoimmune diseases or cancers.

The autoimmune diseases described herein or autoimmune diseases associated with Janus kinase can be, for example, psoriasis, rheumatoid arthritis, inflammatory bowel disease, Sjogren’s syndrome, leukoaraiosis, multiple sclerosis, or systemic lupus erythematosus.

The cancer or cancers associated with Janus kinase described in the present invention may be, for example, Kapozi’s sarcoma, giant lymph node hyperplasia, lymphoma, leukemia multiple myeloma, or myeloproliferative disorders. Myeloproliferative disorders described may be, for example, polycythemia vera (PV), essential thrombocytosis (ET), primary myelofibrosis (PMF), chronic myeloid leukemia (CML), chronic monocytic leukemia (CMML), eosinophilic leukocytosis syndrome (HES), idiopathic myelofibrosis (IMF), or systemic mastocytosis ( SMCD).

Unless otherwise stated, terms used in the present invention have the following definitions, and terms not covered below are defined as common knowledge understood by those skilled in the art of the present invention.

The term "tautomer" refers to a functional group isomer that results from the rapid movement of an atom in two positions in a molecule. For example, acetone and 1-propen-2-ol can transform into each other through the rapid movement of the hydrogen atom between the oxygen atom and $\alpha$ -carbon.

The term "stereoisomers" refers to isomers of molecules in which atoms or groups of atoms are attached to each other in the same order but are in different spatial arrangements, such as *cis-trans* isomers (e.g., Z-isomer, E-isomer), optical isomers (e.g., enantiomeric isomers, diastereomeric isomers), and atropisomers. These stereoisomers can be separated, purified and enriched by asymmetric synthesis methods or chiral separation methods (including but not limited to thin-layer chromatography, rotational chromatography, column chromatography, gas chromatography, high-pressure liquid chromatography, etc.). Stereoisomers can also be obtained by bonding (chemical bonding, etc.) or

salt formation (physical bonding, etc.) with other chiral compounds via chiral resolution. Optical isomers include enantiomers and diastereoisomers. All of these isomers, as well as their mixtures, are included in the scope of the present invention.

i.The term "isotopic derivative" refers to the substitution of one or more atoms in a compound by one or more atoms having a specific atomic mass or mass number. Examples of isotopes that can be incorporated into a compound include, but are not limited to, isotopes of hydrogen, carbon, nitrogen, oxygen, fluorine, sulfur, and chlorine (e.g.,$^{2}H$, $^{3}H$, $^{13}C$, $^{14}C$, $^{15}N$, $^{18}O$, $^{17}O$, $^{18}F$, $^{35}S$, and $^{36}Cl$). Isotopic compounds can typically be prepared by replacing non-isotopically labeled reagents with isotopically labeled reagents according to the methods described herein. Typical examples of isotopic derivatives include deuterated compounds.

The term "pharmaceutically acceptable salt" refers to the salt of a compound prepared with a relatively non-toxic, pharmaceutically acceptable acid or base. When a compound contains a relatively acidic functional group, a base addition salt may be obtained via the exposure of the neutral form of such compound to a sufficient amount of a pharmaceutically acceptable base in a pure solution or a suitable inert solvent. The pharmaceutically acceptable base addition salts include, but are not limited to: lithium, sodium, potassium, calcium, aluminum, magnesium, zinc, bismuth, ammonium, and diethanolamine salts. When the compounds of the present invention contain relatively basic functional groups, the corresponding acid addition salts can be obtained by exposing the neutral form of such compounds to a sufficient amount of a pharmaceutically acceptable acid in a pure solution or a suitable inert solvent. Said pharmaceutically acceptable acids include inorganic acids, said inorganic acids including but not limited to: hydrochloric acid, hydrobromic acid, hydroiodic acid, nitric acid, carbonic acid, phosphoric acid, phosphorous acid, sulfuric acid, etc. Said pharmaceutically acceptable acids include organic acids, said organic acids including, but not limited to: acetic acid, propionic acid, oxalic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citric acid, oleic acid, tannic acid, pantothenic acid, tartaric acid hydrogen, ascorbic acid, gentianic acid, fumaric acid, gluconic acid, glycolic acid, formic acid, ethanesulfonic acid, dihydroxynaphthalic acid (i.e., 4, 4'-methylene-bis(3-hydroxy-2-naphthoic acid)), amino acids (e.g., glutamic acid, arginine), etc. When the compounds contain relatively acidic and relatively basic functional groups, they can be converted into base addition salts or acid addition salts, respectively. For details, see Berge et al. "Pharmaceutical Salts", Journal of Pharmaceutical Science 66: 1-19 (1977), or, Handbook of Pharmaceutical Salts: Properties, Selection, and Use (P. Heinrich Stahl and Camille G. Wermuth, ed., Wiley-VCH, 2002).

In some embodiments, a pharmaceutically acceptable salt of a compound with the structure shown in general formula (I) as described herein may be an acid addition salt formed by a compound of general formula (I) with a pharmaceutically acceptable acid, said acid including, but not limited to: hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid p-toluenesulfonic acid or ferulic acid. A pharmaceutically acceptable salt of said compound with the structure shown in general formula (I) may be prepared by reaction with an equivalent or excess amount of an acid (inorganic or organic) in a suitable solvent or solvent mixture. Said acids include, but are not limited to, hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid, or ferulic acid. Said solvents include, but are not limited to, methanol, ethanol, methylene chloride, acetone, ethyl acetate, toluene, or tetrahydrofuran, or a mixture of any of these acids..

The term "crystalline" is defined as a structure where the ions or molecules are arranged in a defined way, in a three-dimensional space, and in a strictly periodic manner, and have a regular pattern of periodic recurrence at a certain distance apart; because of the periodic arrangement mentioned above, there can be a variety of crystalline forms, that is, the phenomenon of polycrystalline forms, also known as polymorphism.

The term "solvate" refers to a substance formed by combining molecules with a stoichiometric or non-stoichiometric solvent. The solvent molecules in a solvate may exist in an ordered or unordered arrangement. Said solvents include, but are not limited to, water, methanol, ethanol, etc.

The term "halogen" refers to fluorine, chlorine, bromine or iodine, preferably fluorine or chlorine.

The term "amino group" refers to the $-NH_2$ group.

The term "hydroxyl group" refers to the -OH group.

The term "cyano group" refers to the -CN group.

The term "alkyl group" refers to a saturated straight or branched monovalent hydrocarbon group having a certain number of carbon atoms. A $C_{1-4}$ alkyl group refers to an alkyl group having 1-4 carbon atoms, including $C_1$ alkyl group, $C_2$ alkyl group, $C_3$ alkyl group, and $C_4$ alkyl group, such as methyl group, ethyl group, n-propyl group, isopropyl group, n-butyl group, isobutyl group, sec-butyl group, tert-butyl group butyl grouptert-butyl group. Examples of alkyl groups include, but are not limited to, methyl group, ethyl group, n-propyl group, isopropyl group, n-butylgroup, isobutyl group,

sec-butyl group, tert-butyl group, and pentyl group.

The term "haloalkyl group" refers to an alkyl group substituted with one or more (e.g., 1, 2, 3 or 4) halogens (e.g., fluorine, chlorine, bromine or iodine, preferably fluorine), including but not limited to $-CHF_2$, $-CH_2F$, $-CF_3$, $-CHF-CH_2Cl$.

The term "hydroxyalkyl group" refers to an alkyl group substituted with one or more (e.g., 1, 2, 3 or 4) hydroxyl groups, including but not limited to $-CH_2OH$, $-CHOH-CH_2OH$. $C_{1-4}$ hydroxyalkyl groups include but are not limited to $-CH_2OH$, $-CHOH-CH_2OH$, $-CH(CH_2OH)-CH_2OH$.

The term "alkenyl group" refers to a straight or branched monovalent hydrocarbon group with a certain number of carbon atoms and contains at least one carbon-carbon double bond, where the carbon-carbon double bond can be located anywhere within the alkenyl group (e.g.

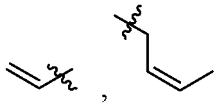

). A $C_2$-$C_6$ alkenyl group refers to an alkenyl group with 2-6 carbon atoms. These include the $C_2$ alkenyl group, the $C_3$ alkenyl group, $C_4$ alkenyl group, $C_5$ alkenyl group, and $C_6$ alkenyl group. Examples of alkenyl groups include, but are not limited to, vinyl group, propenyl group, butenyl group, pentenyl group, hexenyl group, butadienyl group, pentadienyl group, hexadienyl group.

The term "alkoxy group" refers to $-O-R^X$, where $R^X$ is an alkyl group as defined above. In some embodiments, a $C_{1-4}$ alkoxy group can be a methoxy group, ethoxy group, n-propoxy group, isopropoxy group, n-butoxy group, isobutoxy group, sec-butoxy group, or tert-butoxy group.

The term "cycloalkyl group" refers to a saturated monocyclic or polycyclic (e.g., concatenated, spiro, or bridged) hydrocarbon group formed by carbon atoms. In some embodiments, the cycloalkyl group is a monocyclic group. In some embodiments, a $C_{3-6}$ cycloalkyl group can be a cyclopropyl group, cyclobutyl group, cyclopentyl group, or cyclohexyl group. In some embodiments, a $C_{3-8}$ cycloalkyl group can be a $C_{3-6}$ cycloalkyl group, such as a cyclopropyl group, cyclobutyl group, cyclopentyl group, or cyclohexyl group. i.The term "heteroaryl group" or "heteroaromatic ring" refers to an aromatic cyclic group formed by a carbon atom and at least one heteroatom, wherein the heteroatom may be N, O and S. A 5-membered heteroaryl group or heteroaromatic ring is, for example, a furan, thiophene, pyrrole, pyrazole, oxazole, thiazole, imidazole or triazole. 6-membered heteroaryl group or heteroaromatic ring or, for example, pyrazine, pyridazine, pyridine or pyrimidine.

The "x-y membered" in the cyclic group described in the present invitation means that the number of atoms in the ring is x-y. For example, cyclopropyl group is a 3-membered group, tetrahydropyrrolyl group is a 5-membered group, and piperidinyl group is a 6-membered group.

The term "substitution" or "substituent" refers to the substitution of one or more hydrogen atoms by a specified group. When no substitution position is specified, the substitution may be at any position, but only the formation of a stable or chemically viable chemical is permitted.

When any variable (e.g. R) appears more than once in the composition or structure of a compound, its definition in each case is independent. Thus, for example, if a group is substituted with 0-2 R's, said group may optionally be substituted with up to two R's, and there are independent options for R in each case. Furthermore, combinations of substituents and/or variables are only allowed if such combinations would yield stable compounds. For example,

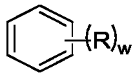

where w is 0, 1 or 2 and each R is independently a methyl group or fluorine, then

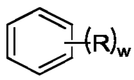

includes

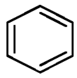, 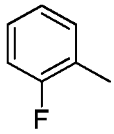 and 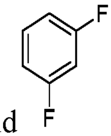

etc.

(i) The term "treatment" refers to curative therapy. In the context of a specific diseaseor condition, treatment means (1) alleviating one or more biological manifestations of the disease or condition, (2) interfering with (a) one or more points in the biological cascade causing or contributing to the disease or condition or (b) one or more biological manifestations of the disease or condition, (3) ameliorating one or more symptoms, effects or side effects associated with the disease or condition, or one or more symptoms, effects or side effects associated with the disease, condition or its treatment, or (4) slowing the progression of the disease, condition or one or more biological manifestations of the disease or condition.

The term "therapeutically effective amount" means an amount of a compound that, when given to a patient, is sufficient to effectively treat or prevent the disease or condition described herein. The "therapeutically effective amount" will vary depending on the compound, the disease or condition, and its severity, and the age of the patient to be treated, but may be adjusted by those skilled in the art as needed. In general, the compounds of the present invention are used in the therapeutic dose range of 1-1000 mg/day for human use. Doses beyond this range may also be used depending on the dosage form and the severity of the disease or condition.

The pharmaceutical compositions can be made into various types of dosing unit dosage forms depending on the therapeutic purpose, such as tablets, pills, powders, liquids, suspensions, emulsions, granules, pellets, capsules and injections (solutions or suspensions), preferably tablets, capsules, liquids, suspensions and injections (solutions or suspensions).

The compounds described in the present invention can be administered clinically by means of oral administration, injection, etc.

The term "subject" means any animal, preferably mammalian, preferably human, that is about to receive or has received the administration of a compound or composition. The term "mammal" includes any mammal. Examples of mammals include, but are not limited to, cattle, horses, sheep, pigs, cats, dogs, mice, rats, rabbits, guinea pigs, monkeys, humans, etc., with humans being preferred.

All patents and published publications covered herein are incorporated herein by reference in their entirety.

Each of the above preferred conditions can be combined in any way to obtain a better example of the invention without violating the common knowledge in the art.

The reagents and raw materials used in this invention are commercially available.

The positive progressive effect of the present invention is that the present invention provides a novel pyridocyclic compound that exhibits JAK inhibitory activity.

DETAILED DESCRIPTION OF THE PREFERRED EMBODIMENT

In order to further illustrate the invention, a series of embodiments are given below, which are entirely illustrative and are used only to describe the invention specifically and should not be construed as a limitation of the invention.

Synthesis of key **intermediate 1.**

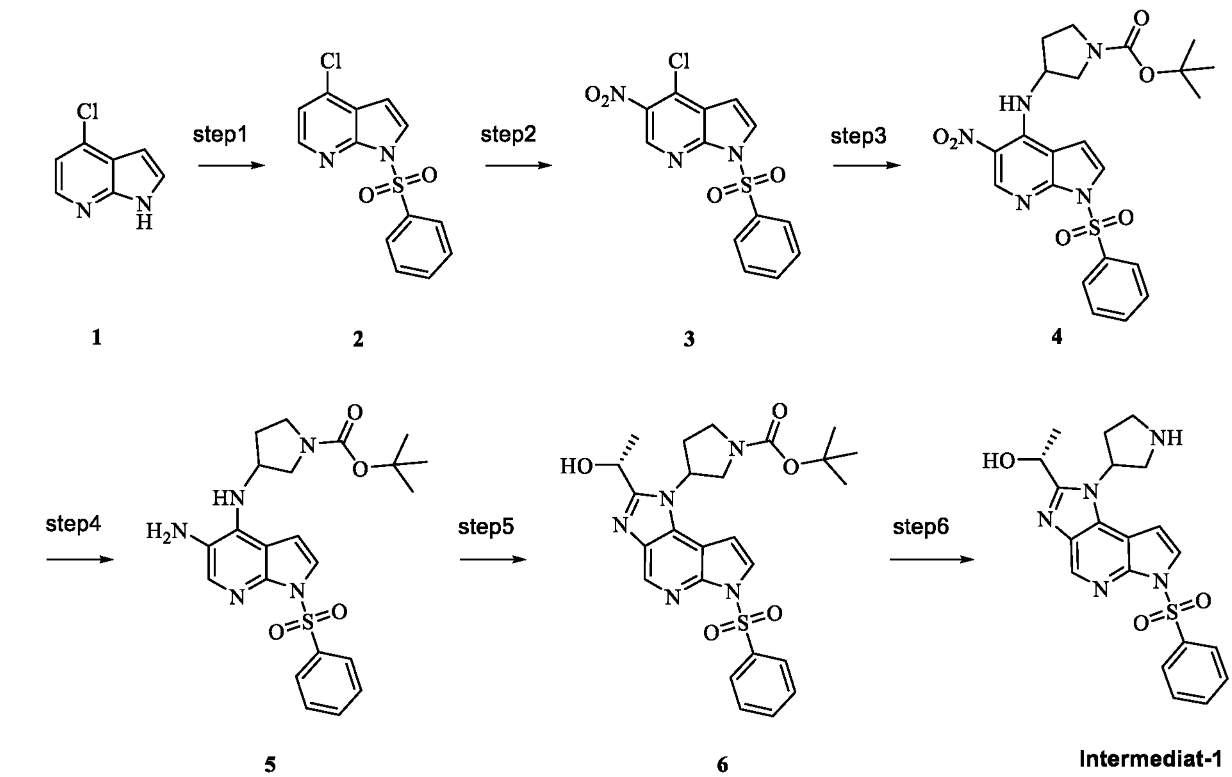


1 2 3 4

5 6 Intermediat-1

Step 1: Dissolve 4-chloro-7-azaindole (25.0 g, 163.8 mmol) in 250 mL of dichloromethane, add DMAP (2.0 g, 16.5 mmol), triethylamine (34.0 mL, 245.8 mmol) and stir at room temperature for 30 min. Dissolve benzenesulfonyl chloride (23.3 mL, 180.3 mmol) in 50 mL of dichloromethane and slowly add dropwise to the above reaction solution, stir for about 4 h at room temperature and then filter, collect the filtrate and concentrate under vacuum to obtain a brown solid. 4-chloro-1-(phenylsulfonyl)-1H-pyrrolo[2,3-b]pyridine is obtained as an off-white solid (43.0 g, 90%) by triturating and curing with an appropriate amount of methanol. The product can be used directly in the next step without further purification. HRMS (ESI): m/z $[M+H]^+$ .$C_{13}H_{10}ClNO_{22}S$ calculated value 293.0146, measured value 293.0139.

Step 2: Dissolve 4-chloro-1-(phenylsulfonyl)-1H-pyrrolo[2,3-b]pyridine (30.0 g, 102.7 mmol) in 300 mL of dichloromethane , add tetramethylammonium nitrate (28.0 g, 205.5 mmol) to the above solution at 25 °C and stir. Add trifluoroacetic anhydride (57.3 mL, 410.8 mmol) slowly dropwise while keeping the temperature of the reaction solution below 30°C. The mixture is stirred at room temperature for 5 h. The completion of the reaction is monitored by TLC. Saturated sodium bicarbonate is then added until the reaction solution was weakly basic, and the organic phase is separated. The aqueous phase is then extracted twice using dichloromethane. Combine the organic phases and wash with saturated NaCl solution, followed by drying over anhydrous sodium sulfate and concentrating under vacuum to yield a yellow solid. Triturating and curing with an appropriate amount of methanol yields 4-chloro-5-nitro-1-(phenylsulfonyl)-1H-pyrrolo[2,3-b]pyridine as a light yellow solid (25.9 g, 75%). The product can be used directly in the next step without further purification.$^1$ HNMR (300 MHz, *DMSO-$d_6$* ): $\delta$ = 9.09 (s, 1H), 8.29 (d, *J* = 3.0 Hz, 1H), 8.17 (t, *J* = 4.5 Hz, 2H), 7.79 (t, *J* = 7.5 Hz, 1H), 7.68 (t, *J* = 7.5 Hz, 2H), 7.11 (d, *J* = 3.0 Hz, 1H) ppm; HRMS (ESI): m/z $[M+Na]^+$ . $C_{13}H_8ClN_3NaO_4S$ calculated value 359.9816, measured value 359.9801.

Step 3: Dissolve 4-chloro-5-nitro-1-(phenylsulfonyl)-1H-pyrrolo[2,3-b]pyridine (10.0 g, 29.7 mmol) in 100 mL of tetrahydrofuran, add DIPEA (7.7 g, 59.3 mmol) and 1-Boc-3-aminopyrrolidine (8.3 g, 44.5 mmol). Stir the mixture under reflux for 4 h at an elevated temperature. The reaction was monitored for completion by TLC. The reaction is then concentrated under vacuum to yield a yellow oily liquid. Tert-butyl 3-((5-nitro-1-(phenylsulfonyl)-1H-pyrrolo[2,3-b]pyridin-4-yl)amino)pyrrolidine-1-carboxylate is obtained as a yellow solid (10.1 g, 70%) by triturating and curing with an appropriate amount of methanol. The product can be used directly in the next step without further purification. HRMS (ESI): m/z $[M+H]^+$ .$C_{22}H_{26}N_5O_6S$ calculated value 488.1598, measured value 488.1607.

Step 4: Dissolve tert-butyl 3-(((5-nitro-1-(phenylsulfonyl)-1H-pyrrolo[2,3-b]pyridin-4-yl)amino)pyrrolidine-1-carboxylate (10.0 g, 20.5 mmol) in 100 mL of methanol, add palladium carbon (1 g, 10%) and keep the reaction under hydrogen atmosphere after replacing the air in the reaction flask with hydrogen gas more than three times.. The reaction is carried out under hydrogen gas atmosphere at room temperature with stirring for 12 h. The completion of reaction is monitored by TLC. After extraction, the filtrate is then collected and concentrated under vacuum to yield 3-(((5-amino-1-(phenylsulfonyl)-1H-pyrrolo[2,3-b]pyridin-4-yl)amino)pyrrolidin-1-tert-butyl carboxylate as a light pink foamy solid (9.2

g, 98%). The product can be used directly in the next step without further purification. HRMS (ESI): m/z $[M+H]^+$ .$C_{22}H_{28}N_5O_4S$ calculated value 458.1857, measured value 458.1862.

Step 5: Dissolve triethyloxonium tetrafluoroborate (11.2 g, 59.1 mmol) and (R)-lactamide (5.3 g, 59.1 mmol) in 100 mL of tetrahydrofuran, stir at room temperature for 3 h and concentrate under vacuum to obtain an oily mixture, followed by the addition of 100 mL of ethanol to dissolve and the addition of tert-butyl 3-(((5-amino-1-(phenyl sulfonyl)-1H-pyrrolo[2,3-b]pyridin-4-yl)amino)pyrrolidine-1-carboxylate (9.0 g, 19.7 mmol) The reaction is then stirred while maintaining reflux for 3 h. The completion of reaction is monitored by TLC. Saturated sodium bicarbonate is then added until the reaction solution is weakly basic, and the organic phase is separated. The aqueous phase is then extracted twice using dichloromethane. Combine the organic phases and wash with saturated NaCl solution, followed by drying over anhydrous sodium sulfate and concentrating under vacuum. The residue is then purified using a silica gel column (petroleum ether: ethyl acetate = 1:1) to yield tert-butyl 3-(2-((R)-1-hydroxyethyl)-6-(phenylsulfonyl)imidazo[4,5-d]pyrrolo[2,3-b]pyridin-1(6H)-yl)pyrrolidine-1-carboxylate as a light green oil (6.0 g, 60%). HRMS (ESI): m/z $[M+H]^+$ .$C_{25}H_{30}N_5O_5S$ calculated value 512.1962, measured value 512.1983.

Step 6: Dissolve tert-butyl 3-(2-((R)-1-hydroxyethyl)-6-(phenylsulfonyl)imidazo[4,5-d]pyrrolo[2,3-b]pyridin-1(6H)-yl)pyrrolidine-1-carboxylate (6.0 g, 11.7 mmol) in 60 mL dichloromethane, slowly add trifluoroacetic acid (13.4 g, 117.4 mmol) and stir at room temperature for 12 h. The product is then concentrated under vacuum to yield **Intermediate-1:** (1R)-1-(6-(phenylsulfonyl)-1-(pyrrolidin-3-yl)-1,6-dihydroimidazo[4,5-d]pyrrolo[2,3-b]pyridin-2-yl) ethanol as a light brown oil (4.8 g, 100%). The product can be used directly in the next step without further purification. HRMS (ESI): m/z $[M+H]^+$ .$C_{20}H_{22}N_5O_3S$ calculated value 412.1438, measured value 412.1448.

Synthesis of key **intermediate 2.**

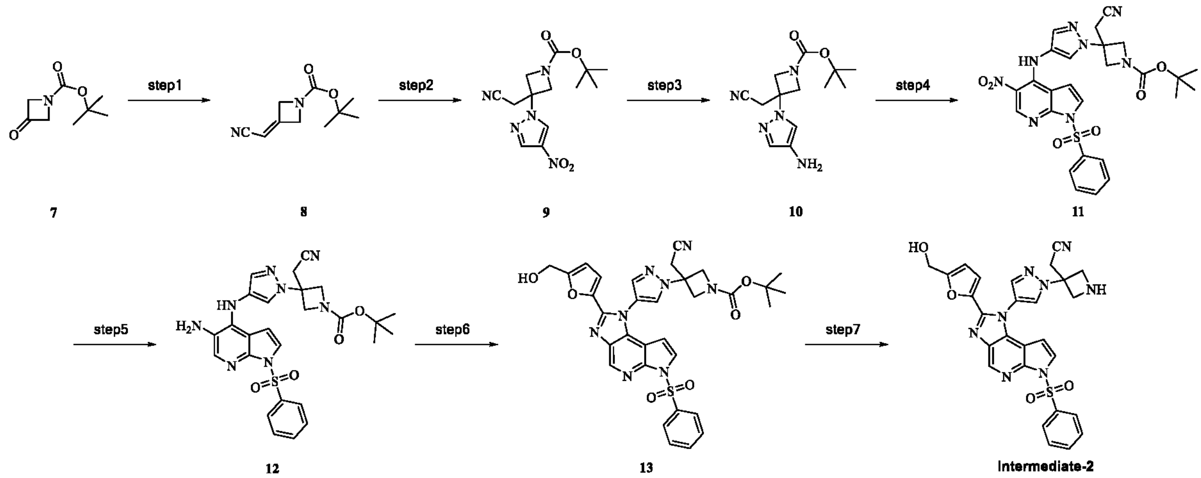


Step 1: A solution of 2-diethoxyphosphoryl acetonitrile (5.7 g, 32.1 mmol) in THF (50 mL) is added to a solution of NaH (1.2 g, 30.7 mmol) in THF (50 mL) at 0 °C. The mixture is then stirred at room temperature for 1 h. Then cool the mixture to 0 °C , and add a solution of tert-butyl 3-oxazetidine-1-carboxylate (5.0 g, 29.2 mmol) in THF (50 mL) within 1 h. Stir the above reaction solution at room temperature for 16 hours. After the reaction is finished, add an appropriate amount of water and extract the aqueous phase twice with EA. Combine the organic layers and wash with saturated NaCl solution, dry over sodium sulfate and concentrate under vacuum to yield tert-butyl 3-(cyanomethyl)azetidine-1-carboxylate as a yellow solid (5.2 g, 78%). The product can be used directly in the next step without further purification. HRMS (ESI): m/z $[M+H]^+$ .$C_{10}H_{15}N_2O_2$ calculated value 195.1128, measured value 195.1121.

Step 2: Add DBU (11.8 g, 77.4 mmol) to a solution of tert-butyl 3-(cyanomethyl)azetidine-1-carboxylate (5.0 g, 25.8 mmol) and 4-nitropyrazole (3.2 g, 28.7 mmol) in acetonitrile (30 mL). Stir the mixture at room temperature for 16 h. At the end of the reaction, add an appropriate amount of water and extract the aqueous phase twice with EA. Combine the organic layers and wash with saturated NaCl solution, dry over sodium sulfate and concentrate under vacuum. The residue is then purified using a silica gel column (petroleum ether: ethyl acetate= 4:1) to yield tert-butyl 3-(cyanomethyl)-3-(4-nitro-1H-pyrazol-1-yl)azetidine-1-carboxylate as a white oil (4.0 g, 50%). HRMS (ESI): m/z $[M+H]^+$.$C_{13}H_{18}N_5O_4$ calculated value 308.1353, measured value 308.1346.

Step 3: Dissolve tert-butyl 3-(cyanomethyl)-3-(4-nitro-1H-pyrazol-1-yl)azetidine-1-carboxylate (4.0 g, 13.0 mmol) in 50 mL of methanol, add palladium carbon (0.4 g, 10%) and then use hydrogen gas to replace the air in the

reaction flask more than three times. Keep the reaction under hydrogen gas atmosphere and stir for 12 h at room temperature. The completion of the reaction is monitored by TLC. The filtrate is then collected and concentrated under vacuum to yield tert-butyl 3-(4-amino-1H-pyrazol-1-yl)-3-(cyanomethyl)azetidine-1-carboxylate as a light brown foamy solid (3.5 g, 98%). The product can be used directly in the next step without further purification. HRMS (ESI): m/z $[M+H]^+$ .$C_{13}H_{20}N_5O_2$ calculated value 278.1612, measured value 278.1610.

Step 4: Dissolve 4-chloro-5-nitro-1-(phenylsulfonyl)-1H-pyrrolo[2,3-b]pyridine (3.5 g, 10.5 mmol) in 100 mL of tetrahydrofuran. Add DIPEA (4.1 g, 31.5 mmol) and tert-butyl 3-(4-amino-1H-pyrazol-1-yl)-3-(cyanomethyl)azetidine-1-carboxylate (3.5 g, 12.6 mmol) and then stir under reflux at an elevated temperature for 4h.. The completion of the reaction is monitored by TLC. The reaction is then concentrated under vacuum to yield a yellow oily liquid. Tert-butyl 3-(cyanomethyl)-3-(4-((5-nitro-1-(phenylsulfonyl)-1H-pyrrolo[2,3-b]pyridin-4-yl)amino)-1H-pyrazol-1-yl)azetidine-1-carboxylate is obtained as a yellow solid (4.3 g, 70%) by triturating and curing using an appropriate amount of methanol. The product can be used directly in the next step without further purification. HRMS (ESI): m/z $[M+H]^+$ .$C_{26}H_{27}N_8O_6S$ calculated value 579.1769, measured value 579.1782.

Step 5: Dissolve tert-butyl 3-(cyanomethyl)-3-(4-((5-nitro-1-(phenylsulfonyl)-1H-pyrrolo[2,3-b]pyridin-4-yl)amino)-1H-pyrazol-1-yl)azetidine-1-carboxylate ester (4.3 g, 7.4 mmol) in 50 mL of methanol, add palladium carbon (0.4 g, 10%) and replace the air in the reaction flask with hydrogen gas more than three times. The reaction is then carried out under hydrogen gas atmosphere with stirring at room temperature for 12 h. The completion of the reaction is monitored by TLC. After extraction, collect the filtrate and concentrate under vacuum to yield tert-butyl 3-(4-((5-amino-1-(phenylsulfonyl)-1H-pyrrolo[2,3-b]pyridin-4-yl)amino)-1H-pyrazol-1-yl)-3-(cyanomethyl)azetidine-1-carboxylate as a light brown foamy solid (4.0 g, 98%). The product can be used directly in the next step without further purification. HRMS (ESI): m/z $[M+H]^+$ . $C_{26}H_{29}N_8O_4S$ calculated value 549.2027, measured value 549.2043.

Step 6: Dissolve tert-butyl 3-(4-((5-amino-1-(phenylsulfonyl)-1H-pyrrolo[2,3-b]pyridin-4-yl)amino)-1H-pyrazol-1-yl)-3-(cyanomethyl)azetidine-1-carboxylate (4.0 g, 7.3 mmol) in 50 mL of DMF. Then add $Na_2S_2O_5$ (6.9 g, 36.5 mmol) and 5-hydroxymethyl furfural (1.8 g, 14.6 mmol), and the reaction is stirred for 12 h at 90 °C. The completion of the reaction is monitored by TLC. Saturated sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and ceoncentrate under vacuum. The residue is then purified using a silica gel column (petroleum ether: ethyl acetate = 1:1) to yield 3-(cyanomethyl)-3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidine-1-carboxylate as a yellow solid (3.3 g, 69%). HRMS (ESI): m/z $[M+H]^+$ $C_{32}H_{31}N_8O_6S$ calculated value 655.2082, measured value 655.2063.

Step 7: Dissolve 3-(cyanomethyl)-3-(4-(2-(5-(hydroxymethyl)furan-2-yl)-6-(phenylsulfonyl)imidazo[4,5-d]pyrrolo[2,3-b pyridin-1(6H)-yl)-1H-pyrazol-1-yl)azetidine-1-carboxylate (3.0 g, 4.6 mmol) in 60 mL of dichloromethane, slowly add trifluoroacetic acid (5.2 g, 46.0 mmol) and stir at room temperature for 12 h. Concentrate under vacuum to yield **Intermediate 2:** 2-(3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl) acetonitrile as a light yellow oil (2.3 g, 91%). The product can be used directly in the next step without further purification. HRMS (ESI): m/z $[M+H]^+$. $C_{27}H_{23}N_8O_4S$ calculated value 555.1557, measured value 555.1555.

Synthesis of key **intermediate 3** and **intermediate 4.**

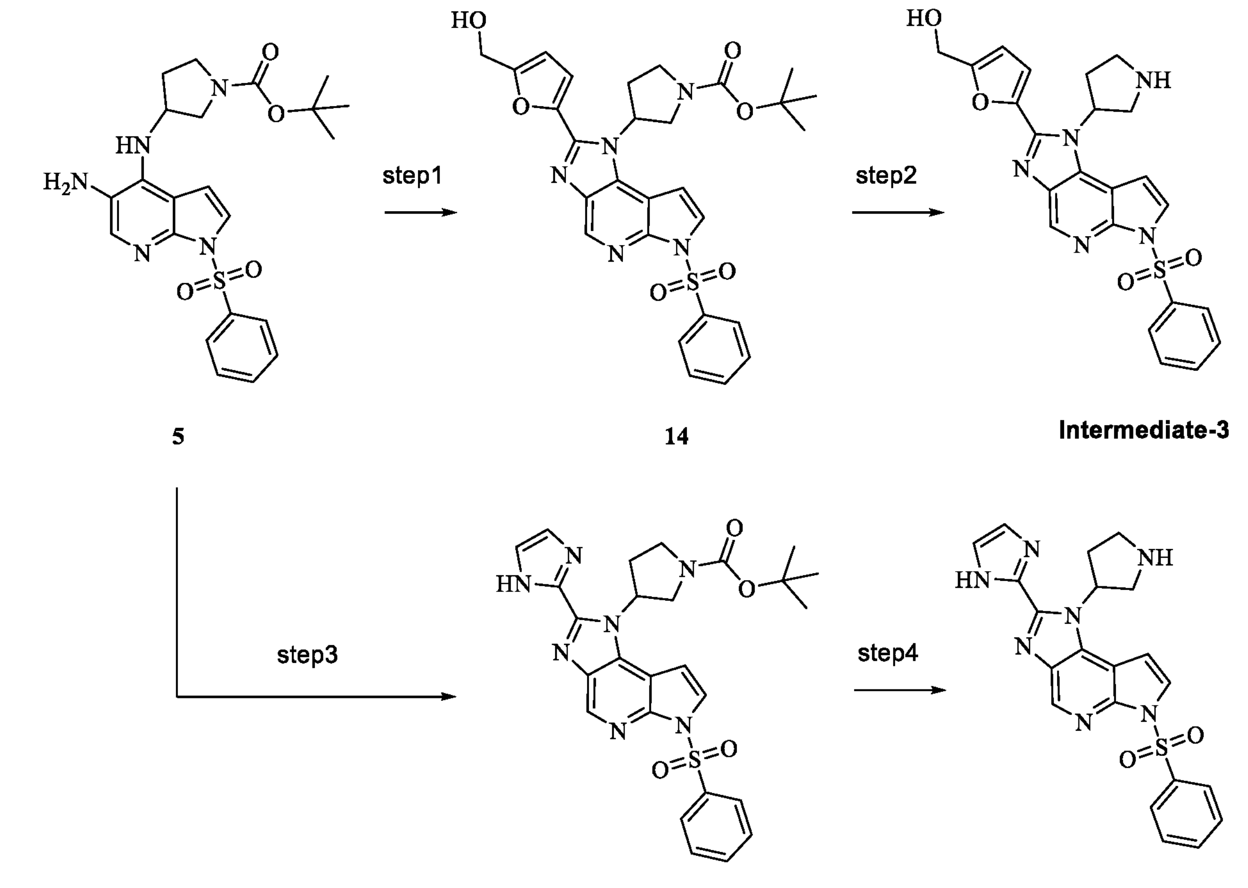


5 14 Intermediate-3

15 Intermediate-4

Step 1: Dissolve tert-butyl 3-(((5-amino-1-(phenylsulfonyl)-1H-pyrrolo[2,3-b]pyridin-4-yl)amino)pyrrolidine-1-carboxylate (3.0 g, 6.6 mmol) in 50 mL of DMF and add $Na_2S_2O_5$ (6.2 g, 32.8 mmol) and 5-hydroxymethylfurfural (1.7 g, 13.2 mmol) . The mixture is then stirred at 90 °C for 12 h. The completion of the reaction is monitored by TLC. Saturated sodium bicarbonate is then added until the reaction solution is weakly basic, and the organic phase is separated. Extract the aqueous phase twice with dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield tert-butyl 3-(2-(5-(hydroxymethyl)furan-2-yl)-6-(phenylsulfonyl)imidazo[4,5-d]pyrrolo[2,3-b]pyridin-1(6H)-yl pyrrolidine-1-carboxylate as a yellow solid (2.4 g, 65%). HRMS (ESI): m/z $[M+H]^+$ .$C_{28}H_{30}N_5O_6S$ calculated value 564.1911, measured value 564.1921.

Step 2: Dissolve tert-butyl 3-(2-(5-(hydroxymethyl)furan-2-yl)-6-(phenylsulfonyl)imidazo[4,5-d]pyrrolo[2,3-b]pyridin-1(6H)-yl pyrrolidine-1-carboxylate (2.0 g, 3.6 mmol) in 50 mL dichloromethane, slowly add trifluoroacetic acid (4.1 g, 36.0 mmol) at room temperature and then stir for 12 h. The product is then concentrated under vacuum to yield

**Intermediate-3:**(5-(6-(phenylsulfonyl)-1-(pyrrolidin-3 -yl)-1,6-dihydroimidazo[4,5-d]pyrrolo[2,3-b]pyridin-2-yl)furan-2- methanol as a light yellow oil (1.5 g, 91%). The product can be used directly in the next step without further purification. HRMS (ESI): m/z $[M+H]^+$ .$C_{23}H_{22}N_5O_4S$ calculated value 464.1387, measured value 464.1385.

Step 3: Dissolve tert-butyl 3-(((5-amino-1-(phenylsulfonyl)-1H-pyrrolo[2,3-b]pyridin-4-yl)amino)pyrrolidine-1-carboxylate (3.0 g, 6.6 mmol) in 50 mL of DMF and add $Na_2S_2O_5$ (6.2 g, 32.8 mmol) and 2-imidazolecarboxaldehyde (1.3 g, 13.2 mmol). The mixture is then stirred at 90 °C for 12 h. The completion of the reaction is monitored by TLC. Saturated sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield tert-butyl 3-(2-(1H-imidazol-2-yl)-6-(phenylsulfonyl)imidazo[4,5-d]pyrrolo[2,3-b]pyridin-1(6H)-yl)pyrrolidine-1-carboxylate as a yellow solid (2.2 g, 63%). HRMS (ESI): m/z $[M+H]^+$ . $C_{26}H_{28}N_7O_4S$ calculated value 534.1918, measured value 534.1910.

Step 4: Dissolve tert-butyl 3-(2-(1H-imidazol-2-yl)-6-(phenylsulfonyl)imidazo[4,5-d]pyrrolo[2,3-b]pyridin-1(6H)-yl)pyrrolidine-1-carboxylate (2.0 g, 3.8 mmol) in 50 mL of dichloromethane, slowly add trifluoroacetic acid (4.3 g, 38.0 mmol) and stir at room temperature for 12 h. The product is then concentrated under vacuum to yield **Intermediate**

**4:** 2-(1H-imidazol-2-yl)-6-(phenylsulfonyl)-1-(pyrrolidin-3-yl)-1,6-dihydroimidazo[4,5-d]pyrrolo[2,3-b]pyridine as a light yellow oil (1.2 g, 74%). The product can be used directly in the next step without further purification. HRMS (ESI): m/z [M+H]$^+$ .$C_{21}H_{20}N_7O_2S$ calculated value 434.1394, measured value 434.1390.

Synthesis of key **intermediates 5, intermediate 6, intermediate** 7 and **intermediate 8.**

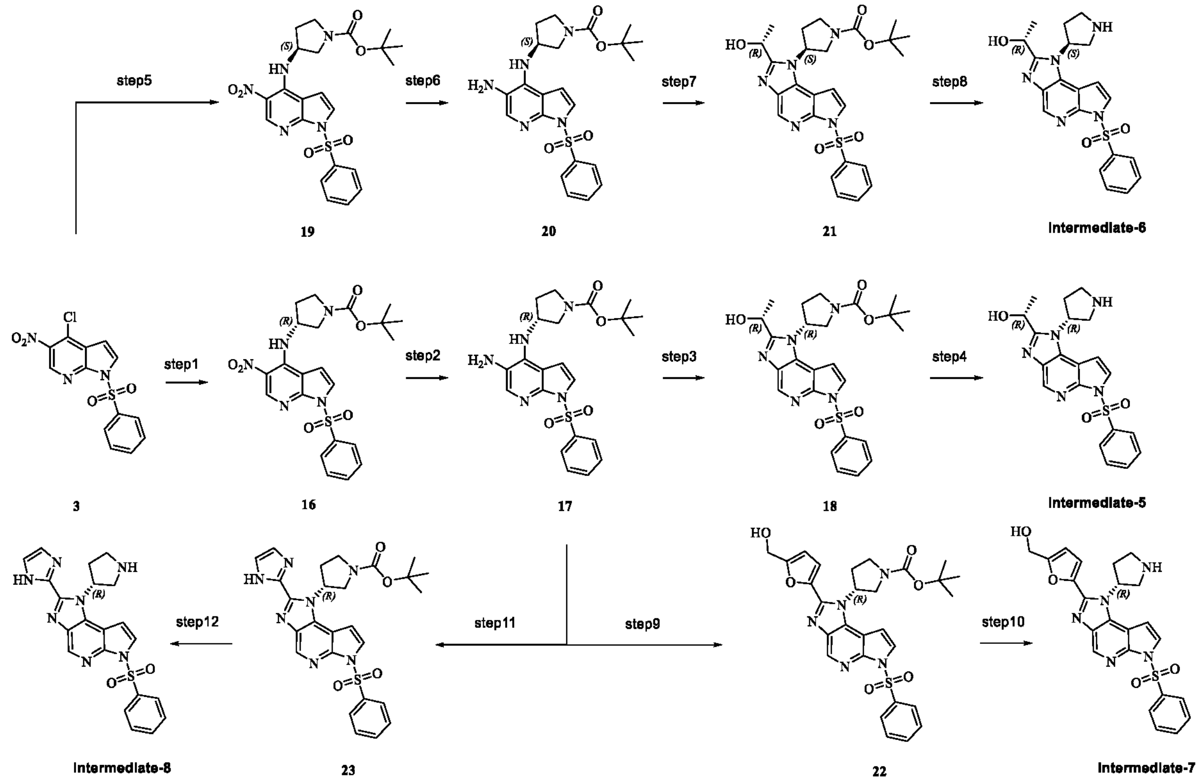


19, 20, 21, Intermediate-6

3, 16, 17, 18, Intermediate-5

Intermediate-8, 23, 22, Intermediate-7

Step 1: Dissolve 4-chloro-5-nitro-1-(phenylsulfonyl)-1H-pyrrolo[2,3-b]pyridine (5.0 g, 14.8 mmol) in 50 mL of tetrahydrofuran, add DIPEA (3.8 g, 29.7 mmol) and (R)-(+)-1-Boc-3-aminopyrrolidine (4.1 g, 22.3 mmol). The mixture is then stirred while maintaining reflux at an elevated temperature for 4 hours. The completion of the reaction is monitored by TLC. Concentrate under vacuum to yield a yellow oily liquid. Triturating and curing with an appropriate amount of methanol yields tert-butyl 3-((5-nitro-1-(phenylsulfonyl)-1H-pyrrolo[2,3-b]pyridin-4-yl)amino)pyrrolidine-1-carboxylate (R) as a yellow solid (4.8 g, 66%). The product can be used directly in the next step without further purification. HRMS (ESI): m/z [M+H]$^+$ .$C_{22}H_{26}N_5O_6S$ calculated value 488.1598, measured value 488.1605.

Step 2: Dissolve tert-butyl 3-((5-nitro-1-(phenylsulfonyl)-1H-pyrrolo[2,3-b]pyridin-4-yl)amino)pyrrolidine-1-carboxylate (R) (4.8 g, 9.9 mmol) in 50 mL of methanol, add palladium carbon (0.5 g, 10%) and replace the air in the reaction flask more than three times using hydrogen gas. The reaction is then carried out under hydrogen gas atmosphere with stirring at room temperature for 12 h. The completion of the reaction is monitored by TLC. The filtrate is then collected and concentrated under vacuum to yield (R) tert-butyl 3-(((5-amino-1-(phenylsulfonyl)-1H-pyrrolo[2,3-b]pyridin-4-yl)amino)pyrrolidine-1-carboxylate as a light pink foamy solid (4.2 g, 93%). The product can be used directly in the next step without further purification. HRMS (ESI): m/z [M+H]$^+$ .$C_{22}H_{28}N_5O_4S$ calculated value 458.1857, measured value 458.1850.

Step 3: Dissolve triethyloxonium tetrafluoroborate (5.0 g, 26.3 mmol) and (R)-lactamide (2.3 g, 26.3 mmol) in 80 mL of tetrahydrofuran, stir for 3 h at room temperature and then concentrate the mixture under vacuum to obtain an oil-like substance, followed by adding 80 mL of ethanol to dissolve and adding (R)tert-butyl 3-(((5-amino-1-(phenylsulfonyl)-1H-pyrrolo[2,3-b]pyridin-4-yl)amino)pyrrolidine-1-carboxylate (4.0 g, 8.8 mmol). The reaction is then stirred while maintaining reflux for 3 h and the completion of the reaction is monitored by TLC. Saturated sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield (R)-tert-butyl 3-(2-((R)-1-hydroxyethyl)-6-(phenylsulfonyl)imidazo[4,5-d]pyrrolo[2,3-b]pyridin-1(6H)-pyrrolidine-1-carboxylate as a light green oil (3.2 g, 72%). HRMS (ESI): m/z $[M+H]^+$ .$C_{25}H_{30}N_5O_5S$ calculated value 512.1962, measured value 512.1974.

Step 4: Dissolve (R)-tert-butyl 3-(2-((R)-1-hydroxyethyl)-6-(phenylsulfonyl)imidazo[4,5-d]pyrrolo[2,3-b]pyridin-1(6H)-pyrrolidine-1-carboxylate (3.0 g, 5.9 mmol) in 50 mL of dichloromethane, slowly add trifluoroacetic acid (6.7 g, 58.6 mmol) and then stir for 12 h at room temperature. Concentrate under vacuum to yield **Intermediate 5:** (R)-1-(6-(phenylsulfonyl)-1-((R-pyrrolidin-3-yl)-1,6-dihydroimidazo[4,5-d]pyrrolo[2,3-b]pyridin-2-yl)ethanol as a light brown oil (2.2 g, 91%). The product can be used directly in the next step without further purification. HRMS (ESI): m/z $[M+H]^+$ .$C_{20}H_{22}N_5O_3S$ calculated value 412.1438, measured value 412.1433.

Step 5: Dissolve 4-chloro-5-nitro-1-(phenylsulfonyl)-1H-pyrrolo[2,3-b]pyridine (8.0 g, 23.7 mmol) in 80 mL of tetrahydrofuran. Add DIPEA (6.1 g, 47.5 mmol) and (S)-(-)-1-Boc-3-aminopyrrolidine (6.6 g, 35.6 mmol). Then, stir the reaction while maintaining reflux at an elevated temperature for 4 hours and monitor the reaction for completion by TLC. Concentrated under vacuum to yield a yellow oily liquid. Triturate and cure with an appropriate amount of methanol to yield tert-butyl 3-((5-nitro-1-(phenylsulfonyl)-1H-pyrrolo[2,3-b]pyridin-4-yl)amino)pyrrolidine-1-carboxylate (S) as a yellow solid (8.1 g, 70%). The product can be used directly in the next step without further purification. HRMS (ESI): m/z $[M+H]^+$ .$C_{22}H_{26}N_5O_6S$ calculated value 488.1598, measured value 488.1603.

Step 6: Dissolve tert-butyl 3-((5-nitro-1-(phenylsulfonyl)-1H-pyrrolo[2,3-b]pyridin-4-yl)amino)pyrrolidine-1-carboxylate (S) (8.0 g, 16.4 mmol) in 80 mL of methanol, add palladium carbon (0.8 g, 10%) and replace the air in the reaction vial more than three times using hydrogen gas. The reaction is then carried out under hydrogen gas atmosphere and stirred at room temperature for 12 h. The completion of the reaction is monitored by TLC. The filtrate is then collected and concentrated under vacuum to yield (S) tert-butyl 3-(((5-amino-1-(phenyl sulfonyl)-1H-pyrrolo[2,3-b]pyridin-4-yl)amino)pyrrolidine-1-carboxylate as a light pink foamy solid (7.4 g, 99%). The product can be used directly in the next step without further purification. HRMS (ESI): m/z $[M+H]^+$ .$C_{22}H_{28}N_5O_4S$ calculated value 458.1857, measured value 458.1850.

Step 7: Dissolve triethyloxonium tetrafluoroborate (8.7 g, 46.0 mmol) and (R)-lactamide (4.1 g, 46.0 mmol) in 100 mL of tetrahydrofuran, stir for 3 h at room temperature and then concentrate the mixture in vacuum to obtain an oily substance, followed by the addition of 100 mL of ethanol to dissolve, and the addition of (S) tert-butyl 3-(((5-amino-1-(phenylsulfonyl)-1H-pyrrolo[2,3-b]pyridin-4-yl)amino)pyrrolidine-1-carboxylate (7.0 g, 15.3 mmol) The mixture is then stirred while maintaining reflux at an elevated temperature for 3 h and the completion of the reaction is monitored by TLC.. Saturated sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield (S)-tert-butyl 3-(2-((R)-1-hydroxyethyl)-6-(phenylsulfonyl)imidazo[4,5-d]pyrrolo[2,3-b]pyridin-1(6H)-pyrrolidine-1-carboxylate as a light green oil (5.3 g, 68%). HRMS (ESI): m/z $[M+H]^+$ .$C_{25}H_{30}N_5O_5S$ calculated value 512.1962, measured value 512.1969.

Step 8: Dissolve (S)-tert-butyl 3-(2-((R)-1-hydroxyethyl)-6-(phenylsulfonyl)imidazo[4,5-d]pyrrolo[2,3-b]pyridin-1(6H)-pyrrolidine-1-carboxylate (5.0 g, 9.8 mmol) in 50 mL of dichloromethane, slowly add trifluoroacetic acid (11.2 g, 97.8 mmol) and then stir for 12 h at room temperature. Concentrate under vacuum to yield **Intermediate 6:** (R)-1-(6-(phenylsulfonyl)-1-((S-pyrrolidin-3-yl)-1,6-dihydroimidazo[4,5-d]pyrrolo[2,3-b]pyridin-2-yl)ethanol as a light brown oil (3.8 g, 94%). The product can be used directly in the next step without further purification. HRMS (ESI): m/z $[M+H]^+$ .$C_{20}H_{22}N_5O_3S$ calculated value 412.1438, measured value 412.1433.

Step 9: Dissolve (R) tert-butyl 3-(((5-amino-1-(phenylsulfonyl)-1H-pyrrolo[2,3-b]pyridin-4-yl)amino)pyrrolidine-1-carboxylate (3.0 g, 6.6 mmol) in 50 mL of DMF. Then, add $NaS_2O_5$ (6.2 g, 32.8 mmol) and 5-hydroxymethylfurfural (1.7 g, 13.2 mmol). Stir the mixture at 90 °C for 12 h. The completion of the reaction is monitored by TLC. Saturated sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield (R)-tert-butyl 3-(2-(5-(hydroxymethyl)furan-2-yl)-6-(phenylsulfonyl)imidazo[4,5-d]pyrrolo[2,3-b]pyridin-1(6H)-yl)pyrrolidine-1-carboxylate as a yellow solid (2.0 g, 54%). HRMS (ESI): m/z $[M+H]^+$ $C_{28}H_{30}N_5O_6S$ calculated value 564.1911, measured value 564.1922.

Step 10: Dissolve (R)-tert-butyl 3-(2-(5-(hydroxymethyl)furan-2-yl)-6-(phenylsulfonyl)imidazo[4,5-d]pyrrolo[2,3-b]pyridin-1(6H)-yl)pyrrolidine-1-carboxylate (2.0 g, 3.6 mmol) in 30 mL of dichloromethane, slowly add trifluoroacetic acid (4.1 g, 36.0 mmol) and stir for 12 h at room temperature. **Intermediate 7,** which is (R)-(5-(6-(phenylsulfonyl)-1-(pyrrolidin-3-yl)-1,6-dihydroimidazo[4,5-d]pyrrolo[2,3-b]pyridin-2-yl) furan-2-yl methanol is then obtained as a light yellow oil (1.6 g, 97%) The product can be used directly in the next step without further purification. HRMS (ESI): m/z $[M+H]^+$ .$C_{23}H_{22}N_5O_4S$ calculated value 464.1387, measured value 464.1380.

Step 11: Dissolve (R) tert-butyl 3-(((5-amino-1-(phenylsulfonyl)-1H-pyrrolo[2,3-b]pyridin-4-yl)amino)pyrrolidine-1-carboxylate (3.0 g, 6.6 mmol)in 50 mL of DMF. Then, add $Na_2S_2O_5$(6.2 g, 32.8 mmol) and 2-imidazolecarboxaldehyde (1.3 g, 13.2 mmol) and stir the mixture at 90 °C for 12 h. The completion of the reaction is monitored by TLC. Saturated

sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield (R)-tert-butyl 3-(2-(1H-imidazol-2-yl)-6-(phenylsulfonyl)imidazo[4,5-d]pyrrolo[2,3-b]pyridin-1(6H)-pyrrolidine-1-carboxylate as a yellow solid (2.5 g, 71%). HRMS (ESI): m/z [M+H]+ .$C_{26}H_{28}N_7O_4S$ calculated value 534.1918, measured value 534.1910.

Step 12: Dissolve (R)-tert-butyl 3-(2-(1H-imidazol-2-yl)-6-(phenylsulfonyl)imidazo[4,5-d]pyrrolo[2,3-b]pyridin-1(6H)-pyrrolidine-1-carboxylate (2.5 g, 4.7 mmol) in 30 mL of dichloromethane, slowly add trifluoroacetic acid (5.3 g, 46.9 mmol) and then stir at room temperature for 12 h. Concentrate under vacuum to obtain **Intermediate** 8: (R)-2-(1H-imidazol-2-yl)-6-(phenylsulfonyl)-1-(pyrrolidin-3-yl)-1,6-dihydroimidazo[4,5-d]pyrrolo[2,3- b]pyridine as a light yellow oil (1.7 g, 84%). The product can be used directly in the next step without further purification. HRMS (ESI): m/z [M+H]+ .$C_{21}H_{20}N_7O_2S$ calculated value 434.1394, measured value 434.1390.

Synthesis of key **intermediate 9.**

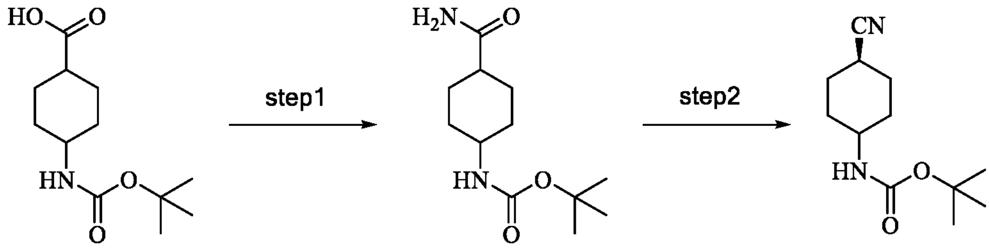


24 25 26

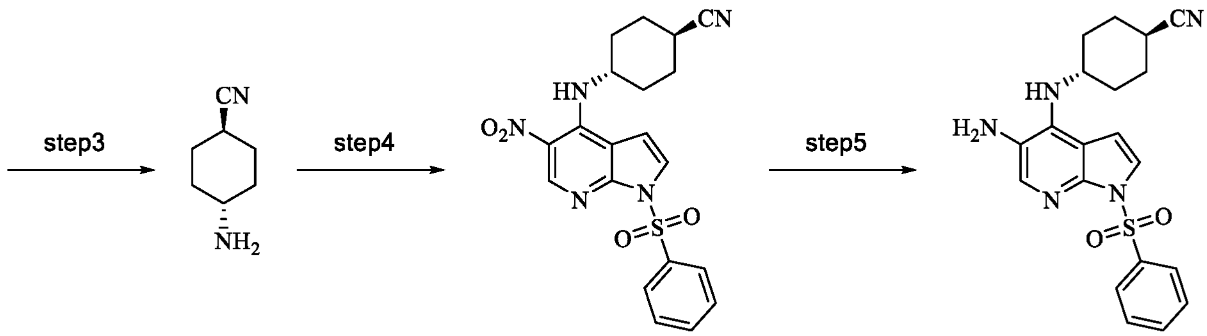


27 28 **Intermediate-9**

Step 1: Dissolve 4-((tert-butoxycarbonyl)amino)cyclohexanecarboxylic acid (10.0 g, 41.1 mmol) in 100 mL of tetrahydrofuran and cool down to -15°C. Add isobutyl chloroformate (11.3 g, 82.2 mmol) at low temperature and stir at low temperature for 1 h. Then, slowly add 100 mL of ammonia and stir at room temperature for 4 h. After the reaction is complete, filter the product and tert-butyl (4-carbamoylcyclohexyl)carbamate is obtained as a white solid (8.0 g, 80%). The product can be used directly in the next step without further purification. HRMS (ESI): m/z [M+H]+ .$C_{12}H_{23}N_2O_3$ calculated value 243.1703, measured value 243.1698.

Step 2: Dissolve tert-butyl (4-carbamoylcyclohexyl)carbamate (8.0 g, 33.1 mmol) in 50 mL of pyridine and cool down to -15°C. Add phosphorus oxychloride (10 g, 66.2 mmol) slowly dropwise at low temperature and stir at low temperature for 3 h. After the reaction is complete, pour the reaction solution slowly into 200 mL of ice-water mixture. Extract the aqueous phase three times with EA. Combine the organic phases and wash with saturated NaCl solution, dry with anhydrous sodium sulfate and concentrate under vacuum to yield tert-butyl (4-cyanocyclohexyl)carbamate as a milky-white solid (5.5 g, 74%). HRMS (ESI): m/z [M+H]+ .$C_{12}H_{21}N_2O_2$ calculated value 225.1598, measured value 225.1603.

Step 3: Dissolve tert-butyl (4-cyanocyclohexyl)carbamate (5.5 g, 24.6 mmol) in 50 mL of dichloromethane and slowly add trifluoroacetic acid (28.0 g, 245.5 mmol). Stir the mixture at room temperature for 12 h. The product is then

concentrated under vacuum to yield trans-4-aminocyclohexanecarbonitrile as a light white solid (2.5 g, 82%). The product can be used directly in the next step without further purification. HRMS (ESI): m/z [M+H]+ .$C_7H_{13}N_2$ calculated value 125.1073, measured value 125.1082.

Step 4: Dissolve 4-chloro-5-nitro-1-(phenylsulfonyl)-1H-pyrrolo[2,3-b]pyridine (4.5 g, 13.4 mmol) in 50 mL of tetrahydrofuran, add DIPEA (3.5 g, 26.9 mmol) and trans-4-aminocyclohexanecarbonitrile (2.5 g, 20.2 mmol) and stir for 4 h at an elevated temperature while maintaining reflux. The completion of the reaction is monitored by TLC. Concentrate under vacuum to obtain a yellow oily liquid. Triturate and cure with an appropriate amount of methanol to yield trans-4-((5-nitro-1-(phenylsulfonyl)-1H-pyrrolo[2,3-b]pyridin-4-yl)amino)cyclohexanecarbonitrile as a yellow solid (4.2 g, 74%). The product can be used directly in the next step without further purification. HRMS (ESI): m/z [M+H]+ .$C_{20}H_{20}N_5O_4S$ calculated value 426.1231, measured value 426.1237.

Step 5: Dissolve trans-4-((5-nitro-1-(phenylsulfonyl)-1H-pyrrolo[2,3-b]pyridin-4-yl)amino)cyclohexanecarbonitrile (4.2 g, 9.9 mmol) in 50 mL of methanol, add palladium carbon (0.4 g, 10%) and replace the air in the reaction flask with hydrogen gas more than three times. The reaction is then carried out under hydrogen gas atmosphere and stirred at room temperature for 12 h. The completion of the reaction is monitored by TLC. After filtration, collect the filtrate and concentrate under vacuum to yield **Intermediate 9:** trans-4-((5-amino-1-(phenylsulfonyl)-1H-pyrrolo[2,3-b]pyridin-4-yl)amino)cyclohexanecarbonitrile as a light brown foamy solid (3.5 g, 90%). The product can be used directly in the next step without further purification. HRMS (ESI): m/z [M+H]+ .$C_{20}H_{22}N_5O_2S$ calculated value 396.1489, measured value 396.1492.

Synthesis of key **intermediate 10** and **intermediate 11.**

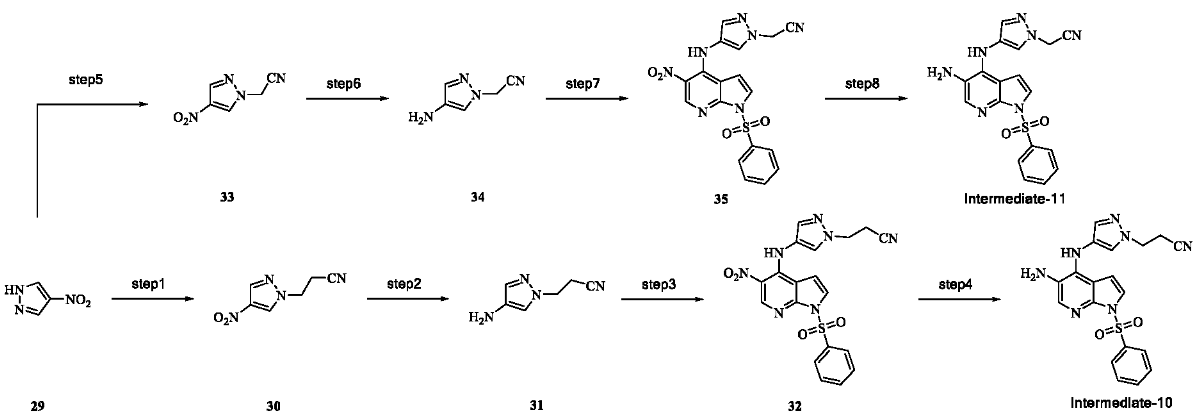


Step 1: Dissolve 4-nitropyrazole (5 g, 44.2 mmol) in 50 mL of DMF, add potassium carbonate (12.2 g, 88.4 mmol) and 3-bromopropionitrile (8.9 g, 66.3 mmol),Then, stir the mixture at room temperature for 12 h. The completion of the reaction is monitored by TLC. Saturated sodium bicarbonate is then added until the reaction solution is weakly basic, and the organic phase is separated. Extract the aqueous phase three times using dichloromethane. Combine the organic phases and wash with saturated NaCl solution, dry over anhydrous sodium sulfate and concentrate under vacuum to yield 3-(4-nitro-1H-pyrazol-1-yl)propanenitrile as a pale yellow solid (4.8 g, 65%) The product can be used directly in the next step without further purification. HRMS (ESI): m/z [M+H]+ .$C_6H_7N_4O_2$ calculated value 167.0564, measured value 167.0569.

Step 2: Dissolve 3-(4-nitro-1H-pyrazol-1-yl)propanenitrile (4.8 g, 28.9 mmol) in 50 mL of methanol, add palladium carbon (0.5 g, 10%) and use hydrogen gas to replace the air in the reaction flask more than three times The reaction is then carried out under hydrogen atmosphere and stirred at room temperature for 12 h. The completion of the reaction is monitored by TLC. After filtration, the filtrate is then collected and concentrated under vacuum to yield 3-(4-amino-1H-pyrazol-1-yl)propanenitrile as a light brown foamy solid (3.5 g, 89%). The product can be used directly in the next step without further purification. HRMS (ESI): m/z [M+H]+ .$C_6H_9N_4$ calculated value 137.0822, measured value 137.0833.

Step 3: Dissolve 4-chloro-5-nitro-1-(phenylsulfonyl)-1H-pyrrolo[2,3-b]pyridine (5.8 g, 17.2 mmol) in 60 mL of tetrahydrofuran. Add DIPEA (4.4 g, 34.4 mmol) and 3-(4-amino-1H-pyrazol-1-yl)propanenitrile (3.5 g, 25.7 mmol). The mixture is stirred while maintaining reflux for 4 hours at an elevated temperature and the completion of the reaction is monitored by TLC. Subsequent concentration under vacuum yields a yellow oily liquid. Triturating and curing with an appropriate amount of methanol yields 3-(4-((5-nitro-1-(phenylsulfonyl)-1H-pyrrolo[2,3-b]pyridin-4-yl)amino)-1H-pyrazol-1-yl)propanenitrile as a yellow solid (5.1 g, 68%). The product can be used directly in the next step without further

purification. HRMS (ESI): m/z $[M+H]^+$ .$C_{19}H_{16}N_7O_4S$ calculated value 438.0979, measured value 438.0985.

Step 4: Dissolve 3-(4-((5-nitro-1-(phenylsulfonyl)-1H-pyrrolo[2,3-b]pyridin-4-yl)amino)-1H-pyrazol-1-yl)propanenitrile (5.0 g, 11.4 mmol) in 50 mL of methanol,add palladium carbon (0.5 g, 10%) and replace the air in the reaction flask more than three times using hydrogen gas. The reaction is then carried out under hydrogen gas atmosphere and stirred at room temperature for 12 h. The completion of the reaction is monitored by TLC. After filtration, collect the filtrate and concentrate under vacuum to yield **Intermediate 10:** 3-(4-((5-amino-1-(phenylsulfonyl)-1H-pyrrolo[2,3-b]pyridin-4-yl)amino)-1H-pyrazol-1-yl)propanenitrile as a light yellow foamy solid (4.5 g, 97%). The product can be used directly in the next step without further purification. HRMS (ESI): m/z $[M+H]^+$ .$C_{19}H_{18}N_7O_2S$ calculated value 408.1237, measured value 408.1241.

Step 5: Dissolve 4-nitropyrazole (2.5 g, 22.1 mmol) in 50 mL of DMF, add potassium carbonate (6.1 g, 44.2 mmol) and 2-bromoacetonitrile (4.0 g, 33.2 mmol) and stir at room temperature for 12 h. The completion of the reaction is monitored by TLC. Saturated sodium bicarbonate is then added until the reaction solution is weakly basic, and the organic phase is separated. Extract the aqueous phase three times using dichloromethane. Combine the organic phases and wash with saturated NaCl solution, dry over anhydrous sodium sulfate and concentrate under vacuum to yield 3-(4-nitro-1H-pyrazol-1-yl)acetonitrile as a pale yellow solid (2.2 g, 65%) The product can be used directly in the next step without further purification. HRMS (ESI): m/z $[M+H]^+$ .$C_5H_5N_4O_2$ calculated value 153.0407, measured value 153.0411.

Step 6: Dissolve 3-(4-nitro-1H-pyrazol-1-yl)acetonitrile (2.2 g, 14.5 mmol) in 30 mL of methanol, add palladium carbon (0.3 g, 10%) and then use hydrogen gas to replace the air in the reaction flask more than three times. The reaction is then carried out under hydrogen gas atmosphere and stirred at room temperature for 12 h. The completion of the reaction is monitored by TLC. After filtration, collect the filtrate and concentrate under vacuum to yield 3-(4-amino-1H-pyrazol-1-yl)acetonitrile as a light brown foamy solid (1.6 g, 91%). The product can be used directly in the next step without further purification. HRMS (ESI): m/z $[M+H]^+$ .$C_5H_7N_4$ calculated value 123.0665, measured value 123.0671.

Step 7: Dissolve 4-chloro-5-nitro-1-(phenylsulfonyl)-1H-pyrrolo[2,3-b]pyridine (2.8 g, 8.2 mmol) in 60 mL of tetrahydrofuran. Add DIPEA (2.1 g, 16.4 mmol) and 3-(4-amino-1H-pyrazol-1-yl)acetonitrile (1.5 g, 12.3 mmol) and then stir the mixture while maintaining reflux at an elevated temperature for 4 h. The completion of the reaction is monitored by TLC. Concentrate under vacuum to yield a yellow oily liquid. Then triturate and cure with an appropriate amount of methanol to yield 3-(4-((5-nitro-1-(phenylsulfonyl)-1H-pyrrolo[2,3-b]pyridin-4-yl)amino)-1H-pyrazol-1-yl)acetonitrile as a yellow solid (2.5 g, 72%). The product can be used directly in the next step without further purification. HRMS (ESI): m/z $[M+H]^+$ .$C_{18}H_{14}N_7O_4S$ calculated value 424.0822, measured value 424.0833.

Step 8: Dissolve 3-(4-((5-nitro-1-(phenylsulfonyl)-1H-pyrrolo[2,3-b]pyridin-4-yl)amino)-1H-pyrazol-1-yl)acetonitrile (2.5 g, 5.9 mmol) in 30 mL of methanol, add palladium carbon (0.3 g, 10%) and replace the air in the reaction flask more than three times using hydrogen gas. The reaction is then carried out under hydrogen gas atmosphere at room temperature with stirring for 12 h. The completion of the reaction is monitored by TLC. Collect the filtrate and concentrate under vacuum to yield **Intermediate 11:** 3-(4-((5-amino-1-(phenylsulfonyl)-1H-pyrrolo[2,3-b]pyridin-4-yl)amino)-1H-pyrazol-1-yl)acetonitrile as a light yellow foamy solid (2.0 g, 86%). The product can be used directly in the next step without further purification. HRMS (ESI): m/z $[M+H]^+$ $C_{18}H_{16}N_7O_2S$ calculated value 394.1081, measured value 394.1084.

Synthesis of key **intermediate 12.**

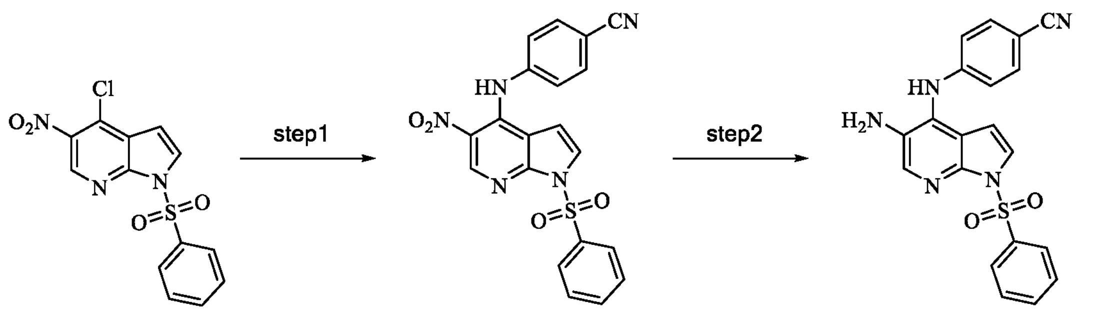


3 36 **Intermediate-12**

Step 1: Dissolve 4-chloro-5-nitro-1-(phenylsulfonyl)-1H-pyrrolo[2,3-b]pyridine (2.0 g, 5.9 mmol) in 50 mL of toluene. Then, add tris(dibenzalacetone)dipalladium (0.2 g, 10%), 4,5-bis(diphenylphosphino)-9,9-dimethylxanthene (0.2 g, 10%) and p-cyananiline (1.4 g, 11.9 mmol). The reaction is then carried out under nitrogen gas protection and

stirred while maintaining reflux at an elevated temperature for 5 h. The completion of the reaction is monitored by TLC. Saturated sodium bicarbonate is then added until the reaction solution is weakly basic, and the organic phase is separated. Extract the aqueous phase three times using dichloromethane. Combine the organic phases and wash with saturated NaCl solution, dry over anhydrous sodium sulfate and concentrate under vacuum. The residue is then purified using a silica gel column (petroleum ether: ethyl acetate= 2:1) to yield 4-((5-nitro-1-(phenylsulfonyl)-1H-pyrrolo[2,3-b]pyridin-4-yl)amino)benzonitrile as a yellow solid (1.2 g, 48%). hRMS (ESI): m/ z $[M+H]^+$ .$C_{20}H_{14}N_5O_4S$ calculated value 420.0761, measured value 420.0771.

Step 2: Dissolve 4-((5-nitro-1-(phenylsulfonyl)-1H-pyrrolo[2,3-b]pyridin-4-yl)amino)benzonitrile (1.2 g, 2.9 mmol) in 30 mL of methanol, add palladium carbon (0.2 g, 10%) and then use hydrogen gas to replace the air in the reaction flask more than three times. The reaction is then carried out under hydrogen gas atmosphere and stirred at room temperature for 12 h. The completion of the reaction is monitored by TLC. Collect the filtrate and concentrate under vacuum to yield **Intermediate 12:** 4-((5-amino-1-(phenyl sulfonyl)-1H-pyrrolo[2,3-b]pyridin-4-yl)amino)benzonitrile as a yellow foamy solid (1.1 g, 99%). The product can be used directly in the next step without further purification. HRMS (ESI): m/z $[M+H]^+$ .$C_{20}H_{16}N_5O_2S$ calculated value 390.1019, measured value 390.1023.

Synthesis of key **intermediate 13.**

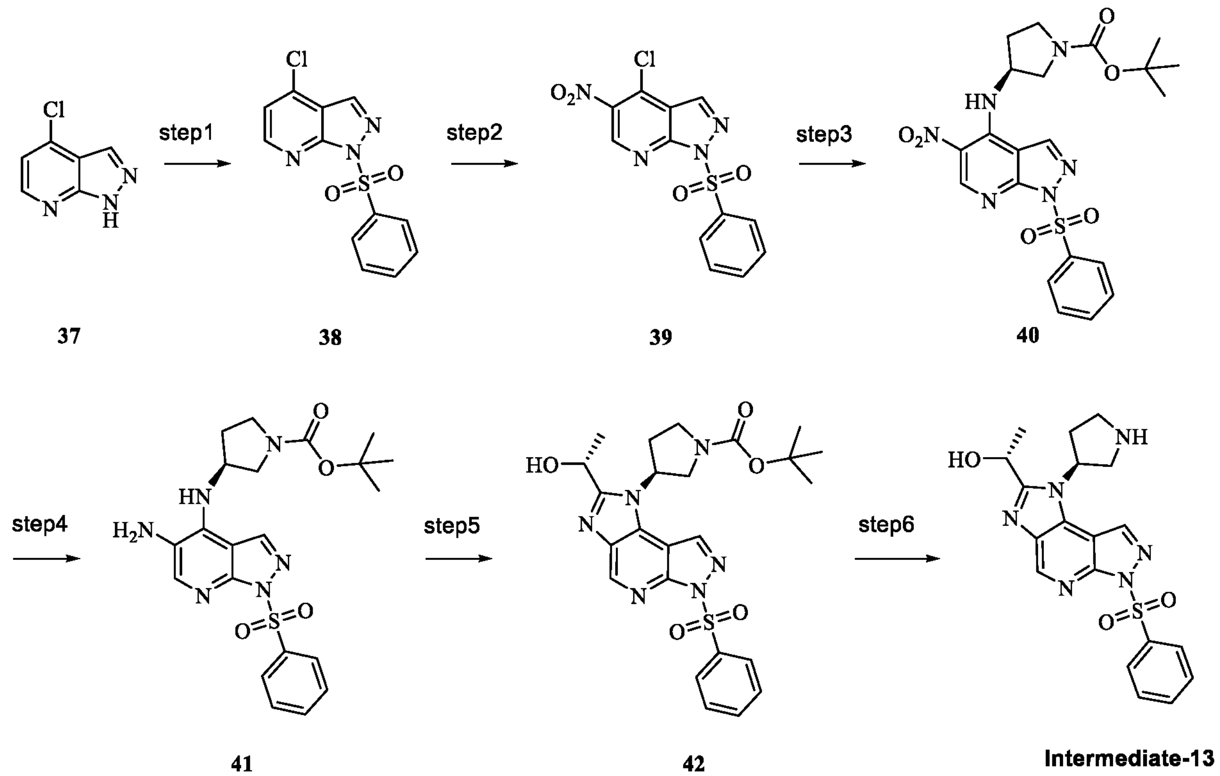


37 38 39 40

41 42 **Intermediate-13**

Step 1: Dissolve 4-chloro-1H-pyrazolo[3,4-b]pyridine (10.0 g, 65.4 mmol) in 100 mL of dichloromethane. Add DMAP (0.8 g, 6.5 mmol), triethylamine (18.0 mL, 130.7 mmol) and stir at room temperature for 30 min. Dissolve benzenesulfonyl chloride (10.1 mL, 78.5 mmol) in 50 mL of dichloromethane and slowly add the solution dropwise to the above reaction solution, stir for about 4 h at room temperature and then filter, collect the filtrate and concentrate under vacuum to obtain a brown solid. 4-chloro-1-(phenylsulfonyl)-1H-pyrazolo[3,4-b]pyridine is then obtained as an off-white solid (17.0 g, 89%) by triturating and curing with an appropriate amount of methanol. The product can be used directly in the next step without further purification. HRMS (ESI): m/z $[M+H]^+$ .$C_{12}H_9ClN_3O_2S$ calculated value 294.0099, measured value 293.0089.

Step 2: Dissolve 4-chloro-1-(phenylsulfonyl)-1H-pyrazolo[3,4-b]pyridine (15.0 g, 51.2 mmol) in 150 mL of dichloromethane, add tetramethylammonium nitrate (13.9 g, 102.3 mmol) to the above solution at 25°C and stir. Trifluoroacetic anhydride (28.6 mL, 204.8 mmol) is then added slowly dropwise while maintaining the temperature of the reaction solution below 30°C. Stir the mixture at room temperature for 5 h. The completion of the reaction is monitored by TLC. Saturated sodium bicarbonate is then added until the reaction solution is weakly basic, and the organic phase is separated.

Extract the aqueous phase twice using dichloromethane. Combine the organic phases and wash with saturated NaCl solution, dry over anhydrous sodium sulfate and concentrate under vacuum to yield a yellow solid. Triturate and cure with an appropriate amount of methanol to yield 4-chloro-5-nitro-1-(phenylsulfonyl)-1H-pyrazolo[2,3-b]pyridine as a light yellow solid (12.3 g, 71%). The product can be used directly in the next step without further purification. HRMS (ESI): m/z $[M+H]^+$ .$C_{12}H_8ClN_4O_4S$ calculated value 338.9949, measured value 338.9930.

Step 3: Dissolve 4-chloro-5-nitro-1-(phenylsulfonyl)-1H-pyrazolo[2,3-b]pyridine (5.0 g, 14.8 mmol) in 50 mL of tetrahydrofuran, followed by the addition of DIPEA (3.8 g, 29.6 mmol) and (S)-(-)-1-Boc-3-aminopyrrolidine (4.2 g, 22.2 mmol). The mixture is then stirred while maintaining reflux at an elevated temperature for 4 hours and the completion of the reaction is monitored by TLC. Concentrate under vacuum to yield a yellow oily liquid. Triturating and curing with an appropriate amount of methanol yields (S)tert-butyl 3-(((5-nitro-1-(phenylsulfonyl)-1H-pyrazolo[3,4-b]pyridin-4-yl)amino)pyrrolidine-1-carboxylate as a yellow solid (5.1 g, 71%). The product can be used directly in the next step without further purification. HRMS (ESI): m/z $[M+H]^+$ .$C_{21}H_{25}N_6O_6S$ calculated value 489.1551, measured value 488.1559.

Step 4: Dissolve (S)tert-butyl 3-(((5-nitro-1-(phenylsulfonyl)-1H-pyrazolo[3,4-b]pyridin-4-yl)amino)pyrrolidine-1-carboxylate (5.0 g, 10.2 mmol) in 50 mL of methanol, add palladium carbon (0.5 g, 10%) and replace the air in the reaction flask with hydrogen gas more than three times. The reaction is then carried out under hydrogen gas atmosphere at room temperature with stirring for 12 h. The completion of the reaction is monitored by TLC. The filtrate is then collected and concentrated under vacuum to yield (S)tert-butyl 3-(((5-amino-1-(phenylsulfonyl)-1H-pyrazolo[3,4-b]pyridin-4-yl)amino)pyrrolidine-1-carboxylate as a pink foamy solid (4.5 g, 96%). The product can be used directly in the next step without further purification. HRMS (ESI): m/z $[M+H]^+$ .$C_{21}H_{27}N_6O_4S$ calculated value 459.1809, measured value 459.1813.

Step 5: Dissolve triethyloxonium tetrafluoroborate (5.0 g, 26.1 mmol) and (R)-lactamide (2.3 g, 26.1 mmol) in 50 mL of tetrahydrofuran, stir at room temperature for 3 h and then concentrate the mixture under vacuum to obtain an oily mixture, followed by the addition of 50 mL of ethanol to dissolve and the addition of (S)tert-butyl 3-(((5-amino-1-(phenylsulfonyl)-1H-pyrazolo[3,4-b]pyridin-4-yl)amino)pyrrolidine-1-carboxylate (4.0 g, 8.7 mmol). The reaction is then stirred while maintaining reflux for 3 h at an elevated temperature. The completion of the reaction is monitored by TLC. Saturated sodium bicarbonate is then added until the reaction solution is weakly basic, and the organic phase is separated. Extract the aqueous phase twice using dichloromethane. Combine the organic phases and wash with saturated NaCl solution, dry over anhydrous sodium sulfate and concentrate under vacuum. The residue is then purified using a silica gel column (petroleum ether: ethyl acetate = 1:1) to yield (S)-tert-butyl 3-(2-((R)-1-hydroxyethyl)-6-(phenylsulfonyl)imidazo[4,5-d]pyrazolo[3,4-b]pyridin-1(6H)-pyrrolidine-1-carboxylate as a green oil (2.2 g, 49%). HRMS (ESI): m/z $[M+H]^+$ .$C_{24}H_{29}N_6O_5S$ calculated value 513.1915, measured value 513.1923.

Step 6: Dissolve (S)-tert-butyl 3-(2-((R)-1-hydroxyethyl)-6-(phenylsulfonyl)imidazo[4,5-d]pyrazolo[3,4-b]pyridin-1(6H)-pyrrolidine-1-carboxylate (2.0 g, 3.9 mmol) in 20 mL of dichloromethane, slowly add trifluoroacetic acid (4.4 g, 39.0 mmol) and then stir for 12 h at room temperature. Concentrate under vacuum to yield **Intermediate 13:** (R)-1-(6-(phenylsulfonyl)-1-((S)-pyrrolidin-3-yl)-1,6-dihydroimidazo[4,5-d]pyrazolo[3,4-b]pyridin-2-yl) ethanol as a light brown oil (1.5 g, 91%). The product can be used directly in the next step without further purification. HRMS (ESI): m/z $[M+H]^+$ .$C_{19}H_{21}N_6O_3S$ calculated value 413.1390, measured value 413.1399.

Example 1

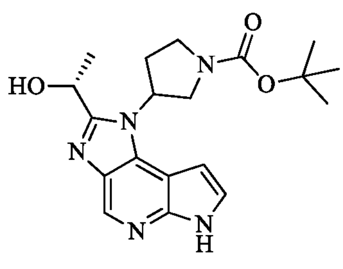

**LXS01**

Tert-butyl 3-(2-((R)-1-hydroxyethyl)imidazo[4,5-d]pyrrolo[2,3-b]pyridin-1(6H)-yl)pyrrolidine-1-carboxylate

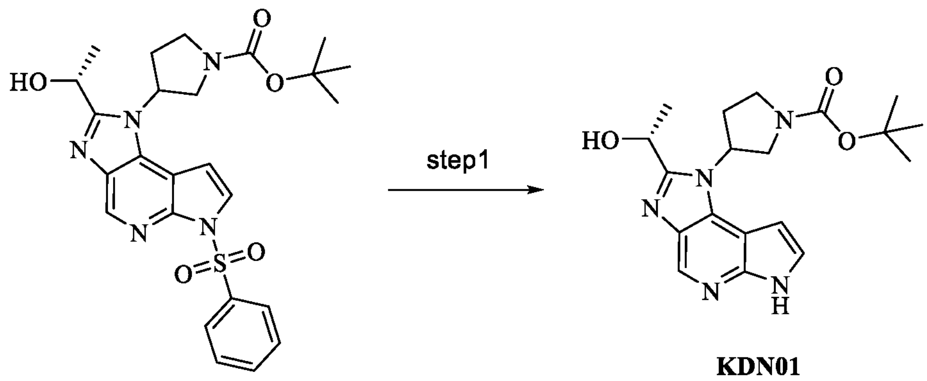


6

KDN01

Step 1: Tert-butyl 3-(2-((R)-1-hydroxyethyl)-6-(phenylsulfonyl)imidazo[4,5-d]pyrrolo[2,3-b]pyridin-1(6H)-yl)pyrrolidine-1-carboxylate (0.4 g, 0.8 mmol) was dissolved in a solvent mixture of 5 mL tetrahydrofuran and 5 mL methanol, and stirred at room temperature for 5 h after adding 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS01:** tert-butyl 3-(2-((R)-1-hydroxyethyl)imidazo[4,5-d]pyrrolo[2,3-b]pyridin-1(6H)-yl)pyrrolidine-1-carboxylate (0.2 g, 69%). 69%).$^1$ HNMR (300 MHz, *DMSO-$d_6$* ): $\delta$ = 11.95 (s, 1H), 8.59 (s, 1H), 7.51 (s, 1H), 6.35 (s, 1H), 5.75 (t, *J* = 3.0 Hz, 2H), 5.17-5.21 (m, 1H), 3.82-3.93 (m, 2H), 3.76 (t, *J = 9.0 Hz, 1H*), 3.40-3.47 (m, 1H), 2.58-2.74 (m, 1H), 2.28-2.41 (m, 1H), 1.65 (d, *J* = 9.0 Hz, 3H), 1.45 (d, *J*= 24.0 Hz, 9H) ppm;$^{13}$ C NMR (75 MHz, *DMSO-$d_6$ )* $\delta$ 153.23, 148.61, 148.53, 142.1, 129.04, 127.15, 120.71, 115.63, 99.37, 79.85, 63.60, 58.31, 53.58, 48.13, 28.47, 27.05, 22.81 ppm; HRMS (ESI): m/z $[M+H]^+$ .$C_{19}H_{26}N_5O_3$ calculated value 372.2030, measured value 372.2018.

Example 2

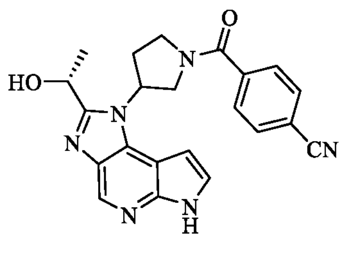

**LXS02**

4-(3-(2-((R)-1-Hydroxyethyl)imidazo[4,5-d]pyrrolo[2,3-b]pyridin-1(6H)-yl)pyrrolidine-1-carbonyl)benzonitrile

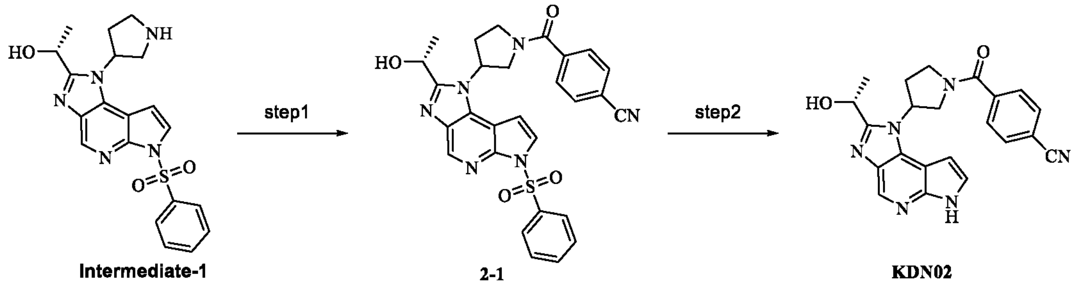


Intermediate-1 2-1 KDN02

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) and then slowly add p-cyanobenzoyl chloride (0.2 g, 1.4 mmol) dropwise. Raise the temperature and stir under reflux for 3 h. Monitor the reaction for completion by TLC. Saturated sodium bicarbonate was added until the reaction solution was weakly basic, and the organic phase was separated. The aqueous phase was then extracted twice using dichloromethane. The organic phase was then combined and washed with saturated saline, dried over anhydrous sodium sulfate and concentrated under vacuum to yield 4-(3-(2-((R)-1-hydroxyethyl)-6-(phenylsulfonyl)imidazo[4, 5-d]pyrrolo[2,3-b]pyridin-1 (6H)-yl)pyrrolidin-1-carbonyl)benzonitrile as a brown oil (0.3 g, 76%). The product can be used directly in the next step without further purification. HRMS (ESI): m/z $[M+H]^+$ .$C_{28}H_{2S}N_6O_4S$ calculated value 541.1653, measured value 541.1643.

Step 2: 4-(3-(2-((R)-1-hydroxyethyl)-6-(phenylsulfonyl)imidazo[4,5-d]pyrrolo[2,3-b]pyrridin-1(6H)-yl)pyrrolidin-1-carbonyl)benzonitrile (0.3 g, 0.6 mmol) was dissolved in a solvent mixture of 5 mL of tetrahydrofuran and 5 mL of methanol and stirred at room temperature for 5 h after the addition of 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS02:** 4-(3-(2-((R)-1-hydroxyethyl)imidazo[4,5-d]pyrrolo[2,3-b]pyridin-1(6H)-yl)pyrrolidin-1-carbonyl)benzonitrile (0.1 g, 45%).$^1$ HNMR (300 MHz, *DMSO-$d_6$*): $\delta$ = 11.96 (s, 1H), 8.87 (s, 1H), 8.21 (d, *J* = 9.0 Hz, 2H), 8.10 (d, *J* = 9.0 Hz, 2H), 7.66 (s, 1H), 6.88 (s, 1H), 4.28-4.69 (m, 1H), 3.81-3.92 (m, 2H), 3.74-3.79 (m, 1H), 3.30-3.45 (m, 2H), 1.93-2.15 (m, 2H), 1.49 (d, *J* = 9.0 Hz, 3H) ppm;$^{13}$ C NMR (75 MHz, *DMSO-$d_6$*) $\delta$ 172.53, 148.69 , 148.51, 142.16, 139.53, 132.09, 129.04, 127.91, 127.16, 120.75, 118.63, 115.61, 113.69, 99.39, 63.60, 58.45, 52.57, 47.13, 27.11, 22.82 ppm; HRMS ( ESI): m/z $[M+H]^+$ .$C_{22}H_{21}N_6O_2$ calculated value 401.1721, measured value 401.1723.

Example 3

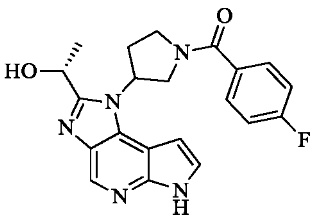

**LXS03**

(4-Fluorophenyl)(3-(2-((R)-1-Hydroxyethyl)imidazo[4,5-d]pyrrolo[2,3-b]pyridin-1(6H)-yl)pyrrolidin-1-yl)methanone

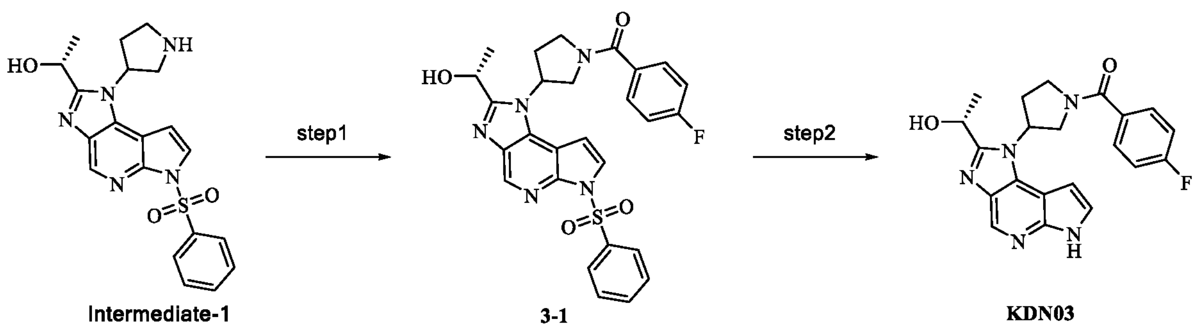


Intermediate-1 3-1 KDN03

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by slow dropwise addition of p-fluorobenzoyl chloride (0.2 g, 1.1 mmol). The reaction was monitored for completion by TLC. Saturated sodium bicarbonate was added until the reaction solution was weakly basic, and the organic phase was separated. The aqueous phase was then extracted twice using dichloromethane. The organic phase was then combined and washed with saturated

saline, dried over anhydrous sodium sulfate and concentrated under vacuum to yield (4-fluorophenyl)(3-(2-((R)-1-hydroxyethyl)-6-(phenylsulfonyl)imidazo[4,5-d]pyrrolo[2,3-b]pyridin-1(6H)-yl)pyrrolidin-1-yl)methanone as a brown oil (0.3 g, 77%). The product can be used directly in the next step without further purification. HRMS (ESI): m/z $[M+H]^+$ .$C_{27}H_{25}FN_5O_4S$ calculated value 534.1606, measured value 534.1619.

Step 2: (4-fluorophenyl)(3-(2-((R)-1-hydroxyethyl)-6-(phenylsulfonyl)imidazo[4,5-d]pyrrolo[2,3-b]pyridin-1(6H)-yl)pyrrolidin-1-yl)methanone (0.3 g, 0.6 mmol) was dissolved in a solvent mixture of 5 mL of tetrahydrofuran and 5 mL of methanol. After the addition of 5 mL of 1 M sodium hydroxide, the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS03:** (4-Fluorophenyl)(3-(2-((R)-1-Hydroxyethyl)imidazo[4,5-d]pyrrolo[2,3-b]pyridin-1(6H)-yl)pyrrolidin-1-yl)methanone (0.1 g, 45%).$^1$ HNMR (300 MHz, *DMSO-$d_6$* ): $\delta$= 11.97 (s, 1H), 8.60 (d, *J* = 6.0 Hz, 1H), 7.74-7-76 (m, 1H), 7.55 (d, *J* = 15.0 Hz, 2H), 7.36 (t, J = 9.0 Hz, 1H), 7.22 (t, *J* = 9.0 Hz, 1H) Hz, 1H), 6.45 (d, J = 48.0 Hz, 1H), 5.77 (s, 2H), 5.15-5.23 (m, 1H), 3.97-4.20 (m, 2H), 3.71-3.93 (m, 2H), 2.66-2.73 (m, 1H), 1.67 (d, *J* = 6.0 Hz, 3H), 1.38 (t, *J* = 37.5 Hz, 2H) ppm;$^{13}$ C NMR (75 MHz, *DMSO-$d_6$ )* $\delta$172.58, 163.91, 148.83, 148.29, 142.54, 130.84, 129.03, 128.83, 127.29, 120.85, 115.61, 115.33, 99.57, 63.67, 58.49, 52.59, 47.83, 27.29, 22.89 ppm; HRMS (ESI): m/z $[M+H]^+$ .$C_{21}H_{21}FN_5O_2$ calculated value 394.1674, measured value 394.1682.,

Example 4

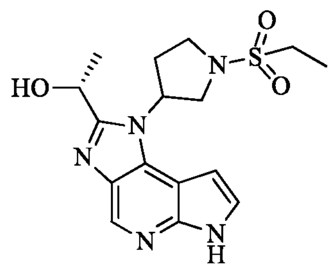

**LXS04**

(1R)-1-(1-(1-(ethylsulfonyl)pyrrolidin-3-yl)-1,6-dihydroimidazo[4,5-d]pyrrolo[2,3-b]pyridin-2-yl)ethan-1-ol

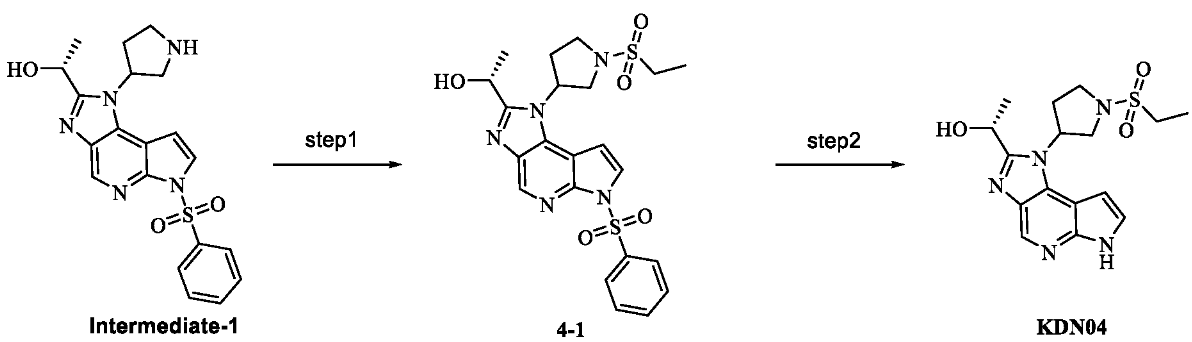


Intermediate-1 4-1 KDN04

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by the slow dropwise addition of ethyl sulfonyl chloride (0.2 g, 1.1 mmol). Then stir under reflux by heating for 3h. The reaction was monitored for completion by TLC. Saturated sodium bicarbonate was then added until the reaction solution was weakly basic, and the organic phase was separated. The aqueous phase was then extracted twice using dichloromethane. The organic phase was then combined and washed with saturated saline, dried over anhydrous sodium sulfate and concentrated under vacuum to yield (1R)-1-(1-(1-(1-(ethylsulfonyl)pyrrolidin-3-yl)-6-(phenylsulfonyl)-1,6-dihydroimidazo[4,5-d]pyrrolo[2,3-b]pyridin-2-yl)ethanol as a yellow oil (0.3 g, 82%). The product can be used directly in the next step without further purification. HRMS (ESI): m/z [M+H]$^+$ .$C_{22}H_{26}N_5O_5S_2$ calculated value 504.1370, measured value 504.1389.

Step 2: (1R)-1-(1-(1-(1-(ethylsulfonyl)pyrrolidin-3-yl)-6-(phenylsulfonyl)-1,6-dihydroimidazo[4,5-d]pyrrolo[2,3-b]pyridin-2-yl) ethanol (0.3 g, 0.6 mmol) was dissolved in a mixture of 5 mL of tetrahydrofuran and 5 mL of methanol, and 5 mL of 1M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate= 1:1) to yield **LXS04:** (1R)-1-(1-(1-(ethylsulfonyl)pyrrolidin-3-yl)-1, 6-dihydroimidazo[4, 5-d]pyrrolo[2,3-b]pyridin-2-yl)ethan-1-ol (0.2 g, 93%).$^1$ HNMR (300 MHz, *DMSO-$d_6$* ): $\delta$ = 12.03 (s, 1H), 8.97 (s, 1H), 7.69 (s, 1H), 6.78 (s, 1H), 4.18-4.77 (m, 1H), 3.60-3.79 (m, 1H), 3.40-3.51 (m, 2H), 3.11-3.32 (m, 2H), 2.74-2.89 (m, 2H), 1.90-2.15 (m, 2H), 1.49 (d, *J* = 6.0 Hz, 3H), 1.22 (t, *J* = 9.0 Hz, 3H) ppm;$^{13}$ C NMR (75 MHz, *DMSO-$d_6$)* $\delta$ 148.77, 148.56, 142.77, 129.19, 127.31, 120.77, 115.68, 113.69, 99.48, 63.29, 57.53, 56.10, 51.11, 50.09, 26.20, 22.89, 2.69 ppm; HRMS (ESI): m/z [M+H]$^+$ .$C_{16}H_{22}N_5O_3S$ calculated value 364.1438, measured value 364.1472.

Example 5

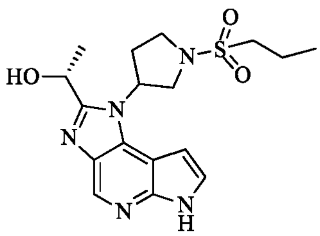

**LXS05**

(1R)-1-(1-(1-(propylsulfonyl)pyrrolidin-3-yl)-1,6-dihydroimidazo[4,5-d]pyrrolo[2,3-b]pyridin-2-yl)ethan-1-ol

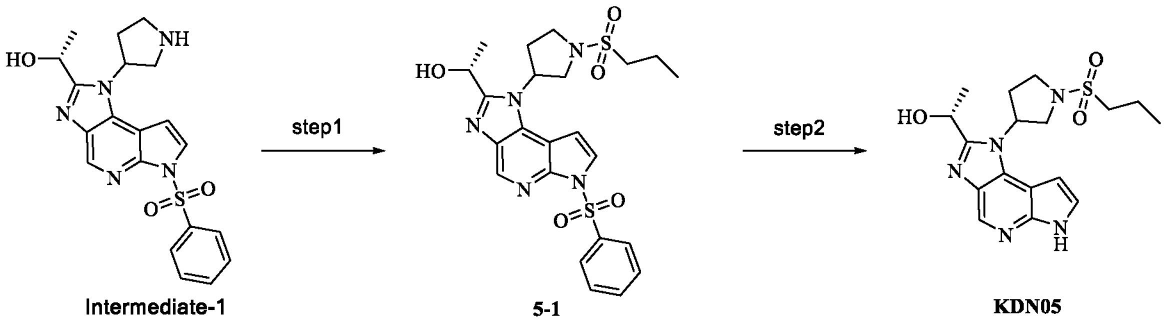


Intermediate-1 5-1 KDN05

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by slow dropwise addition of propylsulfonyl chloride (0.2 g, 1.1 mmol). Then stir under reflux by heating for 3h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (1R)-1-(1-(1-(1-(propylsulfonyl)pyrrolidin-3-yl)-6-(phenylsulfonyl)-1,6-dihydroimidazo[4,5-d]pyrrolo[2,3-b]pyridin-2-yl)ethanol as a yellow oil (0.3 g, 80%). The product can be used directly in the next step without further purification. HRMS (ESI): m/z [M+H]$^+$ .$C_{23}H_{28}N_5O_5S_2$ calculated value 518.1526, measured value 518.1536.

Step 2: (1R)-1-(1-(1-(1-(propylsulfonyl)pyrrolidin-3-yl)-6-(phenylsulfonyl)-1,6-dihydroimidazo[4,5-d]pyrrolo[2,3-b]pyridin-2-yl) ethanol (0.3 g, 0.6 mmol) was dissolved in a mixture of 5 mL of tetrahydrofuran and 5 mL of methanol,

and 5 mL of 1M sodium hydroxide was added, and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate= 1:1) to yield **LXS05:** (1R)-1-(1-(1-(propylsulfonyl)pyrrolidin-3-yl)-1,6-dihydroimidazo[4,5-d]pyrrolo[2,3-b]pyridin-2-yl)ethan-1-ol (0.2 g, 91%).$^{1}$ HNMR (300 MHz, *DMSO-$d_6$* ): $\delta$ = 11.93 (s, 1H), 8.77 (s, 1H), 7.58 (s, 1H), 6.67 (s, 1H), 4.21-4.68 (m, 1H), 3.51-3.73 (m, 1H), 3.21-3.28 (m, 2H), 3.18 (t, *J = 9.0 Hz,* 2H), 2.79-2.99 (m, 2H), 1.83-2.25 (m, 2H), 1.52-1.68 (m, 2H), 1.49 (d, *J* = 9.0 Hz, 3H), 0.90 (t, *J* = 6.0 Hz, 3H) ppm;$^{13}$ C NMR (75 MHz, *DMSO-$d_6$ )* $\delta$ 148.66, 148.59, 142.19, 129.02, 127.15, 120.73, 115.61, 113.69, 99.31, 63.66, 60.40, 57.59, 56.19, 50.02, 26.28, 22.84, 13.33, 12.44 ppm; HRMS (ESI): m/z $[M+H]^+$ .$C_{17}H_{24}N_5O_3S$ calculated value 378.1594, measured value 378.1599.

Example 6

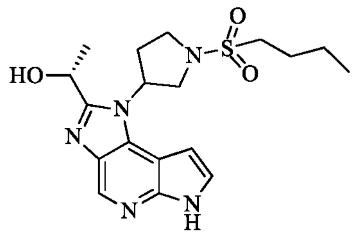

**LXS06**

(1R)-1-(1-(1-(butylsulfonyl)pyrrolidin-3-yl)-1,6-dihydroimidazo[4,5-d]pyrrolo[2,3-b]pyridin-2-yl)ethan-1-ol

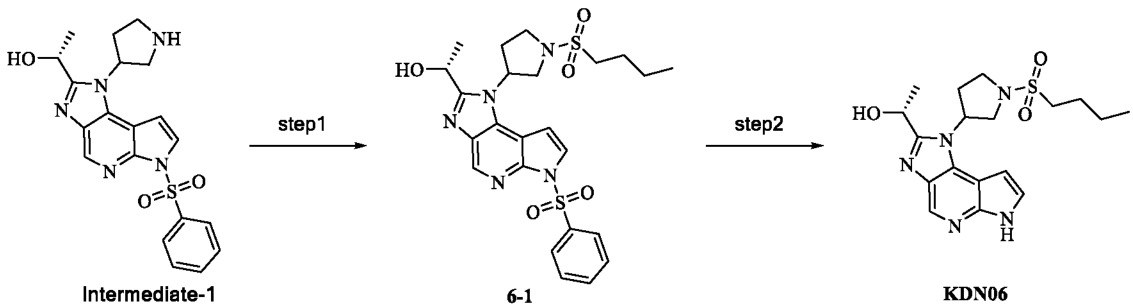


Intermediate-1 6-1 KDN06

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by the slow dropwise addition of butylsulfonyl chloride (0.2 g, 1.1 mmol). Then stir under reflux by heating for 3h. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (1R)-1-(1-(1-(1-(butylsulfonyl)pyrrolidin-3-yl)-6-(phenylsulfonyl)-1,6-dihydroimidazo[4,5-d]pyrrolo[2,3-b]pyridin-2-yl)ethanol as a pale white oil (0.3 g, 77%). The product can be used directly in the next step without further purification. HRMS (ESI): m/z $[M+H]^+$ .$C_{24}H_{30}N_5O_5S_2$ calculated value 532.1683, measured value 532.1703.

Step 2: (1R)-1-(1-(1-(1-(butylsulfonyl)pyrrolidin-3-yl)-6-(phenylsulfonyl)-1,6-dihydroimidazo[4,5-d]pyrrolo[2,3-b]pyridin-2-yl) ethanol (0.3 g, 0.6 mmol) was dissolved in a mixture of 5 mL of tetrahydrofuran and 5 mL of methanol, and 5 mL of 1 M sodium hydroxide was added, and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate= 1:1) to yield **LXS06:** (1R)-1-(1-(1-(butylsulfonyl)pyrrolidin-3-yl)-1,6-dihydroimidazo[4,5-d]pyrrolo[2,3-b]pyridin-2-yl)ethan-1-ol (0.2 g, 91%).$^1$ HNMR (300 MHz, *DMSO-$d_6$* ): $\delta$ = 12.03 (s, 1H), 8.47 (s, 1H), 7.68 (s, 1H), 6.87 (s, 1H), 4.41-4.62 (m, 1H), 3.50-3.79 (m, 1H), 3.11 -3.22 (m, 2H), 3.10 (t, *J = 6.0 Hz,* 2H), 2.77-2.80 (m, 2H), 1.93-2.15 (m, 2H), 1.51-1.61 (m, 2H), 1.48 (d, *J* = 6.0 Hz, 3H), 1.29-1.31 (m, 2H), 0.93 (t, *J* = 9.0 Hz, 3H) ppm;$^{13}$ C NMR (75 MHz, *DMSO-$d_6$ )* $\delta$ 148.65, 148.51, 142.18, 129.04, 127.11, 120.79, 115.61, 99.39, 63.68, 57.93, 57.58, 56.17, 50.01, 26.28, 22.82, 21.93, 21.05, 13.88 ppm; HRMS (ESI): m/z $[M+H]^+$ $C_{18}H_{26}N_5O_3S$ calculated value 392.1751, measured value 392.1755.

Example 7

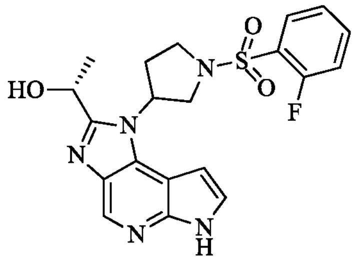

**LXS07**

(1R)-1-(1-(1-((2-fluorophenyl)sulfonyl)pyrrolidin-3-yl)-1,6-dihydroimidazo[4,5-d]pyrrolo[2,3-b]pyridin-2-yl)ethan-1-ol

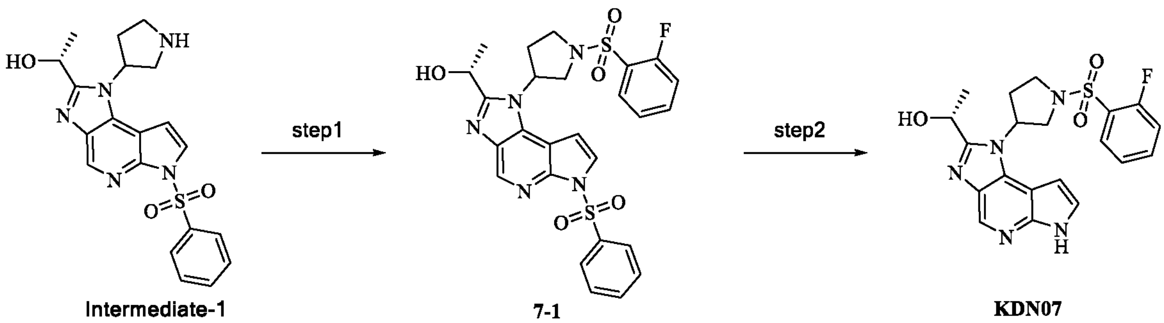


Intermediate-1 7-1 KDN07

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by the slow dropwise addition of o-fluorobenzenesulfonyl chloride (0.2 g, 1.1 mmol). Then stir under reflux by heating for 3h. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (1R)-1-(1-(1-(1-(((2-fluorophenyl)sulfonyl)pyrrolidin-3-yl)-6-(phenylsulfonyl)-1,6-dihydroimidazo[4,5-d]pyrrolo[2,3-b]pyridin-2-yl)ethanol as a light yellow oil (0.3 g, 72%). The product can be used directly in the next step without further purification. HRMS (ESI): m/z $[M+H]^+$ .$C_{26}H_{25}FN_5O_5S_2$ calculated value 570.1276, measured value 570.1257.

Step 2: (1R)-1-(1-(1-(1-(((2-fluorophenyl)sulfonyl)pyrrolidin-3-yl)-6-(phenylsulfonyl)-1,6-dihydroimidazo[4,5-d]pyrrolo[2,3 -b]pyridin-2-yl)ethanol (0.3 g, 0.5 mmol) was dissolved in a mixture of 5 mL of tetrahydrofuran and 5 mL of methanol, and 5 mL of 1 M sodium hydroxide was added, and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS07:** (1R)-1-(1-(1-((2-fluorophenyl)sulfonyl)pyrrolidin-3-yl)-1,6-dihydroimidazo[4,5-d]pyrrolo[2,3-b]pyridin-2-yl)ethan-1-ol (0.1 g, 44%).$^1$ HNMR (300 MHz, *DMSO-$d_6$* ): $\delta$ = 12.16 (s, 1H), 8.87 (s, 1H), 7.80-7.84 (m, 1H), 7.63-7.75

(m, 1H), 7.60 (s, 1H), 7.48-7.52 (m, 1H), 7.21- 7.43 (m, 1H), 6.77 (s, 1H), 4.51-4.70 (m, 1H), 3.63-3.81 (m, 1H), 3.09-3.33 (m, 2H), 2.63-2.83 (m, 2H), 1.89-2.19 (m, 2H), 1.93 (d, *J* = 9.0 Hz, 3H) ppm;$^{13}$ C NMR (75 MHz, *DMSO-$d_6$*) $\delta$ 158.22, 148.66, 148.57, 142.18, 133.53, 129.03, 128.91, 127.11, 126.33, 124.69, 120.74, 115.84, 115.64, 99.38, 63.69, 57.58, 55.70, 49.63, 26.24, 22.89 ppm; HRMS (ESI): m/z $[M+H]^+$ .$C_{20}H_{21}FN_5O_3S$ calculated value 430.1344, measured value 430.1350.

Example 8

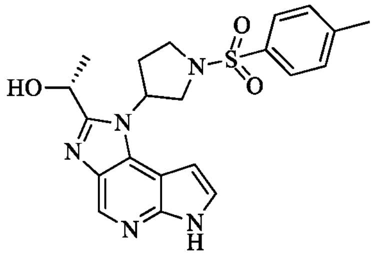

**LXS08**

(1R)-1-(1-(1-tosylpyrrolidin-3-yl)-1,6-dihydroimidazo[4,5-d]pyrrolo[2,3-b]pyridin-2-yl)ethan-1-ol

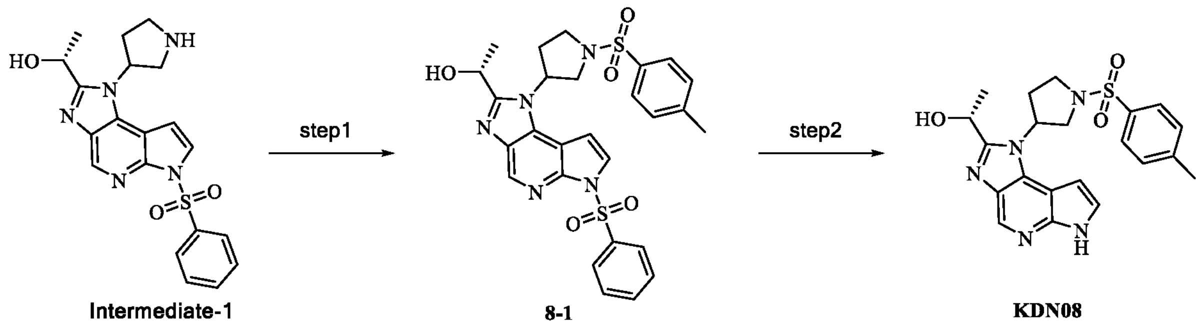


**Intermediate-1** **8-1** **KDN08**

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) and slowly add p-toluenesulfonyl chloride (0.2 g, 1.4 mmol) dropwise, then raise the temperature while maintaining reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (1R)-1-(6-(phenylsulfonyl)-1-(1-toluenesulfonylpyrrolidin-3-yl)-1,6-dihydroimidazo[4,5-d]pyrrolo[2,3-b]pyridin-2-yl)ethanol as a yellow oil (0.3 g, 73%). The product can be used directly in the next step without further purification. HRMS (ESI): m/z $[M+H]^+$ .$C_{27}H_{28}N_5O_5S_2$ calculated value 566.1526, measured value 566.1523.

Step 2: (1R)-1-(6-(phenylsulfonyl)-1-(1-toluenesulfonylpyrrolidin-3-yl)-1,6-dihydroimidazo[4,5-d]pyrrolo[2,3-b]pyridin-2-yl) ethanol (0.3 g, 0.5 mmol) was dissolved in a solvent mixture of 5 mL tetrahydrofuran and 5 mL methanol. Then, 5 mL of 1 M sodium hydroxide was added and the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS08:** (1R)-1-(1-(1-tosylpyrrolidin-3-yl)-1,6-dihydroimidazo[4,5-d]pyrrolo[2,3-b]pyridin-2-yl)ethan-1-ol( 0.2 g, 89%).$^1$ HNMR (300 MHz, *DMSO-$d_6$*): $\delta$ = 11.95 (s, 1H), 8.84 (s, 1H), 7.74 (d, *J* = 7.5 Hz, 2H), 7.68 (s, 1H), 7.40 (d, *J* = 7.5 Hz, 2H), 6.87 (s, 1H), 4.53-4.68 (m, 1H) , 3.55-3.81 (m, 1H), 3.10-3.25 (m, 2H), 2.79-2.89 (m, 2H), 2.34 (s, 3H), 1.91-2.25 (m, 2H), 1.43 (d, *J* = 4.5 Hz, 3H) ppm;$^{13}$ C NMR (75 MHz, *DMSO-$d_6$*) $\delta$ 148.63, 148.59, 143.33, 142.17, 137.66, 129.39, 129.00, 128.33, 127.16, 120.71, 115.65, 99.39, 63.64, 57.58, 55.74, 49.63, 26.23, 22.81, 21.39 ppm. HRMS (ESI): m/z

$[M+H]^+$ .$C_{21}H_{24}N_5O_3S$ calculated value 426.1594, measured value 426.1596.

Example 9

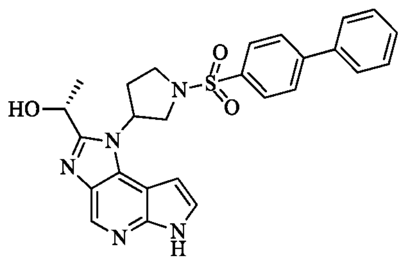

LXS09

(1R)-1-(1-(1-([1,1'-biphenyl]-4-ylsulfonyl)pyrrolidin-3-yl)-1,6-dihydroimidazo[4,5-d]pyrrolo[2,3-b]pyridin-2-yl)ethan-1-ol

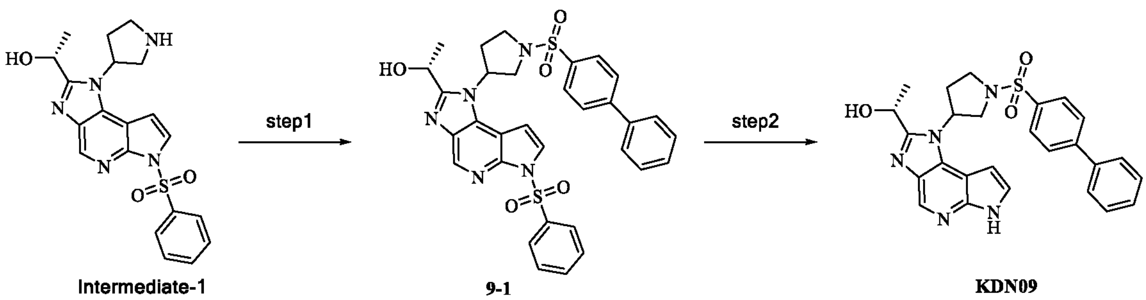


Intermediate-1 9-1 KDN09

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) and slowly add p-phenylbenzenesulfonyl chloride (0.3 g, 1.1 mmol) dropwise. Raise the temperature while maintaining reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (1R)-1-(1-(1-(1-([[1,1'-biphenyl]-4-yl sulfonyl)pyrrolidin-3-yl)-6-(phenylsulfonyl)-1,6-dihydroimidazo[4,5-d]pyrrolo[2,3-b]pyridin-2-yl)ethanol as a yellow oil (0.3 g, 66%). 66%). The product can be used directly in the next step without further purification. HRMS (ESI): m/z $[M+H]^+$ .$C_{32}H_{30}N_5O_5S_2$ calculated value 628.1683, measured value 628.1686.

Step 2: (1R)-1-(1-(1-(1-([[1,1'-Biphenyl] -4-yl sulfonyl)pyrrolidin-3-yl)-6-(phenylsulfonyl)-1,6-dihydroimidazo[4,5-d ]pyrrolo[2,3-b]pyridin-2-yl)ethanol (0.3 g, 0.5 mmol) was dissolved in a solvent mixture of 5 mL tetrahydrofuran and 5 mL methanol. After adding 5 mL of 1 M sodium hydroxide, the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS09:** (1R)-1-(1-(1-([1,1'-biphenyl]-4-ylsulfonyl)pyrrolidin-3-yl)-1,6-dihydroimidazo[4,5-d]pyrrolo[2,3-b]pyridin-2-yl)ethan-1-ol (0.2 g, 86%).$^1$ HNMR (300 MHz, *DMSO-$d_6$* ): $\delta$ = 12.35 (s, 1H), 8.74 (s, 1H), 7.88-7.92 (m, 4H), 7.67 (s, 1H), 7.45-7.52 (m, 4H), 7.28-7.41 (m, 1H), 6.87 (s, 1H), 4.43-4.71 (m, 1H), 3.52-3.79 (m, 1H), 3.11-3.35 (m, 2H), 2.77-2.88 (m, 2H), 1.90-2.15 (m, 2H), 1.49 (d, $J$ = 6.0 Hz, 3H) ppm;$^{13}$ C NMR ( 75 MHz, *DMSO-$d_6$ )* $\delta$ 148.69, 148.53, 142.19, 140.88, 138.62, 133.06, 129.21, 129.04, 127.93, 127.88, 127.11, 127.10, 120.73, 115.63, 99.38, 63.63, 57.58, 55.70, 49.62, 26.25, 22.81 ppm; HRMS (ESI): m/z $[M+H]^+$ .$C_{26}H_{26}N_5O_3S$ calculated value 488.1751, measured value 488.1757.

Example 10

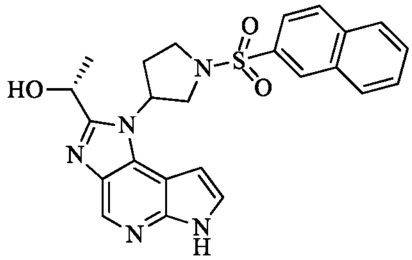

**LXS10**

(1R)-1-(1-(1-(naphthalen-2-ylsulfonyl)pyrrolidin-3-yl)-1,6-dihydroimidazo[4,5-d]pyrrolo[2,3-b]pyridin-2-yl)ethan-1-ol

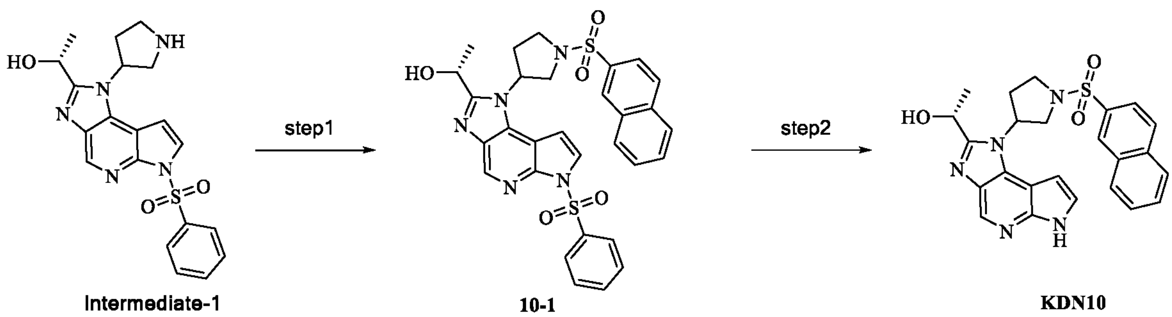


Intermediate-1 10-1 KDN10

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by slow dropwise addition of naphthalenesulfonyl chloride (0.3 g, 1.1 mmol). Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (1R)-1-(1-(1-(1-naphthalen-2-yl sulfonyl)pyrrolidin-3-yl)-6-(phenylsulfonyl)-1,6-dihydroimidazo[4,5-d]pyrrolo[2,3- b]pyridin-2-yl)ethanol as a pale yellow oil (0.3 g, 68%). The product can be used directly in the next step without further purification. HRMS (ESI): m/z $[M+H]^+$ .$C_{30}H_{28}N_5O_5S_2$ calculated value 602.1526, measured value 602.1540.

Step 2: (1R)-1-(1-(1-(1-naphthalen-2-yl sulfonyl)pyrrolidin-3-yl)-6-(benzenesulfonyl)-1,6-dihydroimidazo[4,5-d]pyrrolo[2,3- b]pyridin-2-yl) ethanol (0.3 g, 0.5 mmol) was dissolved in a mixture of 5 mL tetrahydrofuran and 5 mL methanol, and 5 mL of 1 M sodium hydroxide was added, and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate= 1:1) to yield **LXS10:** (1R)-1-(1-(1-(naphthalen-2-ylsulfonyl)pyrrolidin-3-yl)-1,6-dihydroimidazo[4,5-d]pyrrolo[2,3-b]pyridin-2-yl)ethan-1-ol (0.2 g, 87%).$^1$ HNMR (300 MHz, *DMSO-$d_6$* ): $\delta$= 12.04 (s, 1H), 8.87 (s, 1H), 8.80 (s, 1H), 8.30-8.41 (m, 1H), 8.03-8.15 (m, 1H), 8.00 (d, *J* = 7.5 Hz, 2H), 7.59 (d, *J = 7.5 Hz, 2H* = 7.5 Hz, 2H), 7.45 (s, 1H), 6.87 (s, 1H), 4.55-4.69 (m, 1H), 3.45-3.91 (m, 1H), 3.15-3.35 (m, 2H), 2.69-2.74 (m, 2H), 1.96-2.28 (m, 2H), 1.49 (d, *J*= 6.0 Hz, 3H ) ppm;$^{13}$ C NMR (75 MHz, *DMSO-$d_6$ )* $\delta$ 148.62, 148.51, 142.17, 137.03, 136.71, 134.11, 129.44, 129.09, 128.11, 127.11, 126.25, 126.04, 123.41, 120.74, 115.60, 99.38, 63.69, 57.57, 55.76, 49.60, 26.22, 22.81 ppm; HRMS (ESI): m/z $[M+H]^+$ .$C_{24}H_{24}N_5O_3S$ calculated value 462.1594, measured value 462.1607.

Example 11

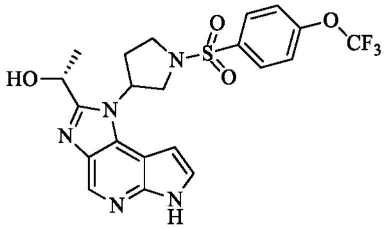

**LXS11**

(1R)-1-(1 -(1 -((4-(trifluoromethoxy)phenyl)sulfonyl)pyrrolidin-3-yl)-1,6-dihydroimidazo[4,5-d]pyrrolo[2,3-b]pyridin-2-yl)ethan-1-ol

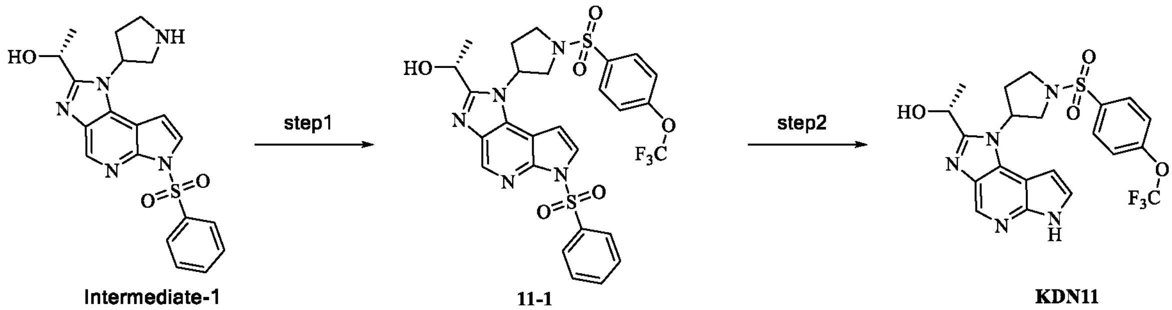


Intermediate-1 11-1 KDN11

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) and then slowly add p-trifluoromethoxy-benzenesulfonyl chloride (0.3 g, 1.1 mmol) dropwise, Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (1R)-1-(6-(phenylsulfonyl)-1-(1-((4-(trifluoromethoxy)phenyl)sulfonyl)pyrrolidin-3-yl)-1,6-dihydroimidazo[4,5-d]pyrrolo[2,3-b]pyridin-2-yl)ethanol as a yellow oil (0.3 g, 65%) . The product can be used directly in the next step without further purification. HRMS (ESI): m/z $[M+H]^+$ .$C_{27}H_{25}F_3N_5O_6S_2$ calculated value 636.1193, measured value 636.1195.

Step 2: (1R)-1-(6-(phenylsulfonyl)-1-(1-((4-(trifluoromethoxy)phenyl)sulfonyl)pyrrolidin-3-yl)-1,6-dihydroimidazo[4,5-d]pyrrolo[2,3-b]pyridin-2-yl)ethanol (0.3 g, 0.5 mmol) was dissolved in a solvent mixture of 5 mL tetrahydrofuran and 5 mL methanol, and 5 mL of 1 M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS11:** (1R)-1-(1-(1-((4-(trifluoromethoxy)phenyl)sulfonyl)pyrrolidin-3-yl)-1,6-dihydroimidazo[4,5-d]pyrrolo[2,3-b]pyridin-2-yl)ethan-1-ol (0.2 g, 86%).$^1$ HNMR (300 MHz, *DMSO-$d_6$* ): $\delta$ = 12.15 (s, 1H), 8.79 (s, 1H), 7.64 (d, *J* = 7.5 Hz, 2H), 7.59 (s, 1H), 7.12 (d, *J* = 7.5 Hz, 2H), 6.74 (s, 1H), 4.33-4.58 (m, 1H), 3.65-3.83 (m, 1H), 3.15-3.31 (m, 2H), 2.74-2.86 (m, 2H), 1.95-2.35 (m, 2H), 1.46 (d, *J* = 9.0 Hz, 3H)ppm;$^{13}$ C NMR (75 MHz, *DMSO-$d_6$)* $\delta$ 153.88, 148.61, 148.59, 142.10, 132.00, 129.79, 129.04, 127.17, 126.11, 120.73, 115.61, 114.67, 99.38, 63.69, 57.58, 55.70, 49.66, 26.25, 22.81 ppm; HRMS (ESI ): m/z $[M+H]^+$ .$C_{21}H_{21}F_3N_5O_4S$ calculated value 496.1261, measured value 496.1262.

Example 12

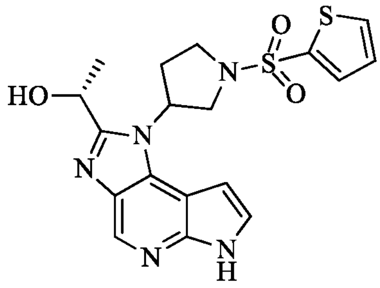

LXS12

(1R)-1-(1-(1-(thiophen-2-ylsulfonyl)pyrrolidin-3-yl)-1,6-dihydroimidazo[4,5- d]pyrrolo[2,3-b]pyridin-2-yl)ethan-1-ol

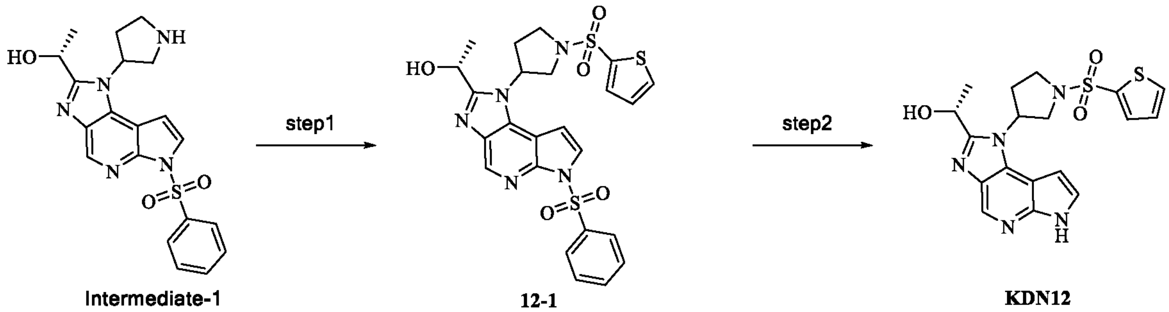


Intermediate-1 12-1 KDN12

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by the slow dropwise addition of thiophene sulfonyl chloride (0.2 g, 1.1 mmol). Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (1R)-1-(6-(phenylsulfonyl)-1-(1-(thiophen-2-yl sulfonyl)pyrrolidin-3-yl)-1,6-dihydroimidazo[4,5-d]pyrrolo[2,3- b]pyridin-2-yl)ethanol as a yellow oil (0.3 g, 74%). The product can be used directly in the next step without further purification. HRMS (ESI): m/z $[M+H]^+$ .$C_{24}H_{24}N_5O_5S_3$ calculated value 558.0934, measured value 558.0949.

Step 2: (1R)-1-(6-(phenylsulfonyl)-1-(1-(thiophen-2-yl sulfonyl)pyrrolidin-3-yl)-1,6-dihydroimidazo[4,5-d]pyrrolo[2,3- b]pyridin-2-yl) ethanol (0.3 g, 0.5 mmol) was dissolved in a mixture of 5 mL of tetrahydrofuran and 5 mL of methanol, and 5 mL of 1 M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS12:** (1R)-1-(1-(1-(thiophen-2-ylsulfonyl)pyrrolidin-3-yl)-1,6-dihydroimidazo[4,5-d]pyrrolo[2,3-b]pyridin-2-yl)ethan-1-ol (0.1 g, 45%).$^1$ HNMR (300 MHz, *DMSO-$d_6$* ): $\delta$ = 12.02 (s, 1H), 8.84 (s, 1H), 7.70 (s, 1H), 7.20 (d, *J* = 7.5 Hz, 1H), 6.96 (d, *J* = 9.0 Hz, 2H), 6.77 (s, 1H), 4.51 -4.69 (m, 1H), 3.57-3.83 (m, 1H), 3.17-3.31 (m, 2H), 2.72-2.85 (m, 2H), 1.93-2.31 (m, 2H), 1.07 (d, *J* = 9.0 Hz, 3H) ppm;$^{13}$ C NMR (75 MHz, *DMSO-$d_6$)* $\delta$ 148.61, 148.57, 142.11, 129.09, 127.21, 127.11, 126.33, 120.73, 115.60, 99.31, 63.61, 57.59, 55.74, 49.62, 26.22, 22.81 ppm; HRMS (ESI): m/z $[M+H]^+$ . $C_{18}H_{20}N_5O_3S_2$ calculated value 418.1002, measured value 418.1012.

Example 13

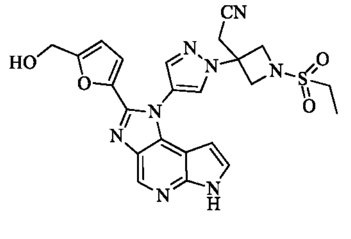

LXS13

2-(1-(Ethylsulfonyl)-3-(4-(2-(5-(hydroxymethyl)furan-2-yl)imidazo[4,5-d]pyrrolo[2,3-b]pyridin-1(6H)-yl)-1H-pyrazol-1-yl)azetidin-3-yl)acetonitrile

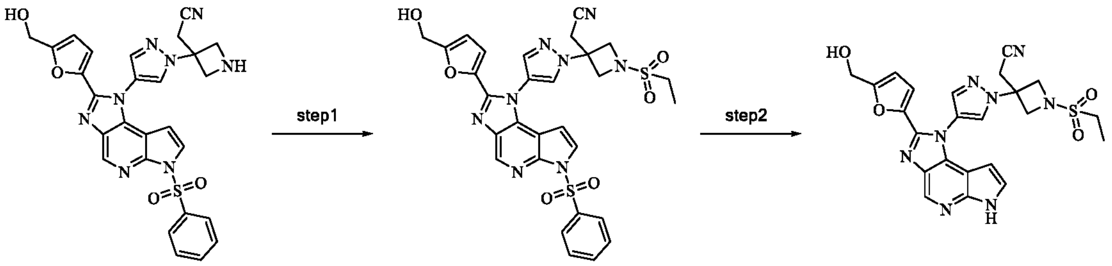


Intermediate-2

13-1

KDN13

Step 1: Dissolve 2-(3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl)acetonitrile (0.3 g, 0.5 mmol) in 10 mL of tetrahydrofuran, add DIPEA (0.2 g, 1.0 mmol) followed by slow addition of ethylsulfonyl chloride (0.1 g, 0.8 mmol) dropwise. Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield 2-(1-(ethylsulfonyl)-3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl)acetonitrile as a yellow oil (0.2 g, 57%). The product can be used directly in the next step without further purification. HRMS (ESI): m/z $[M+H]^+$ . $C_{29}H_{27}N_8O_6S_2$ calculated value 647.1489, measured value 647.1482.

Step 2: 2-(1-(Ethylsulfonyl)-3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl)acetonitrile (0.2 g, 0.3 mmol) was dissolved in a mixture of solvent of 5 mL tetrahydrofuran and 5 mL methanol, 5 mL of 1 M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS13:** 2-(1-(ethylsulfonyl)-3-(4-(2-(5-(hydroxymethyl)furan-2-yl)imidazo[4,5-d]pyrrolo[2,3-b]pyridin-1(6H )-yl)-1H-pyrazol-1-yl)azetidin-3-yl)acetonitrile (0.1 g, 64%).$^1$ HNMR (300 MHz, *DMSO-$d_6$* ): $\delta$= 11.94 (s, 1H), 8.90 (s, 1H), 8.69 (s, 1H), 8.14 (s, 1H), 7.37 (s, 1H), 6.41 (s, 2H), 5.95 ( s, 1H), 5.43 (t, *J* = 6.0 Hz, 1H), 4.62 (d, *J* = 9.0 Hz, 2H), 4.47 (d, *J* = 6.0 Hz, 2H), 4.34 (d, *J* = 9.0 Hz, 2H), 3.78 (s, 2H), 3.25-3.32 (m, 2H), 1.28 (t, *J* = 7.5 Hz, 3H) ppm $^{13}$ C NMR (75 MHz, *DMSO-$d_6$ )* $\delta$153.88, 151.11, 148.67, 142.16, 141.57, 130.77, 129.79, 129.04, 127.12, 120.74, 117.77, 115.65, 107.93, 104.05, 100.53, 99.38 , 59.23, 57.39, 51.60, 50.81, 23.20, 2.62 ppm; HRMS (ESI): m/z $[M+H]^+$ .$C_{23}H_{23}N_8O_4S$ calculated value 507.1557, measured value 507.1549.

Example 14

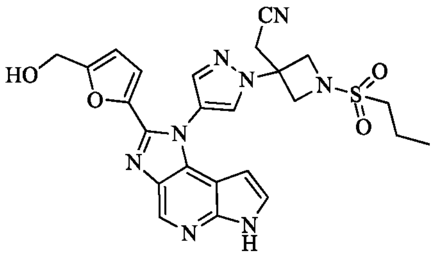

**LXS14**

2-(1-(Propylsulfonyl)-3-(4-(2-(5-(hydroxymethyl)furan-2-yl)imidazo[4,5-d]pyrrolo[2,3-b]pyridin-1(6H)-yl)-1H-pyrazol-1-yl)azetidin-3-yl)acetonitrile

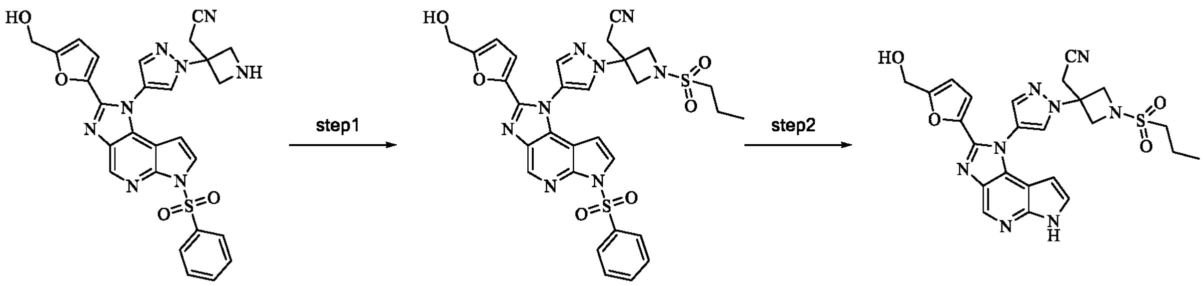


Intermediate-2 14-1 KDN14

Step 1: Dissolve 2-(3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl)acetonitrile (0.3 g, 0.5 mmol) in 10 mL of tetrahydrofuran. After the addition of DIPEA (0.2 g, 1.0 mmol), propylsulfonyl chloride (0.1 g, 0.8 mmol) was added slowly dropwise. Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield 2-(1-(propylsulfonyl)-3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl)acetonitrile as a yellow oil (0.3 g, 84%). The product can be used directly in the next step without further purification. HRMS (ESI): m/z $[M+H]^+$ .$C_{30}H_{29}N_8O_6S_2$ calculated value 661.1646, measured value 661.1649.

Step 2: 2-(1-(propylsulfonyl)-3-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)azetidin-3-yl)acetonitrile (0.3 g, 0.5 mmol) was dissolved in a solvent mixture of 5 mL of tetrahydrofuran and 5 mL of methanol. Then, 5ml of 1 M sodium hydroxide was added and the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS14:** 2-(1-(propylsulfonyl)-3-(4-(2-(5-(hydroxymethyl)furan-2-yl)imidazo[4,5-d]pyrrolo[2,3-b]pyridin-1(6H)-yl)-1H-pyrazol-1-yl)azetidin-3-yl)acetonitrile (0.1 g, 42%).$^1$ HNMR (300 MHz, *DMSO-$d_6$* ): $\delta$ = 11.91 (s, 1H), 8.87 (s, 1H), 8.66 (s, 1H), 8.11 (s, 1H), 7.34 (s, 1H), 6.38 (s, 2H), 5.93 ( s, 1H), 5.40 (t, *J* = 6.0 Hz, 1H), 4.58 (d, *J* = 9.0 Hz, 2H), 4.45 (d, *J* = 3.0 Hz, 2H), 4.31 (d, *J* = 9.0 Hz, 2H), 3.75 (s, 2H), 3.24 (t, *J* = 7.5 Hz, 2H), 1.69-1.77 (m, 2H), 1.18-1.30 (m, 2H), 1.00 (t, *J* = 7.5 Hz, 3H) ppm;$^{13}$ CNMR(75 MHz, *DMSO-$d_6$ )* $\delta$ 157.85, 145.73, 143.25, 143.07, 139.10, 135.78, 135.68, 134.04, 128.63, 124.75, 119.74, 117.03, 113.11, 109.22, 104.52, 96.44, 58.75, 57.15, 56.11, 50.29, 27.30, 16.89, 13.16 ppm; HRMS (ESI): m/z $[M+H]^+$ .$C_{24}H_{25}N_8O_4S$ calculated value 521.1714, measured value 521.1695.

Example 15

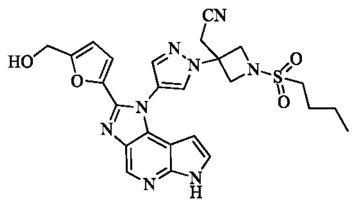

LXS15

2-(1-(Butylsulfonyl)-3-(4-(2-(5-(hydroxymethyl)furan-2-yl)imidazo[4,5-d]pyrrolo[2,3-b]pyridin-1(6H)-yl)-1H-pyrazol-1-yl)azetidin-3-yl)acetonitrile

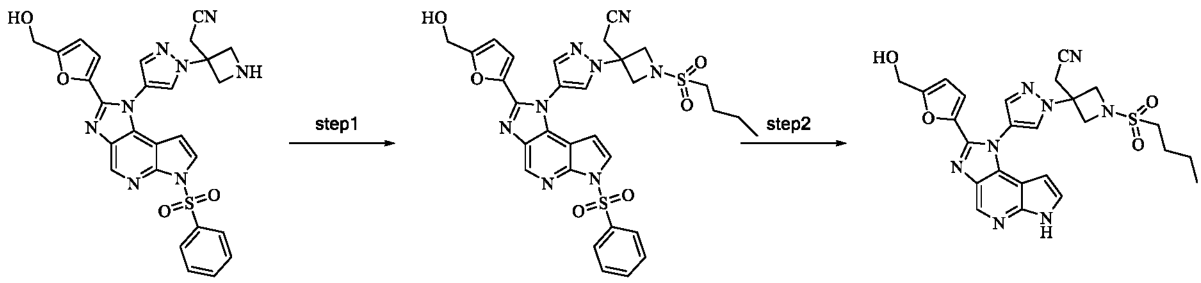


Intermediate-2 15-1 KDN15

Step 1: Dissolve 2-(3-(4-(2-(5-(hydroxymethyl)furan-2-yl)-6-(phenylsulfonyl)imidazo[4,5-d]pyrrolo[2,3-b]pyridin-1(6H )-yl)-1H-pyrazol-1-yl)azetidin-3-yl)acetonitrile (0.3 g, 0.5 mmol) in 10 mL of tetrahydrofuran. After the addition of DIPEA (0.2 g, 1.0 mmol), butylsulfonyl chloride (0.1 g, 0.8 mmol) was added slowly dropwise. Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield 2-(1-(butylsulfonyl)-3-(4-(2-(5-(hydroxymethyl)furan-2-yl)-6-(phenylsulfonyl)imidazo[4,5-d]pyrrolo[2,3-b ]pyridin-1(6H)-yl)-1H-pyrazol-1-yl)azetidin-3-yl)acetonitrile as a yellow oil (0.2 g, 55%). The product can be used directly in the next step without further purification. HRMS (ESI): m/z $[M+H]^+$ .$C_{31}H_{31}N_8O_6S_2$ calculated value 675.1802, measured value 675.1811.

Step 2: 2-(1-(Butylsulfonyl)-3-(4-(2-(5-(hydroxymethyl)furan-2-yl)-6-(phenylsulfonyl)imidazo[4,5-d]pyrrolo[2,3-b ]pyridin-1(6H)-yl)-1H-pyrazol-1-yl)azetidin-3-yl)acetonitrile (0.2 g, 0.3 mmol) was dissolved in a solvent mixture of 5 mL of tetrahydrofuran and 5 mL of methanol, 5ml of 1 M sodium hydroxide was added and the mixture was then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS15:** 2-(1-(butylsulfonyl)-3-(4-(2-(5-(hydroxymethyl)furan-2-yl)imidazo[4,5-d]pyrrolo[2,3-b]pyridin-1(6H )-yl)-1H-pyrazol-1-yl)azetidin-3-yl)acetonitrile (0.1 g, 63%).$^1$ HNMR (300 MHz, *DMSO-$d_6$* ): $\delta$ = 11.95 (s, 1H), 8.90 (s, 1H), 8.69 (s, 1H), 8.14 (s, 1H), 7.37 (s, 1H), 6.41 (s, 2H), 5.96 ( s, 1H), 5.44 (t, *J* = 6.0 Hz, 1H), 4.62 (d, *J* = 9.0 Hz, 2H), 4.48 (d, *J* = 6.0 Hz, 2H), 4.35 (d, *J* = 9.0 Hz, 2H), 3.78 (s, 2H), 3.29 (t, *J* = 7.5 Hz, 2H), 2.53 (s, 2H), 1.66- 1.76 (m, 2H), 1.38-1.50 (m, 2H), 0.92 (t, *J* = 7.5 Hz, 3H) ppm; $^{13}$C NMR (75 MHz, *DMSO-$d_6$)* $\delta$ 157.86, 145.75, 143.27, 143.09, 139.10, 135.80, 135.69, 134.05, 128.65, 124.76, 119.77, 117.06, 113.13, 109.23, 104.54, 96.46, 58.78, 57.16, 56.13, 48.45, 27.33, 25.08, 21.36, 13.94 ppm; HRMS (ESI): m/z $[M+H]^+$ $C_{25}H_{27}N_8O_4S$ calculated value 535.1870, measured value 535.1870.

Example 16

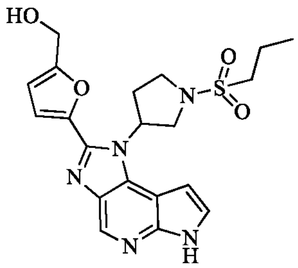

**LXS16**

(5-(1-(1-(thiophen-2-ylsulfonyl)pyrrolidin-3-yl)-1,6-dihydroimidazo[4,5-d]pyrrolo[2,3-b]pyridin-2-yl)furan-2-yl)methanol

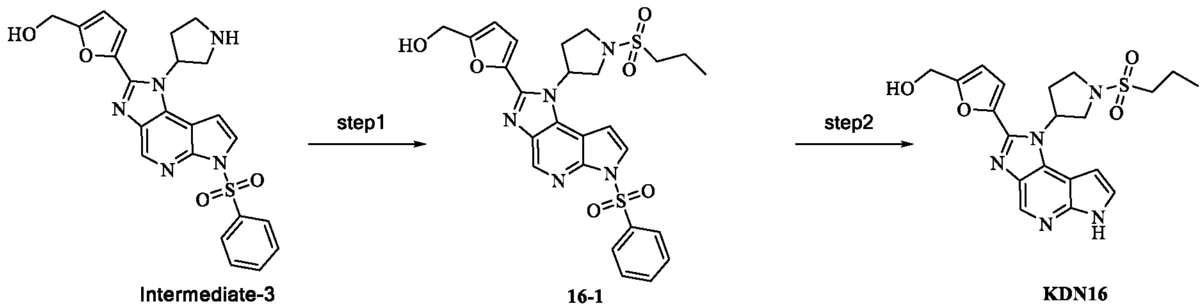


Intermediate-3 16-1 KDN16

Step 1: Dissolve 5-(6-(phenylsulfonyl)-1-(pyrrolidin-3-yl)-1,6-dihydroimidazo[4,5-d]pyrrolo[2,3-b]pyridin-2-yl)furan-2- methanol (0.3 g, 0.6 mmol) in 10 mL of tetrahydrofuran, add DIPEA (0.2 g, 1.2 mmol) followed by slow dropwise addition of propylsulfonyl chloride (0.1 g, 0.9 mmol). Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (5-(6-(phenylsulfonyl)-1-(1-(propylsulfonyl)pyrrolidin-3-yl)-1,6-dihydroimidazo[4,5-d]pyrrolo[2,3-b]pyridin-2-yl furan-2-yl) methanol as a yellow oil (0.2 g, 54%). The product can be used directly in the next step without further purification. HRMS (ESI): m/z $[M+H]^+$ .$C_{26}H_{28}N_5O_6S_2$ calculated value 570.1476, measured value 570.1488.

Step 2: Dissolve (5-(6-(phenylsulfonyl)-1-(1-(propylsulfonyl)pyrrolidin-3-yl)-1,6-dihydroimidazo[4,5-d]pyrrolo[2,3-b]pyridin-2-yl furan-2-yl) methanol (0.2 g, 0.4 mmol) in a solvent mixture of 5 mL of tetrahydrofuran and 5 mL of methanol. Then, add 5 mL of 1 M sodium hydroxide. Stir at room temperature for 5 h. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum, and the residue was purified using a silica gel column (petroleum ether: ethyl acetate = 1:1) to yield **LXS16:** (5-(1-(1-(thiophen-2-ylsulfonyl)pyrrolidin-3-yl)-1,6-dihydroimidazo[4,5-d]pyrrolo[2,3-b]pyridin-2-yl)furan-2-yl)methanol (0.1 g, 66%). $^1$HNMR (300 MHz, *DMSO-$d_6$* ): $\delta$ = 12.11 (s, 1H), 8.69 (s, 1H), 7.62 (s, 1H), 7.20 (d, *J* = 3.0 Hz, 1H), 6.83 (s, 1H), 6.64 (d, *J* = 3.0 Hz, 1H), 5.58-5.92 (m, 1H), 5.52 (t, *J* = 6.64 (d, J = 3.0 Hz, 1H), 5.58-5.92 (m, 1H), 5.52 (t, J = 6.0 Hz, 1H), 4.59 (d, *J* = 6.0 Hz, 2H), 4.04 (t, *J* = 10.5 Hz, 1H), 3.79-3.87 (m, 2H), 3.53-3.62 (m, 1H), 3.24-3.29 (m, 2H), 2.69-2.83 (m, 1H), 1.76-1.88 (m, 2H), 1.07 (t, *J* = 7.5 Hz, 3H) ppm;$^{13}$ C NMR (75 MHz, *DMSO-$d_6$)* $\delta$ 158.09, 144.98, 143.27, 143.04, 136.34, 135.19, 132.29, 125.05, 114.29, 109.37, 105.02, 98.97, 56.21, 54.67, 49.36, 48.97, 46.90, 30.36, 16.90, 13.35 ppm; HRMS (ESI): m/z $[M+H]^+$ .$C_{20}H_{24}N_5O_4S$ calculated value 430.1544, measured value 430.1550.

Example 17

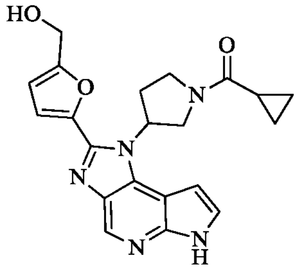

LXS17

Cyclopropyl(3-(2-(5-(hydroxymethyl)furan-2-yl)imidazo[4,5-d]pyrrolo[2,3-b]pyridin-1(6H)-yl)pyrrolidin-1-yl) methanone

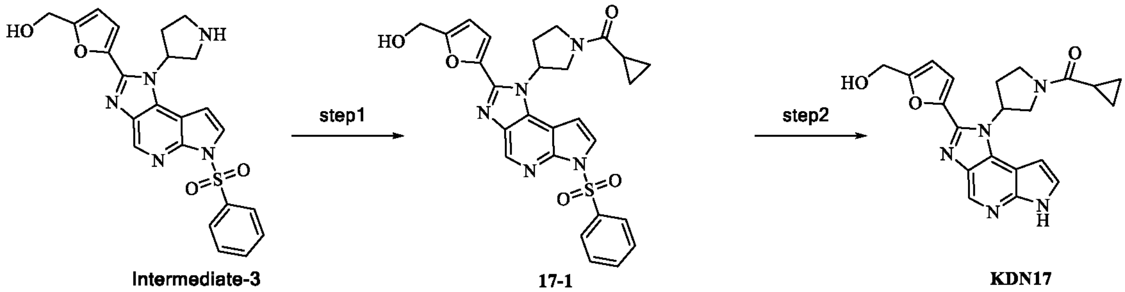


Intermediate-3 17-1 KDN17

Step 1: Dissolve (5-(6-(phenylsulfonyl)-1-(pyrrolidin-3-yl)-1,6-dihydroimidazo[4,5-d]pyrrolo[2,3-b]pyridin-2-yl)furan-2-methanol (0.3 g, 0.6 mmol) in 10 mL of tetrahydrofuran, add DIPEA (0.2 g, 1.2 mmol) followed by slow dropwise addition of cyclopropanecarbonyl chloride (0.1 g, 0.9 mmol). Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield cyclopropyl (3-(2-(5-(hydroxymethyl)furan-2-yl)-6-(phenylsulfonyl)imidazo[4,5-d]pyrrolo[2,3-b]pyridin-1(6H)-yl)pyrrolidin-1-yl)methanone as a brown oil (0.2 g, 58%). The product can be used directly in the next step without further purification. HRMS (ESI): m/z $[M+H]^+$ .$C_{27}H_{26}N_5O_5S$ calculated value 532.1649, measured value 532.1661.

Step 2: Cyclopropyl (3-(2-(5-(hydroxymethyl)furan-2-yl)-6-(phenylsulfonyl)imidazo[4,5-d]pyrrolo[2,3-b]pyridin-1(6H)-yl)pyrrolidin-1-yl) methanone (0.2 g, 0.4 mmol) was dissolved in a mixture of 5 mL of tetrahydrofuran and 5 mL of methanol. Then,5 mL of 1 M sodium hydroxide was added and the mixture was stirred for 5 h at room temperature. The reaction was monitored for completion by TLC. Saturated sodium bicarbonate was then added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield LXS17: Cyclopropyl(3-(2-(5-(hydroxymethyl)furan-2-yl)imidazo[4, 5-d]pyrrolo[2,3-b]pyridin-1 (6H)-yl)pyrrolidin-1-yl) methanone (0.1 g, 68%).$^1$ HNMR (300 MHz, *DMSO-$d_6$* ): $\delta$= 12.10 (s, 1H), 8.69 (d, $J$ = 3.0 Hz, 1H), 7.60 (t, $J$ = 4.5 Hz, 1H), 7.22 (dd, $J_1$ = 9.0 Hz, $J_2$ = 3.0 Hz, 1H), 6.61 (t, $J$ = 4.5 Hz, 1H), 6.46 (d, $J$ = 9.0 Hz, 1H), 5.84-6.03 (m, 1H), 5.50 (t, $J$ = 4.5 Hz, 1H), 4.57 (d, $J$ = 6.0 Hz, 2H), 4.20-4.42 (m, 1H), 4.02-4.12 (m, 1H), 3.81-3.95 (m, 1H), 2.65-2.86 (m, 1H), 1.72-2.01 (m, 1H), 1.25 (s, 2H), 0.84 (t, $J$ = 7.5 Hz, 2H), 0.74 (d, $J$ = 9.0 Hz, 2H) ppm;$^{13}$ C NMR (75 MHz, *DMSO-$d_6$ )* $\delta$ 171.92, 158.10, 158.05, 144.98, 143.47, 142.97, 136.41, 135.27, 132.40, 125.22, 114.32, 109.43, 105.09, 105.01, 98.67, 56.20, 45.58, 12.55, 7.68 ppm; HRMS (ESI): m/z $[M+H]^+$ . $C_{21}H_{22}N_5O_3$ calculated value 392.1717, measured value 392.1727.

Example 18

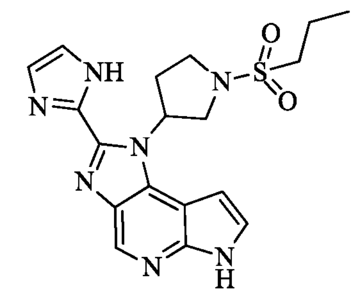

**LXS18**

2-(1H-imidazol-2-yl)-1-(1-(propylsulfonyl)pyrrolidin-3-yl)-1,6-dihydroimidazo[4,5-d]pyrrolo[2,3-b]pyridine

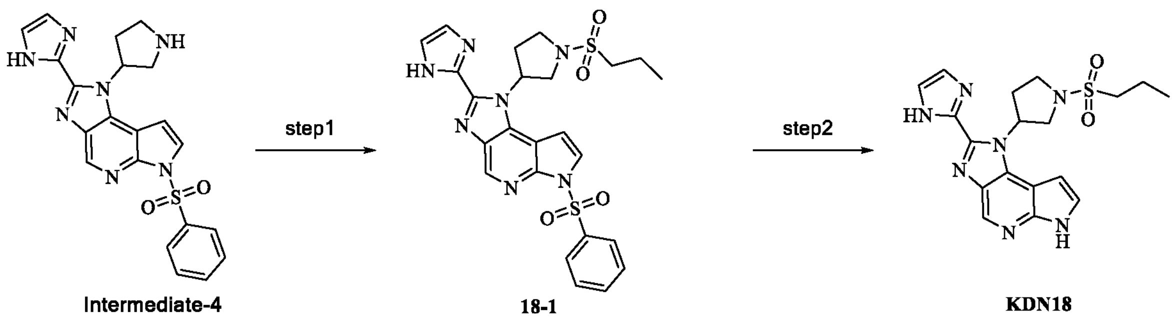


Intermediate-4 18-1 KDN18

Step 1: Dissolve 2-(1H-imidazol-2-yl)-6-(phenylsulfonyl)-1-(pyrrolidin-3-yl)-1,6-dihydroimidazo[4,5-d]pyrrolo[2,3-b]pyridine (0.3 g, 0.7 mmol) in 10 mL of tetrahydrofuran, add DIPEA (0.2 g, 1.4 mmol) followed by the slow dropwise addition of propylsulfonyl chloride (0.2 g, 1.0 mmol). Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield 2-(1H-imidazol-2-yl)-6-(phenylsulfonyl)-1-(1-(propylsulfonyl)pyrrolidin-3-yl)-1,6-dihydroimidazo[4,5-d]pyrrolo[2,3-b]pyridine as a yellow oil (0.2 g, 54%). The product can be used directly in the next step without further purification. HRMS (ESI): m/z $[M+H]^+$ .$C_{24}H_{26}N_7O_4S_2$ calculated value 540.1482, measured value 540.1485.

Step 2: 2-(1H-imidazol-2-yl)-6-(phenylsulfonyl)-1-(1-(propylsulfonyl)pyrrolidin-3-yl)-1,6-dihydroimidazo[4,5-d]pyrrolo[2,3 -b]pyridine (0.2 g, 0.4 mmol) was dissolved in a solvent mixture of 5 mL tetrahydrofuran and 5 mL methanol. 5 mL of 1 M sodium hydroxide was then added and the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS18:** 2-(1H-imidazol-2-yl)-1-(1-(propylsulfonyl)pyrrolidin-3-yl)-1,6-dihydroimidazo[4,5-d]pyrrolo[2,3-b]pyridine ( 0.1 g, 68%).$^1$ HNMR (300 MHz, *DMSO-$d_6$* ): $\delta$ = 13.34 (s, 1H), 12.09 (d, *J* = 9.0 Hz, 1H), 8.72 (d, *J* = 6.0 Hz, 1H), 7.58 (t, *J* = 12.0 Hz, 2 H), 7.41 (s, 1H), 7.25 (d, *J* = 6.0 Hz, 2H), 6.83 (s, 1H), 4.08 (s, 2H), 3.83-3.97 (m, 3H), 3.20-3.32 (m, 2H), 2.79 (t, *J* = 9.0 Hz, 1H), 2.11 (s, 2H), 1.76-1.86 (m, 2H), 1.25 (s, 1H), 1.08 (t, *J* = 6.0 Hz, 3H)ppm;$^{13}$ C NMR (75 MHz, *DMSO-$d_6$* ) $\delta$157.04, 148.61, 142.46, 138.11, 129.09, 127.92, 122.74, 120.71, 115.15, 99.49, 60.42, 56.83, 56.19, 50.01, 36.27, 13.20, 12.62 ppm; HRMS (ESI): m/z $[M+H]^+$ .$C_{18}H_{22}N_7O_2S$ calculated value 400.1550, measured value 400.1561.

Example 19

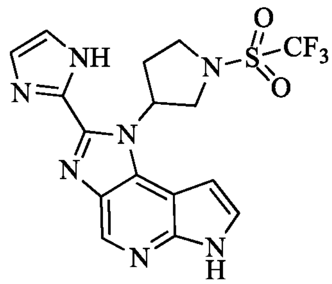

**LXS19**

2-(1H-imidazol-2-yl)-1-(1-((trifluoromethyl)sulfonyl)pyrrolidin-3-yl)-1,6-dihydroimidazo[4,5-d]pyrrolo[2,3-b]pyridine

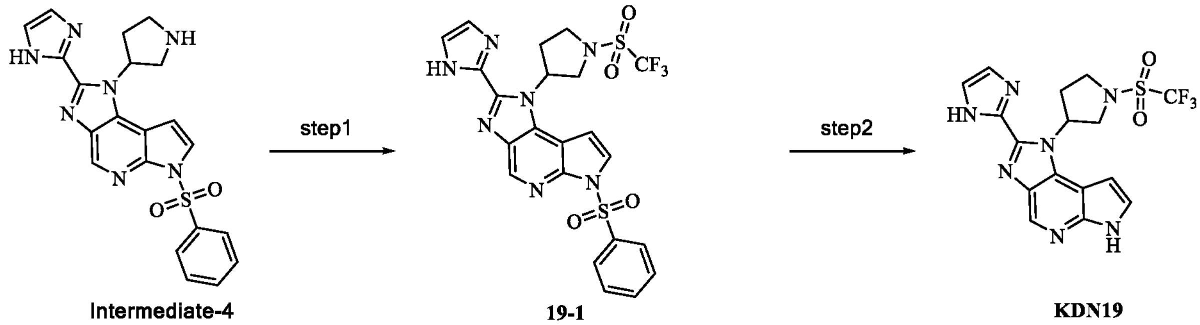


Intermediate-4 19-1 KDN19

Step 1: Dissolve 2-(1H-imidazol-2-yl)-6-(phenylsulfonyl)-1-(pyrrolidin-3-yl)-1,6-dihydroimidazo[4,5-d]pyrrolo[2,3-b]pyridine (0.3 g, 0.7 mmol) in 10 mL of tetrahydrofuran, add DIPEA (0.2 g, 1.4 mmol) followed by the slow dropwise addition of trifluoromethylsulfonyl chloride (0.2 g, 1.0 mmol). Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield 2-(1H-imidazol-2-yl)-6-(phenylsulfonyl)-1-(1-(((trifluoromethyl)sulfonyl)pyrrolidin-3-yl)-1,6-dihydroimidazo[4,5-d]pyrrolo[2,3-b]pyridine as a yellow oil (0.3 g, 77%). The product can be used directly in the next step without further purification. HRMS (ESI): m/z $[M+H]^+$ .$C_{22}H_{19}F_3N_7O_4S_2$ calculated value 566.0887, measured value 566.0894.

Step 2: 2-(1H-imidazol-2-yl)-6-(phenylsulfonyl)-1-(1-(((trifluoromethyl)sulfonyl)pyrrolidin-3-yl)-1,6-dihydroimidazo[4,5-d]pyrrolo[2,3-b]pyridine (0.3 g, 0.5 mmol) was dissolved in a solvent mixture of 5 mL tetrahydrofuran and 5 mL methanol. Then, 5ml of 1M sodium hydroxide was added and then the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS19**: 2-(1H-imidazol-2-yl)-1-(1-((trifluoromethyl)sulfonyl)pyrrolidin-3-yl)-1,6-dihydroimidazo[4,5-d]pyrrolo[2,3-b]pyridine (0.1 g, 44%).$^1$ HNMR (300 MHz, *DMSO-$d_6$* ): $\delta$ = 13.36 (s, 1H), 12.16 (s, 1H), 8.72 (s, 1H), 7.66 (s, 1H), 7.41 (s, 1H), 7.26 (s, 2H), 6.65 (s, 1H), 4.17 (d, *J* = 9.0 Hz, 2H), 3.90 (d, *J* = 6.0 Hz, 1H), 2.57-2.72 (m, 1H), 1.25 (s, 2H) ppm;$^{13}$ C NMR (75 MHz, *DMSO-$d_6$ )* $\delta$ 144.80, 142.52, 138.37, 136.04, 134.54, 132.85, 125.37, 119.82, 104.82, 98.39, 48.52 ppm; HRMS (ESI): m/z $[M+H]^+$ .$C_{16}H_{15}F_3N_7O_2S$ calculated value 426.0955, measured value 426.0959.

Example 20

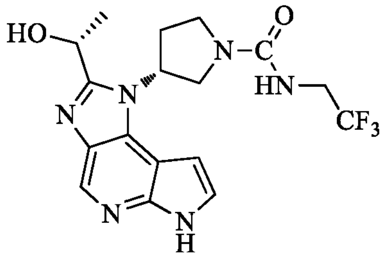

**LXS20**

(R)-3-(2-((R)-1-Hydroxyethyl)imidazo[4,5-d]pyrrolo[2,3-b]pyridin-1(6H)-yl)-N-(2,2,2-trifluoroethyl)pyrrolidine-1-carboxamide

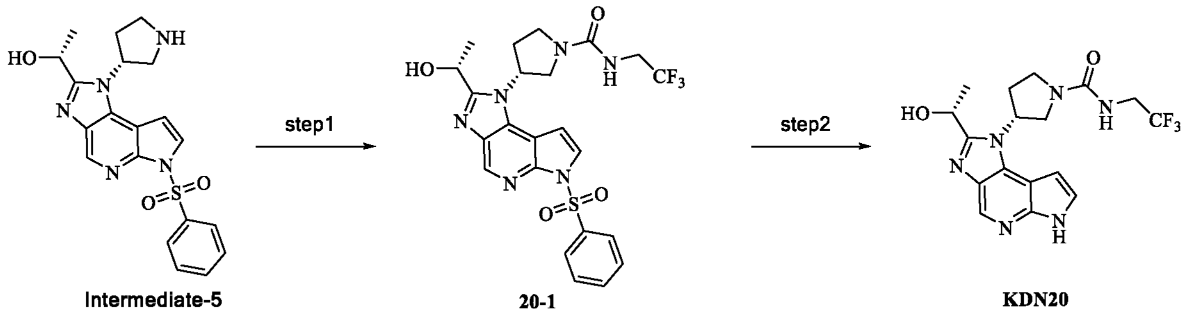


Intermediate-5 20-1 KDN20

Step 1: Dissolve (R)-1-(6-(phenylsulfonyl)-1-((R-pyrrolidin-3-yl)-1,6-dihydroimidazo[4,5-d]pyrrolo[2,3-b]pyridin-2-yl) ethanol (0.3 g, 0.7 mmol) in 10 mL of tetrahydrofuran, add carbonyl diimidazole (0.2 g, 1.4 mmol) and slowly add trifluoromethyl ethylamine (0.2 g, 1.4 mmol) dropwise. Then, stir at room temperature for 3 h and monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (R)-3-(2-((R)-1-hydroxyethyl)-6-(phenylsulfonyl)imidazo[4,5-d]pyrrolo[2,3-b]pyridin-1(6H)-yl)-N-(2,2,2-trifluoroethyl)pyrrolidine-1-carboxamide as a pale yellow oil (0.2 g, 51%). ). The product can be used directly in the next step without further purification. HRMS (ESI): m/z [M+H].$C_{23}H_{24}F_3N_6O_4S$ calculated value 537.1526, measured value 537.1511.

Step 2: Dissolve (R)-3-(2-((R)-1-hydroxyethyl)-6-(phenylsulfonyl)imidazo[4,5-d]pyrrolo[2,3-b]pyridin-1(6H)-yl)-N-(2,2,2-trifluoroethyl)pyrrolidine-1-carboxamide (0.2 g, 0.4 mmol) in a mixture of 5 mL of tetrahydrofuran and 5 mL of methanol, add 5mL of 1 M sodium hydroxide and stir the mixture at room temperature for 5 h. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum, and the residue was purified using a silica gel column (petroleum ether: ethyl acetate = 1:1) to yield **LXS20:** (R)-3-(2-((R)-1-Hydroxyethyl)imidazo[4,5-d]pyrrolo[2,3-b]pyridin-1(6H)-yl)-N-(2,2,2-trifluoroethyl)pyrrolidine-1-carboxamide (0.1 g, 44%).$^1$ HNMR (300 MHz, *DMSO-$d_6$*): $\delta$= 11.96 (s, 1H), 8.62 (s, 1H), 7.51 (s, 1H), 7.09 (s, 1H), 6.37 (s, 1H), 5.80 (d, *J* = 6.0 Hz, 2H), 5.22 (t, *J* = 6.0 Hz, 1H), 3.87-3.96 (m, 5H), 3.49 (d, *J* = 6.0 Hz, 1H), 2.64-2.76 (m, 1H), 2.43 (s, 1H), 1.68 (d, *J* = 3.0 Hz, 3H), 1.53 (s, 1H), 1.26 (s, 3H), 0.89 (d, *J* = 6.0 Hz, 2H) ppm;$^{13}$ C NMR (75 MHz, *DMSO-$d_6$*) $\delta$155.73, 148.66, 148.57, 142.76, 129.09, 127.12, 124.28, 120.70, 115.65, 99.38, 63.64, 57.99, 54.17, 48.74, 41.88, 26.60, 22.82 ppm; HRMS (ESI): m/z $[M+H]^+$ .$C_{17}H_{20}F_3N_6O_2$ calculated value 397.1594, measured value 397.1603.

Example 21

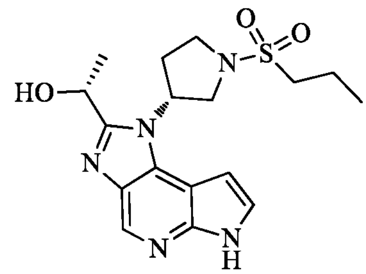

LXS21

(R)-1-(1-((R)-1-(Propylsulfonyl)pyrrolidin-3-yl)-1,6-dihydroimidazo[4,5-d]pyrrolo[2,3-b]pyridin-2-yl)ethanol

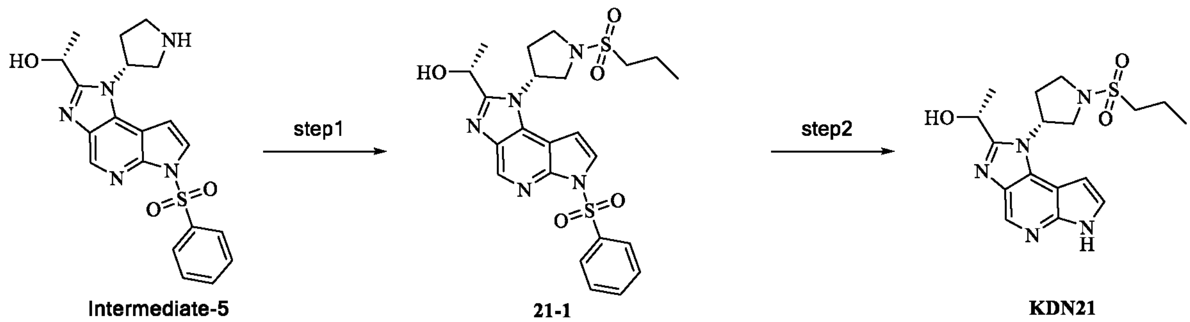


Intermediate-5 21-1 KDN21

Step 1: Dissolve (R)-1-(6-(phenylsulfonyl)-1-((R-pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) and then slowly add propyl sulfonyl chloride (0.2 g, 1.4 mmol) dropwise. Raise the temperature to maintain reflux and stir for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (R)-1-(6-(phenylsulfonyl)-1-((R)-1-(propylsulfonyl)pyrrolidin-3-yl)-1,6-dihydroimidazo[4,5-d]pyrrolo[2,3-b]pyridin-2-yl) ethanol as a yellow oil (0.3 g, 80%). The product can be used directly in the next step without further purification. HRMS (ESI): m/z $[M+H]^+$ .$C_{23}H_{28}N_5O_5S_2$ calculated value 518.1526, measured value 518.1534.

Step 2: (R)-1-(6-(Phenylsulfonyl)-1-((R)-1-(propylsulfonyl)pyrrolidin-3-yl)-1,6-dihydroimidazo[4,5-d]pyrrolo[2,3-b]pyridin-2-yl) ethanol (0.3 g, 0.6 mmol) was dissolved in a solvent mixture of 5 mL tetrahydrofuran and 5 mL methanol. Then, 5 mL of 1 M sodium hydroxide was added and the mixture was then stirred for 5 h at room temperature. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS21:** (R)-1-(1-((R)-1-(Propylsulfonyl)pyrrolidin-3-yl)-1,6-dihydroimidazo[4,5-d]pyrrolo[2,3-b]pyridin-2-yl)ethanol (0.2 g, 91%).$^1$ HNMR (300 MHz, *DMSO-$d_6$* ): $\delta$ = 11.97 (s, 1H), 8.62 (s, 1H), 7.56 (s, 1H), 6.72 (s, 1H), 5.81 (d, $J$ = 6.0 Hz, 2H), 5.22 (t, $J$ = 6.0 Hz, 1H), 3.82-3.94 (m, 2H), 3.74 (t, $J$ = 9.0 Hz, 1H), 3.51-3.59 (m, 1H), 3.28 (t, $J$ = 7.5 Hz, 2H), 2.65-2.75 (m, 1H), 1.78-1.85 (m, 2H), 1.68 (d, $J$ = 6.0 Hz, 3H), 1.53 (s, 1H), 1.26 (s, 1H), 1.08 (t, $J$ = 7.5 Hz, 3H) ppm;$^{13}$ C NMR (75 MHz, *DMSO-$d_6$ )* $\delta$ 155.12, 144.83, 136.22, 134.22, 132.43, 124.69, 104.91, 98.76, 62.52, 53.69, 49.72, 49.02, 46.86, 31.15, 22.08, 16.98, 13.32 ppm; HRMS (ESI): m/z $[M+H]^+$ .$C_{17}H_{24}N_5O_3S$ calculated value 378.1594, measured value 378.1609.

Example 22

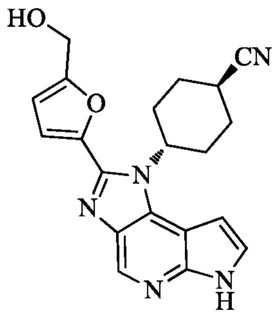

LXS22

trans-4-(2-(5-(Hydroxymethyl)furan-2-yl)imidazo[4,5-d]pyrrolo[2,3-b]pyridin-1(6H)-yl)cyclohexanecarbonitrile

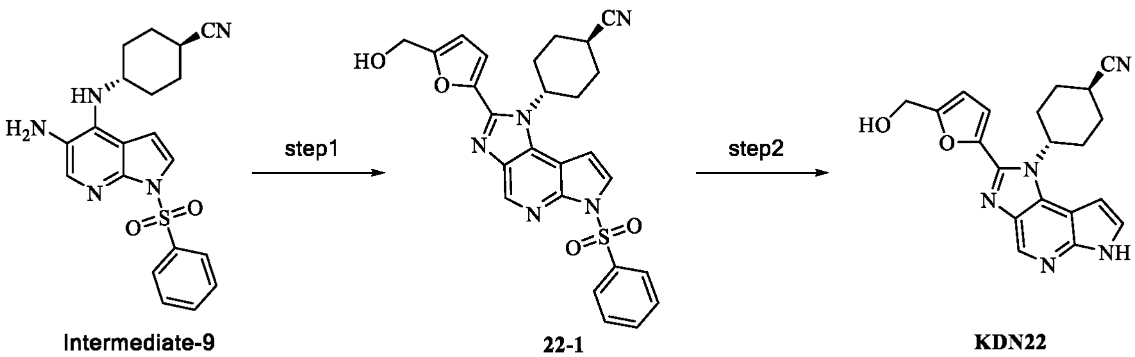


Intermediate-9 22-1 KDN22

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 5-hydroxymethylfurfural (0.2 g, 1.1 mmol) dropwise. Then, raise the temperature to 90°C and stir for 12 hours. The reaction was monitored for completion by TLC.Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was then purified using a silica gel column (petroleum ether: ethyl acetate = 1:1) to yield trans-4-(2-(5-(hydroxymethyl)furan-2-yl)-6-(phenylsulfonyl)imidazo[4,5-d]pyrrolo[2,3-b]pyridin-1(6H)-yl)cyclohexanecarbonitrile as a yellow solid (0.2 g, 53%). HRMS (ESI): m/z $[M+H]^+$ .$C_{26}H_{24}N_5O_4S$ calculated value 502.1544, measured value 502.1556.

Step 2: Dissolve trans-4-(2-(5-(hydroxymethyl)furan-2-yl)-6-(phenylsulfonyl)imidazo[4,5-d]pyrrolo[2,3-b]pyridin-1(6H )-yl)cyclohexanecarbonitrile (0.2 g, 0.4 mmol) in a mixture of 5 mL of tetrahydrofuran and 5 mL of methanol, add 5 mL of 1 M sodium hydroxide and stir at room temperature for 5 hours. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried with anhydrous sodium sulfate and concentrated under vacuum, and the residue was purified using a silica gel column (petroleum ether: ethyl acetate = 1:1) to yield **LXS22:** trans-4-(2-(5-(hydroxymethyl)furan-2-yl)imidazo[4,5-d]pyrrolo[2,3-b]pyridin-1(6H)-yl)cyclohexanecarbonitrile (0.1 g, 69%). 69%).$^1$HNMR (300 MHz, *DMSO-$d_6$ ):* $\delta$= 11.99 (s, 1H), 8.60 (s, 1H), 7.51 (s, 1H), 7.03 (s, 1H), 6.87 (s, 1H), 6.57 (s, 1H), 5.47 (s, 1H), 4.88 (s, 1H), 4.55 (s, 1H), 3.11 (s, 1H), 2.35 (d, *J* = 9.0 Hz, 2H), 2.24 (d, *J* = 12.0 Hz, 2H), 1.98 (s, 3H), 1.88 (d, *J* = 12.0 Hz, 2H), 1.19 (t, *J* = 9.0 Hz, 3H) ppm;$^{13}$C NMR (75 MHz, *DMSO-$d_6$)* $\delta$ 153.81, 150.11, 146.67, 144.93, 142.06, 129.05, 127.92, 122.75, 120.71, 115.65, 105.93, 104.75, 99.68, 64.56, 57.69, 30.22, 26.91, 24.60 ppm; HRMS (ESI): m/z $[M+H]^+$ .$C_{20}H_{20}N_5O_2$ calculated value 362.1612, measured value 362.1625.

Example 23

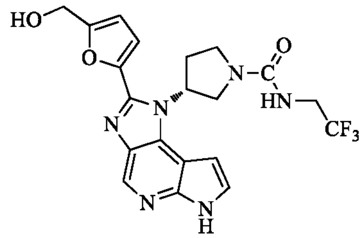

LXS23

(R)-3-(2-(5-(Hydroxymethyl)furan-2-yl)imidazo[4,5-d]pyrrolo[2,3-b]pyridin-1(6H)-yl)-N-(2,2,2-trifluoroethyl)pyrrolidine-1-carboxamide

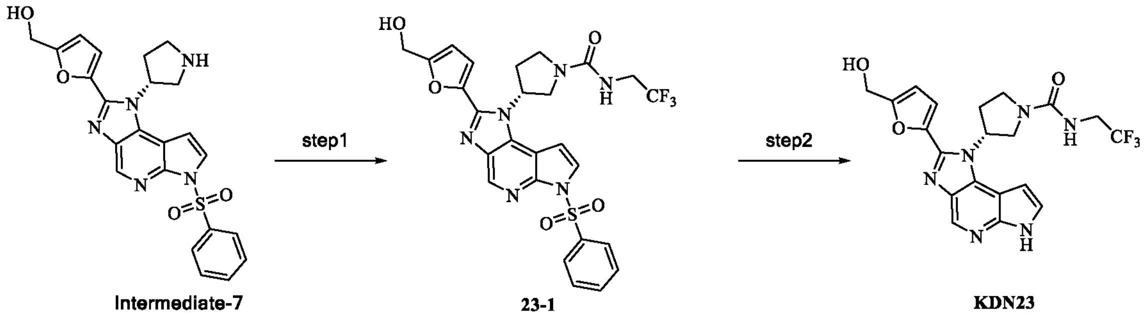


Intermediate-7 23-1 KDN23

Step 1: Dissolve (R)-(5-(6-(phenylsulfonyl)-1-(pyrrolidin-3-yl)-1,6-dihydroimidazo[4,5-d]pyrrolo[2,3-b]pyridin-2-yl) furan-2-yl methanol (0.3 g, 0.6 mmol) in 10 mL of tetrahydrofuran, add carbonyl diimidazole (0.2 g, 1.2 mmol) and then slowly add trifluoromethyl ethylamine (0.2 g, 1.2 mmol) dropwise. Then, stir the mixture at room temperature for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (R)-3-(2-(5-(hydroxymethyl)furan-2-yl)-6-(phenylsulfonyl)imidazo[4,5-d]pyrrolo[2,3-b]pyridin-1(6H)-yl)-N-(2,2,2-trifluoroethyl)pyrrolidine-1-carboxamide as a pale yellow oil (0.2 g (52%). The product can be used directly in the next step without further purification. HRMS (ESI): m/z $[M+H]^+$ .$C_{26}H_{24}F_3N_6O_5S$ calculated value 589.1475, measured value 589.1482.

Step 2: (R)-3-(2-(5-(Hydroxymethyl)furan-2-yl)-6-(phenylsulfonyl)imidazo[4,5-d]pyrrolo[2,3-b]pyridin-1(6H)-yl)-N-(2,2,2-trifluoroethyl)pyrrolidine-1-carboxamide (0.2 g, 0.3 mmol) was dissolved in a solvent mixture of 5 mL tetrahydrofuran and 5 mL methanol. After adding 5 mL of 1M sodium hydroxide, the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS23**: (R)-3-(2-(5-(hydroxymethyl)furan-2-yl)imidazo[4,5-d]pyrrolo[2,3-b]pyridin-1(6H)-yl)-N-(2,2,2- trifluoroethyl)pyrrolidine-1-carboxamide (0.1 g, 66%).$^1$ HNMR (300 MHz, *DMSO-$d_6$):* $\delta$ = 12.08 (s, 1H), 8.66 (s, 1H), 7.57 (s, 1H), 7.19 (s, 1H), 7.09 (s, 1H), 6.61 (s, 1H), 6.47 (s, 1H), 5.94 (d, *J* = 9.0 Hz, 1H), 5.49 (s, 1H), 4.57 (d, *J* = 6.0 Hz, 2H), 3.95 (d, *J* = 9.0 Hz, 6H), 2.71 (t, *J* = 12.0 Hz, 1H), 1.26 (s, 2H) ppm;$^{13}$ C NMR (75 MHz, *DMSO-$d_6$)* $\delta$ 155.75, 153.18, 151.71, 148.07, 144.94, 142.96, 129.54, 127.72, 124.74, 120.77, 115.35, 107.53, 104.35, 99.68, 57.79, 54.88, 53.59, 48.73, 41.81, 26.20 ppm; HRMS (ESI ): m/z $[M+H]^+$ .$C_{20}H_{20}F_3N_6O_3$ calculated value 449.1543, measured value 449.1550.

Example 24

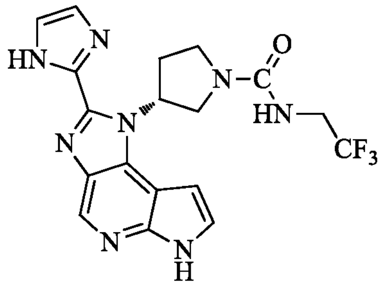

LXS24

(R)-3-(2-(1H-imidazol-2-yl)imidazo[4,5-d]pyrrolo[2,3-b]pyridin-1(6H)-yl)-N-(2,2,2-trifluoroethyl)pyrrolidine- 1 -carboxamide

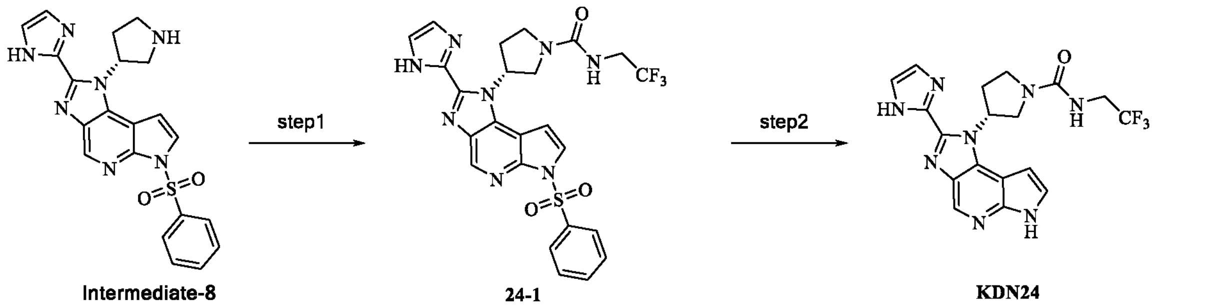


Intermediate-8 24-1 KDN24

Step 1: Dissolve (R)-2-(1H-imidazol-2-yl)-6-(phenylsulfonyl)-1-(pyrrolidin-3-yl)-1,6-dihydroimidazo[4,5-d]pyrrolo[2,3-b]pyridine (0.3 g, 0.7 mmol) in 10 mL of tetrahydrofuran, add carbonyl diimidazole (0.2 g, 1.4 mmol) and then slowly add trifluoromethyl ethylamine (0.2 g, 1.4 mmol) dropwise. Then, stir the mixture at room temperature for 3 h. Monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (R)-3-(2-(1H-imidazol-2-yl)-6-(phenylsulfonyl)imidazo[4,5-d]pyrrolo[2,3-b]pyridin-1(6H)-yl)-*N*-(2,2,2-trifluoroethyl)pyrrolidine-1-carboxamide as a pale yellow oil (0.2 g, 52%). ). The product can be used directly in the next step without further purification. HRMS (ESI): m/z $[M+H]^+$ .$C_{24}H_{22}F_3N_8O_3S$ calculated value 559.1482, measured value 559.1479.

Step 2: Dissolve (R)-3-(2-(1H-imidazol-2-yl)-6-(phenylsulfonyl)imidazo[4,5-d]pyrrolo[2,3-b]pyridin-1(6H)-yl)- N (2,2,2-trifluoroethyl)pyrrolidine-1-carboxamide (0.2 g, 0.4 mmol) in a solvent mixture of 5 mL tetrahydrofuran and 5 mL methanol. Then, add 5 ml of 1 M of sodium hydroxide and stir the mixture at room temperature for 5 h. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum, and the residue was purified using a silica gel column (petroleum ether: ethyl acetate = 1:1) to yield **LXS24**: (R)-3-(2-(1H-imidazol-2-yl)imidazo[4,5-d]pyrrolo[2,3-b]pyridin-1(6H)-yl)-N-(2,2,2-trifluoroethyl ) pyrrolidine-1-carboxamide (0.1 g, 67%).$^1$ HNMR (300 MHz, *DMSO-$d_6$* ): $\delta$= 13.34(s, 1H), 12.10 (s, 1H), 8.72 (s, 1H), 7.58 (s, 1H), 7.41 (s, 1H), 7.25 (s, 2H), 7.10 (s, 1H), 6.47 (s, 1H), 3.89-4.00 (m, 6H), 2.77 (t, *J* = 12.0 Hz, 1H), 2.12 (s, 2H) ppm;$^{13}$ CNMR(75 MHz, *DMSO-$d_6$ )* $\delta$157.08, 155.78, 148.66, 142.49, 138.11, 129.00, 127.19, 124.27, 122.77, 120.75, 115.68, 99.33, 57.26, 54.17, 48.70, 41.80, 26.63 ppm; HRMS (ESI): m/z $[M+H]^+$ .$C_{18}H_{18}F_3N_8O$ calculated value 419.1550, measured value 419.1551.

Example 25

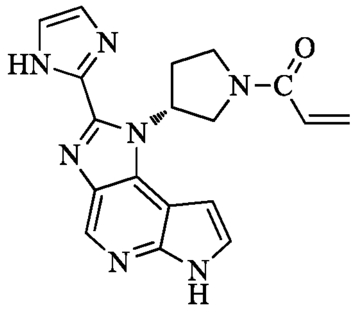

LXS25

(R)-1-(3-(2-(1H-imidazol-2-yl)imidazo[4,5-d]pyrrolo[2,3-b]pyridin-1(6H)-yl)pyrrolidin-1-yl)prop-2-en-1-one

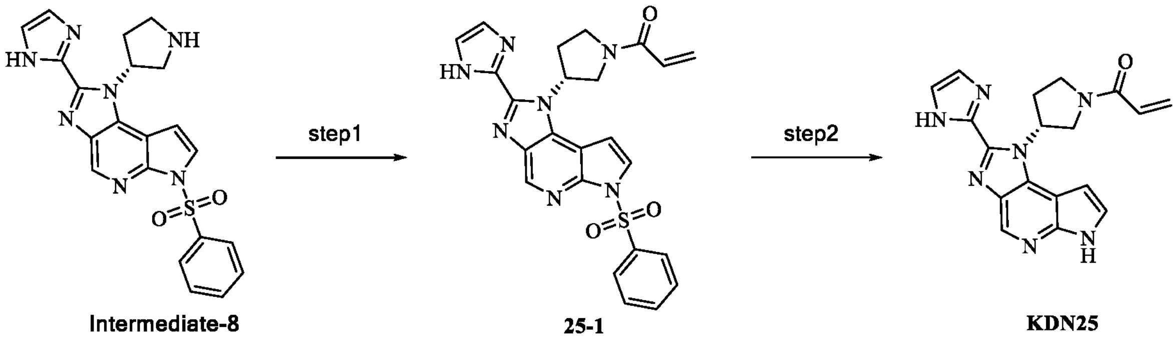


Intermediate-8

25-1

KDN25

Step 1: Dissolve (R)-2-(1H-imidazol-2-yl)-6-(phenylsulfonyl)-1-(pyrrolidin-3-yl)-1,6-dihydroimidazo[4,5-d]pyrrolo[2,3-b]pyridine (0.3 g, 0.7 mmol) in 10 mL of tetrahydrofuran, add DIPEA (0.2 g, 1.4 mmol) followed by the slow dropwise addition of acryloyl chloride (0.1 g, 1.4 mmol). Then, stir the mixture at room temperature for 3 h and monitor the reaction for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum to yield (R)-1-(3-(2-(1H-imidazol-2-yl)-6-(phenylsulfonyl)imidazo[4,5-d]pyrrolo[2,3-b]pyrrolidin-1(6H)-yl)pyrrolidin-1-yl)prop-2-en-1-one as a pale yellow oil (0.2 g, 59%). The product can be used directly in the next step without further purification. HRMS (ESI): m/z $[M+H]^+$ .$C_{24}H_{22}N_7O_3S$ calculated value 488.1499, measured value 488.1511.

Step 2: (R)-1-(3-(2-(1H-imidazol-2-yl)-6-(phenylsulfonyl)imidazo[4,5-d]pyrrolo[2,3-b]pyridin-1(6H)-yl)pyrrolidin-1-yl)prop-2-en-1-one (0.2 g, 0.4 mmol) was dissolved in a mixture of 5 mL of tetrahydrofuran and 5 mL of methanol. Then, 5ml of 1 M sodium hydroxide was added and the mixture was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS25**: (R)-1-(3-(2-(1H-imidazol-2-yl)imidazo[4,5-d]pyrrolo[2,3-b]pyridin-1(6H)-yl)pyrrolidin-1-yl)prop-2-en-1-one (0.1 g, 70%).$^1$ HNMR (300 MHz, *DMSO-$d_6$*): $\delta$ = 13.34 (s, 1H), 12.10 (s, 1H), 8.72 (s, 1H), 7.58 (d, $J$ = 9.0 Hz, 1H), 7.41 (s, 1H), 7.25 (s, 1H), 6.46 (s, 1H), 4.02 -4.22 (m, 2H), 3.95 (t, $J$ = 9.0 Hz, 1H), 3.67 (d, $J$ = 6.0 Hz, 2H), 3.61 (d, $J$ = 6.0 Hz, 1H), 3.34 (s, 2H), 3.23 (s, 1H), 2.74-2.88 (m, 1H) ppm;$^{13}$C NMR (75 MHz, *DMSO-$d_6$* ) $\delta$166.30, 157.05, 148.66, 142.96, 138.17, 131.16, 129.05, 127.17, 126.88, 122.75, 120.74, 115.65, 99.38, 57.73, 52.07, 46.68, 27.17 ppm; HRMS (ESI): m/z $[M+H]^+$ .$C_{18}H_{18}N_7O$ calculated value 348.1567, measured value 348.1557.

Example 26

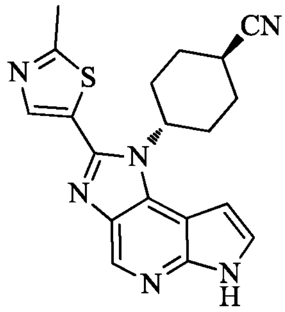

**LXS26**

trans-4-(2-(2-Methylthiazol-5-yl)imidazo[4,5-d]pyrrolo[2,3-b]pyridin-1(6H)-yl)cyclohexanecarbonitrile

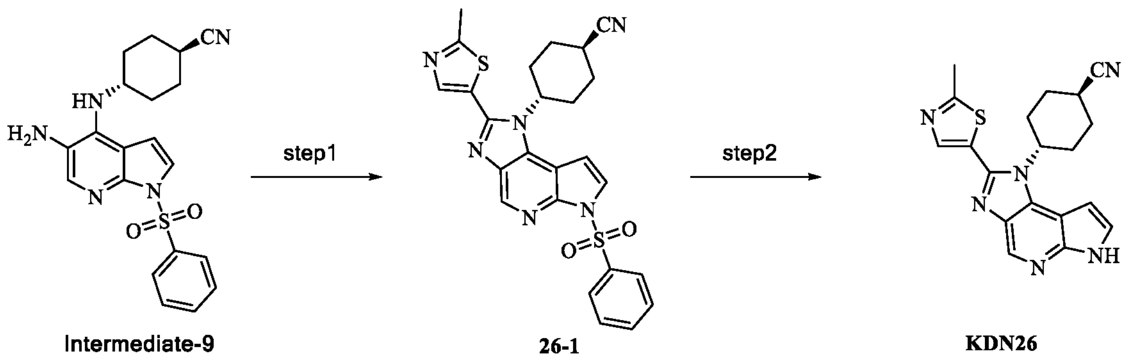


Intermediate-9 26-1 KDN26

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $NaS_2O_5$ (0.8 g, 4 mmol) followed by the dropwise addition of 5-formyl-2-methylthiazole (0.2 g, 1.1 mmol). Stir the mixture for 12 h at 90°C. The reaction was monitored for completion by TLC. Saturated sodium bicarbonate was then added until the reaction solution was weakly basic, and the organic phase was separated. The aqueous phase was then extracted twice with dichloromethanel. The organic phases were then combined and washed with saturated saline, dried over anhydrous sodium sulfate and concentrated under vacuum to yield trans-4-(2-(2-methylthiazol-5-yl)-6-(phenylsulfonyl)imidazo[4,5-d]pyrrolo[2,3-b]pyridin-1(6H)-yl ) cyclohexanecarbonitrile as a yellow oil (0.2 g, 52%). HRMS (ESI): m/z $[M+H]^+$.$C_{25}H_{23}N_6O_2S_2$ calculated value 503.1318, measured value 503.1322.

Step 2: Trans-4-(2-(2-methylthiazol-5-yl)-6-(phenylsulfonyl)imidazo[4,5-d]pyrrolo[2,3-b]pyridin-1(6H)-yl)cyclohexanecarbonitrile (0.2 g, 0.4 mmol) was dissolved in a mixture of 5 mL of tetrahydrofuran and 5 mL of methanol, and then stirred at room temperature for 5 h after the addition of 5 mL of 1M sodium hydroxide. The reaction was monitoredfor completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS26:** trans-4-(2-(2-Methylthiazol-5-yl)imidazo[4,5-d]pyrrolo[2,3-b]pyridin-1(6H)-yl)cyclohexanecarbonitrile (0.1 g, 69%). ).$^1$ HNMR (300 MHz, *DMSO-d6): $\delta$* = 12.05 (s, 1H), 8.65 (s, 1H), 8.10 (s, 1H), 7.56 (s, 1H), 6.89 (s, 1H), 4.76 (s, 1H), 3.20 (t, *J* = *12.0* Hz, 1H), 2.80 (s, 3H), 2.43 (t, *J* = *12.0 Hz, 2H)* = 12.0 Hz, 2H), 2.27 (d, *J* = 12.0 Hz, 2H), 2.10 (d, *J* = 15.0 Hz, 2H), 1.86-1.98 (m, 2H)ppm;$^{13}$ CNMR(75 MHz, *$DMSO-d_6$ ) $\delta$*168.60, 144.99, 143.26, 143.05, 136.18, 135.29, 132.94, 127.33, 124.63, 123.32, 104.45, 100.98, 54.58, 31.18, 29.48, 29.16, 28.49, 26.19, 19.36 ppm; HRMS (ESI): m/z $[M+H]^+$ .$C_{19}H_{19}N_6S$ calculated value 363.1386, measured value 363.1395.

Example 27

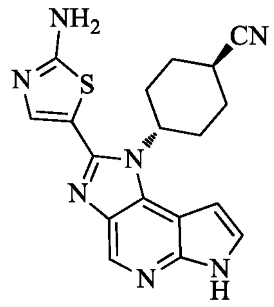

**LXS27**

trans-4-(2-(2-Aminothiazol-5-yl)imidazo[4,5-d]pyrrolo[2,3-b]pyridin-1(6H)-yl)cyclohexanecarbonitrile

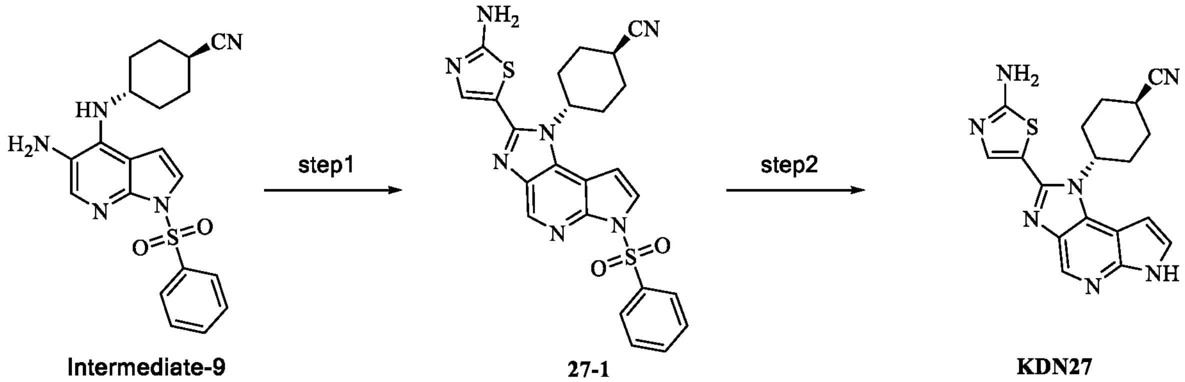


**Intermediate-9** **27-1** **KDN27**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $NaS_2O_5$ (0.8 g, 4 mmol) followed by the addition of 2-aminothiazole-5-carboxaldehyde (0.2 g, 1.1 mmol) dropwise. Then, stir the mixture for 12 h at 90°C. The reaction was monitored for completion by TLC. Saturated sodium bicarbonate was then added until the reaction solution was weakly basic, and the organic phase was separated. The aqueous phase was then extracted twice with dichloromethanel. The organic phases were then combined and washed with saturated saline, dried over anhydrous sodium sulfate and concentrated under vacuum to yield trans-4-(2-(2-aminothiazol-5-yl)-6-(phenylsulfonyl)imidazo[4,5-d]pyrrolo[2,3-b]pyridin-1(6H)-yl)cyclohexanecarbonitrile as a yellow oil (0.2 g, 52%). HRMS (ESI): m/z $[M+H]^+$.$C_{24}H_{22}N_7O_2S_2$ calculated value 504.1271, measured value 504.1277.

Step 2: Trans-4-(2-(2-aminothiazol-5-yl)-6-(phenylsulfonyl)imidazo[4,5-d]pyrrolo[2,3-b]pyridin-1(6H)-yl)cyclohexanecarbonitrile (0.2 g, 0.4 mmol) was dissolved in a mixture of 5 mL of tetrahydrofuran and 5 mL of methanol, and then stirred at room temperature for 5 h after the addition of 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS27**: trans-4-(2-(2-aminothiazol-5-yl)imidazo[4,5-d]pyrrolo[2,3-b]pyridin-1(6H)-yl)cyclohexanecarbonitrile (0.1 g, 69%). ).$^1$ HNMR (300 MHz, *DMSO-d6):* $\delta$ = 11.95 (s, 1H), 8.57 (s, 1H), 7.51 (s, 2H), 7.45 (s, 1H), 6.84 (s, 1H), 4.78 (s, 1H), 3.22 (d, *J* = 12.0 Hz, 1H), 2.44 (t, *J* = 13.5 Hz, 2H) , 2.28 (d, *J* = 12.0 Hz, 2H), 1.01 (d, *J* = 12.0 Hz, 2H), 1.91 (t, *J* = 13.5 Hz, 2H) ppm;$^{13}$ C NMR (75 MHz, *DMSO-$d_6$ )* $\delta$ 168.94, 148.64, 144.94, 142.16, 137.74, 129.05, 127.15, 122.75, 122.29, 120.75, 115.67, 99.39, 65.97, 30.88, 26.95, 24.66 ppm; HRMS (ESI): m/z $[M+H]^+$ .$C_{18}H_{18}N_7S$ calculated value 364.1339, measured value 364.1365.

Example 28

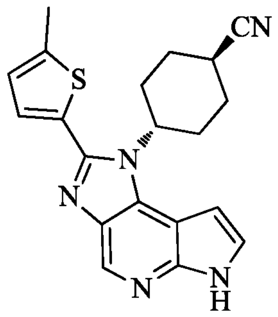

**LXS28**

trans-4-(2-(2-Methylthiophen-5-yl)imidazo[4,5-d]pyrrolo[2,3-b]pyridin-1(6H)-yl)cyclohexanecarbonitrile

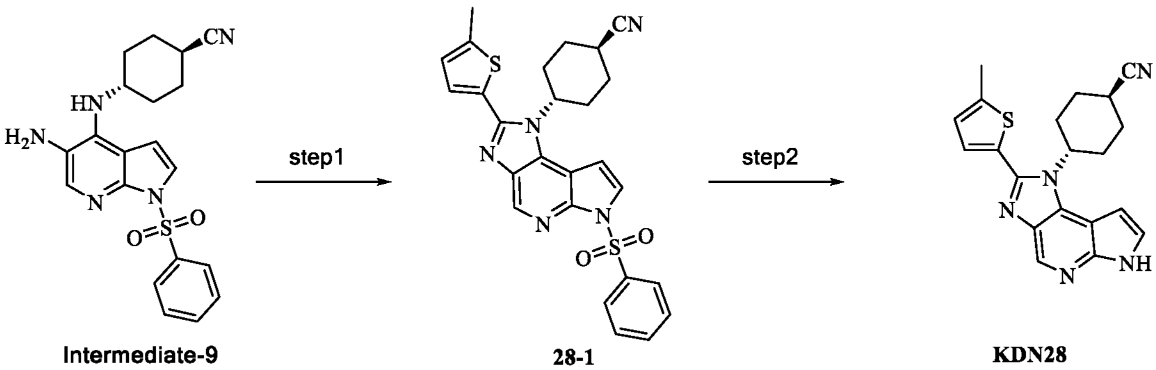


**Intermediate-9** **28-1** **KDN28**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 5-methyl-2-thiophenecarboxaldehyde (0.2 g, 1.1 mmol) dropwise, and stir for 12 h at 90°C. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(5-methylthiophen-2-yl)-6-(phenylsulfonyl)imidazo[4,5-d]pyrrolo[2,3-b]pyridin-1(6H)-yl ) cyclohexanecarbonitrile as a yellow oil (0.2 g, 53%). HRMS (ESI): m/z $[M+H]^+$.$C_{26}H_{24}N_5O_2S_2$ calculated value 502.1366, measured value 502.1377.

Step 2: Dissolve trans-4-(2-(5-methylthiophen-2-yl)-6-(phenylsulfonyl)imidazo[4,5-d]pyrrolo[2,3-b]pyridin-1(6H)-yl ) cyclohexanecarbonitrile (0.2 g, 0.4 mmol) in a mixture of 5 mL of tetrahydrofuran and 5 mL of methanol, add 5 mL of 1 M sodium hydroxide and then stir for 5 h at room temperature. The reaction was monitored for completion by TLC. Saturated sodium bicarbonate was then added until the reaction solution was weakly basic, and the organic phase was separated. The aqueous phase was then extracted twice using dichloromethane. The organic phases were then combined and washed with saturated saline, dried over anhydrous sodium sulfate and concentrated under vacuum. The residue was purified using a silica gel column (petroleum ether: ethyl acetate = 1:1) to yield **LXS28**: trans-4-(2-(2-methylthiophen-5-yl)imidazo[4,5-d]pyrrolo[2,3-b]pyridin-1(6H)-yl)cyclohexanecarbonitrile (0.1 g, 69%). ).$^1$ HNMR (300 MHz, *DMSO-$d_6$* ): $\delta$ = 12.00 (s, 1H), 8.63 (s, 1H), 7.54 (s, 1H), 7.38 (d, $J$ = 3.0 Hz, 1H), 7.02 (s, 1H), 6.87 (s, 1H), 4.84 (d, $J$ = 12.0 Hz, 1H), 3.21 (t, $J$ = 12.0 Hz, 1H), 2.58 (s, 3H), 2.45 (t, $J$ = 12.0 Hz, 2H), 2.29 (d, $J$ = *12.0* Hz, 2H), 2.03 (d, $J$ = 6.0 Hz, 2H), 1.81-1.93 (m, 2H) ppm;$^{13}$ C NMR (75 MHz, *DMSO-$d_6$)* $\delta$ 145.90, 144.93, 143.26, 136.04, 135.11, 132.96, 129.68, 129.49, 127.19, 124.51, 123.28, 104.53, 100.88, 54.70, 31.17, 29.17, 28.65, 26.21, 15.41 ppm; HRMS ( ESI): m/z $[M+H]^+$ .$C_{20}H_{20}N_5S$ calculated value 362.1434, measured value 362.1432.

Example 29

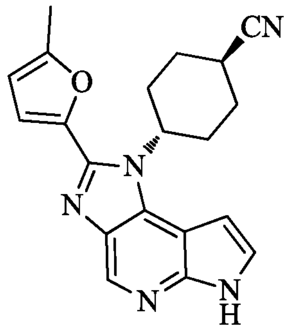

**LXS29**

trans-4-(2-(2-Methylfuran-5-yl)imidazo[4,5-d]pyrrolo[2,3-b]pyridin-1(6H)-yl)cyclohexanecarbonitrile

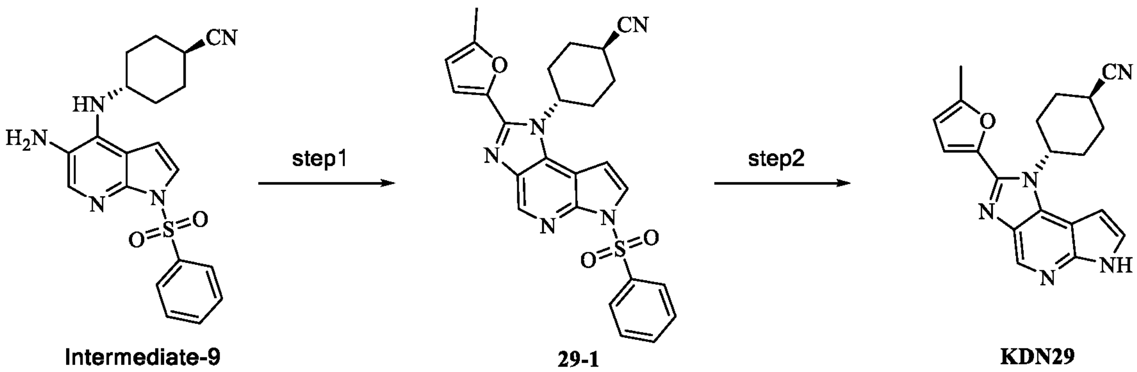


**Intermediate-9** **29-1** **KDN29**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 5-methyl-2-furanformaldehyde (0.2 g, 1.1 mmol)dropwise, and stir the mixture for 12 h at 90 °C. The reaction was monitored for completion by TLC. Saturated sodium bicarbonate was then added until the reaction solution was weakly basic, and the organic phase was separated. The aqueous phase was then extracted twice with dichloromethanel and the organic phases were combined and washed with saturated saline, dried over anhydrous sodium sulfate and concentrated under vacuum to yield trans-4-(2-(5-methylfuran-2-yl)-6-(phenylsulfonyl)imidazo[4,5-d]pyrrolo[2,3-b]pyridin-1(6H)-yl ) cyclohexanecarbonitrile as a yellow oil (0.2 g, 54%). HRMS (ESI): m/z $[M+H]^+$.$C_{26}H_{24}N_5O_3S$ calculated value 486.1594, measured value 486.1601.

Step 2: Dissolve trans-4-(2-(5-methylfuran-2-yl)-6-(phenylsulfonyl)imidazo[4,5-d]pyrrolo[2,3-b]pyridin-1(6H)-yl ) cyclohexanecarbonitrile (0.2 g, 0.4 mmol) in a mixture of 5 mL of tetrahydrofuran and 5 mL of methanol, add 5 mL of 1M sodium hydroxide and stir for 5 h at room temperature. The reaction was monitored for completion by TLC. Saturated sodium bicarbonate was then added until the reaction solution was weakly basic, and the organic phase was separated. The aqueous phase was then extracted twice using dichloromethane and the organic phase were combined and washed with saturated saline, dried over anhydrous sodium sulfate and concentrated under vacuum, and the residue was purified using a silica gel column (petroleum ether: ethyl acetate = 1:1) to yield **LXS29**: trans-4-(2-(2-Methylfuran-5-yl)imidazo[4,5-d]pyrrolo[2,3-b]pyridin-1(6H)-yl)cyclohexanecarbonitrile (0.1 g, 70%). ).$^1$HNMR (300 MHz, *DMSO-d$_6$* ): $\delta$ = 12.00 (s, 1H), 8.62 (s, 1H), 7.53 (s, 1H), 7.01 (s, 1H), 6.89 (s, 1H), 6.41 (s, 1H), 4.89 (t, *J* = 10.5 Hz, 1H), 3.15 (t, *J* = 12.0 Hz, 1H) , 2.53 (s, 3H), 2.40 (d, *J* = 12.0 Hz, 2H), 2.29 (d, *J* = 12.0 Hz, 2H), 2.00 (d, *J* = 10.0 Hz, 2H), 1. 91 (d, *J* = 12.0 Hz, 2H) ppm;$^{13}$C NMR (75 MHz, *DMSO-d$_6$)* $\delta$154.49, 145.07, 143.04, 142.81, 136.20, 135.14, 132.90, 124.49, 123.34, 114.47, 108.68, 104.57, 100.44, 54.96, 31.17, 29.36, 28.78, 26.41, 22.57, 13.91 ppm; HRMS (ESI ): m/z $[M+H]^+$ .$C_{20}H_{20}N_5O$ calculated value 346.1662, measured value 346.1664.

Example 30

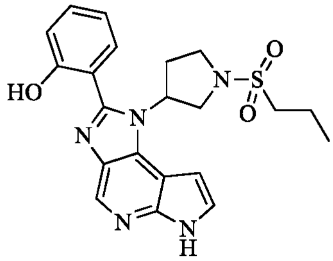

**LXS30**

2-(1-(1-(propylsulfonyl)pyrrolidin-3-yl)-1,6-dihydroimidazo[4,5-d]pyrrolo[2,3-b]pyridin-2-yl)phenol

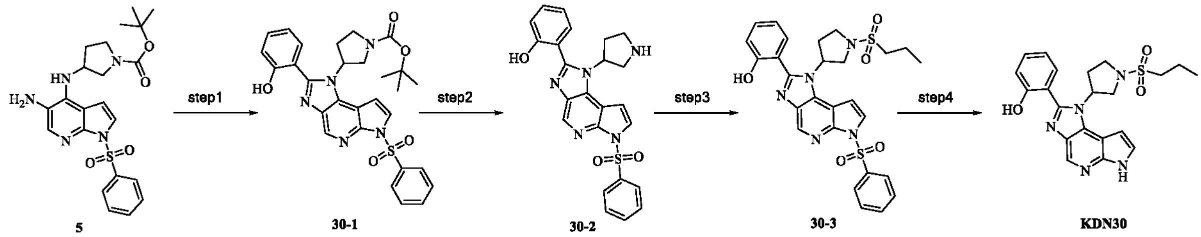


Step 1: Dissolve tert-butyl 3-(((5-amino-1-(phenylsulfonyl)-1H-pyrrolo[2,3-b]pyridin-4-yl)amino)pyrrolidine-1-carboxylate (0.3 g, 0.7 mmol) in 10 mL of DMF, add $Na_2S_2O_5$ (0.7 g, 3.5 mmol) followed by addition of o-hydroxybenzaldehyde (0.2 g, 1.4 mmol). Stir for 12h at 90 °C. The reaction was monitored for completion by TLC. Saturated sodium bicarbonate was then added until the reaction solution was weakly basic, and the organic phase was separated. The aqueous phase was then extracted twice using dichloromethane and the organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(2-(2-hydroxyphenyl)-6-(phenylsulfonyl)imidazo[4,5-d]pyrrolo[2,3-b]pyridin-1(6H)-yl)pyrrolidine-1-carboxylate as a yellow solid (0.2 g, 55%). HRMS (ESI): m/z $[M+H]^+$ .$C_{29}H_{30}N_5O_5S$ calculated value 560.1962, measured value 560.1965.

Step 2: Dissolve tert-butyl 3-(2-(2-hydroxyphenyl)-6-(phenylsulfonyl)imidazo[4,5-d]pyrrolo[2,3-b]pyridin-1(6H)-yl)pyrrolidine-1-carboxylate (0.2 g, 0.4 mmol) in 10 mL dichloromethane, slowly add trifluoroacetic acid (0.5 g, 4.0 mmol) and stir at room temperature for 12 h. The product was concentrated under vacuum to yield 2-(6-(phenylsulfonyl)-1-(pyrrolidin-3-yl)-1,6-dihydroimidazo[4,5-d]pyrrolo[2,3-b]pyridin-2-yl)phenol as a light yellow oil (0.1 g, 61%). The product can be used directly in the next step without further purification. HRMS (ESI): m/z $[M+H]^+$ .$C_{24}H_{22}N_5O_3S$ calculated value 460.1438, measured value 460.1441.

Step 3: Dissolve 2-(6-(phenylsulfonyl)-1-(pyrrolidin-3-yl)-1,6-dihydroimidazo[4,5-d]pyrrolo[2,3-b]pyridin-2-yl)phenol (0.1 g, 0.2 mmol) in 10 mL of tetrahydrofuran, add DIPEA (0.1 g, 0.4 mmol) followed by the slow dropwise addition of propylsulfonyl chloride (0.1 g, 0.3 mmol) and stir for 3 h at room temperature. The reaction was monitored for completion by TLC. Saturated sodium bicarbonate was then added until the reaction solution was weakly basic, and the organic phase was separated. The aqueous phase was then extracted twice using dichloromethane and the organic phases were combined and washed with saturated saline, dried over anhydrous sodium sulfate and concentrated under vacuum to yield 2-(6-(phenylsulfonyl)-1-(1-(propylsulfonyl)pyrrolidin-3-yl)-1,6-dihydroimidazo[4,5 - d]pyrrolo[2,3-b]pyridin-2-yl)phenol as a pale yellow oil (0.1 g, 81%). The product can be used directly in the next step without further purification. HRMS (ESI): m/z $[M+H]^+$ .$C_{27}H_{28}N_5O_5S_2$ calculated value 566.1526, measured value 566.1533.

Step 4: Dissolve 2-(6-(phenylsulfonyl)-1-(1-(propylsulfonyl)pyrrolidin-3-yl)-1,6-dihydroimidazo[4,5-d]pyrrolo[2,3-b]pyridin-2-yl)phenol (0.1 g, 0.2 mmol) in a mixture of 5 mL of tetrahydrofuran and 5 mL of methanol.Then, add 5 mL of 1 M sodium hydroxide and then stir the mixture at room temperature for 5 hours. The reaction was monitored for completion by TLC. Saturated sodium bicarbonate was then added until the reaction solution was weakly basic, and the organic phase was separated. The aqueous phase was then extracted twice using dichloromethane and the organic phases were combined and washed with saturated saline, dried with anhydrous sodium sulfate and concentrated under vacuum, and the residue was purified using a silica gel column (petroleum ether: ethyl acetate = 1:1) to yield **LXS30:** 2-(1-(1-(propylsulfonyl)pyrrolidin-3-yl)-1,6-dihydroimidazo[4,5-d]pyrrolo[2,3-b]pyridin-2-yl)phenol ( 65.0 mg, 86%).$^1$ HNMR (300 MHz, *DMSO-$d_6$ ): $\delta$* = 12.06 (s, 1H), 8.67 (s, 1H), 7.59 (t, *J* = 3.0 Hz, 1H), 7.49-7.52 (m, 1H), 7.41-7.47 (m, 1H),

7.19 (d, $J$ = 9.0 Hz, 1H), 7.01 (t, $J$ = 7.5 Hz, 1H), 6.80 (d, $J$ = 3.0 Hz, 1H), 5.12-5.24 (m, 1H), 3.98-4.10 (m, 1H), 3.74-3.87 (m, 2H), 3.19-3.25 (m, 2H), 2.69-2.83 (m, 1H), 2.33-2.42 (m, 1H), 1.77-1.84 (m, 2H), 1.05 (t, $J$ = 9.0 Hz, 3H) ppm; $^{13}C$ NMR (75 MHz, *DMSO-$d_6$)* $\delta$ 170.82, 156.82, 150.94, 144.75, 136.04, 135.50, 132.54, 131.85, 131.74, 124.82 , 119.58, 117.92, 116.59, 104.91, 98.80, 60.22, 55.10, 49.24, 46.75, 30.10, 21.24, 16.91, 14.55, 13.32 ppm; HRMS (ESI): m/z $[M+H]^+$ .$C_{21}H_{24}N_5O_3S$ calculated value 426.1594, Measured value 426.1462.

Example 31

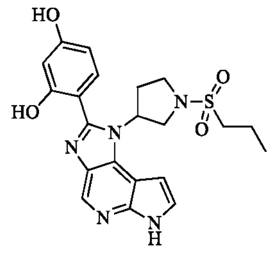

**LXS31**

4-(1-(1-(Propylsulfonyl)pyrrolidin-3-yl)-1,6-dihydroimidazo[4,5-d]pyrrolo[2,3-b]pyridin-2-yl)benzene-1,3-diol

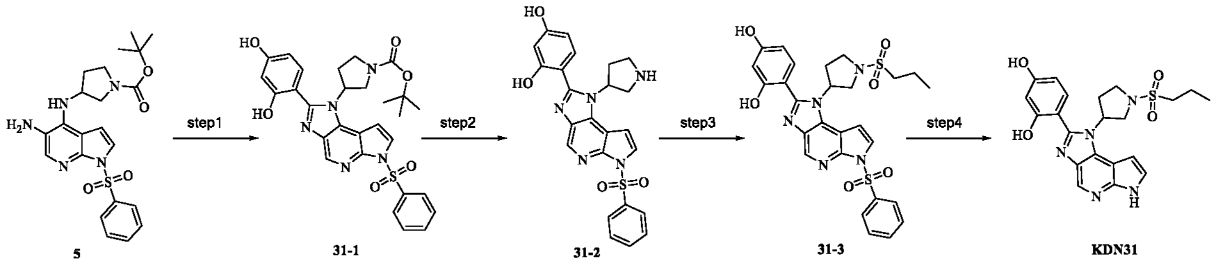


Step 1: Dissolve tert-butyl 3-(((5-amino-1-(phenylsulfonyl)-1H-pyrrolo[2,3-b]pyridin-4-yl)amino)pyrrolidine-1-carboxylate (0.3 g, 0.7 mmol) in 10 mL of DMF, add $Na_2S_2O_5$ (0.7 g, 3.5 mmol) followed by addition of 2,4-dihydroxybenzaldehyde (0.2 g, 1.4 mmol). Stir for 12h at 90 °C. The reaction was monitored for completion by TLC. Saturated sodium bicarbonate was then added until the reaction solution was weakly basic, and the organic phase was separated. The aqueous phase was then extracted twice using dichloromethane and the organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(2-(2,4-dihydroxyphenyl)-6-(phenylsulfonyl)imidazo[4,5-d]pyrrolo[2,3-b]pyridin-1(6H)-yl)pyrrolidine-1-carboxylate as a yellow solid (0.3 g, 79%). HRMS (ESI): m/z $[M+H]^+$ .$C_{29}H_{30}N_5O_6S$ calculated value 576.1911, measured value 576.1919.

Step 2: Dissolve tert-butyl 3-(2-(2,4-dihydroxyphenyl)-6-(phenylsulfonyl)imidazo[4,5-d]pyrrolo[2,3-b]pyridin-1(6H)-yl)pyrrolidine-1-carboxylate (0.3 g, 0.5 mmol) in 10 mL dichloromethane, slowly add trifluoroacetic acid (0.6 g, 5.0 mmol) and stir at room temperature for 12 hours. The product is then concentrated under vacuum to yield 4-(6-(phenylsulfonyl)-1-(pyrrolidin-3-yl)-1,6-dihydroimidazo[4,5-d]pyrrolo[2,3-b]pyridin-2-yl)benzene-1,3-diol as a light yellow oil (0.2 g, 81%). The product can be used directly in the next step without further purification. HRMS (ESI): m/z $[M+H]^+$ .$C_{24}H_{22}N_5O_4S$ calculated value 476.1387, measured value 476.1391.

Step 3: Dissolve 4-(6-(phenylsulfonyl)-1-(pyrrolidin-3-yl)-1,6-dihydroimidazo[4,5-d]pyrrolo[2,3-b]pyridin-2-yl)benzene-1,3-diol (0.2 g, 0.4 mmol) in 10 mL of tetrahydrofuran, add DIPEA (0.1 g, 0.8 mmol) followed by the slow dropwise addition of propylsulfonyl chloride (0.1 g, 0.6 mmol). Stir at room temperature for 3h. The reaction was monitored by TLC for completion. Saturated sodium bicarbonate was then added until the reaction solution was weakly basic, and the organic phase was separated. The aqueous phase was then extracted twice using dichloromethane and the organic phases were combined and washed with saturated saline, dried over anhydrous sodium sulfate and concentrated under vacuum to yield 4-(6-(phenylsulfonyl)-1-(1-(propylsulfonyl)pyrrolidin-3-yl)-1,6-dihydroimidazo[4,5 - d]pyrrolo[2,3-b]pyridin-2-yl) benzene-1,3-diol as a pale yellow oil (0.2 g, 82%). The product can be used directly in the next step without

further purification. HRMS (ESI): m/z [M+H]+ .$C_{27}H_{28}N_5O_6S_2$ calculated value 582.1476, measured value 582.1501.

Step 4: Dissolve 4-(6-(phenylsulfonyl)-1-(1-(propylsulfonyl)pyrrolidin-3-yl)-1,6-dihydroimidazo[4,5-d]pyrrolo[2,3-b]pyridin-2-yl) benzene-1,3-diol (0.2 g, 0.3 mmol) in a solvent mixture of 5 mL tetrahydrofuran and 5 mL methanol, after adding 5 mL of 1 M sodium hydroxide, stir the mixture for 5 h at room temperature and monitor the reaction for completion by TLC. Saturated sodium bicarbonate was then added until the reaction solution was weakly basic, and the organic phase was separated. The aqueous phase was then extracted twice using dichloromethane and the organic phases were combined and washed with saturated saline, dried over anhydrous sodium sulfate and concentrated under vacuum, and the residue was purified using a silica gel column (petroleum ether: ethyl acetate = 1:1) to yield **LXS31:** 4-(1-(1-(propylsulfonyl)pyrrolidin-3-yl)-1,6-dihydroimidazo[4,5-d]pyrrolo[2,3-b]pyridin-2-yl)benzene-1,3-diol (0.1 g, 66%).$^1$ HNMR (300 MHz, *DMSO-$d_6$* ): $\delta$ = 11.97 (s, 1H), 10.15 (s, 1H), 9.84 (s, 1H), 8.63 (s, 1H), 7.57 (s, 1H), 7.29 (d, *J* = 9.0 Hz, 1H), 6.76 (d, *J* = 3.0 Hz, 1H), 6.53 (d, *J* = 3.0 Hz, 1H), 6.42-6.46 (m, 1H), 5.12-5.34 (m, 1H), 3.95 (t, *J* = 9.0 Hz, 1H), 3.73-3.81 (m, 2H), 3.17-3.23 (m, 2H), 2.66-2.80 (m, 1H), 2.30-2.38 (m, 1H), 1.72-1.85 (m, 2H), 1.28-1.31 (m, 2H), 1.08-1.19 (m, 2H), 1.02 (t, *J* = 9.0 Hz, 3H) ppm;$^{13}$ C NMR (75 MHz, *DMSO-$d_6$ )* $\delta$ 160.74, 157.62, 151.30, 144.70, 135.81, 135.33, 133.33, 131.70, 124.76, 108.69, 107.72, 104.91, 102.94, 98.78, 54.98, 53.91, 49.43, 46.70, 18.47, 16.95, 13.34 ppm; HRMS (ESI) : m/z [M+H]+ .$C_{21}H_{24}N_5O_4S$ calculated value 442.1544, measured value 442.1405.

Example 32

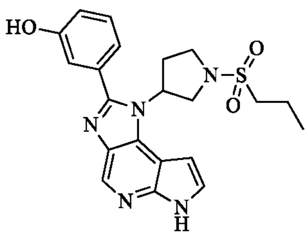

**LXS32**

3-(1-(1-(propylsulfonyl)pyrrolidin-3-yl)-1,6-dihydroimidazo[4,5-d]pyrrolo[2,3-b]pyridin-2-yl)phenol

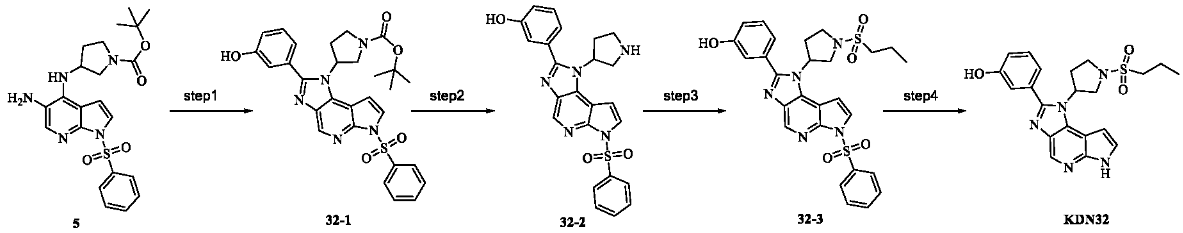


Step 1: Dissolve tert-butyl 3-(((5-amino-1-(phenylsulfonyl)-1H-pyrrolo[2,3-b]pyridin-4-yl)amino)pyrrolidine-1-carboxylate (0.3 g, 0.7 mmol) in 10 mL of DMF, add $Na_2S_2O_5$ (0.7 g, 3.5 mmol) followed by adding 3-hydroxybenzaldehyde (0.2 g, 1.4 mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(2-(3-hydroxyphenyl)-6-(phenylsulfonyl)imidazo[4,5-d]pyrrolo[2,3-b]pyridin-1(6H)-yl)pyrrolidine-1-carboxylate as a yellow solid ( 0.3 g, 82%). HRMS (ESI): m/z [M+H]+ .$C_{29}H_{30}N_5O_5S$ calculated value 560.1962, measured value 560.1969.

Step 2: Dissolve tert-butyl 3-(2-(3-hydroxyphenyl)-6-(phenylsulfonyl)imidazo[4,5-d]pyrrolo[2,3-b]pyridin-1(6H)-yl)pyrrolidine-1-carboxylate (0.3 g, 0.5 mmol) in 10 mL of dichloromethane, slowly add trifluoroacetic acid (0.6 g, 5.0 mmol) and stir at room temperature for 12 h. The product was concentrated under vacuum to yield 3-(6-(phenylsulfonyl)-1-(pyrrolidin-3-yl)-1,6-dihydroimidazo[4,5-d]pyrrolo[2,3-b]pyridin-2-yl) phenol as a light yellow oil (0.2 g, 81%). The product can be used directly in the next step without further purification. HRMS (ESI): m/z [M+H]+ .$C_{24}H_{22}N_5O_3S$ cal-

culated value 460.1438, measured value 460.1441.

Step 3: Dissolve 3-(6-(phenylsulfonyl)-1-(pyrrolidin-3-yl)-1,6-dihydroimidazo[4,5-d]pyrrolo[2,3-b]pyridin-2-yl)phenol (0.2 g, 0.4 mmol) in 10 mL of tetrahydrofuran, add DIPEA (0.1 g, 0.8 mmol) followed by slow dropwise addition of propylsulfonyl chloride (0.1 g, 0.6 mmol) and then stir for 3 h at room temperature. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(6-(phenylsulfonyl)-1-(1-(propylsulfonyl)pyrrolidin-3-yl)-1,6-dihydroimidazo[4,5-d]pyrrolo[2,3-b]pyridin-2-yl) phenol as a pale yellow oil (0.2 g, 81%). The product can be used directly in the next step without further purification. HRMS (ESI): m/z $[M+H]^+$ .$C_{27}H_{28}N_5O_5S_2$ calculated value 566.1526, measured value 566.1529.

Step 4: Dissolve 3-(6-(phenylsulfonyl)-1-(1-(propylsulfonyl)pyrrolidin-3-yl)-1,6-dihydroimidazo[4,5-d]pyrrolo[2,3-b]pyridin-2-yl) phenol (0.2 g, 0.4 mmol) in a mixture of 5 mL of tetrahydrofuran and 5 mL of methanol, add 5 mL of 1M sodium hydroxide and then stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS32**: 3-(1-(1-(propylsulfonyl)pyrrolidin-3-yl)-1,6-dihydroimidazo[4,5-d]pyrrolo[2,3-b]pyridin-2-yl)phenol ( 0.1 g, 66%).$^1$ HNMR (300 MHz, *DMSO-$d_6$* ): $\delta$ = 12.06 (s, 1H), 10.04 (s, 1H), 8.68 (s, 1H), 7.60 (t, $J$ = 3.0 Hz, 1H), 7.43 (t, $J$ = 7.5 Hz, 1H), 7.20 (t, $J$ = 6.0 Hz, 1H), 7.00- 7.03 (m, 1H), 6.80 (d, $J$ = 3.0 Hz, 1H), 5.41-5.53 (m, 1H), 3.99-4.09 (m, 1H), 3.76-3.89 (m, 2H), 3.46-3.55 (m, 1H), 3.16-3.32 (m, 2H), 2.71- 2.85 (m, 1H), 2.40-2.49 (m, 1H), 1.72-1.84 (m, 2H), 1.05 (t, $J$ = 9.0 Hz, 3H) ppm;$^{13}$ C NMR (75 MHz, *DMSO-$d_6$ )* $\delta$ 158.03, 152.38, 144.82, 136.27, 135.14, 132.22, 131.63, 130.44, 120.62, 117.30, 116.85, 111.64, 105.14, 55.07, 49.05, 30.03, 16.88, 13.35 ppm; HRMS (ESI): m/z $[M+H]^+$ .$C_{21}H_{24}N_5O_3S$ calculated value 426.1594, measured value 426.1464.

Example 33

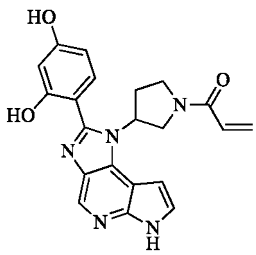

**LXS33**

1-(3-(2-(2,4-Dihydroxyphenyl)imidazo[4,5-d]pyrrolo[2,3-b]pyridin-1(6H)-yl)pyrrolidin-1 -yl)prop-2-en-1 -one

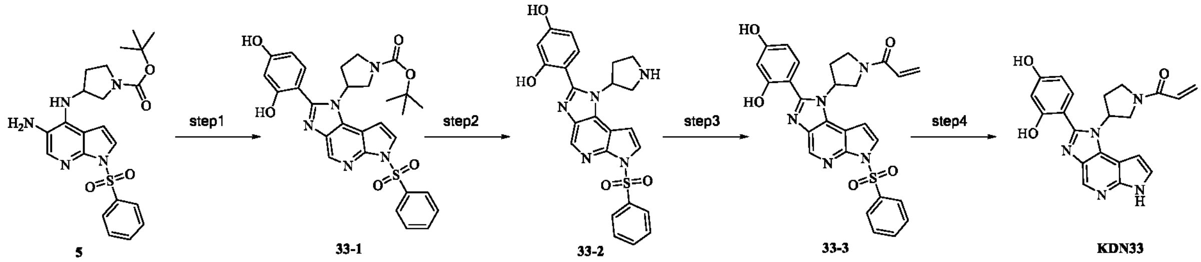


5 33-1 33-2 33-3 KDN33

Step 1: Dissolve tert-butyl 3-(((5-amino-1-(phenylsulfonyl)-1H-pyrrolo[2,3-b]pyridin-4-yl)amino)pyrrolidine-1-carboxylate (0.3 g, 0.7 mmol) in 10 mL of DMF, add $Na_2S_2O_5$ (0.7 g, 3.5 mmol) followed by adding 2,4-dihydroxybenzaldehyde (0.2 g, 1.4 mmol). Stir for 12h at 90 °C. The reaction was monitored for completion by TLC. Saturated sodium bicarbonate was added until the reaction solution was weakly basic, and the organic phase was separated. The aqueous

phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(2-(2,4-dihydroxyphenyl)-6-(phenylsulfonyl)imidazo[4,5-d]pyrrolo[2,3-b]pyridin-1(6H)-yl)pyrrolidine-1-carboxylate as a yellow solid (0.3 g, 79%). HRMS (ESI): m/z $[M+H]^+$ .$C_{29}H_{30}N_5O_6S$ calculated value 576.1911, measured value 576.1918.

Step 2: Dissolve tert-butyl 3-(2-(2,4-dihydroxyphenyl)-6-(phenylsulfonyl)imidazo[4,5-d]pyrrolo[2,3-b]pyridin-1(6H)-yl)pyrrolidine-1-carboxylate (0.3 g, 0.5 mmol) in 10 mL dichloromethane, slowly add trifluoroacetic acid (0.6 g, 5.0 mmol) and stir at room temperature for 12 hours. The product was concentrated under vacuum to yield 4-(6-(phenylsulfonyl)-1-(pyrrolidin-3-yl)-1,6-dihydroimidazo[4,5-d]pyrrolo[2,3-b]pyridin-2-yl)benzene-1,3-diol as a light yellow oil (0.2 g, 81%). The product can be used directly in the next step without further purification. HRMS (ESI): m/z $[M+H]^+$ .$C_{24}H_{22}N_5O_4S$ calculated value 476.1387, measured value 476.1375.

Step 3: Dissolve 4-(6-(phenylsulfonyl)-1-(pyrrolidin-3-yl)-1,6-dihydroimidazo[4,5-d]pyrrolo[2,3-b]pyridin-2-yl)benzene-1,3-diol (0.2 g, 0.4 mmol) in 10 mL of tetrahydrofuran, add DIPEA (0.1 g, 0.8 mmol) followed by slow dropwise addition of acryloyl chloride (0.1 g, 0.6 mmol). Stir at room temperature for 3h. The reaction was monitored by TLC for completion. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 1-(3-(2-(2,4-dihydroxyphenyl)-6-(phenylsulfonyl)imidazo[4,5-d]pyrrolo[2,3-b]pyridin-1(6H)-yl)pyrrolidin-1-yl)prop-2-en-1-one as a pale yellow oil (0.2 g, 90%). The product can be used directly in the next step without further purification. HRMS (ESI): m/z $[M+H]^+$ .$C_{27}H_{24}N_5O_5S$ calculated value 530.1493, measured value 530.1488.

Step 4: Dissolve 1-(3-(2-(2,4-dihydroxyphenyl)-6-(phenylsulfonyl)imidazo[4,5-d]pyrrolo[2,3-b]pyridin-1(6H)-yl)pyrrolidin-1-yl)prop-2-en-1-one (0.2 g, 0.4 mmol) in a mixture of 5 mL of tetrahydrofuran and 5 mL of methanol, add 5 mL of 1 M sodium hydroxide. Then, stir the mixture for 5 h at room temperature, the reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS33**: 1-(3-(2-(2,4-Dihydroxyphenyl)imidazo[4,5-d]pyrrolo[2,3-b]pyridin-1(6H)-yl)pyrrolidin-1-yl)prop-2-en-1-one (0.1 g, 68%). $^1$HNMR (300 MHz, *DMSO-$d_6$*): $\delta$ = 11.96 (s, 1H), 8.62 (s, 1H), 7.52 (d, *J* = 6.0 Hz, 1H), 7.26 (d, *J* = 6.0 Hz, 1H), 6.55 (s, 1H), 6.42 (d, *J* = 9.0 Hz, 1H), 4.03 -4.10 (m, 4H), 3.55-3.66 (m, 3H), 2.68-2.79 (m, 1H), 2.32 (s, 1H), 2.02 (s, 4H), 1.21 (t, *J* = 6.0 Hz, 4H) ppm; $^{13}$C NMR (75 MHz, *DMSO-$d_6$*) $\delta$ 166.39, 159.94, 156.62, 153.78, 148.64, 142.18, 131.16, 130.11, 129.05, 127.14, 126.89, 120.74, 115.69, 110.95, 109.05, 105.64, 99.38, 57.77, 52.05, 46.69, 27.17 ppm; HRMS ( ESI): m/z $[M+H]^+$ .$C_{21}H_{20}N_5O_3$ calculated value 390.1561, measured value 390.1692.

Example 34

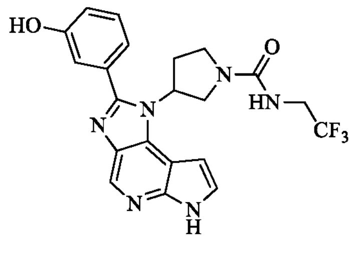

**LXS34**

3-(2-(3-Hydroxyphenyl)imidazo[4,5-d]pyrrolo[2,3-b]pyridin-1(6H)-yl)-N-(2,2,2-trifluoroethyl)pyrrolidine-1-carboxamide

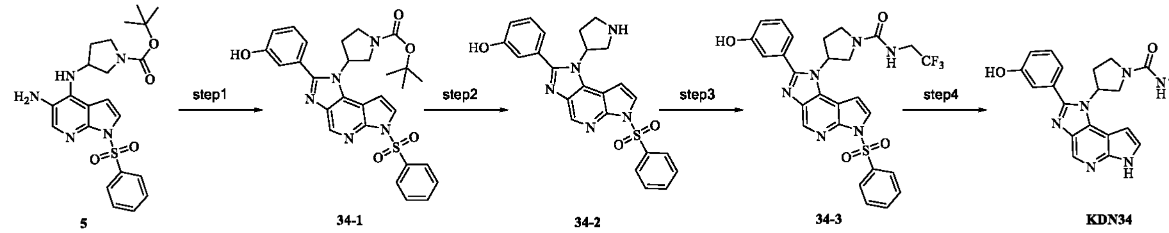

5 34-1 34-2 34-3 KDN34

Step 1: Dissolve tert-butyl 3-(((5-amino-1-(phenylsulfonyl)-1H-pyrrolo[2,3-b]pyridin-4-yl)amino)pyrrolidine-1-carboxylate (0.3 g, 0.7 mmol) in 10 mL of DMF, add $Na_2S_2O_5$ (0.7 g, 3.5 mmol) followed by adding 3-hydroxybenzaldehyde (0.2 g, 1.4 mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate= 1:1) to yield tert-butyl 3-(2-(3-hydroxyphenyl)-6-(phenylsulfonyl)imidazo[4,5-d]pyrrolo[2,3-b]pyridin-1(6H)-yl)pyrrolidine-1-carboxylate as a yellow solid ( 0.3 g, 82%). HRMS (ESI): m/z $[M+H]^+$ .$C_{29}H_{30}N_5O_5S$ calculated value 560.1962, measured value 560.1977.

Step 2: Dissolve tert-butyl 3-(2-(3-hydroxyphenyl)-6-(phenylsulfonyl)imidazo[4,5-d]pyrrolo[2,3-b]pyridin-1(6H)-yl)pyrrolidine-1-carboxylate (0.3 g, 0.5 mmol) in 10 mL of dichloromethane, slowly add trifluoroacetic acid (0.6 g, 5.0 mmol) and then stir at room temperature for 12 h. The product was concentrated under vacuum to yield 3-(6-(phenylsulfonyl)-1-(pyrrolidin-3-yl)-1,6-dihydroimidazo[4,5-d]pyrrolo[2,3-b]pyridin-2-yl) phenol as a light yellow oil (0.2 g, 81%). The product can be used directly in the next step without further purification. HRMS (ESI): m/z $[M+H]^+$ .$C_{24}H_{22}N_5O_3S$ calculated value 460.1438, measured value 460.1441.

Step 3: Dissolve (R)-2-(1H-imidazol-2-yl)-6-(phenylsulfonyl)-1-(pyrrolidin-3-yl)-1,6-dihydroimidazo[4,5-d]pyrrolo[2,3-b]pyridine (0.2 g, 0.4 mmol) in 10 mL of tetrahydrofuran, add carbonyl diimidazole (0.2 g, 1.0 mmol) and then slowly add trifluoromethyl ethylamine (0.2 g, 1.0 mmol) dropwise. Then, stir the mixture at room temperature for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(2-(3-hydroxyphenyl)-6-(phenylsulfonyl)imidazo[4,5-d]pyrrolo[2,3-b]pyridin-1(6H)-yl)-N-(2,2,2-trifluoroethyl)pyrrolidine-1-carboxamide as a pale yellow oil (0.2 g, 79%). The product can be used directly in the next step without further purification. HRMS (ESI): m/z $[M+H]^+$ .$C_{27}H_{24}F_3N_6O_4S$ calculated value 585.1526, measured value 585.1531.

Step 4: Dissolve 3-(2-(3-hydroxyphenyl)-6-(phenylsulfonyl)imidazo[4,5-d]pyrrolo[2,3-b]pyridin-1(6H)-yl)-N-(2,2,2-trifluoroethyl)pyrrolidine-1-carboxamide (0.2 g, 0.3 mmol) in a mixture of 5 mL of tetrahydrofuran and 5 mL of methanol, followed by adding 5 mL of 1M sodium hydroxide. Stir for 5 h at room temperature. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS34**: 3-(2-(3-hydroxyphenyl)imidazo[4,5-d]pyrrolo[2,3-b]pyridin-1(6H)-yl)-N-(2,2,2-trifluoroethyl)pyrrolidine-1 -carboxamide (0.1 g, 66%). $^1$HNMR (300 MHz, *DMSO-$d_6$* ): $\delta$= 12.04 (s, 1H), 8.69 (s, 1H), 7.56 (t, *J* = 3.0 Hz, 1H), 7.43 (t, *J* = 7.5 Hz, 1H), 7.15 (t, *J* = 4.5 Hz, 2H), 6.99-7.08 (m, 2H), 6.43 (d, *J* = 3.0 Hz, 1H), 5.46 (t, *J* = 9.0 Hz, 1H), 3.86-3.98 (m, 6H), 2.71-2.78 (m, 1H), 2.30-2.41 (m, 1H), 1.26 (s, 1H) ppm; $^{13}$C NMR (75 MHz, DMSO- $d_6$) $\delta$158.03, 156.37, 152.81, 152.40, 144.80, 136.37, 135.20, 132.31, 131.66, 130.50, 125.03, 120.61, 117.57, 117.33, 116.78, 111.67, 105.18, 98.76, 91.18, 76.29, 73.64, 73.34, 67.45, 30.10 ppm; HRMS (ESI): m/z $[M+H]^+$ .$C_{21}H_{20}F_3N_6O_2$ calculated value 445.1594, measured value 445.1459.

Example 35

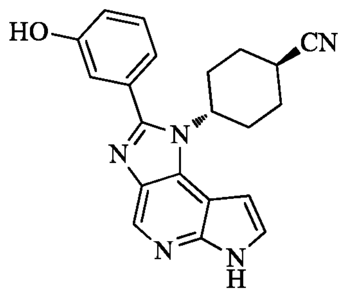

LXS35

trans-4-(2-(3-Hydroxyphenyl)imidazo[4,5-d]pyrrolo[2,3-b]pyridin-1(6H)-yl)cyclohexanecarbonitrile

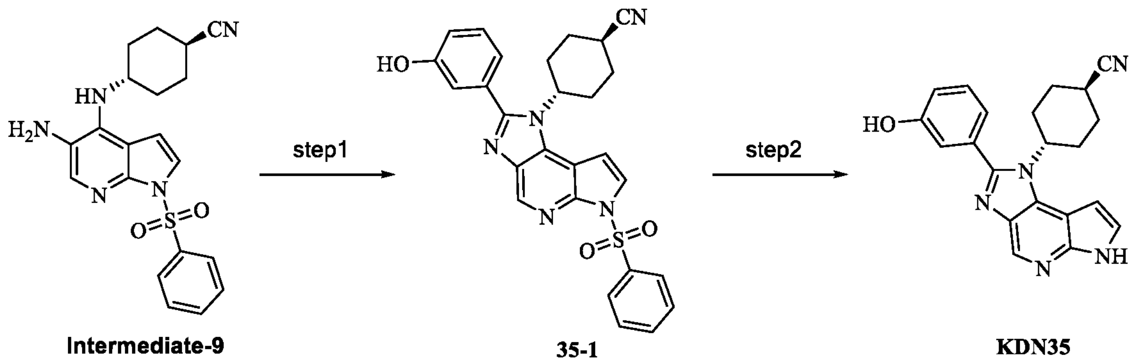


Intermediate-9 35-1 KDN35

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by adding 3-hydroxybenzaldehyde (0.2 g, 1.6 mmol) dropwise, then raise the temperature to 90°C and stir for 12 hours. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(3-hydroxyphenyl)-6-(phenylsulfonyl)imidazo[4,5-d]pyrrolo[2,3-b]pyridin-1(6H)-yl)cyclohexanecarbonitrile as a yellow oil (0.3 g, 79%). HRMS (ESI): m/z $[M+H]^+$ . $C_{27}H_{24}N_5O_3S$ calculated value 498.1594, measured value 498.1588.

Step 2: Dissolve trans-4-(2-(3-hydroxyphenyl)-6-(phenylsulfonyl)imidazo[4,5-d]pyrrolo[2,3-b]pyridin-1(6H)-yl)cyclohexanecarbonitrile (0.3 g, 0.6 mmol) in a solvent mixture of 5 mL tetrahydrofuran and 5 mL methanol. Then add 5 mL of 1 M sodium hydroxideand stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS35:** trans-4-(2-(3-Hydroxyphenyl)imidazo[4,5-d]pyrrolo[2,3-b]pyridin-1(6H)-yl)cyclohexanecarbonitrile (0.1 g, 46%). $^1$HNMR (300 MHz, *DMSO-$d_6$): $\delta$* = 11.98 (s, 1H), 9.86 (s, 1H), 8.64 (s, 1H), 7.54 (t, *J* = 3.0 Hz, 1H), 7.41 (t, *J* = 7.5 Hz, 2H), 7.10 (t, *J* = 4.5 Hz, 2H), 6.98-7.01 (m, 1H), 6.87 (s, 1H), 4.48-4.56 (m, 1H), 3.21 (s, 1H), 3.19 (s, 1H), 2.41 (t, *J* = 12.0 Hz, 2H), 2.26 (d, *J* = 12.0 Hz, 2H), 2.02 (t, *J* = 4.5 Hz, 2H), 1.67-1.79 (m, 2H) ppm;$^{13}$ C NMR (75 MHz, *DMSO-$d_6$) $\delta$* 157.90, 152.05, 144.92, 136.19, 135.11, 132.69, 132.29, 130.35, 124.39, 123.15, 120.52, 117.15, 116.76, 104.60, 100.64, 55.37, 54.78, 49.05, 29.34, 28.75, 26.27 ppm; HRMS (ESI): m/z $[M+H]^+$ .$C_{21}H_{20}N_5O$ calculated value 358.1662, measured value 358.1655.

Example 36

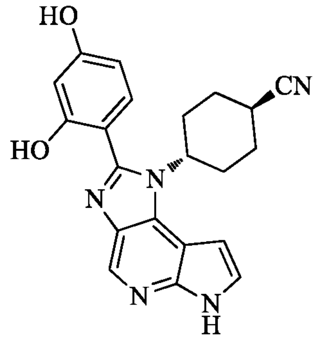

**LXS36**

trans-4-(2-(2,4-Dihydroxyphenyl)imidazo[4,5-d]pyrrolo[2,3-b]pyridin-1(6H)-yl)cyclohexanecarbonitrile

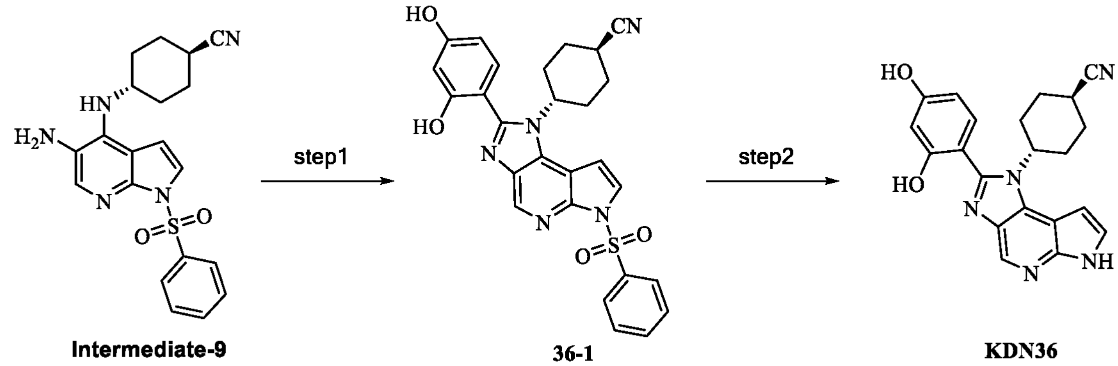


**Intermediate-9** **36-1** **KDN36**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g,4 mmol) followed by adding 2,4-dihydroxybenzaldehyde (0.2 g, 1.6 mmol) dropwise. Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield trans-4-(2-(2,4-dihydroxyphenyl)-6-(phenylsulfonyl)imidazo[4,5-d]pyrrolo[2,3-b]pyridin-1(6H)-yl)cyclohexanecarbonitrile as a yellow oil (0.3 g, 77%). HRMS (ESI): m/z $[M+H]^+$ . $C_{27}H_{24}N_5O_4S$ calculated value 514.1544, measured value 514.1560.

Step 2: Trans-4-(2-(2,4-dihydroxyphenyl)-6-(phenylsulfonyl)imidazo[4,5-d]pyrrolo[2,3-b]pyridin-1(6H)-yl)cyclohexanecarbonitrile (0.3 g, 0.6 mmol) was dissolved in a mixture of 5 mL of tetrahydrofuran and 5 mL of methanol, and then stirred at room temperature for 5 h after the addition of 5 mL of 1M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS36**: trans-4-(2-(2,4-Dihydroxyphenyl)imidazo[4,5-d]pyrrolo[2,3-b]pyridin-1(6H)-yl)cyclohexanecarbonitrile (0.1 g, 46%). .$^1$ HNMR (300 MHz, *DMSO-$d_6$ ):* $\delta$ = 11.92 (s, 1H), 8.58 (s, 1H), 7.49 (s, 1H), 7.19 (d, *J* = 9.0 Hz, 1H), 6.84 (s, 1H), 6.58 (s, 1H), 6.41-6.44 (m, 1H), 4.23 (d, *J = 9.0 Hz, 1H),* 3.14 (t, *J* = 12.0 Hz, 1H), 2.79 (d, *J* = 18.0 Hz, 1H), 2.36 (d, *J*= 9.0 Hz, 2H), 2.26 (d, *J* = 15.0 Hz, 2H), 2.02 (s, 1H), 1.62-1.74 (m, 2H), 1.18-1.26 (m, 2H) ppm;$^{13}$ C NMR (75 MHz, *DMSO-$d_6$ )* $\delta$ 160.50, 157.73, 150.96, 144.77, 135.36, 133.04, 132.21, 124.09, 123.21, 109.32, 107.49, 104.42, 85.32, 73.67, 60.22, 54.89, 29.20, 28.95, 26.39, 14.55 ppm; HRMS (ESI): m/z $[M+H]^+$ .$C_{21}H_{20}N_5O_2$ calculated value 374.1612, measured value 374.1600.

Example 37

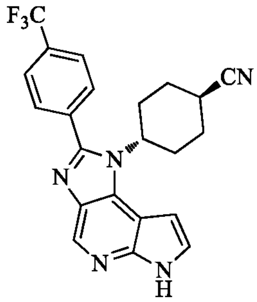

LXS37

trans-4-(2-(4-(Trifluoromethyl)phenyl)imidazo[4,5-d]pyrrolo[2,3-b]pyridin-1(6H)-yl)cyclohexanecarbonitrile

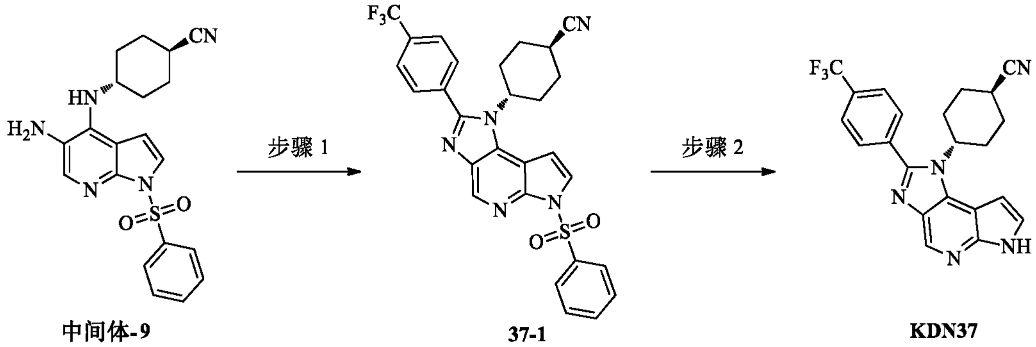


中间体-9 37-1 KDN37

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by adding 4-(trifluoromethyl)benzaldehyde (0.3 g, 1.6 mmol) dropwise. Stir for 12h at 90 °C. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(6-(phenylsulfonyl)-2-(4-(trifluoromethyl)phenyl)imidazo[4,5-d]pyrrolo[2,3-b]pyridin-1(6H)-yl) cyclohexanecarbonitrile as a yellow oil (0.3 g, 72%). HRMS (ESI): m/z $[M+H]^+$.$C_{28}H_{23}F_3N_5O_2S$ calculated value 550.1519, measured value 550.1521.

Step 2: Trans-4-(6-(phenylsulfonyl)-2-(4-(trifluoromethyl)phenyl)imidazo[4,5-d]pyrrolo[2,3-b]pyridin-1(6H)-yl) cyclohexanecarbonitrile (0.3 g, 0.5 mmol) was dissolved in a mixture of 5 mL of tetrahydrofuran and 5 mL of methanol, 5 mL of 1 M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield LXS37: trans-4-(2-(4-(trifluoromethyl)phenyl)imidazo[4,5-d]pyrrolo[2,3-b]pyridin-1(6H)-yl)cyclohexanecarbonitrile (0.1 g, 45%). ).$^1$HNMR (300 MHz, *DMSO-$d_6$):* $\delta$ = 12.04 (s, 1H), 8.68 (s, 1H), 7.97 (d, $J$ = 3.0 Hz, 4H), 7.56 (t, $J$ = 3.0 Hz, 1H), 6.89 (s, 1H), 4.45-4.53 (m, 1H), 3.17 (t, $J$ = 13.5 Hz, 1H ), 2.40 (t, $J$ = 10.5 Hz, 2H), 2.23 (d, $J$ = 12.0 Hz, 2H), 2.03 (t, $J$ = 10.5 Hz, 2H), 1.74-1.86 (m, 2H), 1.25 (s, 1H) ppm;$^{13}$C NMR (75 MHz, *DMSO-$d_6$ )* $\delta$ 150.58, 145.02, 136.44, 135.27, 132.89, 131.00, 130.63, 129.90, 126.38, 126.10, 124.55, 123.21, 104.58, 100.85, 72.19, 54.92, 29.32, 28.50, 26.22 ppm; HRMS (ESI): m/z $[M+H]^+$ .$C_{22}H_{19}F_3N_5$ calculated value 410.1587, measured value 410.1590.

Example 38

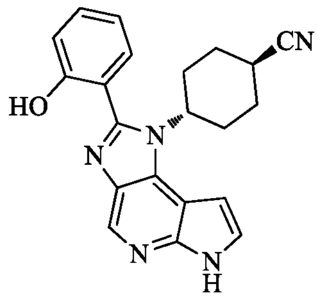

**LXS38**

trans-4-(2-(2-Hydroxyphenyl)imidazo[4,5-d]pyrrolo[2,3-b]pyridin-1(6H)-yl)cyclohexanecarbonitrile

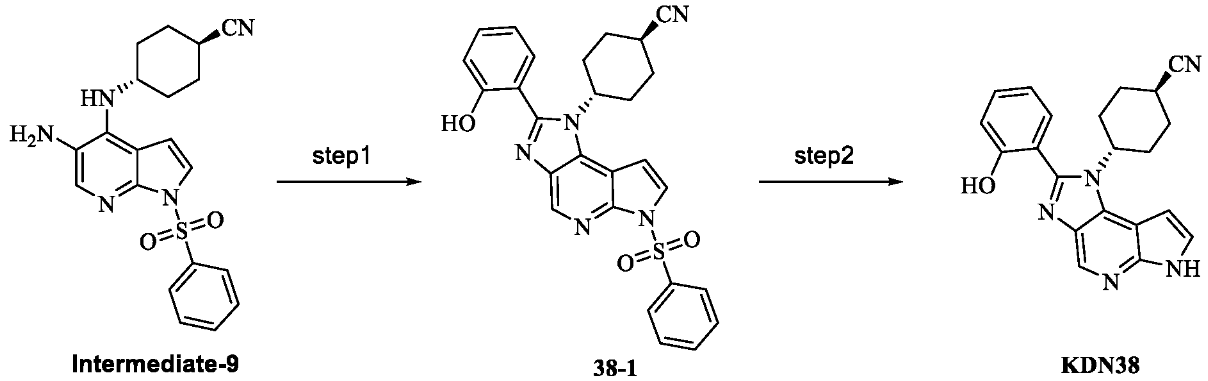


**Intermediate-9** **38-1** **KDN38**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by adding 2-hydroxybenzaldehyde (0.2 g, 1.6 mmol) dropwise, raise the temperature to 90°C and stir for 12 hours. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(2-hydroxyphenyl)-6-(phenylsulfonyl)imidazo[4,5-d]pyrrolo[2,3-b]pyridin-1(6H)-yl)cyclohexanecarbonitrile as a yellow oil (0.3 g, 79%). HRMS (ESI): m/z $[M+H]^+$ . $C_{27}H_{24}N_5O_3S$ calculated value 498.1594, measured value 498.1600.

Step 2: Dissolve trans-4-(2-(2-hydroxyphenyl)-6-(phenylsulfonyl)imidazo[4,5-d]pyrrolo[2,3-b]pyridin-1(6H)-yl)cyclohexanecarbonitrile (0.3 g, 0.6 mmol) in a solvent mixture of 5 mL tetrahydrofuran and 5 mL methanol, add 5 mL of 1 M sodium hydroxide and stir at room temperature for 5 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate= 1:1) to yield **LXS38**: trans-4-(2-(2-Hydroxyphenyl)imidazo[4,5-d]pyrrolo[2,3-b]pyridin-1(6H)-yl)cyclohexanecarbonitrile (0.1 g, 46%). $^1$HNMR (300 MHz, *DMSO-$d_6$* ): $\delta$= 11.94 (s, 1H), 10.17 (s, 1H), 8.62 (s, 1H), 7.52 (t, *J* = 3.0 Hz, 1H), 7.40-7.46 (m, 2H), 6.98-7.08 (m, 2H), 6.87 (d, *J* = 3.0 Hz, 1H), 4.18-4.26 (m, 1H), 3.14 (t, *J* = 12.0 Hz, 1H), 2.38 (t, *J* = 12.0 Hz, 2H), 2.26 (d, *J* = 10.5 Hz, 2H), 1.60-1.72 (m, 2H), 1.18-1.26 (m, 2H) ppm; $^{13}$CNMR(75 MHz, *DMSO-$d_6$ )* $\delta$156.49, 150.28, 144.85, 135.95, 135.45, 132.47, 132.24, 131.75, 124.19, 123.16, 119.68, 118.54, 116.23, 104.41, 100.53, 72.30, 55.03, 29.22, 28.89, 26.37 ppm; HRMS (ESI): m/z $[M+H]^+$ .$C_{21}H_{20}N_5O$ calculated value 358.1662, measured value 358.1658.

Example 39

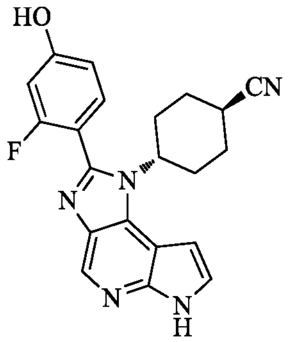

**LXS39**

trans-4-(2-(2-Fluoro-4-hydroxyphenyl)imidazo[4,5-d]pyrrolo[2,3-b]pyridin-1(6H)-yl)cyclohexanecarbonitrile

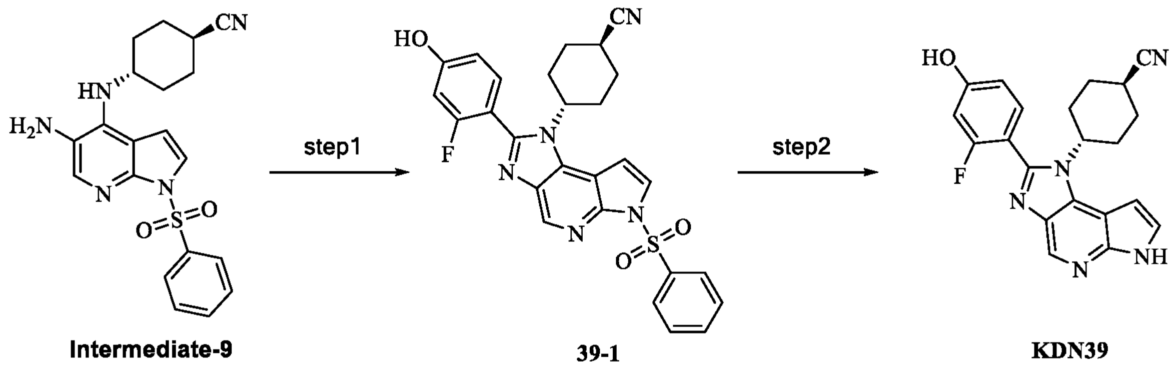


**Intermediate-9** **39-1** **KDN39**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 2-fluoro-4-hydroxybenzaldehyde (0.2 g, 1.6 mmol) dropwise and then stir for 12 h at 90°C. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(2-fluoro-4-hydroxyphenyl)-6-(phenylsulfonyl)imidazo[4,5-d]pyrrolo[2,3-b]pyridin-1(6H)-yl) cyclohexanecarbonitrile as a pale yellow oil (0.3 g, 77%). HRMS (ESI): m/z $[M+H]^+$ .$C_{27}H_{23}FN_5O_3S$ calculated value 516.1500, measured value 516.1511.

Step 2: Dissolve trans-4-(2-(2-fluoro-4-hydroxyphenyl)-6-(phenylsulfonyl)imidazo[4,5-d]pyrrolo[2,3-b]pyridin-1(6H)-yl ) cyclohexanecarbonitrile (0.3 g, 0.6 mmol) in a mixture of 5 mL of tetrahydrofuran and 5 mL of methanol, add 5 mL of 1 M sodium hydroxide and then stir for 5 h at room temperature. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS39**: trans-4-(2-(2-Fluoro-4-hydroxyphenyl)imidazo[4,5-d]pyrrolo[2,3-b]pyridin-1(6H)-yl)cyclohexanecarbonitrile (0.1 g, 46%). ).$^1$ HNMR (300 MHz, *DMSO-d6):* $\delta$ = 11.99 (s, 1H), 8.63 (s, 1H), 7.54 (t, *J* = 3.0 Hz, 1H), 7.43-7.49 (m, 1H), 6.88 (d, *J* = 3.0 Hz, 1H), 6.82-6.85 (m, 1H), 6.80 (d, *J* = 3.0 Hz, 1H), 4.24 (d, *J* = 12.0 Hz, 1H), 3.13 (t, *J* = 13.5 Hz, 1H), 2.34 (d, *J* = 12.0 Hz, 2H), 2.23 (d, *J* = 12.0 Hz, 2H), 2.02 (s, 1H), 1.94 (d, *J* = 9.0 Hz, 2H), 1.70-1.82 (m, 2H), 1.18-1.26 (m, 1H) ppm;$^{13}$ C NMR (75 MHz, *DMSO-$d_6$)* $\delta$ 160.11, 159.75, 153.77, 148.62, 142.15, 130.54, 129.04, 127.16, 122.75, 120.77, 116.16, 115.64, 112.04, 104.54, 99.38, 65.93, 30.88, 26.91, 23.69 ppm; HRMS (ESI): m/z $[M+H]^+$ .$C_{21}H_{19}FN_5O$ calculated value 376.1568, measured value 376.1562.

Example 40

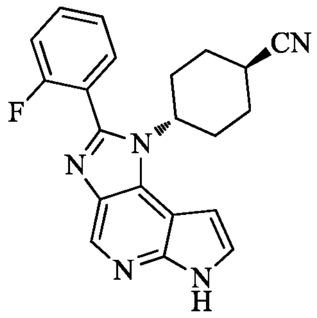

**LXS40**

trans-4-(2-(2-Fluorophenyl)imidazo[4,5-d]pyrrolo[2,3-b]pyridin-1(6H)-yl)cyclohexanecarbonitrile

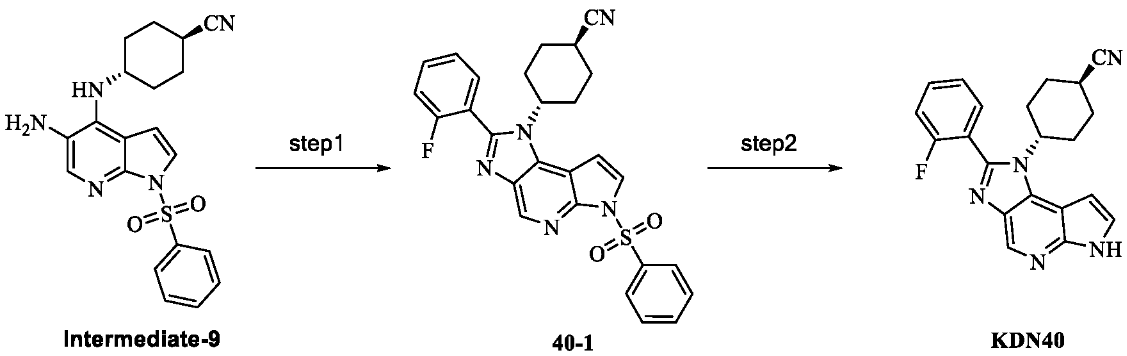


**Intermediate-9** **40-1** **KDN40**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 2-fluorobenzaldehyde (0.2 g, 1.6 mmol) dropwise, raise the temperature to 90 °C and stir for 12 hours. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield trans-4-(2-(2-fluorophenyl)-6-(phenylsulfonyl)imidazo[4,5-d]pyrrolo[2,3-b]pyridin-1(6H)-yl)cyclohexanecarbonitrile as a pale yellow oil (0.3 g, 79%). HRMS (ESI): m/z $[M+H]^+$ . $C_{27}H_{23}FN_5O_2S$ calculated value 500.1551, measured value 500.1563.

Step 2: Dissolve trans-4-(2-(2-fluorophenyl)-6-(phenylsulfonyl)imidazo[4,5-d]pyrrolo[2,3-b]pyridin-1(6H)-yl ) cyclohexanecarbonitrile (0.3 g, 0.6 mmol) in a solvent mixture of 5 mL tetrahydrofuran and 5 mL methanol, add 5 mL of 1 M sodium hydroxide and then stir at room temperature for 5 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate= 1:1) to yield **LXS40**: trans-4-(2-(2-Fluorophenyl)imidazo[4,5-d]pyrrolo[2,3-b]pyridin-1(6H)-yl)cyclohexanecarbonitrile (0.1 g, 46%). $^1$HNMR (300 MHz, *DMSO-$d_6$ ):* $\delta$ = 12.06 (s, 1H), 8.70 (s, 1H), 7.70 (t, *J* = 6.0 Hz, 2H), 7.57 (s, 1H), 7.49 (t, *J* = 9.0 Hz, 2H), 6.91 (s, 1H), 4.27 (s, 1H), 3.13 (s, 1H), 2.35 (d, *J* = 12.0 Hz, 2H), 2.24 (d, *J* = 9.0 Hz, 2H), 2.01 (d, *J* = 6.0 Hz, 2H), 1.75 (d, *J* = 12.0 Hz, 2H) ppm;$^{13}$ C NMR (75 MHz, *DMSO-$d_6$)* $\delta$ 162.07, 158.80, 146.49, 145.00, 136.27, 135.41, 133.08, 132.92, 132.42, 125.44, 124.54, 123.11, 119.40, 119.19, 116.62, 116.34, 104.40, 100.51, 60.22, 55.18, 29.42, 28.59, 26.29, 14.51 ppm; HRMS (ESI): m/z $[M+H]^+$ .$C_{21}H_{19}FN_5$ calculated value 360.1619, measured value 360.1615.

Example 41

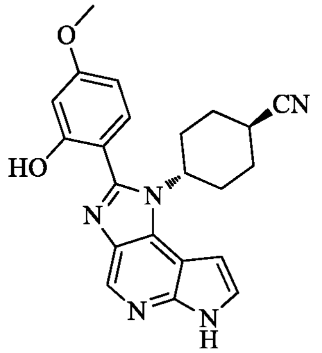

**LXS41**

trans-4-(2-(2-Hydroxy-4-methoxyphenyl)imidazo[4,5-d]pyrrolo[2,3-b]pyridin-1(6H)-yl)cyclohexanecarbonitrile

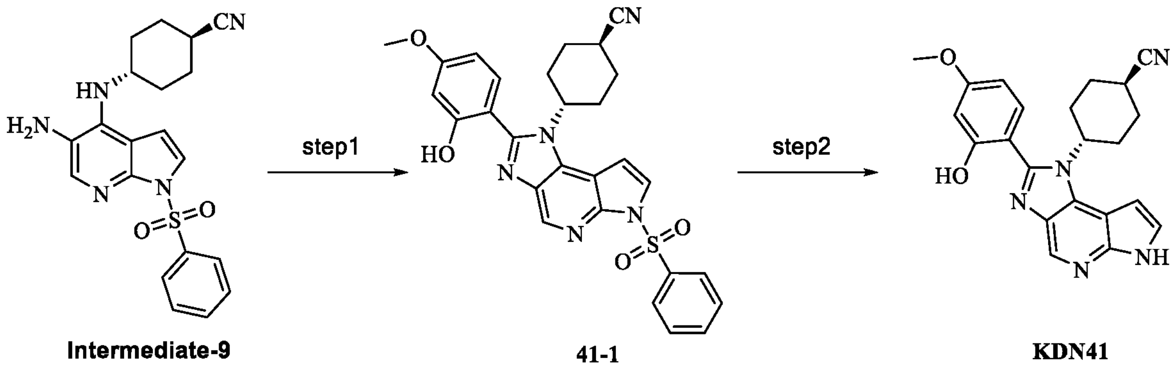


**Intermediate-9** **41-1** **KDN41**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by adding 2-fluoro 4-methoxybenzaldehyde (0.2 g, 1.6 mmol) dropwise, and then stir for 12 h at 90 °C. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(2-hydroxy-4-methoxyphenyl)-6-(phenylsulfonyl)imidazo[4,5-d]pyrrolo[2,3-b]pyridin-1(6H)-yl )cyclohexanecarbonitrile as a yellow oil (0.3 g, 75%). HRMS (ESI): m/z $[M+H]^+$ .$C_{28}H_{26}N_5O_4S$ calculated value 528.1700, measured value 528.1711.

Step 2: Trans-4-(2-(2-hydroxy-4-methoxyphenyl)-6-(phenylsulfonyl)imidazo[4,5-d]pyrrolo[2,3-b]pyridin-1(6H)-yl) cyclohexanecarbonitrile (0.3 g, 0.6 mmol) was dissolved in a mixture of 5 mL of tetrahydrofuran and 5 mL of methanol, and then stirred at room temperature for 5 h after the addition of 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS41:** trans-4-(2-(2-hydroxy-4-methoxyphenyl)imidazo[4,5-d]pyrrolo[2,3-b]pyridin-1(6H)-yl)cyclohexanecarbonitrile (0.1 g, 46%). 46%).$^1$ HNMR (300 MHz, *DMSO-d$_6$* ): $\delta$ = 11.91 (s, 1H), 10.26 (s, 1H), 8.60 (s, 1H), 7.51 (t, *J* = 3.0 Hz, 1H), 7.34 (d, *J* = 9.0 Hz, 1H), 6.86 (d, *J* = 3.0 Hz, 1H), 6.58-6.61 (m , 2H), 4.24 (t, *J* = 13.5 Hz, 1H), 3.83 (s, 3H), 3.15 (t, *J* = 12.0 Hz, 1H), 2.39 (t, *J* = 12.0 Hz, 2H), 2.25 (d, *J* = 12.0 Hz, 2H), 2.01 (d, *J* = 12.0 Hz, 2H), 1.62-1.74 (m, 2H ) ppm;$^{13}$ C NMR (75 MHz, *DMSO-d$_6$ )* $\delta$162.03, 157.72, 150.38, 144.83, 135.83, 135.37, 133.21, 132.24, 124.14, 123.19, 111.08, 105.80, 104.42, 101.63, 55.60, 54.94, 29.20, 28.92, 26.38 ppm; HRMS (ESI): m/z $[M+H]^+$ .$C_{22}H_{22}N_5O_2$ calculated

value 388.1768, measured value 366.1782.

Example 42

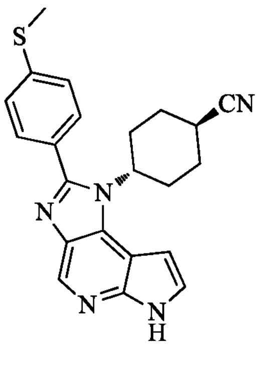

LXS42

trans-4-(2-(4-(Methylthio)phenyl)imidazo[4,5-d]pyrrolo[2,3-b]pyridin-1(6H)-yl)cyclohexanecarbonitrile

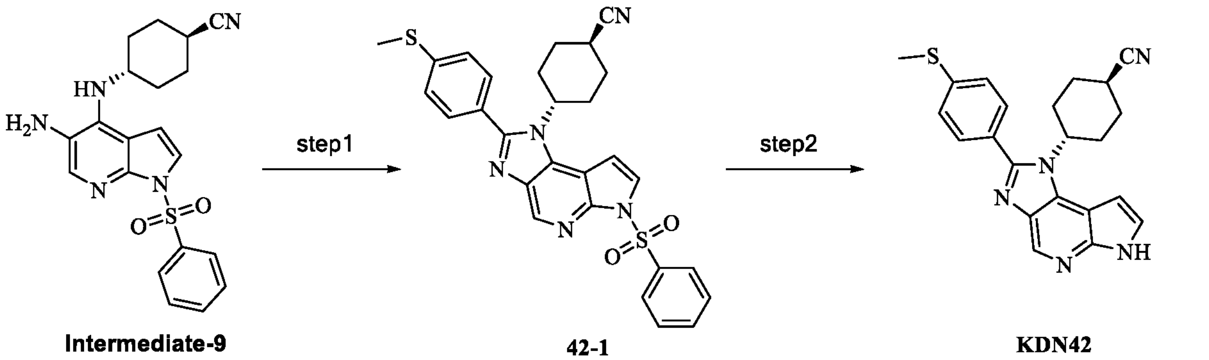


Intermediate-9 42-1 KDN42

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by adding 4-(methylthio)benzaldehyde (0.2 g, 1.6 mmol) dropwise. Stir for 12h at 90 °C. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(4-(methylthio)phenyl)-6-(phenylsulfonyl)imidazo[4,5-d]pyrrolo[2,3-b]pyridin-1(6H)-yl) cyclohexanecarbonitrile as a yellow oil (0.3 g, 75%). HRMS (ESI): m/z $[M+H]^+$ . $C_{28}H_{26}N_5O_2S_2$ calculated value 528.1522, measured value 528.1533.

Step 2: Trans-4-(2-(4-(methylthio)phenyl)-6-(phenylsulfonyl)imidazo[4,5-d]pyrrolo[2,3-b]pyridin-1(6H)-yl) cyclohexanecarbonitrile (0.3 g, 0.6 mmol) was dissolved in a mixture of 5 mL of tetrahydrofuran and 5 mL of methanol, 5 mL of 1 M sodium hydroxidewas added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS42**: trans-4-(2-(4-(methylthio)phenyl)imidazo[4,5-d]pyrrolo[2,3-b]pyridin-1(6H)-yl)cyclohexanecarbonitrile (0.1 g, 46%). .$^1$ HNMR (300 MHz, *DMSO-$d_6$):* $\delta$ = 11.98 (s, 1H), 8.65 (s, 1H), 7.66 (d, *J* = 9.0 Hz, 2H), 7.54 (t, *J* = 3.0 Hz, 1H), 7.48 (d, *J* = 9.0 Hz, 2H), 6.88 (d, *J* = 3.0 Hz, 1H), 4.49 (t, *J* = 13.5 Hz, 1H), 3.17 (t, *J* = 12.0 Hz, 1H), 2.61 (s, 3H), 2.45 (t, *J* = 12.0 Hz, 2H), 2.25 (d, *J* = 12.0 Hz, 2H), 2.03 (d, *J* = 6.0 Hz, 2H), 1.71-1.83 (m, 2H) ppm;$^{13}$ C NMR ( 75 MHz, *DMSO-$d_6$)* $\delta$ 153.77, 148.43, 142.89, 139.43, 129.04, 127.77, 127.32, 127.17, 127.01, 122.74, 120.73, 115.64, 99.49, 65.93, 31.96, 26.94, 24.68, 14.88 ppm; HRMS (ESI): m/z $[M+H]^+$ .$C_{22}H_{22}N_5O_2$ calculated value 388.1590, measured value 388.1599.

Example 43

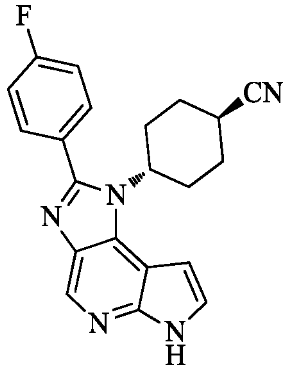

**LXS43**

trans-4-(2-(4-Fluorophenyl)imidazo[4,5-d]pyrrolo[2,3-b]pyridin-1(6H)-yl)cyclohexanecarbonitrile

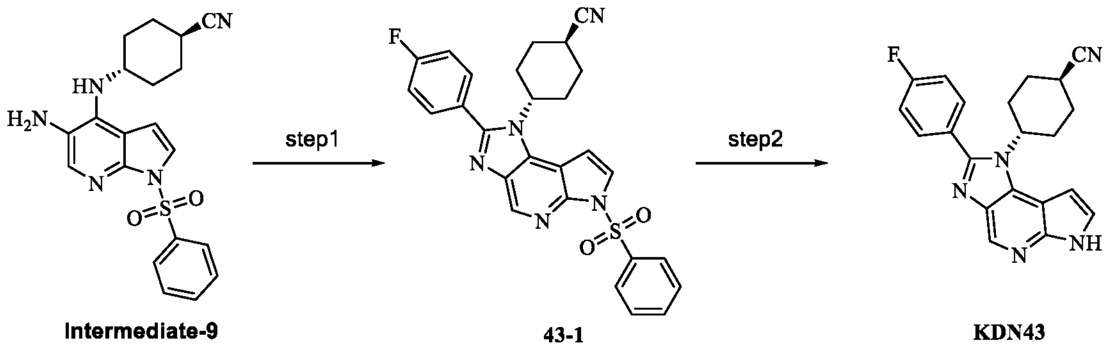


**Intermediate-9** **43-1** **KDN43**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 4-fluorobenzaldehyde (0.2 g, 1.6 mmol) dropwise, raise the temperature to 90 °C and stir for 12 hours. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield trans-4-(2-(4-fluorophenyl)-6-(phenylsulfonyl)imidazo[4,5-d]pyrrolo[2,3-b]pyridin-1(6H)-yl) cyclohexanecarbonitrile as a yellow oil (0.3 g, 79%). HRMS (ESI): m/z $[M+H]^+$ . $C_{27}H_{23}FN_5O_2S$ calculated value 500.1551, measured value 500.1559.

Step 2: Dissolve trans-4-(2-(4-fluorophenyl)-6-(phenylsulfonyl)imidazo[4,5-d]pyrrolo[2,3-b]pyridin-1(6H)-yl) cyclohexanecarbonitrile (0.3 g, 0.6 mmol) in a mixture of 5 mL of tetrahydrofuran and 5 mL of methanol, followed by the addition of 5 mL of 1M sodium hydroxide. Then, stir the mixture at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS43**: trans-4-(2-(4-Fluorophenyl)imidazo[4,5-d]pyrrolo[2,3-b]pyridin-1(6H)-yl)cyclohexanecarbonitrile (0.1 g, 46%). $^1$HNMR (300 MHz, *DMSO-$d_6$*): $\delta$= 11.99 (s, 1H), 8.65 (s, 1H), 7.74-7.79 (m, 2H), 7.54 (t, *J* = 3.0 Hz, 1H), 7.46 (t, *J* = 9.0 Hz, 2H), 6.88 (d, *J* = 3.0 Hz, 1H), 4.44 (t, *J* = 12.0 Hz, 1H), 3.15 (t, *J* = 12.0 Hz, 1H), 2.34-2.47 (m, 2H), 2.24 (d, *J* = 12.0 Hz, 2H), 2.04 (d, *J* = 9.0 Hz, 2H), 1.70-1.81 (m, 2H) ppm; $^{13}$C NMR (75 MHz, *DMSO-$d_6$*) $\delta$162.99 , 153.74, 148.65, 142.11, 129.19, 129.03, 127.43, 126.22, 122.74, 120.75, 116.43, 115.64, 99.40, 65.93, 30.82, 26.94, 21.54 ppm; HRMS (ESI): m/z $[M+H]^+$ . $C_{21}H_{19}FN_5$ calculated value 360.1619, measured value 360.1683.

Example 44

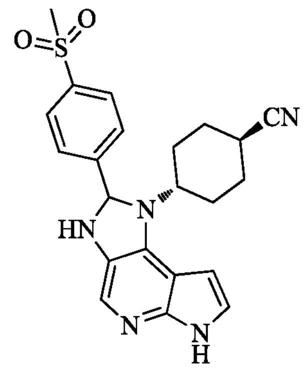

**LXS44**

trans-4-(2-(4-(Methylsulfonyl)phenyl)-2,3-dihydroimidazo[4,5-d]pyrrolo[2,3-b]pyridin-1 (6H)-yl)cyclohexanecarbonitrile

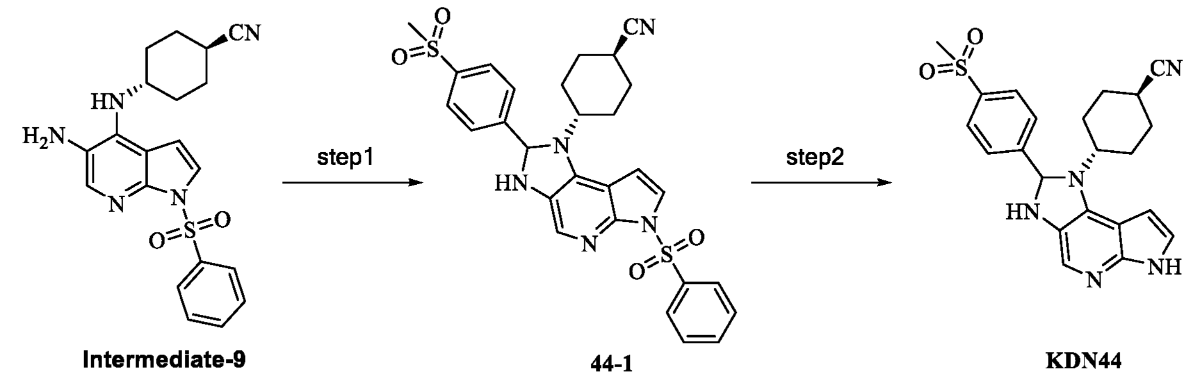


**Intermediate-9** **44-1** **KDN44**

Step 1: Dissolve trans-4-((5-amino-1-(phenylsulfonyl)-1H-pyrrolo[2,3-b]pyridin-4-yl)amino)cyclohexanecarbonitrile (0.3 g, 0.8 mmol) in 10 mL of DMF, add $Na_2S_2O_5$ (0.8 g, 4 mmol) followed by the addition of 4-methylsulfonylbenzaldehyde (0.3 g, 1.6 mmol) dropwise. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(4-(methylsulfonyl)phenyl)-6-(phenylsulfonyl)-2,3-dihydroimidazo[4,5-d]pyrrolo[2,3-b]pyridin-1(6H)-yl)cyclohexanecarbonitrile as a yellow oil (0.3 g, 70%). HRMS (ESI) : m/z $[M+H]^+$ .$C_{28}H_{28}N_5O_4S_2$ calculated value 562.1577, measured value 562.1588.

Step 2: Trans-4-(2-(4-(methylsulfonyl)phenyl)-6-(phenylsulfonyl)-2,3-dihydroimidazo[4,5-d]pyrrolo[2,3-b]pyridin-1(6H)-yl) cyclohexanecarbonitrile (0.3 g, 0.5 mmol) was dissolved in a solvent mixture of 5 mL of tetrahydrofuran and 5 mL of methanol, followed by the addition of 5 mL of 1 M sodium hydroxide. The mixture is then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS44**: trans-4-(2-(4-(Methylsulfonyl)phenyl)-2,3-dihydroimidazo[4,5-d]pyrrolo[2,3-b]pyridin-1(6H)-yl)cyclohexanecarbonitrile ( 0.2 g, 89%). $^{1}$HNMR (300 MHz, *DMSO-d*$_6$ ): $\delta$ = 11.40 (s, 1H), 8.93 (s, 1H), 8.22 (d, *J* = 9.0 Hz, 2H), 8.14 (s, 1H), 8.06 (d, *J* = 6.0 Hz, 2H), 7.22 (t, *J* = 3.0 Hz, 1H), 6.63 (d, *J* = 3.0 Hz, 1H), 6.07 (d, *J* = 9.0 Hz, 1H), 4.02-4.10 (m, 1H), 3.30 (s, 3H), 2.73-2.81 (m, 1H), 2.12 (d, *J* = 9.0 Hz, 2H), 2.03 (s, 2H), 1.76-1.87 (m, 2H), 1.44-1.55 (m, 2H) ppm; $^{13}$ C NMR (75 MHz, *DMSO-d*$_6$ *)* $\delta$ 144.28, 139.38, 138.74, 134.46, 131.18, 128.94, 128.11, 127.19, 123.38, 122.74, 108.17, 99.36, 83.56, 61.28, 47.74, 28.48, 26.94, 24.93 ppm; HRMS (ESI): m/z $[M+H]^+$ .$C_{22}H_{24}N_5O_2S$ calculated value 422.1645, measured value 422.1679.

Example 45

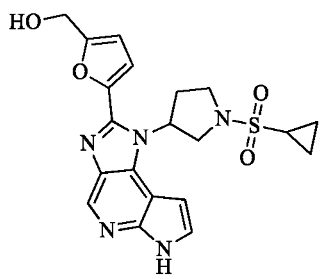

**LXS45**

(5-(1-(1-(cyclopropylsulfonyl)pyrrolidin-3-yl)-1,6-dihydroimidazo[4,5-d]pyrrolo[2,3-b]pyridin-2-yl)furan-2-yl)methanol

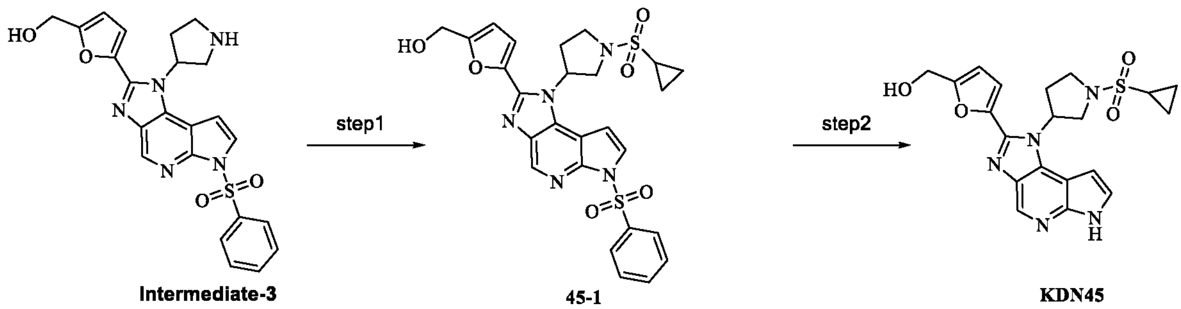


Intermediate-3 45-1 KDN45

Step 1: Dissolve 5-(6-(phenylsulfonyl)-1-(pyrrolidin-3-yl)-1,6-dihydroimidazo[4,5-d]pyrrolo[2,3-b]pyridin-2-yl)furan-2-yl) methanol (0.3 g, 0.6 mmol) in 10 mL of tetrahydrofuran, add DIPEA (0.2 g, 1.2 mmol) and then slowly add cyclopropylsulfonyl chloride (0.1 g, 0.9 mmol) dropwise. Then raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (5-(6-(phenylsulfonyl)-1-(1-(cyclopropylsulfonyl)pyrrolidin-3-yl)-1,6-dihydroimidazo[4,5-d]pyrrolo[2,3-b]pyridin-2-yl) furan-2-yl) methanol as a yellow oil (0.3 g, 82%). The product can be used directly in the next step without further purification. HRMS (ESI): m/z $[M+H]^+$ .$C_{26}H_{26}N_5O_6S_2$ calculated value 568.1319, measured value 568.1321.

Step 2: Dissolve (5-(6-(phenylsulfonyl)-1-(1-(cyclopropylsulfonyl)pyrrolidin-3-yl)-1,6-dihydroimidazo[4,5-d]pyrrolo[2,3-b]pyridin-2-yl) furan-2-yl) methanol (0.3 g, 0.5 mmol) in a solvent mixture of 5 mL of tetrahydrofuran and 5 mL of methanol, followed by the addition of 5 mL 1 M sodium hydroxide. Stir the mixture for 5 h at room temperature and the reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS45**: (5-(1-(1-(cyclopropylsulfonyl)pyrrolidin-3-yl)-1,6-dihydroimidazo[4,5-d]pyrrolo[2,3-b]pyridin-2-yl)furan-2-yl)methanol (0.1 g, 44%).$^1$ HNMR (300 MHz, *DMSO-$d_6$*): $\delta$ = 12.09 (s, 1H), 8.67 (s, 1H), 7.61 (t, $J$ = 3.0 Hz, 1H), 7.19 (d, $J$ = 3.0 Hz, 1H), 6.85 (s, 1H), 6.63 (d, $J$ = 3.0 Hz, 1H), 5.80-5.92 (m, 1H), 5.51 (t, $J$ = 6.0 Hz, 1H), 4.58 (d, $J$ = 6.0 Hz, 2H), 4.04-4.10 (m, 1H), 3.81-3.90 (m, 2H), 3.58-3.67 (m, 1H), 2.89-2.97 (m, 1H), 2.71-2.80 (m, 2H), 1.26 (s, 1H), 1.06 (t, $J$ = 6.0 Hz, 4H) ppm;$^{13}$ C NMR (75 MHz, *DMSO-$d_6$)* $\delta$ 153.88, 151.18, 148.60, 144.53, 142.17, 129.04, 127.16, 120.88, 115.63, 107.95, 104.05, 99.38, 57.39, 56.43, 54.43, 49.75, 37.55, 24.66, 4.07 ppm; HRMS (ESI): m/z $[M+H]^+$ .$C_{20}H_{22}N_5O_4S$ calculated value 428.1387, measured value 428.1384.

Example 46

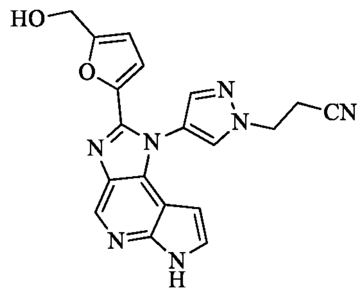

**LXS46**

3-(4-(2-(5-(Hydroxymethyl)furan-2-yl)imidazo[4,5-d]pyrrolo[2,3-b]pyridin-1(6H)-yl)-1H-pyrazol-1 -yl)propanenitrile

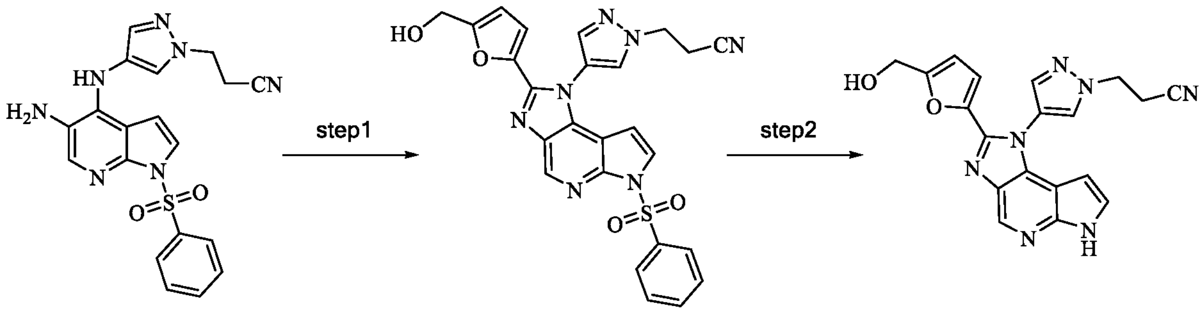


**Intermediate-10** **46-1** **KDN46**

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3 g, 0.7 mmol) in 10 mL of DMF, add $Na_2S_2O_5$ (0.8 g, 4 mmol) followed by adding 5-hydroxymethylfurfural (0.2 g, 1.4 mmol) and then stir for 12 h at 90°C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(4-(2-(5-(hydroxymethyl)furan-2-yl)-6-(phenylsulfonyl)imidazo[4,5-d]pyrrolo[2,3-b]pyridin-1(6H)-yl )-1H-pyrazol-1-yl)propanenitrile as a yellow oil (0.3 g, 79%). HRMS ( ESI): m/z $[M+H]^+$ .$C_{25}H_{20}N_7O_4S$ calculated value 514.1292, measured value 514.1299.

Step 2: Dissolve 3-(4-(2-(5-(hydroxymethyl)furan-2-yl)-6-(phenylsulfonyl)imidazo[4,5-d]pyrrolo[2,3-b]pyridin-1(6H)-yl )-1H-pyrazol-1-yl)propanenitrile (0.3 g, 0.6 mmol) in a mixture of 5 mL of tetrahydrofuran and 5 mL of methanol, then add 5 mL of 1 M sodium hydroxide. After stirring for 5 h at room temperature, the reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum. The residue was purified using a silica gel column (petroleum ether: ethyl acetate = 1:1) to yield **LXS46**: 3-(4-(2-(5-(Hydroxymethyl)furan-2-yl)imidazo[4,5-d]pyrrolo[2,3-b]pyridin-1(6H)-yl)-1H-pyrazol-1-yl) propanenitrile (0.1 g, 46%).$^1$ HNMR (300 MHz, *DMSO-d$_6$*): $\delta$ = 11.96 (s, 1H), 8.79 (s, 1H), 8.06 (s, 1H), 7.94 (s, 1H), 7.59 (s, 1H), 7.02 (d, *J* = 6.0 Hz, 1H), 6.78 (s, 1H), 6.59 (d, *J* = 6.0 Hz, 1H), 5.04 (t, *J* = 7.5 Hz, 2H), 4.39 (s, 2H), 3.28 (t, *J* = 7.5 Hz, 2H) ppm;$^{13}$ C NMR (75 MHz, *DMSO-d$_6$ )* $\delta$153.89, 151.18, 148.69, 142.17, 141.89, 130.74, 129.74, 129.04, 127.17, 120.73, 117.77, 115.63, 107.94, 104.05, 100.05, 99.49, 57.40, 49.27, 15.93 ppm; HRMS (ESI): m/z $[M+H]^+$ .$C_{19}H_{16}N_7O_2$ calculated value 374.1360, measured value 374.1377.

Example 47

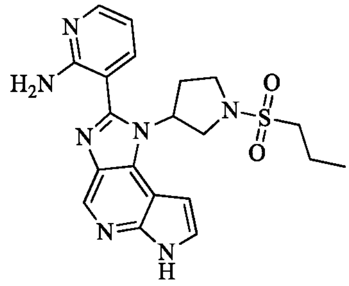

LXS47

3-(1-(1-(propylsulfonyl)pyrrolidin-3-yl)-1,6-dihydroimidazo[4,5-d]pyrrolo[2,3-b]pyridin-2-yl)pyridin-2-amine

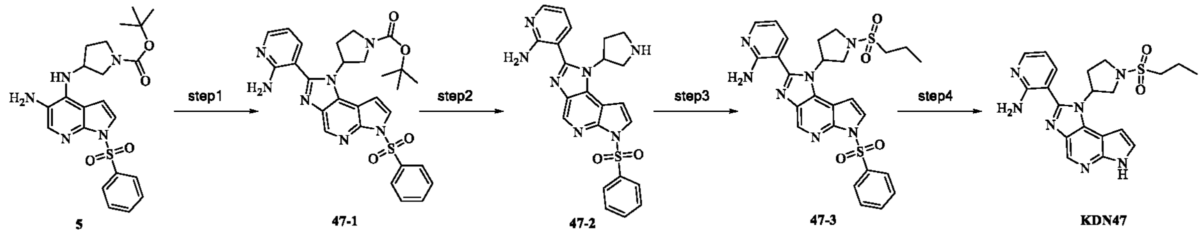


5 47-1 47-2 47-3 KDN47

Step 1: Dissolve tert-butyl 3-(((5-amino-1-(phenylsulfonyl)-1H-pyrrolo[2,3-b]pyridin-4-yl)amino)pyrrolidine-1-carboxylate (0.3 g, 0.7 mmol) in 10 mL of DMF, add $Na_2S_2O_5$ (0.7 g, 3.5 mmol) followed by adding 2-amino-3-pyridinecarboxaldehyde (0.2 g, 1.4 mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(2-(2-aminopyridin-3-yl)-6-(phenylsulfonyl)imidazo[4,5-d]pyrrolo[2,3-b]pyridin-1(6H)-yl)pyrrolidine-1-carboxylate as a yellow solid (0.3 g, 82%). HRMS (ESI): m/z $[M+H]^+$ .$C_{28}H_{30}N_7O_4S$ calculated value 560.2074, measured value 560.2077.

Step 2: Dissolve tert-butyl 3-(2-(2-aminopyridin-3-yl)-6-(phenylsulfonyl)imidazo[4,5-d]pyrrolo[2,3-b]pyridin-1(6H)-yl)pyrrolidine-1-carboxylate (0.3 g, 0.5 mmol) in 10 mL dichloromethane, slowly add trifluoroacetic acid (0.6 g, 5.0 mmol) and stir at room temperature for 12 hours. The product was concentrated under vacuum and 3-(6-(phenylsulfonyl)-1-(pyrrolidin-3-yl)-1,6-dihydroimidazo[4,5-d]pyrrolo[2,3-b]pyridin-2-yl)pyridin-2-amine was obtained as a light yellow oil (0.2 g, 81%). The product can be used directly in the next step without further purification. HRMS (ESI): m/z $[M+H]^+$ .$C_{23}H_{22}N_7O_2S$ calculated value 460.1550, measured value 460.1566.

Step 3: Dissolve 3-(6-(phenylsulfonyl)-1-(pyrrolidin-3-yl)-1,6-dihydroimidazo[4,5-d]pyrrolo[2,3-b]pyridin-2-yl)pyridin-2-amine (0.2 g, 0.4 mmol) in 10 mL of tetrahydrofuran, add DIPEA (0.1 g, 0.8 mmol) followed by the slow dropwise addition of propylsulfonyl chloride (0.1 g, 0.6 mmol) and then stir for 3 h at room temperature. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(6-(phenylsulfonyl)-1-(1-(propylsulfonyl)pyrrolidin-3-yl)-1,6-dihydroimidazo[4,5-d]pyrrolo[2,3-b]pyridin-2-yl) pyridin-2-amine as a pale yellow oil (0.2 g, 81%). The product can be used directly in the next step without further purification. HRMS (ESI): m/z $[M+H]^+$ .$C_{26}H_{28}N_7O_4S_2$ calculated value 566.1639, measured value 566.1645.

Step 4: 3-(6-(Phenylsulfonyl)-1-(1-(propylsulfonyl)pyrrolidin-3-yl)-1,6-dihydroimidazo[4,5-d]pyrrolo[2,3-b]pyridin-2-yl) pyridin-2-amine (0.2 g, 0.4 mmol) was dissolved in a solvent mixture of 5 mL tetrahydrofuran and 5 mL methanol, followed by adding 5 mL of 1 M sodium hydroxide. Stir the mixture at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield LXS47: 3-(1-(1-(propylsulfonyl)pyrrolidin-3-yl)-1,6-dihydroimidazo[4,5-d]pyrrolo[2,3-b]pyridin-2-yl)pyridin-2-amine (0.1 g, 67%).$^1$ HNMR (300 MHz, *DMSO-$d_6$*): $\delta$ = 12.06 (s, 1H), 8.83 (s, 1H), 8.03 (t, $J$ = 7.5 Hz, 1H), 7.74 (s, 2H), 7.66 (d, $J$ =

6.0 Hz, 1H), 7.56 (s, 1H), 6.81 (s, 1H), 6.68 (t, *J* = 7.5 Hz, 1H), 3.65-3.79 (m, 1H), 3.12-3.36 (m, 2H), 3.10 (t, *J* = 9.0 Hz, 2H), 2.70-2.81 (m, 2H), 1.85-2.14 (m, 2H), 1.69 (m, 2H), 0.93 (t, *J* = 7.5 Hz, 3H) ppm;$^{13}$ C NMR (75 MHz, *DMSO-d*$_6$ ) $\delta$ 156.68, 153.77, 148.64, 146.69, 142.19, 134.84, 129.04, 127.16, 120.74, 118.74, 115.63, 113.59, 99.49, 60.47, 56.83, 56.18, 50.04, 26.25, 13.38, 12.48 ppm; HRMS (ESI): m/z $[M+H]^+$ .$C_{20}H_{24}N_7O_2S$ calculated value 426.1707, measured value 426.1705.

Example 48

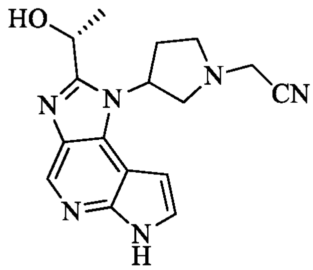

**LXS48**

2-(3-(2-((R)-1-Hydroxyethyl)imidazo[4,5-d]pyrrolo[2,3-b]pyridin-1(6H)-yl)pyrrolidin-1-yl)acetonitrile

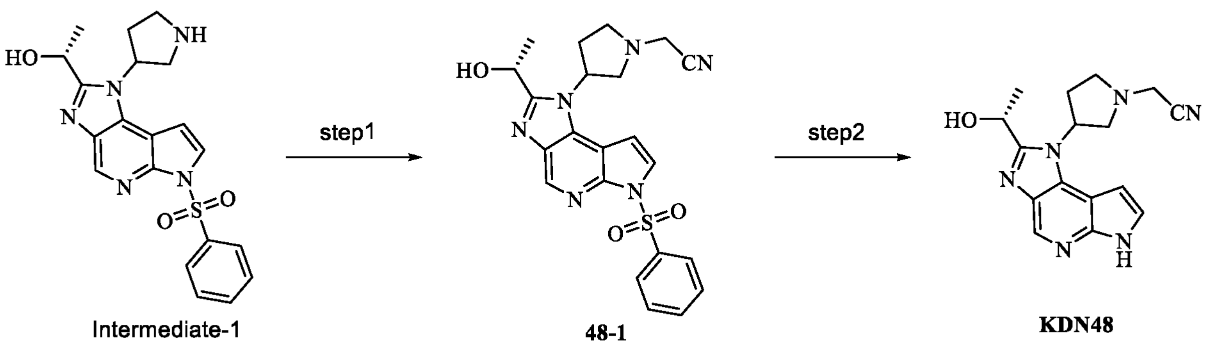


Intermediate-1 48-1 KDN48

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by the slow dropwise addition of 2-bromoacetonitrile (0.2 g, 1.1 mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(3-(2-((R)-1-hydroxyethyl)-6-(phenylsulfonyl)imidazo[4,5-d]pyrrolo[2,3-b]pyridin-1(6H)-yl)pyrrolidin-1-yl)acetonitrile as a pale yellow oil (0.2 g, 61%). The product can be used directly in the next step without further purification. HRMS (ESI): m/z $[M+H]^+$ .$C_{22}H_{23}N_6O_3S$ calculated value 451.1547, measured value 451.1550.

Step 2: Dissolve 2-(3-(2-((R)-1-hydroxyethyl)-6-(phenylsulfonyl)imidazo[4,5-d]pyrrolo[2,3-b]pyridin-1(6H)-yl)pyrrolidin-1-yl)acetonitrile (0.2 g, 0.4 mmol) in a mixture of 5 mL of tetrahydrofuran and 5 mL of methanol, and stir at room temperature for 5 hours after adding 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS48**: 2-(3-(2-((R)-1-Hydroxyethyl)imidazo[4,5-d]pyrrolo[2,3-b]pyridin-1(6H)-yl)pyrrolidin-1-yl)acetonitrile (0.1 g , 73%).$^1$ HNMR (300 MHz, *DMSO-d*$_6$*)*: $\delta$ = 11.86 (s, 1H), 8.87 (s, 1H), 7.59 (s, 1H), 6.89 (s, 1H), 4.53-4.68 (m, 1H), 3.68-3.78 (m, 1H), 3.48 (s, 2H), 2.56-2.81 (m, 2H), 2.20-2.30 (m, 2H), 1.90-2.15 (m, 2H), 1.93 (t, *J* = 6.0 Hz, 3H) ppm;$^{13}$ C NMR (75 MHz, *DMSO-d*$_6$ *)* $\delta$ 148.59, 142.17, 129.04, 127.16, 120.75, 115.63, 114.78, 99.28, 63.69, 58.18, 57.39, 54.83, 50.04, 26.97, 22.86 ppm; HRMS

(ESI): m/z [M+H]+ .$C_{16}H_{19}N_6O$ calculated value 311.1615, measured value 311.1618.

Example 49

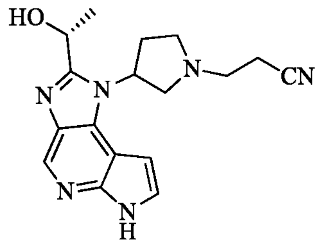

**LXS49**

3-(3-(2-((R)-1-Hydroxyethyl)imidazo[4,5-d]pyrrolo[2,3-b]pyridin-1 (6H)-yl)pyrrolidin-1-yl)propanenitrile

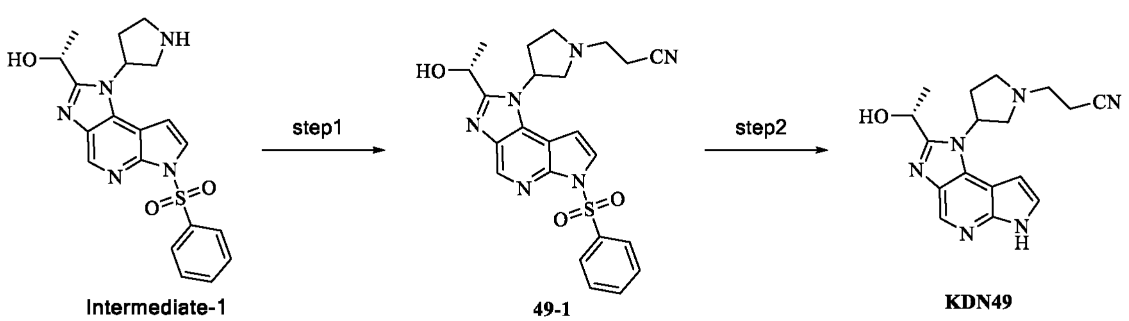


Intermediate-1 49-1 KDN49

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3 g, 0.7 mmol) in 10 mL of tetrahydrofuran, add DIPEA (0.2 g, 1.4 mmol) followed by the slow dropwise addition of 3-bromopropionitrile (0.2 g, 1.1 mmol). Raise the temperature to maintain reflux and stir for 3 h. The reaction was monitored by TLC for completion. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(3-(2-((R)-1-hydroxyethyl)-6-(phenylsulfonyl)imidazo[4,5-d]pyrrolo[2,3-b]pyridin-1(6H)-yl)pyrrolidin-1-yl)propanenitrile as a pale yellow oil (0.2 g, 59%). The product can be used directly in the next step without further purification. HRMS (ESI): m/z [M+H]+ .$C_{23}H_{25}N_6O_3S$ calculated value 465.1703, measured value 465.1709.

Step 2: Dissolve 2-(3-(2-((R)-1-hydroxyethyl)-6-(phenylsulfonyl)imidazo[4,5-d]pyrrolo[2,3-b]pyridin-1(6H)-yl)pyrrolidin-1-yl)propanenitrile (0.2 g, 0.4 mmol) in a mixture of 5 mL of tetrahydrofuran and 5 mL of methanol, and stir at room temperature for 5 hours after adding 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS49**: 3-(3-(2-((R)-1-Hydroxyethyl)imidazo[4,5-d]pyrrolo[2,3-b]pyridin-1(6H)-yl)pyrrolidin-1-yl)propanenitrile (0.1 g , 72%).$^1$ HNMR (300 MHz, *DMSO-d*$_6$ ): $\delta$ = 12.14 (s, 1H), 8.97 (s, 1H), 7.53 (s, 1H), 6.91 (s, 1H), 4.58-4.72 (m, 1H), 3.69-3.81 (m, 1H), 3.01 (t, *J* = 9.0 Hz, 2H), 2.72- 2.85 (m, 2H), 2.70 (t, *J* = 9.0 Hz, 2H), 2.22-2.36 (m, 2H), 1.93-2.19 (m, 2H), 1.58 (t, *J* = 7.5 Hz, 3H) ppm;$^{13}$ C NMR (75 MHz, *DMSO-d*$_6$ ) $\delta$ 148.66, 142.98, 129.03, 127.17, 120.77, 119.04, 115.69, 99.38, 63.69, 58.94, 57.69, 55.16, 27.64, 22.85, 17.17 ppm; HRMS (ESI): m/z [M+H]+ .$C_{17}H_{21}N_6O$ calculated value 325.1771, measured value 325.1777.

Example 50

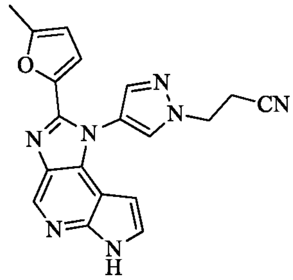

**LXS50**

3-(4-(2-(5-Methylfuran-2-yl)imidazo[4,5-d]pyrrolo[2,3-b]pyridin-1(6H)-yl)-1H-pyrazol-1-yl)propanenitrile

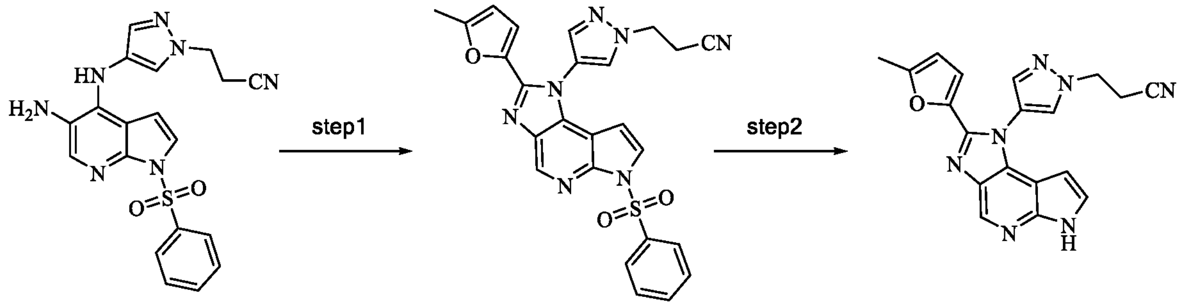


**Intermediate-10** **50-1** **KDN50**

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3 g, 0.7 mmol) in 10 mL of DMF, add $Na_2S_2O_5$ (0.8 g, 4 mmol) followed by adding 5-methylfurfural (0.2 g, 1.4 mmol). Stir for 12h at

90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(4-(2-(5-methylfuran-2-yl)-6-(phenylsulfonyl)imidazo[4,5-d]pyrrolo[2,3-b]pyridin-1(6H)-yl)-1H -pyrazol-1-yl)propanenitrile as a yellow oil (0.3 g, 82%).HRMS (ESI): m/z $[M+H]^+$ .$C_{25}H_{20}N_7O_3S$ calculated value 498.1343, measured value 498.1350.

Step 2: Dissolve 3-(4-(2-(5-methylfuran-2-yl)-6-(phenylsulfonyl)imidazo[4,5-d]pyrrolo[2,3-b]pyridin-1(6H)-yl)-1H -pyrazol-1-yl)propanenitrile (0.3 g, 0.6 mmol) in a solvent mixture of 5 mL of tetrahydrofuran and 5 mL of methanol, followed by adding 5 mL of 1 M sodium hydroxide. The mixture was then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS50**: 3-(4-(2-(5-methylfuran-2-yl)imidazo[4,5-d]pyrrolo[2,3-b]pyridin-1(6H)-yl)-1H-pyrazol-1-yl)propanenitrile (0.1 g, 46%).$^1$ HNMR (300 MHz, *DMSO-$d_6$* ): $\delta$ = 11.87 (s, 1H), 8.65 (s, 1H), 8.48 (s, 1H), 7.95 (s, 1H), 7.34 (s, 1H), 6.32 (d, *J* = 3.0 Hz, 1H), 6.21 (s, 1H), 5.95 (s, 1H), 4.61 (t, *J* = 4.5 Hz, 2H), 3.26 (t, *J* = 6.0 Hz, 2H), 2.36 (s, 3H) ppm;$^{13}$ C NMR (75 MHz, *DMSO-$d_6$* ) $\delta$ 154.31, 145.68, 143.29, 142.72, 138.24, 135.85, 135.54, 134.05, 129.75, 124.57, 118.82, 118.75, 113.43, 108.51, 104.57, 96.45, 48.05, 19.20, 13.73 ppm; HRMS (ESI): m/z $[M+H]^+$ .$C_{19}H_{16}N_7O$ calculated value 358.1411, measured value 358.1414.

Example 51

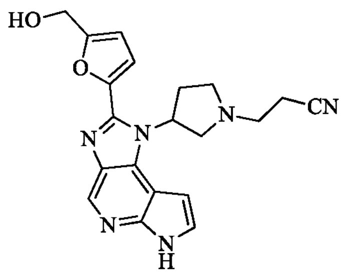

**LXS51**

3-(3-(2-(5-(Hydroxymethyl)furan-2-yl)imidazo[4,5-d]pyrrolo[2,3-b]pyridin-1(6H)-yl)pyrrolidin-1-yl)propanenitrile

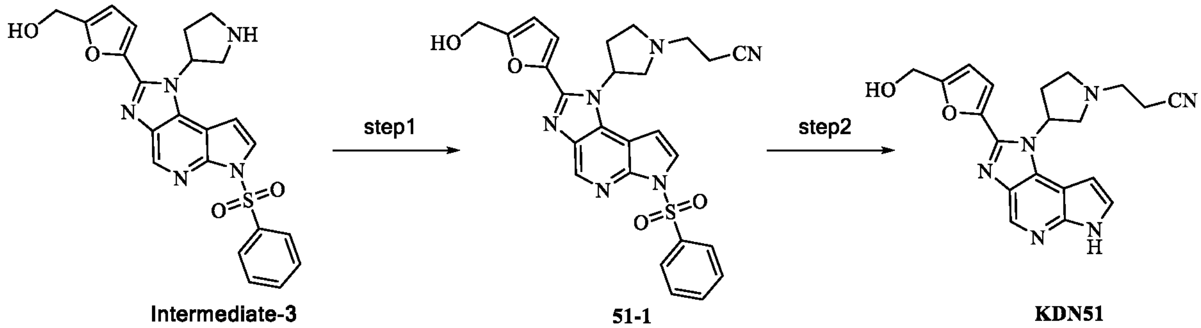


**Intermediate-3** **51-1** **KDN51**

Step 1: Dissolve (5-(6-(phenylsulfonyl)-1-(pyrrolidin-3-yl)-1,6-dihydroimidazo[4,5-d]pyrrolo[2,3-b]pyridin-2-yl)furan-2- methanol (0.3 g, 0.6 mmol) in 10 mL of tetrahydrofuran, add DIPEA (0.2 g, 1.2 mmol) followed by the slow dropwise addition of 3-bromopropionitrile (0.2 g, 0.9 mmol).Raise the temperature to maintain reflux and stir for 3 h. The reaction was monitored by TLC for completion. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(3-(2-(5-(hydroxymethyl)furan-2-yl)-6-(phenylsulfonyl)imidazo[4,5-d]pyrrolo[2,3-b]pyridin-1(6H)-yl )pyrrolidin-1-yl)propanenitrile as a pale yellow oil (0.2 g, 60%). The product can be used directly in the next step without further purification. HRMS (ESI): m/z $[M+H]^+$ .$C_{26}H_{25}N_6O_4S$ calculated value 517.1653, measured value 517.1659.

Step 2: Dissolve 3-(3-(2-(5-(hydroxymethyl)furan-2-yl)-6-(phenylsulfonyl)imidazo[4,5-d]pyrrolo[2,3-b]pyridin-1(6H)-yl )pyrrolidin-1-yl)propanenitrile (0.2 g, 0.4 mmol) in a solvent mixture of 5 mL tetrahydrofuran and 5 mL methanol, followed by adding 5 mL of 1 M sodium hydroxide. Stir for 5 h at room temperature and monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS51:** 3-(3-(2-(5-(Hydroxymethyl)furan-2-yl)imidazo[4,5-d]pyrrolo[2,3-b]pyridin-1(6H)-yl)pyrrolidin-1-yl)propanenitrile(0.1g, 69%).$^1$ HNMR(300 MHz, *DMSO-$d_6$*): $\delta$=12.03 (s, 1H), 8.88 (s, 1H), 7.59 (s, 1H), 7.02 (d, *J*= 9.0 Hz, 1H), 6.84 (s, 1H), 6.59 (d, *J*= 9.0 Hz, 1H), 4.39 (s, 2H), 3.64-3.78 (m, 1H), 3.03 (t, *J*= 9.0 Hz, 2H), 2.74-2.86 (m, 2H), 2.73 (t, *J*= 9.0 Hz, 2H), 2.20-2.35 (m, 2H), 1.90-2.15 (m, 2H) ppm;$^{13}$ CNMR (75MHz, *DMSO-$d_6$)* $\delta$153.88, 151.18, 148.64, 144.94, 142.17, 129.04, 127.18, 120.79, 119.04, 115.63, 107.94, 104.06, 99.38, 57.39, 57.01, 55.84, 55.19, 27.05, 17.16 ppm; HRMS (ESI): m/z $[M+H]^+$. $C_{20}H_{21}N_6O_2$ calculated value 377.1721, measured value 377.1728.

Example 52

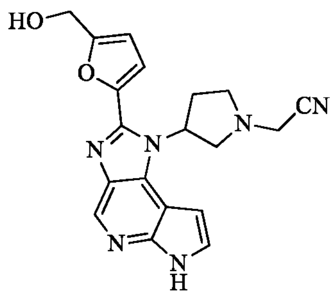

**LXS52**

2-(3-(2-(5-(hydroxymethyl)furan-2-yl)imidazo[4,5-d]pyrrolo[2,3-b]pyridin-1(6H)-yl)pyrrolidin-1-yl)acetonitrile

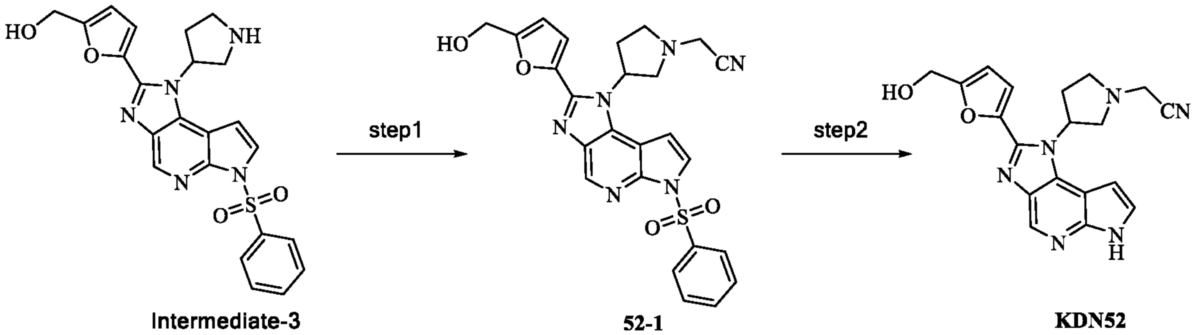


Intermediate-3 52-1 KDN52

Step 1: Dissolve (5-(6-(phenylsulfonyl)-1-(pyrrolidin-3-yl)-1,6-dihydroimidazo[4,5-d]pyrrolo[2,3-b]pyridin-2-yl)furan-2- methanol (0.3 g, 0.6 mmol) in 10 mL of tetrahydrofuran, add DIPEA (0.2 g, 1.2 mmol) followed by the slow dropwise addition of 2-bromoacetonitrile (0.2 g, 0.9 mmol). Raise the temperature to maintain reflux and stir for 3 h.The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(3-(2-(5-(hydroxymethyl)furan-2-yl)-6-(phenylsulfonyl)imidazo[4,5-d]pyrrolo[2,3-b]pyridin-1(6H)-yl)pyrrolidin-1-yl) acetonitrile as a pale yellow oil (0.2g, 61%). The product can be used directly in the next step without further purification. HRMS (ESI): m/z $[M+H]^+$. $C_{25}H_{23}N_6O_4S$ calculated value 503.1496, measured value 503.1500.

Step 2: Dissolve 3-(3-(2-(5-(hydroxymethyl)furan-2-yl)-6-(phenylsulfonyl)imidazo[4,5-d]pyrrolo[2,3-b]pyridin-1(6H)-yl)pyrrolidin-1-yl)acetonitrile (0.2g, 0.4mmol) in a solvent mixture of 5 mL of tetrahydrofuran and 5 mL of methanol, followed by adding 5 mL of 1 M sodium hydroxide. Stir for 5 h at room temperature and monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS52:** 2-(3-(2-(5-(hydroxymethyl)furan-2-yl)imidazo[4,5-d]pyrrolo[2,3-b]pyridin-1(6H)-yl)pyrrolidin-1-yl)acetonitrile (0.1g, 69%).$^1$ HNMR (300 MHz, *DMSO-$d_6$* ): $\delta$= 11.99 (s, 1H), 8.67 (s, 1H), 7.58 (s, 1H), 7.06 (d, *J* = 7.5 Hz, 1H), 6.88 (s, 1H), 6.54 (d, *J*= 7.5 Hz, 1H), 4.33 (s, 2H), 3.69 -3.83 (m, 1H), 3.48 (s, 2H), 2.56-2.81 (m, 2H), 2.20-2.30 (m, 2H), 1.93-2.18 (m, 2H) ppm; $^{13}$CNMR (75MHz, *DMSO-$d_6$)* $\delta$ 153.97, 151.19, 148.63, 144.95, 142.18, 129.03, 127.17, 120.75, 115.69, 114.84, 107.94, 104.05, 99.39, 57.36, 56.72, 55.09, 54.81, 50.05, 26.29 ppm; HRMS (ESI): m/z $[M+H]^+$. $C_{19}H_{19}N_6O_2$ calculated value 363.1564, Measured value 363.1569.

Example 53

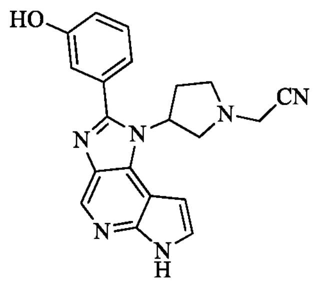

LXS53

2-(3-(2-(3-Hydroxyphenyl)imidazo[4,5-d]pyrrolo[2,3-b]pyridin-1(6H)-yl)pyrrolidin-1-yl)acetonitrile

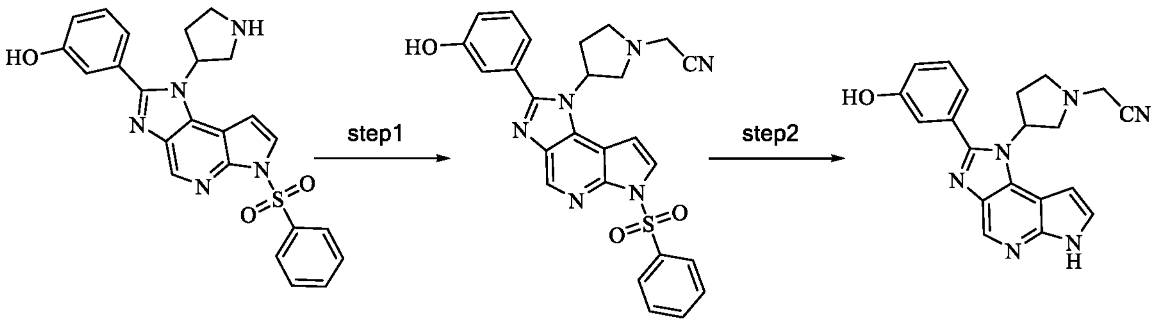


34-2 53-1 KDN53

Step 1: Dissolve 3-(6-(phenylsulfonyl)-1-(pyrrolidin-3-yl)-1,6-dihydroimidazo[4,5-d]pyrrolo[2,3-b]pyridin-2-yl)phenol (0.3g, 0.7mmol) in 10 mL of tetrahydrofuran, add DIPEA (0.2g, 1.4mmol) followed by slow dropwise addition of 2-bromoacetonitrile (0.2g, 1.1mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(3-(2-(3-hydroxyphenyl)-6-(phenylsulfonyl)imidazo[4,5-d]pyrrolo[2,3-b]pyridin-1(6H)-yl)pyrrolidin-1-yl) acetonitrile as a yellow oil (0.2 g, 61%). The product can be used directly in the next step without further purification. HRMS (ESI): m/z $[M+H]^+$ . $C_{26}H_{23}N_6O_3S$ calculated value 499.1547, measured value 499.1550.

Step 2: Dissolve (3-(2-(3-hydroxyphenyl)-6-(phenylsulfonyl)imidazo[4,5-d]pyrrolo[2,3-b]pyridin-1(6H)-yl)pyrrolidin-1-yl)acetonitrile (0.2g, 0.4mmol) in a solvent mixture of 5 mL tetrahydrofuran and 5 mL methanol, add 1 M sodium hydroxide 5 mL and stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS53:** 2-(3-(2-(3-Hydroxyphenyl)imidazo[4,5-d]pyrrolo[2,3-b]pyridin-1(6H)-yl)pyrrolidin-1-yl)acetonitrile (0.1g, 70%). $^1$HNMR (300 MHz, *DMSO-$d_6$* ): $\delta$= 12.09 (s, 1H), 8.83 (s, 1H), 7.84 (d, *J* = 6.0 Hz, 1H), 7.66 (s, 1H), 7.34 (t, *J* = 6.0 Hz, 1H), 7.04 (s, 1H), 6.91 (d, *J*= 6.0 Hz, 1H), 6.83 (s, 1H), 3.71-3.82 (m, 1H), 3.49 (s, 2H), 2.58-2.83 (m, 2H), 2.17-2.36 (m, 2H), 1.90-2.16 (m, 2H) ppm; $^{13}$CNMR (75 MHz, *DMSO-$d_6$*) $\delta$ 157.59, 153.75, 148.63, 142.17, 132.05, 130.65, 129.05, 127.16, 120.75, 120.11, 115.94, 115.65, 114.85, 112.94, 99.38, 57.3, 54.84, 50.04, 26.84 ppm; HRMS (ESI): m/z $[M+H]^+$. $C_{20}H_{19}N_6O$ calculated value 359.1615, measured value 359.1619.

Example 54

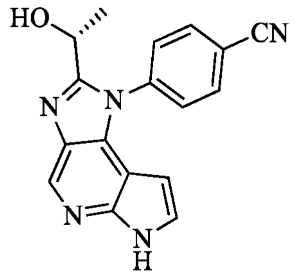

**LXS54**

(R)-4-(2-(1-Hydroxyethyl)imidazo[4,5-d]pyrrolo[2,3-b]pyridin-1(6H)-yl)benzonitrile

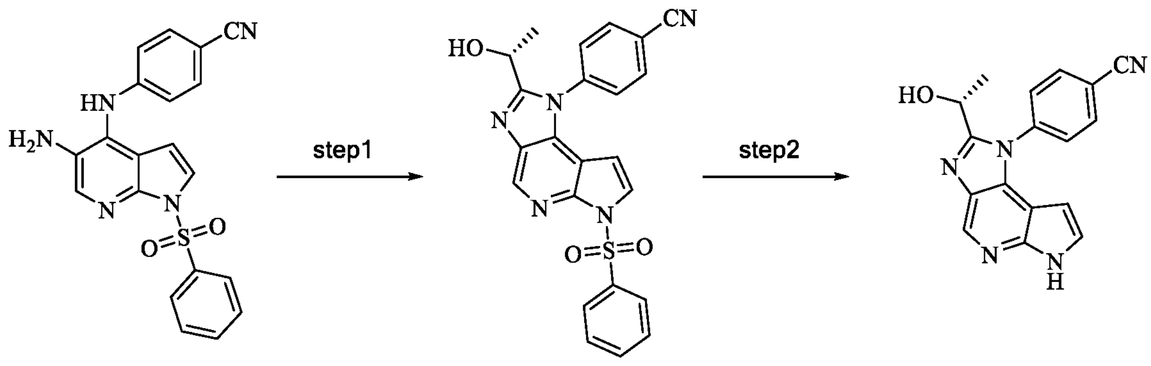


Intermediate-12 54-1 KDN54

Step 1: Triethyloxonium tetrafluoroborate (0.5g, 2.4mmol) and (R)-lactamide (0.2g, 2.4mmol) were dissolved in 10 mL of tetrahydrofuran, stirred at room temperature for 3 h and concentrated under vacuum to obtain the mixture as an oil, followed by the addition of 10 mL of ethanol to dissolve and the addition of 4-((5-amino-1-(phenylsulfonyl)-1H-pyrrolo[2,3-b]pyridin-4-yl)amino)benzonitrile (0.3g, 0.8mmol). Then, raise the temperature while maintaining reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (R)-4-(2-(1-hydroxyethyl)-6-(phenylsulfonyl)imidazo[4,5-d]pyrrolo[2,3-b]pyridin-1(6H)-yl)benzonitrile as a light green oil (0.3 g, 88%). HRMS (ESI): m/z $[M+H]^+$. $C_{23}H_{18}N_5O_3S$ calculated value 444.1125, measured value 444.1129.

Step 2: Dissolve (R)-4-(2-(1-hydroxyethyl)-6-(phenylsulfonyl)imidazo[4,5-d]pyrrolo[2,3-b]pyridin-1(6H)-yl)benzonitrile (0.3g, 0.7mmol) in a solvent mixture of 5 mL tetrahydrofuran and 5 mL methanol, add 1 M sodium hydroxide 5 mL and stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS54:** (R)-4-(2-(1-Hydroxyethyl)imidazo[4,5-d]pyrrolo[2,3-b]pyridin-1(6H)-yl)benzonitrile (0.1 g, 49%). $^1$HNMR (300MHz, *DMSO-$d_6$*): $\delta$ = 12.01 (s, 1H), 8.91 (s, 1H), 7.80 (d, *J* = 9.0 Hz, 2H), 7.64 (d, *J*= 9.0 Hz, 2H), 7.56 (s, 1H), 6.88 (s, 1H), 3.98-4.68 (m, 1H), 1.49 (d, *J*= 7.5 Hz, 3H) ppm; $^{13}$CNMR (75 MHz, *DMSO-$d_6$)* $\delta$ 151.75, 148.69, 142.16, 140.48, 134.27, 129.04, 127.18, 122.84, 120.73, 118.64, 115.67, 112.17, 99.38, 62.95, 22.84 ppm; HRMS(ESI): m/z $[M+H]^+$. $C_{17}H_{14}N_5O$ calculated value 304.1193, measured value 304.1203.

Example 55

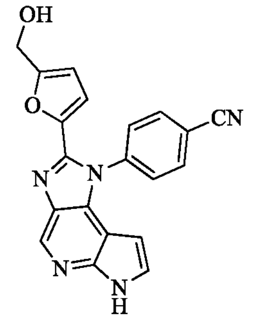

LXS55

4-(2-(5-(Hydroxymethyl)furan-2-yl)imidazo[4,5-d]pyrrolo[2,3-b]pyridin-1(6H)-yl)benzonitrile

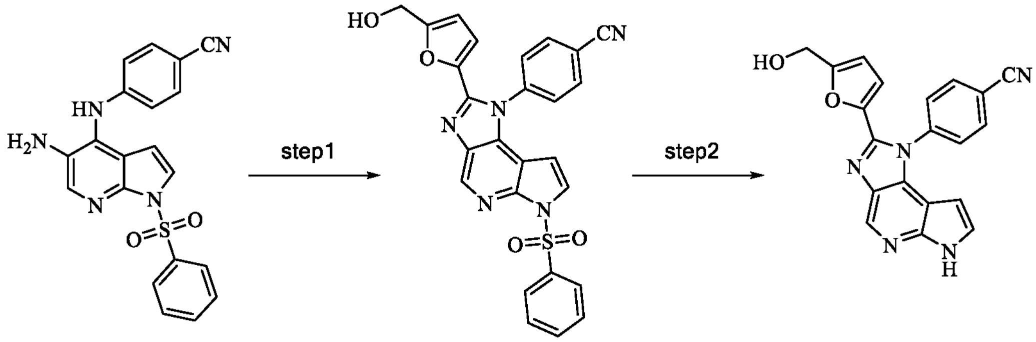


Intermediate-12 55-1 KDN55

Step 1: Dissolve 4-((5-amino-1-(phenylsulfonyl)-1H-pyrrolo[2,3-b]pyridin-4-yl)amino)benzonitrile (0.3g, 0.8mmol) in 10 mL of DMF, add $Na_2S_2O_5$ (0.8g, 4mmol) followed by the addition of 5-hydroxymethylfurfural (0.2g, 1.6mmol) dropwise, then raise the temperature to 90°C and stir for 12 h. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield 4-(2-(5-(hydroxymethyl)furan-2-yl)-6-(phenylsulfonyl)imidazo[4,5-d]pyrrolo[2,3-b]pyridin-1(6H)-yl)benzonitrile as a yellow oil (0.3g, 79%). HRMS(ESI): m/z $[M+H]^+$. $C_{26}H_{18}N_5O_4S$ calculated value 496.1074, measured value 496.1080.

Step 2: Dissolve 4-(2-(5-(hydroxymethyl)furan-2-yl)-6-(phenylsulfonyl)imidazo[4,5-d]pyrrolo[2,3-b]pyridin-1(6H)-yl)benzonitrile (0.3g, 0.6mmol) in a solvent mixture of 5 mL tetrahydrofuran and 5 mL methanol, add 5 mL of 1 M sodium hydroxide and then stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield **LXS55:** 4-(2-(5-(hydroxymethyl)furan-2-yl)imidazo[4,5-d]pyrrolo[2,3-b]pyridin-1(6H)-yl)benzonitrile (0.1 g, 47%). $^1$HNMR (300 MHz, *DMSO-d$_6$*): $\delta$= 11.91 (s, 1H), 8.73 (s, 1H), 7.83 (d, *J*= 6.0 Hz, 2H), 7.68 (d, *J*= 6.0 Hz, 2H), 7.56 (s, 1H), 7.02 (d, *J* = 9.0 Hz, 1H), 6.83 (s, 1H), 6.59 (d , *J*= 9.0 Hz, 1H), 4.39 (s, 2H) ppm; $^{13}$CNMR (75MHz, *DMSO-d$_6$*) $\delta$ 153.85, 151.17, 148.64, 142.59, 142.31, 134.27, 129.05, 127.17, 122.85, 120.73, 118.65, 115.69, 112.15, 107.94, 104.06, 99.38, 57.39 ppm; HRMS (ESI): m/z $[M+H]^+$. $C_{20}H_{14}N_5O_2$ calculated value 356.1142, measured value 356.1149.

Example 56

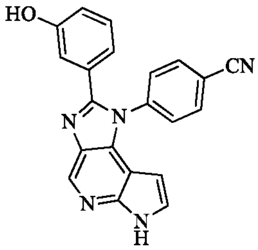

LXS56

4-(2-(3-Hydroxyphenyl)imidazo[4,5-d]pyrrolo[2,3-b]pyridin-1(6H)-yl)benzonitrile

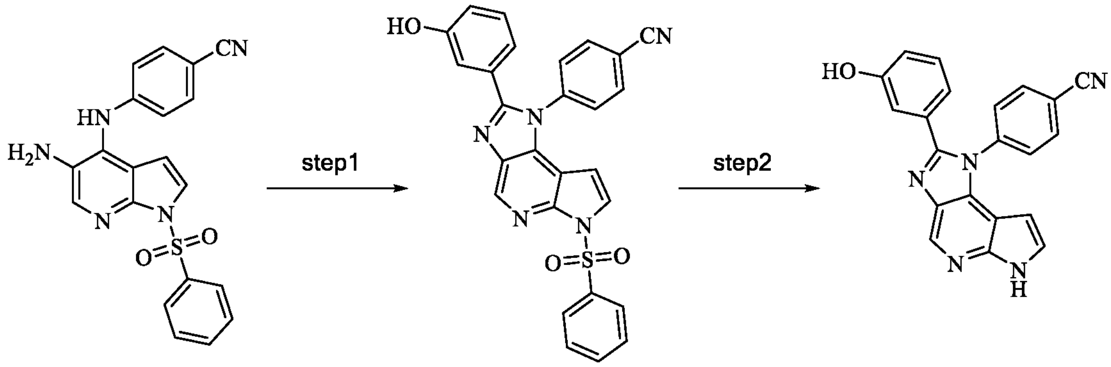


Intermediate-12 56-1 KDN56

Step 1: Dissolve 4-((5-amino-1-(phenylsulfonyl)-1H-pyrrolo[2,3-b]pyridin-4-yl)amino)benzonitrile (0.3g, 0.8mmol) in 10 mL of DMF, add $Na_2S_2O_5$ (0.8g, 4mmol) followed by the addition of 3-hydroxybenzaldehyde (0.2g, 1.6mmol) dropwise, raise the temperature to 90°C and stir for 12 h. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield 4-(2-(3-hydroxyphenyl)-6-(phenylsulfonyl)imidazo[4,5-d]pyrrolo[2,3-b]pyridin-1(6H)-yl)benzonitrile as a yellow oil (0.3g, 79%). HRMS(ESI): m/z $[M+H]^+$. $C_{27}H_{18}N_5O_3$ S calculated value 492.1125, measured value 492.1134.

Step 2: 4-(2-(3-Hydroxyphenyl)-6-(phenylsulfonyl)imidazo[4,5-d]pyrrolo[2,3-b]pyridin-1(6H)-yl)benzonitrile (0.3g, 0.6mmol) was dissolved in a solvent mixture of 5 mL tetrahydrofuran and 5 mL methanol, and the mixture was stirred at room temperature for 5 h after the addition of 5 mL 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS56:** 4-(2-(3-hydroxyphenyl)imidazo[4,5-d]pyrrolo[2,3-b]pyridin-1(6H)-yl)benzonitrile (0.1 g, 47%). $^1$HNMR (300 MHz, *DMSO-$d_6$*): $\delta$ = 12.06 (s, 1H), 8.83 (s, 1H), 7.84-7.93 (m, 1H), 7.81 (d, *J* = 7.5 Hz, 2H), 7.64 (d, *J* = 7.5 Hz, 2H), 7.58 (s, 1H), 7.34 (t, *J* = 9.0 Hz, 1H), 7.08 (s, 1H), 6.91 (d, *J* = 9.0 Hz, 1H), 6.88 (s, 1H) ppm; $^{13}$CNMR (75 MHz, *DMSO-$d_6$*) $\delta$ 157.89, 148.99, 144.47, 142.59, 142.16, 134.27, 132.04, 130.64, 129.05, 127.49, 122.84 , 120.75, 120.16, 118.69, 115.96, 115.61, 112.94, 112.15, 99.54 ppm; HRMS (ESI): m/z $[M+H]^+$. $C_{21}H_{14}N_5O$ calculated value 352.1193, measured value 352.1199.

Example 57

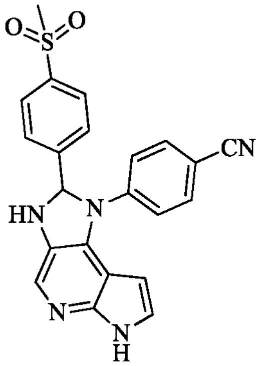

LXS57

4-(2-(4-(Methylsulfonyl)phenyl)-2,3-dihydroimidazo[4,5-d]pyrrolo[2,3-b]pyridin-1(6H)-yl)benzonitrile

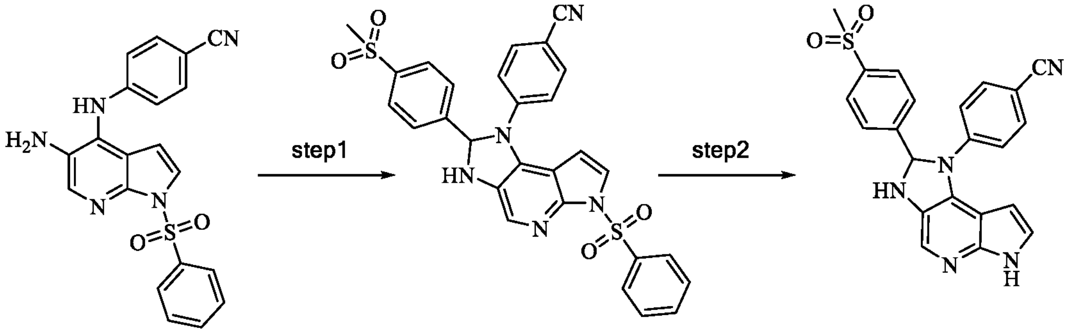


Intermediate-12 57-1 KDN57

Step 1: Dissolve 4-((5-amino-1-(phenylsulfonyl)-1H-pyrrolo[2,3-b]pyridin-4-yl)amino)benzonitrile (0.3g, 0.8mmol) in 10 mL of DMF, add $Na_2S_2O_5$ (0.8g, 4mmol) followed by adding 4-methylsulfonyl benzaldehyde (0.3g, 1.6mmol) dropwise, then raise the temperature to 90°C and stir for 12 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 4-(2-(4-(methylsulfonyl)phenyl)-6-(phenylsulfonyl)-2,3-dihydroimidazo[4,5-d]pyrrolo[2,3-b]pyridin-1(6H)-yl)benzonitrile as a yellow oil (0.3g, 70%).HRMS (ESI): m/z $[M+H]^+$. $C_{28}H_{22}N_5O_4S_2$ calculated value 556.1108, measured value 556.1113.

Step 2: Dissolve 4-(2-(4-(methylsulfonyl)phenyl)-6-(phenylsulfonyl)-2,3-dihydroimidazo[4,5-d]pyrrolo[2,3-b]pyridin-1(6H)-yl)benzonitrile (0.3g, 0.5mmol) in a mixture of 5 mL of tetrahydrofuran and 5 mL of methanol, add 5 mL of 1 M sodium hydroxide and then stir at room temperature for 5 hours. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield**LXS57:** 4-(2-(4-(methylsulfonyl)phenyl)-2,3-dihydroimidazo[4,5-d]pyrrolo[2,3-b]pyridin-1(6H)-yl)benzonitrile (0.1g, 45%). $^1$HNMR(300 MHz, *DMSO-$d_6$*): $\delta$ = 11.86 (s, 1H), 7.89 (s, 1H), 7.74 (d, $J$= 6.0 Hz, 2H), 7.63 (s, 1H), 7.54 (d, $J$= 6.0 Hz, 2H), 7.42 (d, $J$= 9.0 Hz, 2H), 6.88 (s, 1H), 6.78 (d, $J$ = 9.0 Hz, 2H), 5.08 (s, 1H), 3.32 (s, 1H) ppm; $^{13}$CNMR (75 MHz, *DMSO-$d_6$)* $\delta$153.77, 149.47, 139.04, 138.79, 134.41, 133.05, 131.18, 128.15, 127.93, 127.16, 123.38, 118.66, 114.28, 108.15, 101.04, 99.37, 87.84, 47.72 ppm; HRMS (ESI): m/z $[M+H]^+$. $C_{22}H_{18}N_5O_2S$ calculated value 416.1176, measured value 416.1180.

Example 58

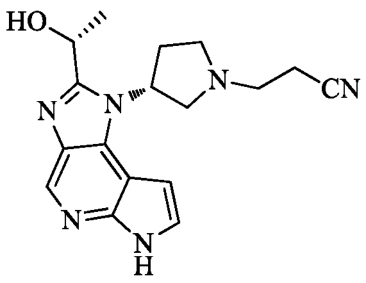

LXS58

3-((R)-3-(2-((R)-1-Hydroxyethyl)imidazo[4,5-d]pyrrolo[2,3-b]pyridin-1(6H)-yl)pyrrolidin-1-yl)propanenitrile

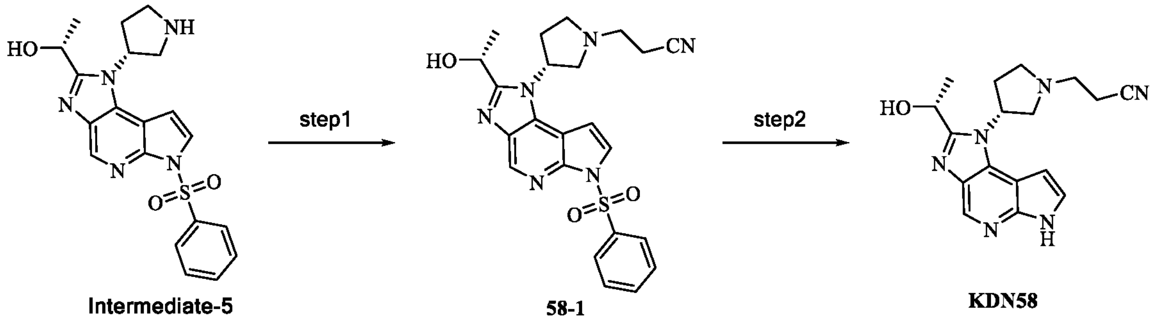


Intermediate-5

58-1

KDN58

Step 1: Dissolve (R)-1-(6-(phenylsulfonyl)-1-((R-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) and then slowly add 3-bromopropanenitrile (0.2g, 1.1mmol) dropwise. Raise the temperature to maintain reflux and stir for 3 h. Monitor the reactionfor completion by TLC. Saturated sodium bicarbonate was added until the reaction solution was weakly basic, and the organic phase was separated. The aqueous phase was extracted twice using dichloromethane. The organic phases were then combined and washed with saturated saline, dried over anhydrous sodium sulfate and concentrated under vacuum to yield 3-((R)-3-(2-((R)-1-hydroxyethyl)-6-(phenylsulfonyl)imidazo[4,5-d]pyrrolo[2,3-b]pyridin-1(6H)-yl)pyrrolidin-1-yl propanenitrile as a pale yellow oil (0.2g, 59%). The product can be used directly in the next step without further purification. HRMS (ESI): m/z $[M+H]^+$. $C_{23}H_{25}N_6O_3S$ calculated value 465.1703, measured value 465.1709.

Step 2: 3-((R)-3-(2-((R)-1-Hydroxyethyl)-6-(phenylsulfonyl)imidazo[4,5-d]pyrrolo[2,3-b]pyridin-1(6H)-yl)pyrrolidin-1-yl) propanenitrile (0.2g, 0.4mmol) was dissolved in a mixture of 5 mL tetrahydrofuran and 5 mL methanol, followed by adding 5 mL of 1 M sodium hydroxide. Stir the mxiture at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS58:** 3-((R)-3-(2-((R)-1-Hydroxyethyl)imidazo[4,5-d]pyrrolo[2,3-b]pyridin-1(6H)-yl)pyrrolidin-1-yl)propanenitrile (0.1g, 72%). $^1$HNMR (300 MHz, *DMSO-d$_6$*): $\delta$ = 11.98 (s, 1H), 8.73 (s, 1H), 7.56 (s, 1H), 6.78 (s, 1H), 4.61-4.75 (m, 1H), 3.65-3.83 (m, 1H), 3.04 (t, $J$ = 6.0 Hz, 2H), 2.70-2.83 (m, 2H), 2.73 (t, $J$= 6.0 Hz, 2H), 2.20-2.31 (m, 2H), 1.90-2.15 (m, 2H), 1.49 (t, $J$= 7.5 Hz, 3H) ppm; $^{13}$CNMR (75 MHz, *DMSO-d$_6$)* $\delta$ 148.64, 148.59, 142.19, 129.03, 127.16, 120.74, 119.04, 115.69, 99.39, 63.62, 58.91, 57.63, 55.16, 27.63, 22.84, 17.19 ppm; HRMS (ESI): m/z $[M+H]^+$. $C_{17}H_{21}N_6O$ calculated value 325.1771, measured value 325.1777.

Example 59

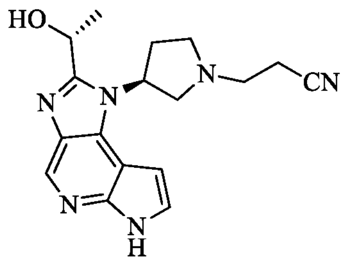

**LXS59**

3-((S)-3-(2-((R)-1-hydroxyethyl)imidazo[4,5-d]pyrrolo[2,3-b]pyridin-1(6H)-yl)pyrrolidin-1-yl)propanenitrile

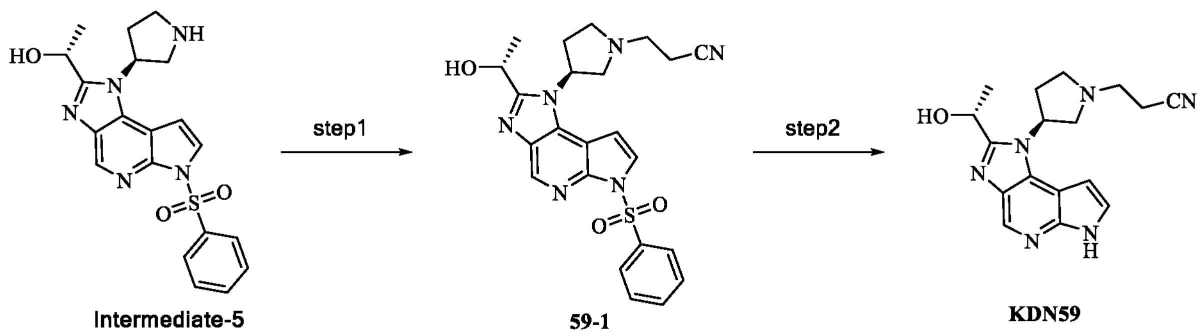


Intermediate-5 59-1 KDN59

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) and then slowly add 3-bromopropanenitrile (0.2g, 1.1mmol) dropwise. Raise the temperature to maintain reflux and stir for 3 h. Monitor the reactionfor completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-((S)-3-(2-((R)-1-hydroxyethyl)-6-(phenylsulfonyl)imidazo[4,5-d]pyrrolo[2,3-b]pyridin-1(6H)-yl)pyrrolidin-1-yl propanenitrile as a pale yellow oil (0.2g, 59%). The product can be used directly in the next step without further purification. HRMS (ESI): m/z $[M+H]^+$. $C_{23}H_{25}N_6O_3S$ calculated value 465.1703, measured value 465.1711.

Step 2: 3-((S)-3-(2-((R)-1-Hydroxyethyl)-6-(phenylsulfonyl)imidazo[4,5-d]pyrrolo[2,3-b]pyridin-1(6H)-yl)pyrrolidin-1-yl propanenitrile (0.2g, 0.4mmol) was dissolved in a mixture of 5 mL tetrahydrofuran and 5 mL methanol, followed by adding 5 mL of 1 M sodium hydroxide, stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS59:** 3-((S)-3-(2-((R)-1-hydroxyethyl)imidazo[4,5-d]pyrrolo[2,3-b]pyridin-1(6H)-yl)pyrrolidin-1-yl)propanenitrile (0.1g, 72%). $^1$HNMR (300 MHz, *DMSO-d$_6$*): $\delta$ = 11.99 (s, 1H), 8.83 (s, 1H), 7.59 (s, 1H), 6.88 (s, 1H), 4.51-4.69 (m, 1H), 3.65-3.85 (m, 1H), 3.05 (t, *J*= 9.0 Hz, 2H), 2.76-2.89 (m, 2H), 2.73 (t, *J* = 9.0 Hz, 2H), 2.20-2.41 (m, 2H), 1.95-2.22 (m, 2H), 1.43 (t, *J* = 7.5 Hz, 3H) ppm; $^{13}$CNMR (75 MHz, *DMSO-d$_6$*) $\delta$ 148.61, 148.57, 142.11, 128.64, 126.11, 120.73, 118.57, 115.62, 99.38, 63.62, 58.93, 57.62, 55.17, 27.64, 22.89, 16.14 ppm; HRMS (ESI): m/z $[M+H]^+$. $C_{17}H_{21}N_6O$ calculated value 325.1771, measured value 325.1780.

Example 60

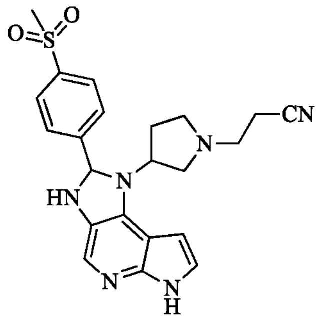

**LXS60**

3-(3-(2-(4-(Methylsulfonyl)phenyl)-2,3-dihydroimidazo[4,5-d]pyrrolo[2,3-b]pyridin-1(6H)-yl)pyrrolidin-1-yl)propanenitrile

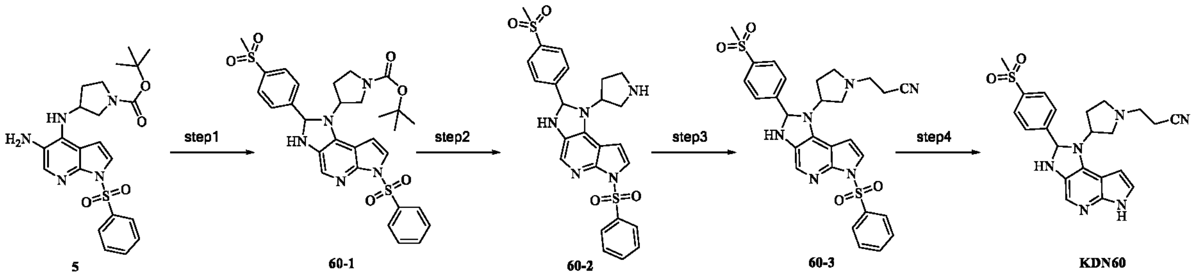


5 60-1 60-2 60-3 KDN60

Step 1: Dissolve tert-butyl 3-(((5-amino-1-(phenylsulfonyl)-1H-pyrrolo[2,3-b]pyridin-4-yl)amino)pyrrolidine-1-carboxylate (0.3g, 0.7mmol) in 10 mL of DMF, add $Na_2S_2O_5$(0.7g, 3.5mmol) followed by adding 4-methylsulfonylbenzaldehyde (0.2g, 1.4mmol). Stir for 12h at 90°C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield 3-(2-(4-(methylsulfonyl)phenyl)-6-(phenylsulfonyl)-2,3-dihydroimidazo[4,5-d]pyrrolo[2,3-b]pyridin-1(6H)-pyrrolidine-1-carboxylate as a yellow solid (0.2g, 49%). HRMS(ESI): m/z $[M+H]^+$. $C_{30}H_{34}N_5O_6S_2$ calculated value 624.1945, measured value 624.1955.

Step 2: Dissolve 3-(2-(4-(methylsulfonyl)phenyl)-6-(phenylsulfonyl)-2,3-dihydroimidazo[4,5-d]pyrrolo[2,3-b]pyridin-1(6H)-pyrrolidine-1-carboxylate (0.2g, 0.3mmol) in 10 mL of dichloromethane, slowly add trifluoroacetic acid (0.4g, 3 .0mmol) and then stir for 12 h at room temperature. The product was then concentrated under vacuum to yield 2-(4-(methylsulfonyl)phenyl)-6-(phenylsulfonyl)-1-(pyrrolidin-3-yl)-1,2,3,6-tetrahydroimidazo[4,5-d]pyrrolo[2,3-b]pyridine as a light yellow oil (0.1g, 60%). The product can be used directly in the next step without further purification. HRMS (ESI): m/z $[M+H]^+$. $C_{25}H_{26}N_5O_4S_2$ calculated value 524.1421, measured value 524.1427.

Step 3: Dissolve 2-(4-(methylsulfonyl)phenyl)-6-(phenylsulfonyl)-1-(pyrrolidin-3-yl)-1,2,3,6-tetrahydroimidazo[4,5-d]pyrrolo[2,3-b]pyridine (0.1g, 0.2mmol) in 10 mL of tetrahydrofuran, add DIPEA (0.1g, 0.4mmol) followed by slow dropwise addition of 3-bromopropyl cyanide (0.1g, 0.3mmol). Stir at room temperature for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(3-(2-(4-(methylsulfonyl)phenyl)-6-(phenylsulfonyl)-2,3-dihydroimidazo[4,5-d]pyrrolo[2,3-b]pyridin-1(6H)-yl)pyrrolidin-1-yl propanenitrile as a pale yellow oil (0.1g, 91%). The product can be used directly in the next step without further purification. HRMS (ESI): m/z $[M+H]^+$ .$C_{28}H_{29}N_6O_4S_2$ calculated value 577.1686, measured value 577.1688.

Step 4: Dissolve 3-(3-(2-(4-(methylsulfonyl)phenyl)-6-(phenylsulfonyl)-2,3-dihydroimidazo[4,5-d]pyrrolo[2,3-b]pyridin-1(6H)-yl)pyrrolidin-1-yl propanenitrile (0.1g, 0.2mmol) in a mixture of 5 mL tetrahydrofuran and 5 mL methanol, add 5 mL of 1 M sodium hydroxide and then stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue

was purified using a silica gel column (petroleum ether: ethyl acetate = 1:1) to yield **LXS60:** 3-(3-(2-(4-(Methylsulfonyl)phenyl)-2,3-dihydroimidazo[4,5-d]pyrrolo[2,3-b]pyridin-1(6H)-yl)pyrrolidin-1-yl)propanenitrile (60.0mg, 79%). $^1$HNMR (300 MHz, *DMSO-d$_6$*): $\delta$ = 11.46 (s, 1H), 7.89 (s, 1H), 7.74 (d, *J* = 9.0 Hz, 2H), 7.56 (s, 1H), 7.51 (d, *J* = 9.0 Hz, 2H), 6.88 (s, 1H), 5.04 (s, 1H), 3.32 (s, 3H), 3.03 (t, *J* = 9.0 Hz, 2H), 2.75 (t, *J*= 9.0 Hz, 2H), 2.66-2.71 (m, 1H), 2.31-2.56 (m, 2H), 2.19-2.33 (m, 2H), 1.66-2.02 (m, 2H) ppm; $^{13}$CNMR (75 MHz, *DMSO-d$_6$*) $\delta$ 144.28, 139.28, 138.73, 134.49, 131.18, 128.95, 128.15, 127.15, 123.38, 119.04, 108.16, 99.38, 83.28, 62.18, 59.28, 55.41, 55.14, 47.72, 33.58, 17.10 ppm; HRMS (ESI): m/z $[M+H]^+$. $C_{22}H_{25}N_6O_2S$ calculated value 437.1754, measured value 437.1759.

Example 61

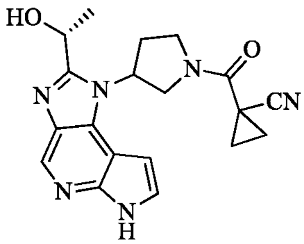

**LXS61**

1-(3-(2-((R)-1-Hydroxyethyl)imidazo[4,5-d]pyrrolo[2,3-b]pyridin-1(6H)-yl)pyrrolidin-1-carbonyl)cyclopropanecarbonitrile

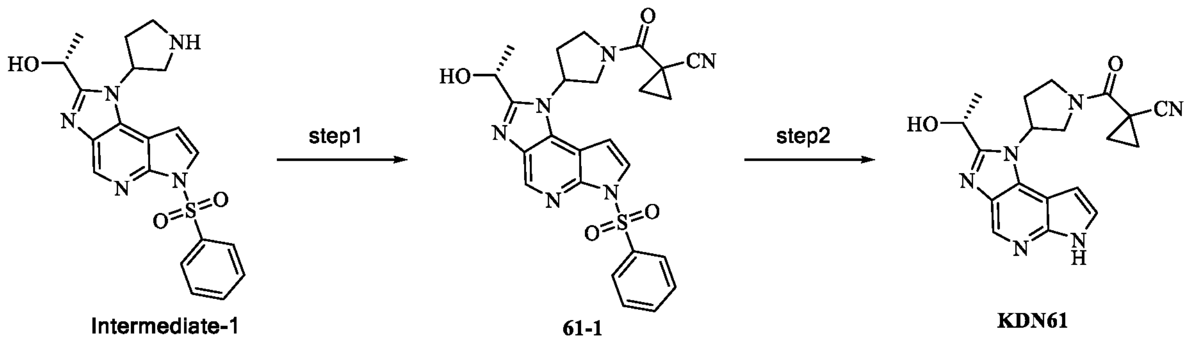


Intermediate-1 61-1 KDN61

Step 1: Dissolve (1R)-1-(6-(phenylsulfonyl)-1-(pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) followed by slow dropwise addition of 3-bromopropacyanine (0.2g, 1.1mmol). Stir at room temperature for 3h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 1-(3-(2-(R)-1-hydroxyethyl)-6-(phenylsulfonyl)imidazo[4,5-d]pyrrolo[2,3-b]pyridin-1 (6H)-yl)pyrrolidin-1-carbonyl)cyclopropanecarbonitrile as a yellow oil (0.3g, 82%). The product can be used directly in the next step without further purification. HRMS (ESI): m/z $[M+H]^+$. $C_{25}H_{25}N_6O_4S$ calculated value 505.1653, measured value 505.1660.

Step 2: 1-(3-(2-((R)-1-Hydroxyethyl)-6-(phenylsulfonyl)imidazo[4,5-d]pyrrolo[2,3-b]pyridin-1(6H)-yl)pyrrolidin-1-carbonyl)cyclopropanecarbonitrile (0.3g, 0.6mmol) was dissolved in a mixture of 5 mL tetrahydrofuran and 5 mL methanol, followed by the addition of 5 mL of 1 M sodium hydroxide. Stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum. The residue was purified using a silica gel column (petroleum ether: ethyl acetate = 1:1) to yield **LXS61:** 1-(3-(2-((R)-1-Hydroxyethyl)imidazo[4,5-d]pyrrolo[2,3-b]pyridin-1(6H)-yl)pyrrolidin-1-carbonyl)cyclopropanecarbonitrile (0.1g, 46%). $^1$HNMR (300 MHz, *DMSO-d$_6$*): $\delta$ = 12.06 (s, 1H), 7.55 (s, 1H), 6.87 (s, 1H), 4.33-4.68 (m, 1H), 3.89-4.17 (m, 1H), 3.77-4.03 (m, 2H), 3.41 -3.51 (m, 2H), 2.29-2.54 (m, 2H), 1.48 (d, *J* = 9.0 Hz, 3H), 0.67-0.92 (m, 4H) ppm;

$^{13}$CNMR (75MHz, *DMSO-$d_6$*) δ 180.77, 148.66, 148.53, 142.17, 129.00, 127.17, 120.73, 115.63, 114.47, 99.39, 63.69, 58.42, 51.88, 46.48, 27.15, 22.84, 13.63, 10.06 ppm; HRMS (ESI): m/z $[M+H]^+$. $C_{19}H_{21}N_6O_2$ calculated value 365.1721, measured value 365.1711.

Example 62

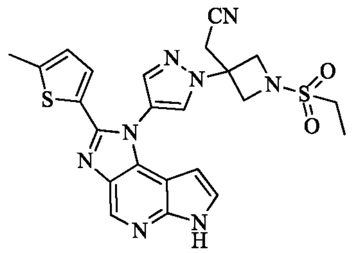

**LXS62**

2-(1 -(Ethyl sulfonyl)-3 -(4-(2-(5-methylthien-2-yl)imidazo[4,5 -d]pyrrolo[2,3-b]pyridin-1(6H)-yl)-1H-pyrazol-1-yl)azetidin-3-yl)acetonitrile

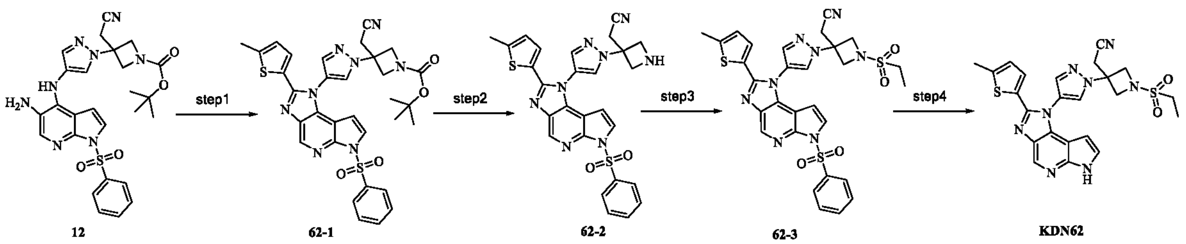


12 62-1 62-2 62-3 KDN62

Step 1: Tert-butyl 3-(4-((5-amino-1-(phenylsulfonyl)-1H-pyrrolo[2,3-b]pyridin-4-yl)amino)-1H-pyrazol-1-yl)-3-(cyanomethyl)azetidine-1-carboxylate (0.3g, 0.6mmol) was dissolved in 10 mL of DMF, $Na_2S_2O_5$ (0.6g, 3.0mmol) was added, followed by the addition of 5-methyl-2-thiophenecarboxaldehyde (0.2 g, 0.9 mmol) dropwise and stirring for 12 h at 90°C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(cyanomethyl)-3-(4-(2-(5-methylthien-2-yl)-6-(phenylsulfonyl)imidazo[4,5-d]pyrrolo[2,3-b]pyridin-1(6H)-yl) -1H-pyrazol-1-yl) azetidine-1-carboxylate as a yellow solid (0.2g, 56%). HRMS (ESI): m/z $[M+H]^+$. $C_{32}H_{31}N_8O_4S_2$ calculated value 655.1904, measured value 655.1909.

Step 2: Tert-butyl 3-(cyanomethyl)-3-(4-(2-(5-methylthien-2-yl)-6-(phenylsulfonyl)imidazo[4,5-d]pyrrolo[2,3-b]pyridin-1(6H)-yl)-1H-pyrazol-1-yl)azetidine-1-carboxylate (0.2g, 0.3mmol) was dissolved in 10 mL of dichloromethane and then trifluoroacetic acid (0.4g, 3.0mmol) was slowly added and stirred at room temperature for 12 h. The product was concentrated under vacuum to yield 2-(3-(4-(2-(5-methylthien-2-yl)-6-(phenylsulfonyl)imidazo[4,5-d]pyrrolo[2,3-b]pyridin-1(6H)-yl)-1H-pyrazol-1-yl)azetidin-3-yl)acetonitrile as a light yellow oil (0.1g, 59%). The product can be used directly in the next step without further purification. HRMS (ESI): m/z $[M+H]^+$. $C_{27}H_{23}N_8O_2S_2$ calculated value 555.1380, measured value 555.1390.

Step 3: Dissolve 2-(3-(4-(2-(5-methylthien-2-yl)-6-(phenylsulfonyl)imidazo[4,5-d]pyrrolo[2,3-b]pyridin-1(6H)-yl)-1H-pyrazol-1-yl)azetidin-3-yl)acetonitrile (0.1g, 0.2mmol) in 10 mL of tetrahydrofuran, add DIPEA (0.1g, 0.4mmol), followed by the slow dropwise addition of ethylsulfonyl chloride (0.1g, 0.3mmol), stir the mixture slowly for 3 h at room temperature. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(1-(ethylsulfonyl)-3-(4-(2-(5-methylthien-2-yl)-6-(phenylsulfonyl)imidazo[4,5-d]pyrrolo[2,3-b]pyridin-1(6H)-yl)-1H-pyrazol-1-yl)azetidin-3-yl)acetonitrile as a light yellow oil (0.1g,

86%). The product can be used directly in the next step without further purification. HRMS(ESI): m/z [M+H]+. $C_{29}H_{27}N_8O_4S_3$ calculated value 647.1312, measured value 647.1318.

Step 4: 2-(1-(Ethylsulfonyl)-3-(4-(2-(5-methylthien-2-yl)-6-(phenylsulfonyl)imidazo[4,5-d]pyrrolo[2,3-b]pyridin-1(6H)-yl)-1H-pyrazol-1-yl)azetidin-3-yl)acetonitrile (0.1g, 0.2mmol) was dissolved in a solvent mixture of 5 mL tetrahydrofuran and 5 mL methanol. Add 5 mL of 1 M sodium hydroxide and then stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS62:** 2-(1-(Ethylsulfonyl)-3-(4-(2-(5-methylthien-2-yl)imidazo[4,5-d]pyrrolo[2,3-b]pyridin-1(6H)-yl)-1H-pyrazol-1-yl)azetidin-3-yl)acetonitrile (65.0 mg, 83%).$^1$ HNMR (300 MHz, *DMSO-$d_6$* ): $\delta$ = 12.03 (s, 1H), 8.89 (s, 1H), 8.08 (s, 2H), 7.65 (s, 1H), 7.49 (d, *J*= 9.0 Hz, 1H), 6.89 (s, 1H), 6.84 (d, *J* = 9.0 Hz, 1H), 3.89-4.06 (m, 4H), 3.45 (m, 2H), 2.81 (s, 2H), 2.36 (s, 3H), 1.22 (t, *J*= 7.5 Hz, 3H) ppm;$^{13}$ C NMR (75 MHz, *DMSO-$d_6$)* $\delta$ 148.69, 142.17, 141.66, 141.61, 134.33, 130.77, 129.73, 129.01, 127.49, 127.11, 120.73, 117.74, 115.63, 100.54, 99.31, 59.29, 51.66, 50.83, 23.28, 15.22, 2.69 ppm. HRMS (ESI): m/z [M+H]+. $C_{23}H_{23}N_8O_2S_2$ calculated value 507.1380, measured value 507.1390.

Example 63

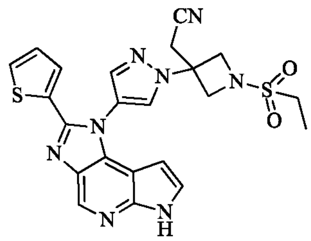

**LXS63**

2-(1-(Ethylsulfonyl)-3-(4-(2-(thiophen-2-yl)imidazo[4,5-d]pyrrolo[2,3-b]pyridin-1(6H)-yl)- 1H-pyrazol-1-yl)azetidin-3-yl)acetonitrile

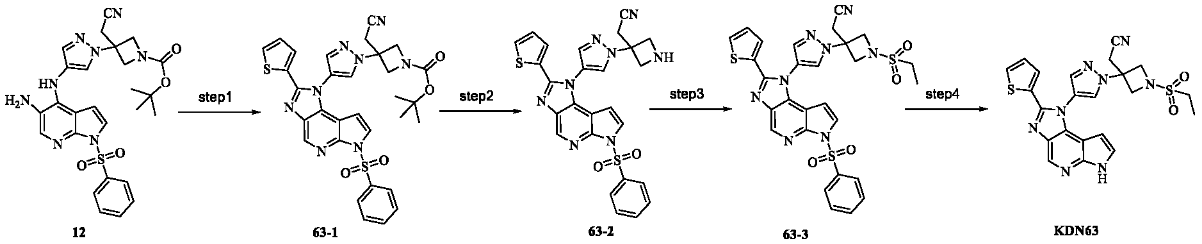


Step 1: Dissolve tert-butyl 3-(4-((5-amino-1-(phenylsulfonyl)-1H-pyrrolo[2,3-b]pyridin-4-yl)amino)-1H-pyrazol-1-yl)-3-(cyanomethyl)azetidine-1-carboxylate (0.3g, 0.6mmol) in 10 mL of DMF, add $Na_2S_2O_5$ (0.6g, 3.0mmol). After adding 2-thiophenecarboxaldehyde (0.2g, 0.9mmol) dropwise, raise the temperature to 90°C and stir for 12 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield tert-butyl 3-(cyanomethyl)-3-(4-(6-(phenylsulfonyl)-2-(thiophen-2-yl)imidazo[4,5-d]pyrrolo[2,3-b]pyridin-1(6H)-yl)-1H-pyrazol-1-yl)azetidine-1-carboxylate as a yellow solid (0.2g, 57%). HRMS (ESI): m/z [M+H]+. $C_{31}H_{29}N_8O_4S_2$ calculated value 641.1748, measured value 641.1751.

Step 2: Tert-butyl 3-(cyanomethyl)-3-(4-(6-(phenylsulfonyl)-2-(thiophen-2-yl)imidazo[4,5-d]pyrrolo[2,3-b]pyridin-1 (6H)-yl)-1H-pyrazol-1-yl)azetidine-1-carboxylate (0.2g, 0.3mmol) was dissolved in 10 mL of dichloromethane, and then trifluoroacetic acid (0.4g, 3.0mmol) was slowly added and stirred at room temperature for 12 hours. The product

was concentrated under vacuum to yield 2-(3-(4-(6-(phenylsulfonyl)-2-(thiophen-2-yl)imidazo[4,5-d]pyrrolo[2,3-b]pyridin-1(6H)-yl)-1H-pyrazol-1-yl)azetidin-3-yl)acetonitrile as a light yellow oil (0.1 g, 59%). The product can be used directly in the next step without further purification. HRMS (ESI): m/z $[M+H]^+$. $C_{26}H_{21}N_8O_2S_2$ calculated value 541.1223, measured value 541.1230.

Step 3: Dissolve 2-(3-(4-(6-(phenylsulfonyl)-2-(thiophen-2-yl)imidazo[4,5-d]pyrrolo[2,3-b]pyridin-1(6H)-yl)- 1H-pyrazol-1-yl)azetidin-3-yl)acetonitrile (0.1g, 0.2mmol) in 10 mL of tetrahydrofuran, add DIPEA (0.1g, 0.4mmol), followed by the dropwise addition of ethylsulfonyl chloride (0.1 g, 0.3 mmol), and then stir slowly for 3 h at room temperature. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(3-(4-(6-(phenylsulfonyl)-2-(thiophen-2-yl)imidazo[4,5-d]pyrrolo[2,3-b]pyridin-1(6H)-yl)-1H-pyrazol-1-yl)azetidin-3-yl)acetonitrile as a pale yellow oil (0.1g, 85%). The product can be used directly in the next step without further purification. HRMS (ESI): m/z $[M+H]^+$. $C_{28}H_{25}N_8O_4S_3$ calculated value 633.1155, measured value 633.1160.

Step 4: 2-(3-(4-(6-(Phenylsulfonyl)-2-(thiophen-2-yl)imidazo[4,5-d]pyrrolo[2,3-b]pyridin-1(6H)-yl)- 1H-pyrazol-1-yl)azetidin-3-yl)acetonitrile (0.1g, 0.2mmol) was dissolved in a solvent mixture of 5 mL tetrahydrofuran and 5 mL methanol, and 5 mL of 1M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS63:** 2-(1-(Ethylsulfonyl)-3-(4-(2-(thiophen-2-yl)imidazo[4,5-d]pyrrolo[2,3-b]pyridin-1(6H)-yl)- 1H-pyrazol-1-yl)azetidin-3-yl)acetonitrile (60.0 mg, 77%). $^1$HNMR (300 MHz, *DMSO-$d_6$*): $\delta$ = 11.96 (s, 1H), 8.74 (s, 1H), 8.10 (s, 2H), 7.85 (d, *J* = 7.5 Hz, 1H), 7.69 (d, *J*= 7.5 Hz, 1H), 7.67 ( s, 1H), 7.13-7.21 (m, 1H), 6.79 (s, 1H), 3.88-4.16 (m, 4H), 3.55 (m, 2H), 2.86 (s, 2H), 1.22 (t, *J* = 7.5 Hz, 3H) ppm; $^{13}$CNMR (75 MHz, *DMSO-$d_6$*) $\delta$ 148.67, 143.88, 142.19, 141.53, 130.77, 129.73, 129.04, 128.63, 128.04, 127.17, 120.73, 117.72, 115.69, 100.53, 99.38, 59.28, 51.64, 50.88, 23.37, 5.77 ppm; HRMS (ESI ): m/z $[M+H]^+$. $C_{22}H_{21}N_8O_2S_2$ calculated value 493.1223, measured value 493.1230.

Example 64

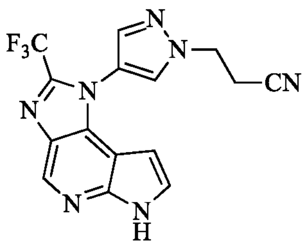

**LXS64**

3-(4-(2-(Trifluoromethyl)imidazo[4,5-d]pyrrolo[2,3-b]pyridin-1(6H)-yl)-1H-pyrazol-1-yl)propanenitrile

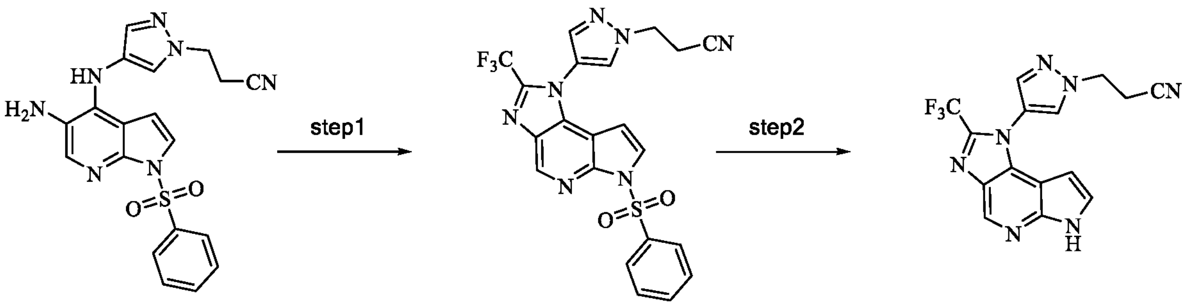


Intermediate-10 64-1 KDN64

Step 1: Triethyloxonium tetrafluoroborate (0.4g, 2.1mmol) and 2,2,2-trifluoroacetamide (0.2g, 2.1mmol) were dissolved in 10 mL of tetrahydrofuran, stirred at room temperature for 3 h and concentrated under vacuum to obtain the mixture in the form of oil, followed by the addition of 10 mL of ethanol to dissolve and the addition of 3-(4-((5-amino-1-(phenylsulfonyl)-1H-pyrrolo[2,3-b]pyridin-4-yl)amino-1H-pyrazol-1-yl)propanenitrile(0.3g , 0.7mmol). Raise the temperature to maintain reflux and stir for 3 h. The reaction was monitored by TLC for completion. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-(4-(6-(phenylsulfonyl)-2-(trifluoromethyl)imidazo[4,5-d]pyrrolo[2,3-b]pyridin-1(6H)-yl)-1H-pyrazol-1-yl)propanenitrile as a milk white oil (0.3g, 84%). HRMS (ESI): m/z $[M+H]^+$. $C_{21}H_{15}F_3N_7O_2S$ calculated value 486.0955, measured value 486.0961.

Step 2: Dissolve 3-(4-(6-(phenylsulfonyl)-2-(trifluoromethyl)imidazo[4,5-d]pyrrolo[2,3-b]pyridin-1(6H)-yl)-1H-pyrazol-1-yl) propanenitrile (0.3g, 0.6mmol) in a mixture of 5 mL of tetrahydrofuran and 5 mL of methanol, and stir for 5 h at room temperature after adding 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS64:** 3-(4-(2-(Trifluoromethyl)imidazo[4,5-d]pyrrolo[2,3-b]pyridin-1(6H)-yl)-1H-pyrazol-1-yl)propanenitrile (0.1g. 47%). $^1$HNMR (300 MHz, *DMSO-$d_6$*): $\delta$ = 11.96 (s, 1H), 8.89 (s, 1H), 8.06 (s, 1H), 7.94 (s, 1H), 7.56 (s, 1H), 6.86 (s, 1H), 5.04 (t, *J* = 9.0 Hz, 2H), 3.24 (t, *J* = 9.0 Hz, 2H) ppm; $^{13}$CNMR (75 MHz, *DMSO-$d_6$*) $\delta$ 148.69, 144.57, 142.18, 130.77, 129.84, 129.04, 127.11, 120.73, 117.77, 116.73, 115.69, 100.52, 99.67, 49.28, 16.88 ppm. HRMS (ESI): m/z $[M+H]^+$. $C_{15}H_{11}F_3N_7$ calculated value 346.1023, measured value 346.1029.

Example 65

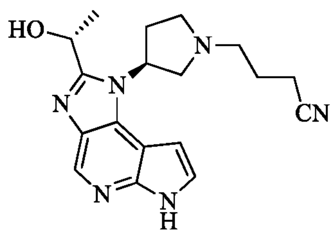

**LXS65**

4-((S)-3-(2-((R)-1-Hydroxyethyl)imidazo[4,5-d]pyrrolo[2,3-b]pyridin-1(6H)-yl)pyrrolidin-1-yl)butanenitrile

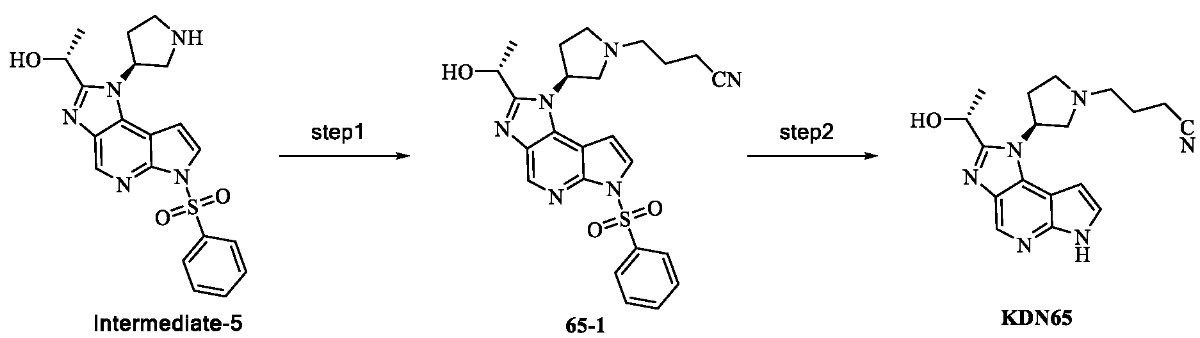


Intermediate-5 65-1 KDN65

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) and then slowly add 4-bromobutyronitrile (0.2g, 1.1mmol) dropwise. Raise the temperature to maintain reflux and stir for 3 h. Monitor the reaction for completion by TLC. Saturated sodium bicarbonate was added until the reaction solution was weakly basic, and the

organic phase was separated. The aqueous phase was extracted twice using dichloromethane. The organic phases were combined and washed with saturated saline, dried over anhydrous sodium sulfate and concentrated under vacuum to yield 4-((S)-3-(2-((R)-1-hydroxyethyl)-6-(phenylsulfonyl)imidazo[4,5-d]pyrrolo[2,3-b]pyridin-1 (6H)-yl)pyrrolidin-1-yl)butyronitrile as a pale yellow oil (0.2g, 57%). The product can be used directly in the next step without further purification. HRMS(ESI): m/z $[M+H]^+$. $C_{24}H_{27}N_6O_3S$ calculated value 479.1860, measured value 479.1871.

Step 2: 4-((S)-3-(2-((R)-1-Hydroxyethyl)-6-(phenylsulfonyl)imidazo[4,5-d]pyrrolo[2,3-b]pyridin-1(6H)-yl)pyrrolidin-1-yl)butyronitrile (0.2g, 0.4mmol) was dissolved in a mixture of 5 mL of tetrahydrofuran and 5 mL of methanol, followed by the addition of 5 mL of 1M sodium hydroxide. Stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS65:** 4-((S)-3-(2-((R)-1-Hydroxyethyl)imidazo[4, 5-d]pyrrolo[2,3 -b]pyridin-1 (6H)-yl)pyrrolidin-1-yl)butanenitrile (0.1g, 71%). $^1$HNMR (300 MHz, *DMSO-d$_6$* ): $\delta$ = 12.16 (s, 1H), 8.89 (s, 1H), 7.69 (s, 1H), 6.88 (s, 1H), 4.53-4.69 (m, 1H), 3.69-3.80 (m, 1H), 2.56-2.81 (m, 2H), 2,43 (t, *J* = 7.5 Hz, 2H), 2.21-2.31 (m, 2H), 1.92-2.16 (m, 2H), 1.87 (t, *J*= 7.5 Hz, 2H), 1.66-1.78 (m, 2H), 1.44 (d, *J* = 9.0 Hz, 3H) ppm; $^{13}$CNMR (75 MHz, DMSO -*d$_6$*) $\delta$ 148.65, 148.51, 142.17, 129.04, 127.19, 120.77, 119.37, 115.62, 99.74, 63.67, 58.92, 58.49, 55.91, 55.53, 27.63, 22.81, 15.92, 15.19 ppm; HRMS ( ESI): m/z $[M+H]^+$. $C_{18}H_{23}N_6O$ calculated value 339.1928, measured value 339.1931.

Example 66

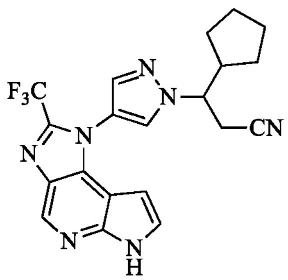

**LXS66**

3-Cyclopentyl-3-(4-(2-(trifluoromethyl)imidazo[4,5-d]pyrrolo[2,3-b]pyridin-1(6H)-yl)-1H-pyrazol-1-yl)propanenitrile

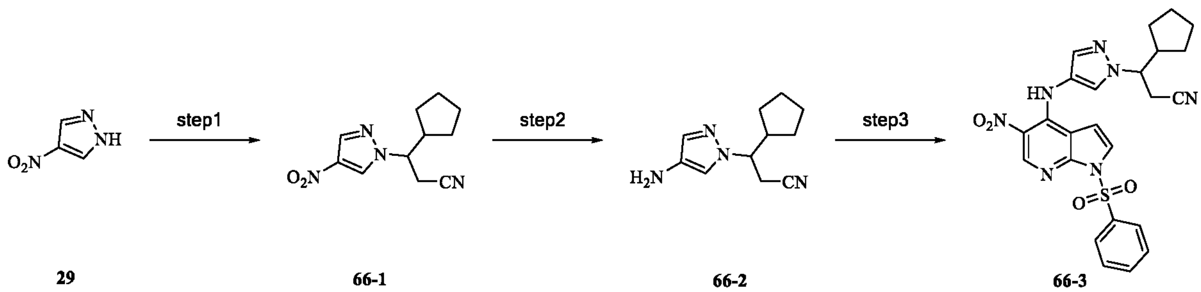


29 66-1 66-2 66-3

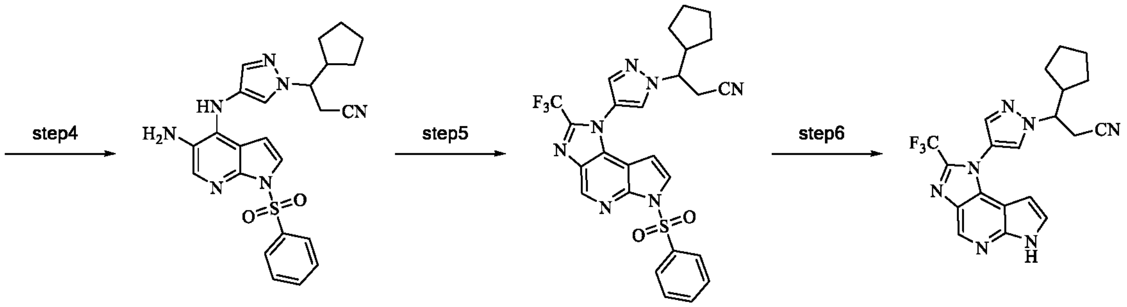


66-4 66-5 KDN66

Step 1: Dissolve 4-nitropyrazole (0.5g, 4.4mmol) in 10 mL of tetrahydrofuran, add DIPEA (1.2g, 8.8mmol) followed by slow dropwise addition of 3-bromo-3-cyclopentylpropanenitrile (1.3g, 6.6mmol). Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 3-cyclopentyl-3-(4-nitro-1H-pyrazol-1-yl)propanenitrile as a pale yellow oil (0.8 g, 77%). The product can be used directly in the next step without further purification. HRMS(ESI): m/z $[M+H]^+$. $C_{11}H_{15}N_4O_2$ calculated value 235.1190, measured value 235.1194.

Step 2: 3-Cyclopentyl-3-(4-nitro-1H-pyrazol-1-yl)propanenitrile (0.8g, 3.4mmol) was dissolved in 10 mL of methanol, after adding palladium carbon (0.1g, 10%), use hydrogen to replace the air in the reaction flask more than three times and the reaction was kept in hydrogen atmosphere. Stir at room temperature for 12 h. The reaction was monitored for completion by TLC. After filtration, the filtrate was collected and concentrated under vacuum to obtain 3-cyclopentyl-3-(4-amino-1H-pyrazol-1-yl)propanenitrile as a pink foamy solid (0.7g, 100%). The product can be used directly in the next step without further purification. HRMS (ESI): m/z $[M+H]^+$. $C_{11}H_{17}N_4$ calculated value 205.1448, measured value 205.1552.

Step 3: Dissolve 3-cyclopentyl-3-(4-amino-1H-pyrazol-1-yl)propanenitrile (0.7g, 3.4 mmol) in 10 mL of tetrahydrofuran, add DIPEA (0.9g, 6.8 mmol) followed by adding 4-chloro-5-nitro-1-(phenylsulfonyl)-1H-pyrrolo[2,3-b]pyridine (0.8g, 2.3 mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum. Triturate and cure with an appropriate amount of methanol to yield 3-cyclopentyl-3-(4-((5-nitro-1-(phenylsulfonyl)-1H-pyrrolo[2,3-b]pyridin-4-yl)amino)-1H-pyrazol-1-yl)propanenitrile as a pale yellow oil (0.6g, 50%). The product can be used directly in the next step without further purification. HRMS (ESI): m/z $[M+H]^+$. $C_{24}H_{24}N_7O_4S$ calculated value 506.1605, measured value 506.1613.

Step 4: 3-Cyclopentyl-3-(4-((5-nitro-1-(phenylsulfonyl)-1H-pyrrolo[2,3-b]pyridin-4-yl)amino)-1H-pyrazol-1-yl)propanenitrile (0.6g, 1.2mmol) was dissolved in 10 mL of methanol, palladium carbon (0.1g, 10%) was added and the air in the reaction flask was replaced more than three times using hydrogen. The reaction was carried out in hydrogen atmosphere and stirred at room temperature for 12 h. The reaction was monitored for completion by TLC. After filtration, the filtrate was collected and concentrated under vacuum to yield 3-cyclopentyl-3-(4-((5-amino-1-(phenylsulfonyl)-1H-pyrrolo[2,3-b]pyridin-4-yl)amino)-1H-pyrazol-1-yl)propanenitrile as a pink foamy solid (0.5g, 88%). The product can be used directly in the next step without further purification. HRMS (ESI): m/z $[M+H]^+$. $C_{24}H_{26}N_7O_2S$ calculated value 476.1863, measured value 476.1870.

Step 5: Triethyloxonium tetrafluoroborate (0.6g, 3.3mmol) and 2,2,2-trifluoroacetamide (0.1g, 3.3mmol) were dissolved in 10 mL of tetrahydrofuran, stirred at room temperature for 3 h and concentrated under vacuum to obtain the mixture as an oil, followed by the addition of 10 mL of ethanol to dissolve and the addition of 3-cyclopentyl-3-(4-((5-amino-1-(phenylsulfonyl)-1H-pyrrolo[2,3-b]pyridin-4-yl)amino)-1H-pyrazol-1-yl)propanenitrile (0.5g, 1.1mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 2:1) to yield 3-cyclopentyl-3-(4-(6-(phenylsulfonyl)-2-(trifluoromethyl)imidazo[4,5-d]pyrrolo[2,3-b]pyridin-1(6H)-yl)-1H-pyrazol-1-yl) propanenitrile as a light green oil (0.2g, 34%). HRMS(ESI): m/z [M+H]$^+$. $C_{26}H_{23}F_3N_7O_2S$ calculated value 554.1581, measured value 554.1593.

Step 6: 3-Cyclopentyl-3-(4-(6-(phenylsulfonyl)-2-(trifluoromethyl)imidazo[4,5-d]pyrrolo[2,3-b]pyridin-1(6H)-yl)-1H-pyrazol-1-yl)propanenitrile (0.2g, 0.4mmol) was dissolved ina solvent mixture of 5 mL tetrahydrofuran and 5 mL methanol, followed by the addition of 5 mL of 1 M sodium hydroxide. Stir for 5 h at room temperature and monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS66:** 3-Cyclopentyl-3-(4-(2-(trifluoromethyl)imidazo[4,5-d]pyrrolo[2,3-b]pyridin-1(6H)-yl)-1H-pyrazol-1-yl)propanenitrile (0.1g, 67%). $^1$HNMR (300 MHz, *DMSO-d$_6$*): $\delta$ = 11.96 (s, 1H), 8.79 (s, 1H), 8.06 (s, 1H), 7.94 (s, 1H), 7.58 (s, 1H), 6.77 (s, 1H), 3.53-3.70 (m, 1H), 2.80 (d, *J* = 9.0 Hz, 2H), 1.35-1.60 (m, 9H) ppm; $^{13}$CNMR (75 MHz, *DMSO-d$_6$*) $\delta$ 148.66, 144.67, 142.19, 130.78, 129.77, 129.03, 127.16, 120.74, 117.74, 116.74, 115.64, 100.58, 99.39, 63.95, 34.28, 30.95, 25.16, 18.88 ppm; HRMS (ESI): m/z [M+H]$^+$. $C_{20}H_{19}F_3N_7$ calculated value 414.1649, measured value 414.1654.

Example 67

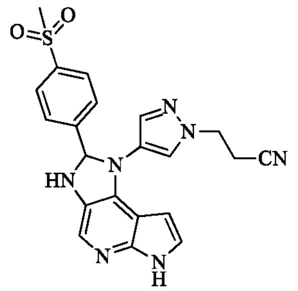

**LXS67**

3-(4-(2-(4-(Methylsulfonyl)phenyl)-2,3-dihydroimidazo[4,5-d]pyrrolo[2,3-b]pyridin-1 (6H)-yl)-1H-pyrazol-1-yl)propanenitrile

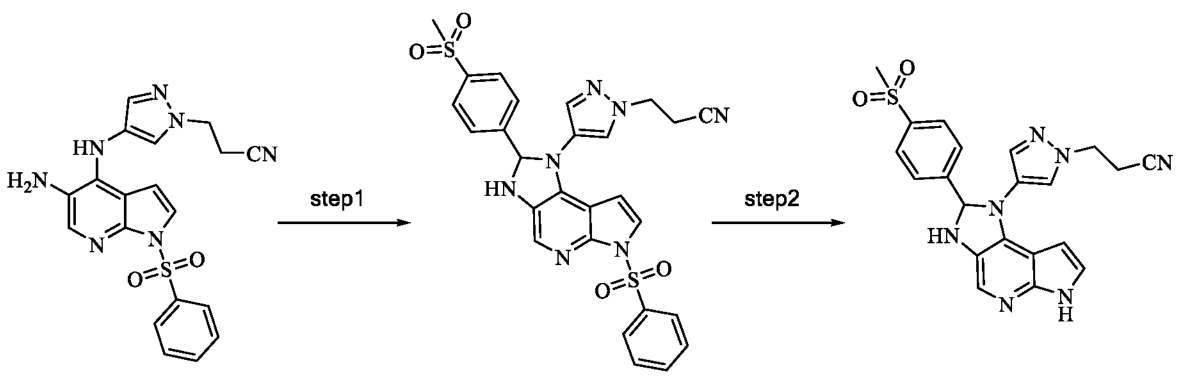


Intermediate-10 67-1 KDN67

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3g, 0.7mmol) in 10 mL of DMF, add $Na_2S_2O_5$ (0.7g, 3.5mmol) followed by adding 4-methylsulfonylbenzaldehyde (0.2g, 0.9mmol) and stir for 12 h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline and dried over anhydrous sodium sulfate to yield 3-(4-(2-(4-(methylsulfonyl)phenyl)-6-(phenylsulfonyl)-2,3-dihydroimidazo[4,5-d]pyrrolo[2,3-b]pyridin-1(6H)-yl)-1H-pyrazol-1-yl)propanenitrile as a yellow solid (0.2g, 47%). HRMS (ESI): m/z $[M+H]^+$. $C_{27}H_{24}N_7O_4S_2$ S calculated value 574.1326, measured value 574.1331.

Step 2: 3-(4-(2-(4-(Methylsulfonyl)phenyl)-6-(phenylsulfonyl)-2,3-dihydroimidazo[4,5-d]pyrrolo[2,3-b]pyridin-1(6H)-yl)-1H-pyrazol-1-yl)propanenitrile (0.2g, 0.3mmol) was dissolved in a solvent mixture of 5 mL tetrahydrofuran and 5 mL methanol followed by adding 1 M sodium hydroxide 5 mL and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS67:** 3-(4-(2-(4-(methylsulfonyl)phenyl)-2,3-dihydroimidazo[4,5-d]pyrrolo[2,3-b]pyridin-1(6H)-yl)-1H-pyrazol-1-yl)propanenitrile (0.1g, 66%).$^{1}$HNMR (300 MHz, *DMSO-$d_6$* ): $\delta$ = 11.06 (s, 1H), 7.89 (s, 1H), 7.86 (s, 1H), 7.74 (d, $J$ = 9.0 Hz, 2H), 7.58 (s, 1H), 7.51 (d, $J$= 9.0 Hz, 2H), 7.18 (s, 1H), 6.87 (s, 1H), 5.04 (t, $J$ = 7.5 Hz, 3H), 3.34 (s, 3H), 3.20 (t, $J$ = 7.5 Hz, 2H) ppm; $^{13}$CNMR (75 MHz, *DMSO-$d_6$)* $\delta$ 149.78, 139.07, 138.77, 134.44, 131.17, 130.48, 130.15, 128.19, 127.94, 127.18, 123.34, 117.79, 115.75, 108.15, 99.43, 87.59, 49.22, 47.78, 17.88 ppm; HRMS (ESI): m/z $[M+H]^+$. $C_{21}H_{20}N_7O_2S$ calculated value 434.1394, measured value 434.1400.

Example 68

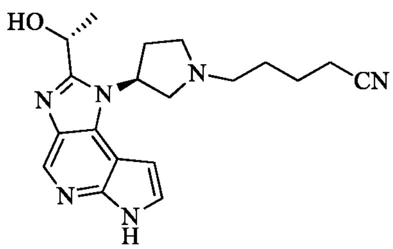

**LXS68**

5-((S)-3-(2-((R)-1-Hydroxyethyl)imidazo[4,5-d]pyrrolo[2,3-b]pyridin-1(6H)-yl)pyrrolidin-1-yl)pentanonitrile

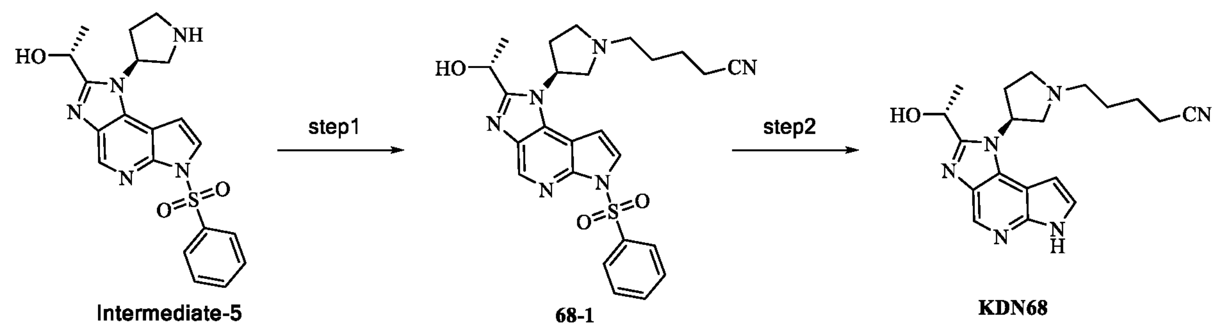


**Intermediate-5** **68-1** **KDN68**

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) and then slowly add 5-bromopentanonitrile (0.2g, 1.1mmol) dropwise. Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum

to yield 5-((S)-3-(2-((R)-1-hydroxyethyl)-6-(phenylsulfonyl)imidazo[4,5-d]pyrrolo[2,3-b]pyridin-1(6H)-yl)pyrrolidin-1-yl) pentanonitrile as a pale yellow oil (0.2g, 56%). The product can be used directly in the next step without further purification. HRMS (ESI): m/z $[M+H]^+$. $C_{25}H_{29}N_6O_3S$ calculated value 493.2016, measured value 493.2020.

Step 2: Dissolve 5-((S)-3-(2-((R)-1-hydroxyethyl)-6-(phenylsulfonyl)imidazo[4,5-d]pyrrolo[2,3-b]pyridin-1(6H)-yl)pyrrolidin-1-yl) pentanonitrile (0.2g, 0.4mmol) in a mixture of 5 mL tetrahydrofuran and 5 mL methanol, followed by adding 5 mL of 1 M sodium hydroxide and stirring at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS68:** 5-((S)-3-(2-((R)-1-Hydroxyethyl)imidazo[4,5-d]pyrrolo[2,3-b]pyridin-1(6H)-yl)pyrrolidin-1-yl)pentanonitrile (0.1 g, 70%). $^1$HNMR (300 MHz, *DMSO-$d_6$*): $\delta$ = 12.03 (s, 1H), 8.87 (s, 1H), 7.58 (s, 1H), 6.85 (s, 1H), 4.59-4.68 (m, 1H), 3.65-3.79 (m, 1H), 2.58-2.83 (m, 2H), 2,44 (t, *J = 9.0 Hz,* 2H), 2.19-2.28 (m, 2H), 1.90-2.13 (m, 2H), 1.90 (t, *J* = 9.0 Hz, 2H), 1.63-1.77 (m, 2H), 1.48 (d, *J*= 9.0 Hz, 3H), 1.25-1.30 (m, 2H) ppm; $^{13}$CNMR (75 MHz, *DMSO-$d_6$)* $\delta$ 148.94, 148.51, 142.11, 129.75, 127.63, 120.71, 119.39, 115.67, 99.52, 62.68, 58.93, 58.41, 55.96, 55.81, 27.63, 27.21, 23.22, 22.84, 17.18 ppm; HRMS (ESI): m/z $[M+H]^+$. $C_{19}H_{25}N_6O$ calculated value 353.2084, measured value 353.2088.

Example 69

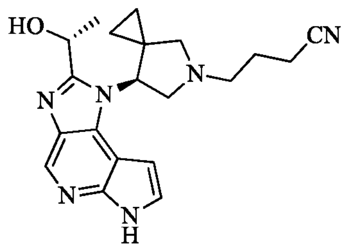

**LXS69**

4-((S)-7-(2-((R)-1-Hydroxyethyl)imidazo[4,5-d]pyrrolo[2,3-b]pyridin-1(6H)-yl)-5-azaspiro[2.4]hept-5-yl)butanenitrile

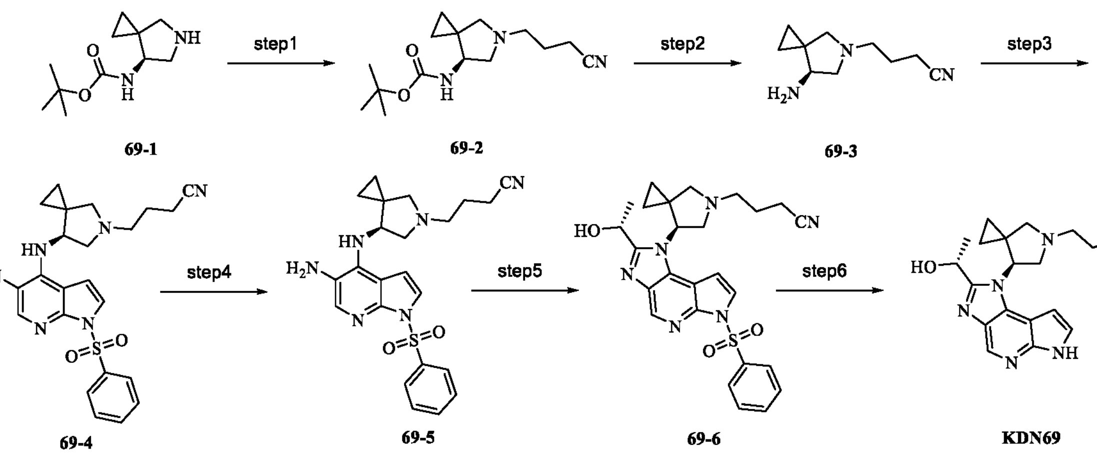

69-1
69-2
69-3
69-4
69-5
69-6
KDN69

Step 1: Dissolve tert-butyl (S)5-azaspiro[2.4]hept-7-carbamate(0.3g, 1.4mmol) in 10 mL of DMF, add potassium carbonate (0.4g, 2.8mmol) and 4-bromobutanenitrile (0.3g, 2.1mmol) and stir at room temperature for 12 h. Monitor the reaction for completion by MS. Saturated sodium bicarbonate was added until the reaction solution was weakly basic, and the organic phase was separated. The aqueous phase was extracted three times using dichloromethane. The organic phases were combined and washed with saturated saline, dried over anhydrous sodium sulfate and concentrated under vacuum to yield (S)-tert-butyl (5-(3-cyanopropyl)-5-azaspiro[2.4]hept-7-yl)carbamate as a pale yellow oil (0.3g,

76%) The product can be used directly in the next step without further purification. HRMS (ESI): m/z $[M+H]^+$. $C_{15}H_{26}N_3O_2$ calculated value 280.2020, measured value 280.2029.

Step 2: Dissolve (S)-tert-butyl (5-(3-cyanopropyl)-5-azaspiro[2.4]hept-7-yl)carbamate (0.3g, 1.1mmol) in 10 mL dichloromethane, slowly add trifluoroacetic acid (1.3g, 11.0mmol) and then stir at room temperature for 12 h. Concentrate under vacuum to obtain (S)-4-(7-amino-5-azaspiro[2.4]hept-5-yl)butanenitrile as a light yellow oil (0.2g, 100%). The product can be used directly in the next step without further purification. HRMS (ESI): m/z $[M+H]^+$.$C_{10}H_{18}N_3$ calculated value 180.1495, measured value 180.1499.

Step 3: 4-Chloro-5-nitro-1-(phenylsulfonyl)-1H-pyrrolo[2,3-b]pyridine (0.3g, 0.9mmol) was dissolved in 100 mL of tetrahydrofuran followed by adding DIPEA (0.7g, 5.5mmol) and (S)-4-(7-amino-5-azaspiro[2.4]hept-5-yl)butanenitrile (0.2g, 1.1mmol). Raise the temperature to maintain reflux and stir for 4 h. The reaction was monitored for completion by TLC. Concentrate under vacuum to yield a yellow oily liquid. Triturate and cure with an appropriate amount of methanol to yield (S)-4-(7-((5-nitro-1-(phenylsulfonyl)-1H-pyrrolo[2,3-b]pyridin-4-yl)amino)-5-azaspiro[2.4]hept-5-yl)butanenitrile as a yellow solid (0.3g, 70%). The product can be used directly in the next step without further purification. HRMS (ESI): m/z $[M+H]^+$. $C_{23}H_{25}N_6O_4S$ calculated value 481.1653, measured value 481.1659.

Step 4: Dissolve (S)-4-(7-((5-nitro-1-(phenylsulfonyl)-1H-pyrrolo[2,3-b]pyridin-4-yl)amino)-5-azaspiro[2.4]hept-5-yl)butanenitrile (0.3g, 0.6mmol) in 10 mL of methanol, add palladium carbon (0.1g, 10%) and replace the air in the reaction flask more than three times with hydrogen gas. The reaction was kept in hydrogen atmosphere and the reaction was stirred at room temperature for 12 h. The reaction was monitored for completion by TLC. The filtrate was collected and concentrated under vacuum to yield (S)-4-(7-(((5-amino-1-(phenylsulfonyl)-1H-pyrrolo[2,3-b]pyridin-4-yl)amino)-5-azaspiro[2.4]hept-5-yl)butanenitrile as a light pink foamy solid (0.3g, 100%). The product can be used directly in the next step without further purification. HRMS (ESI): m/z $[M+H]^+$. $C_{23}H_{27}N_6O_2S$ calculated value 451.1911, measured value 451.1922.

Step 5: Triethyloxonium tetrafluoroborate (0.4g, 2.0mmol) and (R)-lactamide (0.2g, 2.0mmol) were dissolved in 20 mL of tetrahydrofuran, stirred at room temperature for 3 h and concentrated under vacuum to obtain the mixture as an oil, followed by the addition of 20 mL of ethanol to dissolve and the addition of (S)-4-(7-(((5-amino-1-(phenylsulfonyl)-1H-pyrrolo[2,3-b]pyridin-4-yl)amino)-5-azaspiro[2.4]hept-5-yl)butanenitrile (0.3g, 0.7mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield 4-((S)-7-(2-((R)-1-Hydroxyethyl)-6-(phenylsulfonyl)imidazo[4,5-d]pyrrolo[2,3-b]pyridin-1(6H)-yl)-5-azaspiro [2.4] hept-5-yl)butanenitrile as a light yellow oil (0.2g, 60%). HRMS (ESI): m/z $[M+H]^+$. $C_{26}H_{29}N_6O_3S$ calculated value 505.2016, measured value 505.2020.

Step 6: 4-((S)-7-(2-((R)-1-Hydroxyethyl)-6-(phenylsulfonyl)imidazo[4,5-d]pyrrolo[2,3-b]pyridin-1(6H)-yl)-5-azaspiro[2.4]hept-5-yl)butanenitrile (0.2g, 0.4mmol) was dissolved in a solvent mixture of 5 mL tetrahydrofuran and 5 mL methanol, followed by adding 5 mL of 1 M sodium hydroxide and then stirring at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield LXS69: 4-((S)-7-(2-((R)-1-Hydroxyethyl)imidazo[4,5-d]pyrrolo[2,3-b]pyridin-1(6H)-yl)-5-azaspiro[2.4]hept-5-yl)butanenitrile (0.1g, 69%). $^1$HNMR(300 MHz, *DMSO-$d_6$*): $\delta$ = 11.96 (s, 1H), 8.79 (s, 1H), 7.59 (s, 1H), 6.85 (s, 1H), 4.58-4.71 (m, 1H), 3.70-3.77 (m, 1H), 2.66-2.83 (m , 2H), 2,47 (t, $J$ = 7.5 Hz, 2H), 2.12-2.22 (m, 2H), 1.88 (t, $J$ = 9.0 Hz, 2H), 1.76-1.80 (m, 2H), 1.48 (d, $J$= 9.0 Hz, 3H), 0.05-0.27 (m, 4H) ppm; $^{13}$CNMR (75 MHz, *DMSO-$d_6$*) $\delta$149.33, 148.58, 141.66, 129.05, 127.99, 120.78, 119.38, 115.64, 99.56, 71.22, 68.47, 63.68, 56.19, 53.48, 25.59, 22.83, 15.98, 15.11, 4.22 ppm; HRMS (ESI): m/z $[M+H]^+$. $C_{20}H_{25}N_6O$ calculated value 365.2084, measured value 365.2089.

Example 70

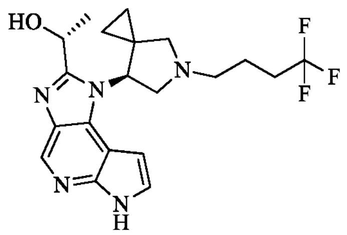

**LXS70**

(R)-1-(1-((S)-5-(4,4,4-Trifluorobutyl)-5-azaspiro[2.4]hept-7-yl)-1,6-dihydroimidazo[4,5-d]pyrrolo[2,3-b]pyridin-2-yl)ethanol

**LXS70** was prepared in a similar preparation way to Example 69 and the crude product was purified using a silica gel column (petroleum ether: ethyl acetate = 1:1) to yield **LXS70:** (R)-1-(1-((S)-5-(4,4,4-Trifluorobutyl)-5-azaspiro[2.4]hept-7-yl)-1,6-dihydroimidazo[4,5-d]pyrrolo[2,3-b]pyridin-2-yl) ethanol (0.1g, 66%). $^1$HNMR (300 MHz, *DMSO-$d_6$* ): $\delta$ = 12.03 (s, 1H), 8.77 (s, 1H), 7.61 (s, 1H), 6.93 (s, 1H), 4.60-4.73 (m, 1H), 3.73-3.79 (m, 1H), 2.56-2.81 (m, 2H), 2,43 (t, *J*= 9.0 Hz, 2H), 2.15-2.26 (m, 2H), 1.77-1.81 (m, 2H), 1.43 (d, *J*= 4.5 Hz, 3H), 1.26-1.38 (m, 2H), 0.02-0.22 (m, 4H) ppm; $^{13}$CNMR (75 MHz, *DMSO-$d_6$)* $\delta$148.93, 148.51, 142.16, 129.04, 127.11, 126.83, 120.77, 115.61, 99.78, 71.21, 67.54, 62.59, 56.93, 53.76, 37.74, 25.78, 22.81, 10.16, 5.83 ppm; HRMS (ESI): m/z [M+H]$^+$. $C_{20}H_{25}F_3N_5O$ calculated value 408.2006, measured value 408.2010.

Example 71

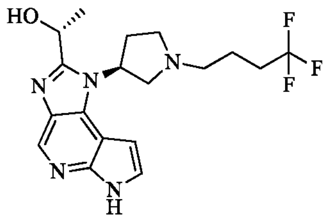

**LXS71**

(R)-1-(1-((S)-1-(4,4,4-Trifluorobutyl)pyrrolidin-3-yl)-1,6-dihydroimidazo[4,5-d]pyrrolo[2,3-b]pyridin-2-yl)ethanol

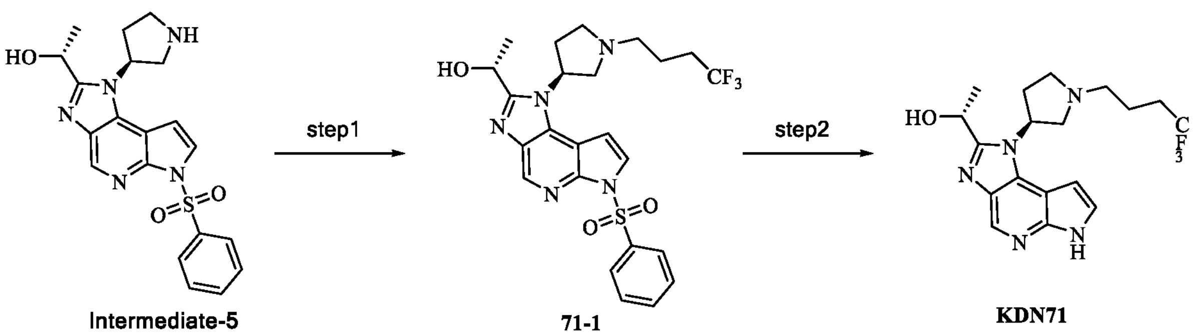


**Intermediate-5** **71-1** **KDN71**

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl) ethanol (0.3g, 0.7mmol) in 10 mL of tetrahydrofuran, add DIPEA (0.2g, 1.4mmol) followed by slow dropwise addition of 4,4,4-trifluoro-1-iodobutane(0.3g, 1.1mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The

organic phases were combined and washed with saturated saline, dried over anhydrous sodium sulfate and concentrated under vacuum to yield (R)-1-(6-(phenylsulfonyl)-1-((S)-1-(4,4,4-trifluorobutyl)pyrrolidin-3-yl)-1,6-dihydroimidazo[4,5-d]pyrrolo[2,3-b]pyridin-2-yl) ethanol as a pale yellow oil (0.3g, 79%). The product can be used directly in the next step without further purification. HRMS (ESI): m/z $[M+H]^+$. $C_{24}H_{27}F_3N_5O_3S$ calculated value 522.1781, measured value 522.1788.

Step 2: (R)-1-(6-(phenylsulfonyl)-1-((S)-1-(4,4,4-trifluorobutyl)pyrrolidin-3-yl)-1,6-dihydroimidazo[4,5-d] pyrrolo[2,3-b]pyridin-2-yl) ethanol (0.3g, 0.6mmol) was dissolved in a solvent mixture of 5 mL tetrahydrofuran and 5 mL methanol, and 5 mL of 1M sodium hydroxide was added and then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS71:** (R)-1-(1-((S)-1-(4,4,4-trifluorobutyl)pyrrolidin-3-yl)-1,6-dihydroimidazo[4,5-d]pyrrolo[2,3-b]pyridin-2-yl)ethanol (0.1 g, 46%). $^1$HNMR (300 MHz, *DMSO-$d_6$* ): $\delta$ = 12.04 (s, 1H), 8.83 (s, 1H), 7.56 (s, 1H), 6.89 (s, 1H), 4.61-4.74 (m, 1H), 3.71-3.79 (m, 1H), 2.56-2.81 (m, 2H), 2,41 (t, *J*= 4.5 Hz, 2H), 2.20-2.32 (m, 2H), 1.90-2.15 (m, 2H), 1.80-1.87 (m, 2H), 1.44 (d, *J*= 6.0 Hz, 3H), 1.35-1.40 (m, 2H) ppm; $^{13}$CNMR (75 MHz, *DMSO-$d_6$)* $\delta$ 150.11, 149.33, 144.34, 129.88, 127.19, 126.53, 120.77, 116.53, 98.57, 64.78, 58.93, 58.42, 56.32, 55.93, 37.77, 27.61, 22.84, 10.56 ppm; HRMS (ESI): m/z $[M+H]^+$. $C_{18}H_{23}F_3N_5O$ calculated value 382.1849, measured value 382.1852.

Example 72

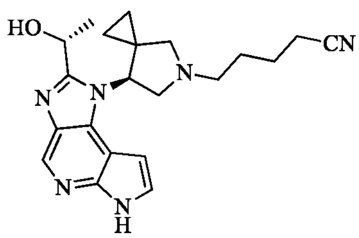

**LXS72**

5-((S)-7-(2-((R)-1-Hydroxyethyl)imidazo[4,5-d]pyrrolo[2,3-b]pyridin-1(6H)-yl)-5-azaspiro[2.4]hept-5-yl)pentanonitrile

LXS72 was prepared in a similar preparation way to Example 69 and the crude product was purified using a silica gel column (petroleum ether: ethyl acetate = 1:1) to yield LXS72: 5-((S)-7-(2-((R)-1-Hydroxyethyl)imidazo[4,5-d]pyrrolo[2,3-b]pyridin-1(6H)-yl)-5-azaspiro[2.4]hept-5-yl)pentanonitrile (0.1 g, 45%). $^1$HNMR (300 MHz, *DMSO-$d_6$* ): $\delta$ = 11.99 (s, 1H), 8.87 (s, 1H), 7.59 (s, 1H), 6.72 (s, 1H), 4.63-4.77 (m, 1H), 3.73-3.82 (m, 1H), 2.52-2.74 (m, 2H), 2,47 (t, *J = 6.0 Hz,* 2H), 2.12-2.23 (m, 2H), 1.87 (t, *J*= 6.0 Hz, 2H), 1.59-1.68 (m, 2H), 1.48 (d, *J* = 9.0 Hz, 3H), 1.33-1.41 (m, 2H), 0.08-0.22 (m, 4H) ppm; $^{13}$CNMR (75 MHz, *DMSO-$d_6$ )* $\delta$ 150.68, 149.47, 142.19, 129.33, 127.86, 120.73, 119.39, 115.62, 98.56, 71.29, 68.40, 65.27, 56.44, 53.49, 27.28, 25.58, 23.29, 22.81, 17.11, 5.88 ppm; HRMS (ESI): m/z $[M+H]^+$. $C_{21}H_{27}N_6O$ calculated value 379.2241, measured value 379.2243.

Example 73

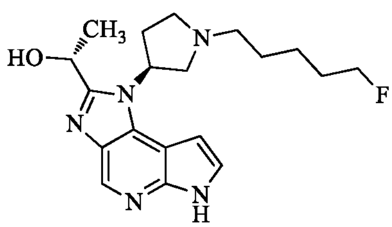

**LXS73**

(R)-1-(1-((S)-1-(5-Fluoropentyl)pyrrolidin-3-yl)-1,6-dihydroimidazo[4,5-d]pyrrolo[2,3-b]pyridin-2-yl)ethanol

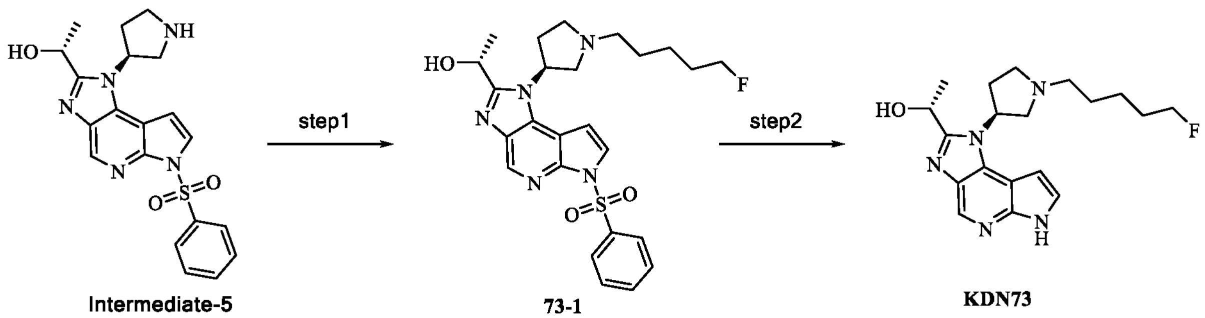


Intermediate-5 73-1 KDN73

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4 mmol) followed by slow dropwise addition of 1-bromo-5-fluoropentane (0.2g, 1.1mmol), raise the temperature to reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (R)-1-(1-((S)-1-(5-fluoropentyl)pyrrolidin-3-yl)-6-(phenylsulfonyl)-1,6-dihydroimidazo[4,5-d]pyrrolo[2,3-b]pyridin-2-yl)ethanol as a pale yellow oil (0.3g, 82%). The product can be used directly in the next step without further purification. HRMS (ESI): m/z $[M+H]^+$. $C_{25}H_{31}FN_5O_3S$ calculated value 500.2126, measured value 500.2130.

Step 2: (R)-1-(1-((S)-1-(5-Fluoropentyl)pyrrolidin-3-yl)-6-(phenylsulfonyl)-1,6-dihydroimidazo[4,5-d]pyrrolo[2,3-b]pyridin-2-yl) ethanol (0.3g, 0.6mmol) was dissolved in a mixture of 5 mL of tetrahydrofuran and 5 mL of methanol, and 5 mL of 1 M sodium hydroxide was added, then stir for 5 h at room temperature, the reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield **LXS73:** (R)-1-(1-((S)-1-(5-fluoropentyl)pyrrolidin-3-yl)-1,6-dihydroimidazo[4,5-d]pyrrolo[2,3-b]pyridin-2-yl) ethanol (0.1 g, 46%). $^1$HNMR (300 MHz, *DMSO-d$_6$*): $\delta$= 11.98 (s, 1H), 8.88 (s, 1H), 7.63 (s, 1H), 6.84 (s, 1H), 4.68-4.76 (m, 1H), 4.09-4.13 (m, 2H), 3.74-3.79 (m, 1H), 2.59-2.84 (m, 2H), 2,49 (t, *J* = 9.0 Hz, 2H), 2.19-2.30 (m, 2H), 1.91-2.18 (m, 2H), 1.49 (d, *J*= 9.0 Hz, 3H), 1.40-1.45 (m, 2H), 1.36-1.40 (m, 2H), 1.27-1.31 (m, 2H) ppm; $^{13}$CNMR (75 MHz, *DMSO-d$_6$*) $\delta$ 151.08, 148.53, 142.19, 129.04, 127.88, 120.78, 115.62, 98.67, 83.69, 64.55, 58.92, 58.44, 56.61, 55.92, 30.71, 28.09, 27.66, 19.45 ppm; HRMS (ESI): m/z $[M+H]^+$. $C_{19}H_{27}FN_5O$ calculated value 360.2194, measured value 360.2199.

Example 74

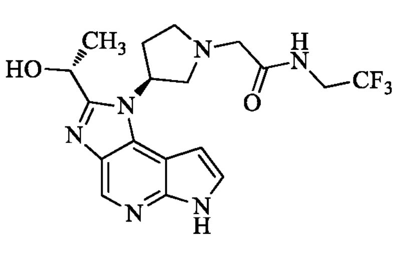

LXS74

2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)acetamide

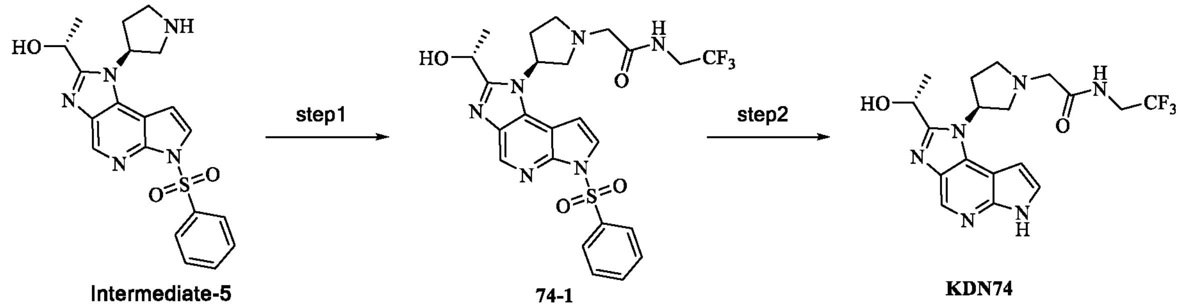


Intermediate-5 74-1 KDN74

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) followed by slow dropwise addition of 2-bromo-N-(2,2,2-trifluoroethyl)acetamide (0.3g, 1.1mmol).Raise the temperature to reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-((S)-3-(2-((R)-1-Hydroxyethyl)-6-(phenylsulfonyl)imidazo[4,5-d]pyrrolo[2,3-b]pyridin-1(6H)-yl)pyrrolidin-1-yl)-N-(2,2,2-trifluoroethyl)acetamide as a pale yellow oil (0.3 g, 75%). The product can be used directly in the next step without further purification. HRMS (ESI): m/z $[M+H]^+$. $C_{24}H_{26}F_3N_6O_4S$ calculated value 551.1683, measured value 552.1688.

Step 2: Dissolve 2-((S)-3-(2-((R)-1-hydroxyethyl)-6-(phenylsulfonyl)imidazo[4,5-d]pyrrolo[2,3-b]pyridin-1(6H)-yl)pyrrolidin-1-yl)-N-(2,2,2-trifluoroethyl)acetamide (0.3g, 0.5mmol) in 5 mL of a mixture of tetrahydrofuran and 5 mL of methanol. Add 5 mL of 1 M sodium hydroxide and then stir at room temperature for 5 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS74:** 2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)acetamide (0.1g, 45%). $^1$HNMR (300 MHz, *DMSO-$d_6$*): $\delta$ = 12.11 (s, 1H), 8.89 (s, 1H), 8.03 (s, 1H), 7.58 (s, 1H), 6.91 (s, 1H), 4.65-4.78 (m, 1H), 3.72-3.79 (m, 3H), 3.25 (s, 2H), 2.58-2.83 (m, 2H), 2.21-2.34 (m, 2H), 1.94-2.18 (m, 2H), 1.49 (d, *J* = 9.0 Hz, 3H) ppm; $^{13}$CNMR (75 MHz, *DMSO-$d_6$*) $\delta$ 170.55, 152.67, 148.55, 143.18, 129.04, 127.84, 124.79, 120.76, 115.69, 98.47, 64.87, 59.54, 58.28, 57.48, 54.94, 39.41, 27.88, 22.81 ppm; HRMS (ESI): m/z $[M+H]^+$. $C_{18}H_{22}F_3N_6O_2$ calculated value 411.1751, measured value 411.1777.

Example 75

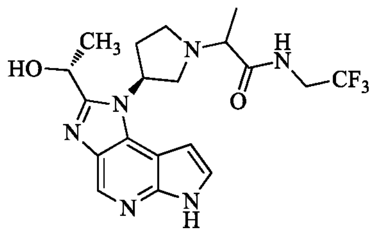

**LXS75**

2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)propanamide

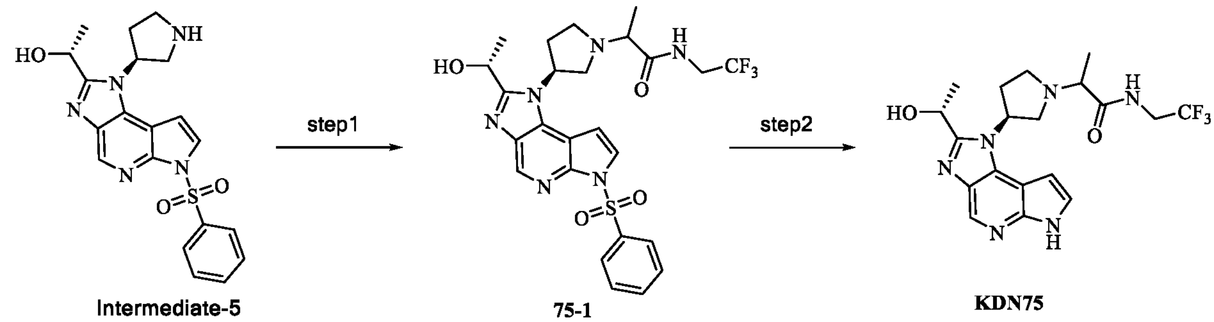


Intermediate-5 75-1 KDN75

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran. Add DIPEA (0.2g, 1.4mmol) and then slowly add *2-bromo-N-(2,2,2*-trifluoroethyl)propanamide (0.3g, 1.1mmol) dropwise, raise the temperature to reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-((S)-3-(2-((R)-1-hydroxyethyl)-6-(phenylsulfonyl)imidazo[4,5-d]pyrrolo[2,3-b]pyridin-1(6H)-yl)pyrrolidin-1-yl)-N-(2,2,2-trifluoroethyl)propanamide as a pale yellow oil (0.3g, 73%). The product can be used directly in the next step without further purification. HRMS (ESI): m/z $[M+H]^+$. $C_{25}H_{28}F_3N_6O_4S$ calculated value 565.1839, measured value 565.1841.

Step 2: Dissolve 2-((S)-3-(2-((R)-1-hydroxyethyl)-6-(phenylsulfonyl)imidazo[4,5-d]pyrrolo[2,3-b]pyridin-1(6H)-yl)pyrrolidin-1-yl)-N-(2,2,2-trifluoroethyl)propanamide (0.3g, 0.5mmol) in a mixture of 5 mL of tetrahydrofuran and 5 mL of methanol. Add 5 mL of 1 M sodium hydroxide and stir at room temperature for 5 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS75:** 2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)propanamide (0.1g, 44%). $^1$HNMR (300 MHz, *DMSO-d$_6$*): $\delta$ = 11.91 (s, 1H), 8.77 (s, 1H), 8.05 (s, 1H), 7.68 (s, 1H), 6.86 (s, 1H), 4.67-4.79 (m, 1H), 3.65-3.79 ( m, 4H), 2.56-2.81 (m, 2H), 2.18-2.31 (m, 2H), 1.90-2.15 (m, 2H), 1.41 (d, *J* = 7.5 Hz, 3H), 1.28 (d, *J* = 7.5 Hz, 3H) ppm; $^{13}$CNMR (75 MHz, *DMSO-d$_6$*) $\delta$ 171.96, 148.67, 148.51, 142.18, 129.04, 127.18, 124.75, 120.75, 115.64, 97.56, 69.29, 63.64, 58.59, 54.92, 52.48, 39.47, 27.36, 22.86, 18.44 ppm; HRMS (ESI): m/z $[M+H]^+$. $C_{19}H_{24}F_3N_6O_2$ calculated value 425.1907, measured value 425.1911.

Example 76

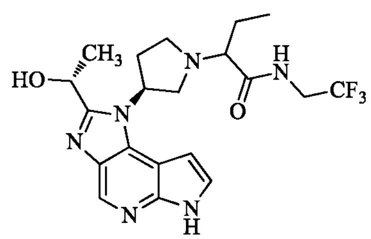

**LXS76**

2-((S)-3-(2-((R)-1-Hydroxyethyl)imidazo[4,5-d]pyrrolo[2,3-b]pyridin-1(6H)-yl)pyrrolidin-1-yl)-N-(2,2,2-trifluoroethyl)butanamide

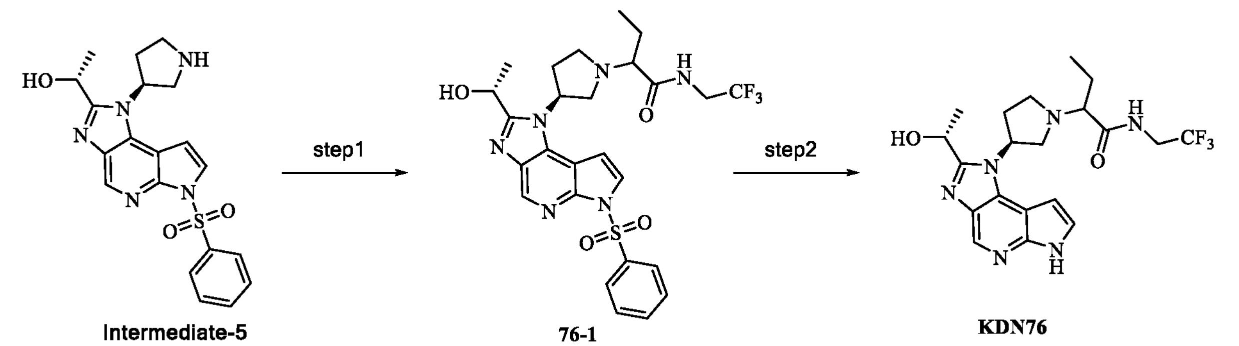


Intermediate-5 76-1 KDN76

Step 1: Dissolve (R)-1-(6-(phenylsulfonyl)-1-((S-pyrrolidin-3-yl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) followed by the slow dropwise addition of 2-bromo-N-(2,2,2-trifluoroethyl)butanamide (0.3g, 1.1mmol). Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-((S)-3-(2-((R)-1-hydroxyethyl)-6-(phenylsulfonyl)imidazo[4,5-d]pyrrolo[2,3-b]pyridin-1(6H)-yl)pyrrolidin-1-yl)-N-(2,2,2-trifluoroethyl)butanamide as a pale yellow oil (0.3g, 71%). The product can be used directly in the next step without further purification. HRMS (ESI): m/z $[M+H]^+$. $C_{26}H_{30}F_3N_6O_4S$ calculated value 579.1996, measured value 579.2001.

Step 2: Dissolve 2-((S)-3-(2-((R)-1-hydroxyethyl)-6-(phenylsulfonyl)imidazo[4,5-d]pyrrolo[2,3-b]pyridin-1(6H)-yl)pyrrolidin-1-yl)-N-(2,2,2-trifluoroethyl)butanamide (0.3g, 0.5mmol) in a mixture of 5 mL tetrahydrofuran and 5 mL methanol. Add 5 mL of 1M sodium hydroxide and stir at room temperature for 5 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS76:** 2-((S)-3-(2-((R)-1-Hydroxyethyl)imidazo[4,5-d]pyrrolo[2,3-b]pyridin-1(6H)-yl)pyrrolidin-1-yl)-N- (2,2,2-trifluoroethyl)butanamide (0.1 g, 44%). $^1$HNMR (300 MHz, *DMSO-d$_6$*): $\delta$= 12.01 (s, 1H), 8.78 (s, 1H), 8.08 (s, 1H), 7.53 (s, 1H), 6.79 (s, 1H), 4.68-4.74 (m, 1H), 3.56-3.79 ( m, 4H), 2.57-2.85 (m, 2H), 2.20-2.30 (m, 2H), 1.93-2.17 (m, 2H), 1.57-1.64 (m, 2H), 1.48 (d, *J* = 7.5 Hz, 3H), 0.91 (t, *J* = 9.0 Hz, 3H) ppm; $^{13}$CNMR (75 MHz, *DMSO-d$_6$)* $\delta$ 173.88, 151.77, 148.53, 142.11, 129.04, 127.17, 124.77, 120.71, 115.63, 98.56, 75.93, 64.65, 58.53, 55.22, 52.71, 39.48, 27.63, 23.66, 22.84, 12.89 ppm; HRMS (ESI): m/z $[M+H]^+$. $C_{20}H_{26}F_3N_6O_2$ calculated value 439.2064, measured value 439.2072.

Example 77

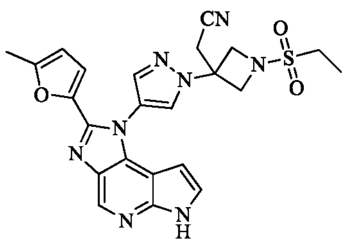

LXS77

2-(1-(Ethylsulfonyl)-3-(4-(2-(5-methylfuran-2-yl)imidazolyl[4,5-d]pyrrolo[2,3-b]pyridin-1 (6H)-yl)-1H-pyrazol-1-yl)azetidin-3-yl)acetonitrile

**LXS77** was prepared using a similar preparation way to Example 63 and the crude product was purified using a silica gel column (petroleum ether: ethyl acetate = 1:1) to yield **LXS77:** 2-(1-(ethylsulfonyl)-3-(4-(2-(5-methylfuran-2-yl)imidazolyl[4,5-d]pyrrolo[2,3-b]pyridin-1(6H)-yl)-1H-pyrazol-1-yl) azetidin-3-yl) acetonitrile (0.1 g, 53%). $^1$HNMR (300 MHz, *DMSO-d$_6$*): $\delta$= 12.07 (s, 1H), 8.88 (s, 1H), 8.08 (s, 2H), 7.58 (s, 1H), 6.95 (d, *J* = 7.5 Hz, 1H), 6.83 (s, 1H), 6.08

(d, $J$ = 7.5 Hz, 1H), 4.80-4.14 (m, 4H), 3.45 (t, $J$ = 9.0 Hz, 2H), 2.85 (s, 2H), 2.30 (s, 3H), 1.22 (t, $J$= 7.5 Hz, 3H) ppm; $^{13}$CNMR (75 MHz, *DMSO-d$_6$)* $\delta$ 152.28, 151.49, 149.11, 143.19, 141.55, 130.75, 129.78, 129.01, 128.66, 121.67, 117.78, 115.61, 107.81, 107.62, 100.57, 99.56, 59.27, 51.63, 50.83, 23.27, 15.98, 4.77 ppm. HRMS (ESI): m/z [M+H]$^+$. $C_{23}H_{23}N_8O_3S$ calculated value 491.1608, measured value 491.1612.

Example 78

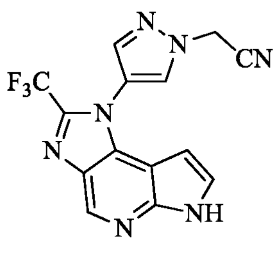

**LXS78**

2-(4-(2-(Trifluoromethyl)imidazo[4,5-d]pyrrolo[2,3-b]pyridin-1(6H)-yl)-1H-pyrazol-1-yl)acetonitrile

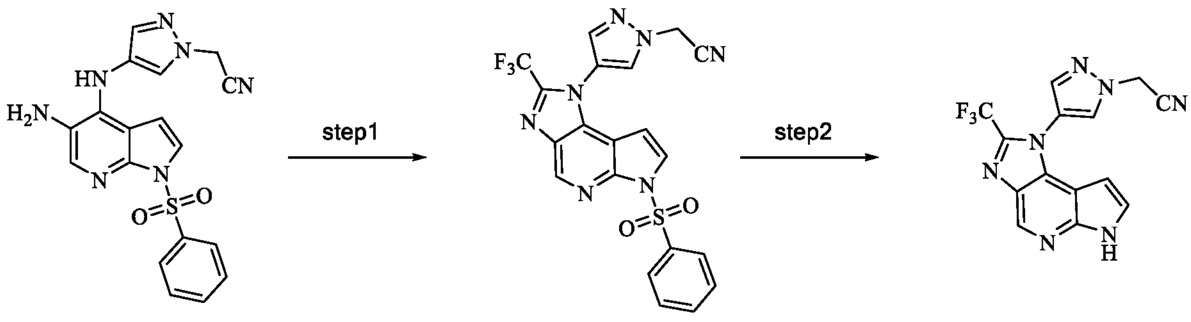


Intermediate-11 78-1 KDN78

Step 1: Triethyloxonium tetrafluoroborate (0.5g, 2.4mmol) and 2,2,2-trifluoroacetamide (0.2g, 2.4mmol) were dissolved in 10 mL of tetrahydrofuran, stirred at room temperature for 3 h and concentrated under vacuum to obtain the mixture in the form of oil, followed by the addition of 10 mL of ethanol to dissolve and the addition of 2-(4-((5-amino-1-(phenylsulfonyl)-1H-pyrrolo[2,3-b]pyridin-4-yl)amino)-1H-pyrazol-1-yl)acetonitrile(0.3g , 0.8mmol). Raise the temperature to maintain reflux and stir for 3 h. The reaction was monitored by TLC for complete reaction. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(4-(6-(phenylsulfonyl)-2-(trifluoromethyl)imidazo[4,5-d]pyrrolo[2,3-b]pyridin-1(6H)-yl)-1H-pyrazol-1-yl) acetonitrile as a milk white oil (0.3 g, 83%). HRMS (ESI): m/z [M+H]$^+$. $C_{20}H_{13}F_3N_7O_2S$ calculated value 472.0798, measured value 472.0802.

Step 2: Dissolve 2-(4-(6-(phenylsulfonyl)-2-(trifluoromethyl)imidazo[4,5-d]pyrrolo[2,3-b]pyridin-1(6H)-yl)-1H-pyrazol-1-yl)acetonitrile (0.3g, 0.6mmol) in a solvent mixture of 5 mL tetrahydrofuran and 5 mL methanol, and stir at room temperature for 5 h after adding 5 mL of 1 M sodium hydroxide. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS78:** 2-(4-(2-(trifluoromethyl)imidazo[4,5-d]pyrrolo[2,3-b]pyridin-1(6H)-yl)-1H-pyrazol-1-yl)acetonitrile (0.1g, 47%). $^1$HNMR (300 MHz, *DMSO-d$_6$*): $\delta$ = 11.97 (s, 1H), 8.87 (s, 1H), 8.05 (s, 1H), 7.91 (s, 1H), 7.56 (s, 1H), 6.83 (s, 1H), 4.85 (s, 2H) ppm; $^{13}$CNMR (75 MHz, *DMSO-d$_6$)* $\delta$ 148.11, 144.33, 130.74, 129.74, 129.04, 127.65, 120.73, 116.73, 116.32, 115.62, 100.53, 98.45, 45.65 ppm; HRMS (ESI): m/z [M+H]$^+$. $C_{14}H_9F_3N_7$ calculated value 332.0866, measured value 332.0871.

Example 79

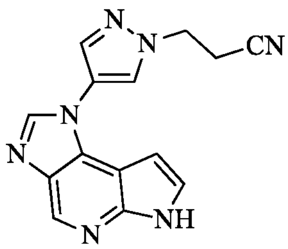

**LXS79**

3-(4-(Imidazo[4,5-d]pyrrolo[2,3-b]pyridin-1(6H)-yl)-1H-pyrazol-1-yl)propanenitrile

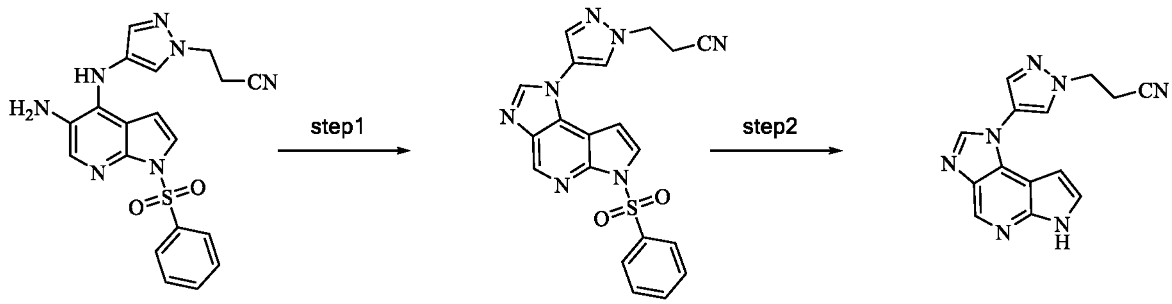


Intermediate-10 79-1 KDN79

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3g, 0.7mmol) in 10 mL of toluene, followed by the addition of triethyl orthoformate (0.2g, 1.4mmol), p-toluenesulfonic acid (30.0mg, 10%), and raise the temperature to maintain reflux and stir for 3 h.. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield 3-(4-(6-(phenylsulfonyl)imidazo[4,5-d]pyrrolo[2,3-b]pyridin-1(6H)-yl)-1H-pyrazol-1-yl)propanenitrile as a pale yellow oil (0.3g, 98%). HRMS (ESI): m/z $[M+H]^+$. $C_{20}H_{16}N_7O_2S$ calculated value 418.1081, measured value 418.1088.

Step 2: Dissolve 3-(4-(6-(phenylsulfonyl)imidazo[4,5-d]pyrrolo[2,3-b]pyridin-1(6H)-yl)-1H-pyrazol-1-yl)propanenitrile (0.3g, 0.7mmol) in a solvent mixture of 5 mL tetrahydrofuran and 5 mL methanol, add 5 mL of 1 M sodium hydroxide and then stir at room temperature for 5 h. Monitor the reaction by TLC for completion. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS79:** 3-(4-(imidazo[4,5-d]pyrrolo[2,3-b]pyridin-1(6H)-yl)-1H-pyrazol-1-yl)propanenitrile (0.1g, 50%). $^1$HNMR (300 MHz, *DMSO-d$_6$*): $\delta$ = 11.99 (s, 1H), 8.89 (s, 1H), 8.15 (s, 1H), 7.95 (s, 1H), 7.56 (s, 1H), 7.15 (s, 1H), 6.89 (s, 1H), 5.04 (t, $J$ = 7.5 Hz, 2H), 3.21 (t, $J$= 7.5 Hz, 2H) ppm; $^{13}$CNMR (75 MHz, *DMSO-d$_6$)* $\delta$ 150.44, 142.88, 134.58, 130.74, 129.73, 129.04, 127.16, 120.74, 117.73, 115.60, 100.54, 98.62, 49.37, 17.50 ppm; HRMS (ESI): m/z $[M+H]^+$. $C_{14}H_{12}N_7$ calculated value 278.1149, measured value 278.1151.

Example 80

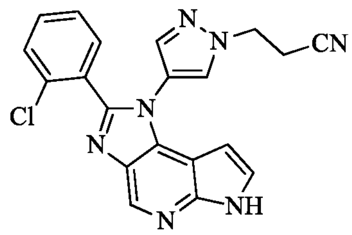

**LXS80**

3-(4-(2-(2-Chlorophenyl)imidazo[4,5-d]pyrrolo[2,3-b]pyridin-1(6H)-yl)-1H-pyrazol-1-yl)propanenitrile

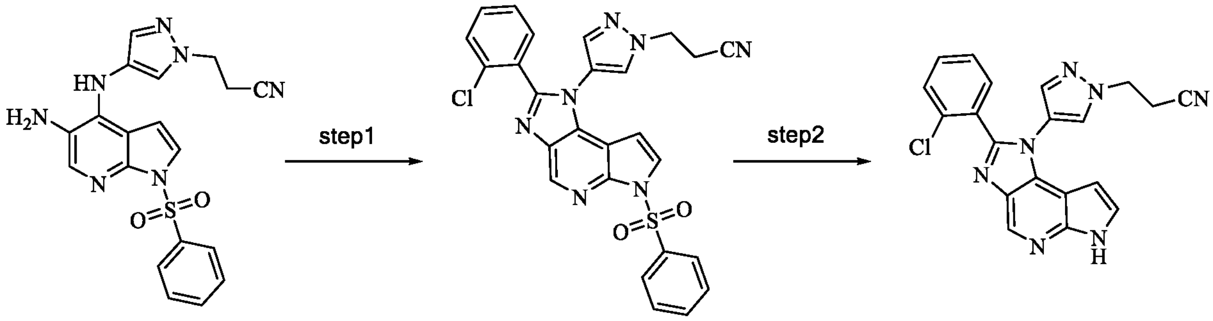


**Intermediate-10** **80-1** **KDN80**

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3g, 0.7mmol) in 10 mL of DMF, add $Na_2S_2O_5$ (0.7g, 3.5mmol) followed by the addition of 2-chlorobenzaldehyde (0.2g, 1.0mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline and dried over anhydrous sodium sulfate to yield 3-(4-(2-(2-chlorophenyl)-6-(phenylsulfonyl)imidazo[4,5-d]pyrrolo[2,3-b]pyridin-1(6H)-yl)-1H-pyrazol-1-yl)propanenitrile as a yellow solid (0.3g, 77%). HRMS (ESI): m/z $[M+H]^+$. $C_{26}H_{19}ClN_7O_2S$ calculated value 528.1004, measured value 528.1014.

Step 2: Dissolve 3-(4-(2-(2-chlorophenyl)-6-(phenylsulfonyl)imidazo[4,5-d]pyrrolo[2,3-b]pyridin-1(6H)-yl)-1H-pyrazol-1-yl)propanenitrile (0.3g, 0.6mmol) in a mixture of 5 mL tetrahydrofuran and 5 mL methanol, add 5 mL of 1 M sodium hydroxide and then stir at room temperature for 5 hours. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS80:** 3-(4-(2-(2-Chlorophenyl)imidazo[4,5-d]pyrrolo[2,3-b]pyridin-1(6H)-yl)-1H-pyrazol-1-yl)propanenitrile (0.1g, 45%). $^1$HNMR (300 MHz, *DMSO-$d_6$*): $\delta$ = 12.03 (s, 1H), 8.78 (s, 1H), 8.08 (s, 1H), 7.99 (s, 1H), 7.73 (d, *J* = 7.5 Hz, 1H), 7.58 (s, 1H), 7.55 (d, *J*= 7.5 Hz, 1H), 7.35-7.39 (m, 2H), 6.85 (s, 1H), 5.09 (t, *J* = 9.0 Hz, 2H), 3.28 (t, *J*= 9.0 Hz, 2H) ppm; $^{13}$CNMR (75 MHz, *DMSO-$d_6$)* $\delta$ 149.21, 143.48, 142.19, 138.56, 132.27, 130.77, 130.16, 129.75, 129.38, 129.05, 127.30, 127.17, 120.77, 117.74, 115.64, 100.63, 98.56, 50.45, 17.54 ppm; HRMS (ESI): m/z $[M+H]^+$. $C_{20}H_{15}ClN_7$ calculated value 388.1072, measured value 388.1077.

Example 81

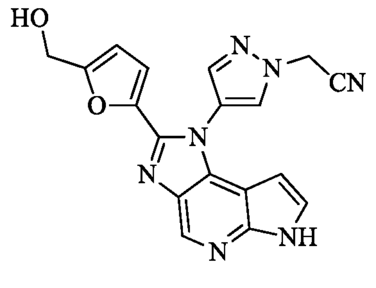

**LXS81**

2-(4-(2-(5-(Hydroxymethyl)furan-2-yl)imidazo[4,5-d]pyrrolo[2,3-b]pyridin-1(6H)-yl)-1H-pyrazol-1-yl)acetonitrile

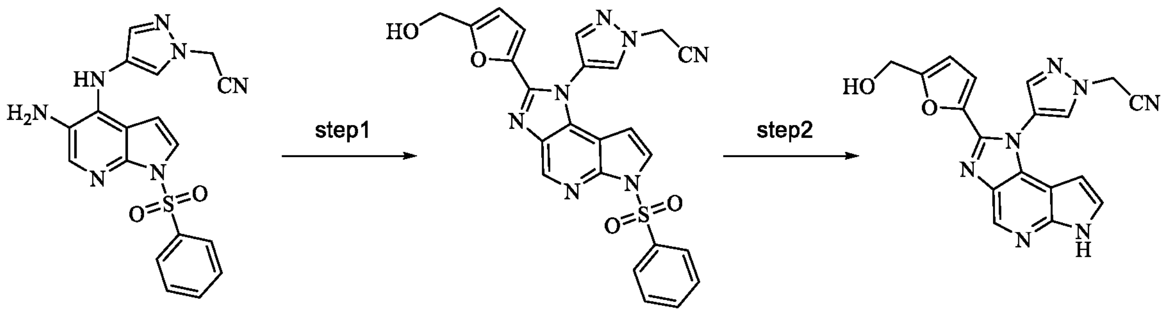


**Intermediate-11** **81-1** **KDN81**

Step 1: Dissolve 2-(4-((5-amino-1-(phenylsulfonyl)-1H-pyrrolo[2,3-b]pyridin-4-yl)amino)-1H-pyrazol-1-yl)acetonitrile (0.3g, 0.8mmol) in 10 mL of DMF, add $Na_2S_2O_5$ (0.8g, 4.0mmol) followed by adding 5-hydroxymethylfurfural (0.3g, 1.6mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 2-(4-(2-(5-(hydroxymethyl)furan-2-yl)-6-(phenylsulfonyl)imidazo[4,5-d]pyrrolo[2,3-b]pyridin-1(6H)-yl )-1H-pyrazol-1-yl) acetonitrile as a pale yellow oil (0.3g, 79%). HRMS (ESI): m/z $[M+H]^+$. $C_{24}H_{18}N_7O_4S$ calculated value 500.1135, measured value 500.1140.

Step 2: Dissolve 2-(4-(2-(5-(hydroxymethyl)furan-2-yl)-6-(phenylsulfonyl)imidazo[4,5-d]pyrrolo[2,3-b]pyridin-1(6H)-yl)-1H-pyrazol-1-yl)acetonitrile (0.3g, 0.6mmol) in a mixture of 5 mL of tetrahydrofuran and 5 mL of methanol, then add 5 mL of 1 M sodium hydroxide. Stir for 5 h at room temperature. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum. The residue was purified using a silica gel column (petroleum ether: ethyl acetate = 1:1) to yield **LXS81:** 2-(4-(2-(5-(Hydroxymethyl)furan-2-yl)imidazo[4,5-d]pyrrolo[2,3-b]pyridin-1(6H)-yl)-1H-pyrazol-1-yl) acetonitrile (0.1g, 46%). $^1$HNMR (300 MHz, *DMSO-$d_6$* ): $\delta$ = 11.93 (s, 1H), 8.91 (s, 1H), 8.05 (s, 1H), 7.91 (s, 1H), 7.56 (s, 1H), 7.02 (d, *J*= 7.5 Hz, 1H), 6.89 (s, 1H), 6.59 (d, *J*= 7.5 Hz, 1H), 4.85 (s, 2H), 4.39 (s, 2H) 3.65 (s, 1H) ppm; $^{13}$CNMR (75 MHz, *DMSO-$d_6$)* $\delta$ 153.88, 151.17, 149.67, 142.18, 141.33, 130.75, 129.75, 129.07, 127.19, 120.77, 116.36, 115.63, 107.96, 104.07, 100.58, 98.67, 57.38, 46.17 ppm; HRMS (ESI): m/z $[M+H]^+$. $C_{18}H_{14}N_7O_2$ calculated value 360.1203, measured value 360.1211.

Example 82

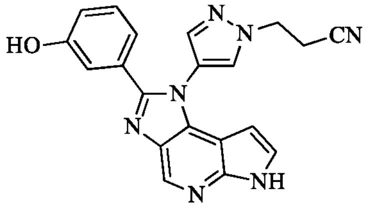

**LXS82**

3-(4-(2-(3-Hydroxyphenyl)imidazo[4,5-d]pyrrolo[2,3-b]pyridin-1(6H)-yl)-1H-pyrazol-1-yl)propanenitrile

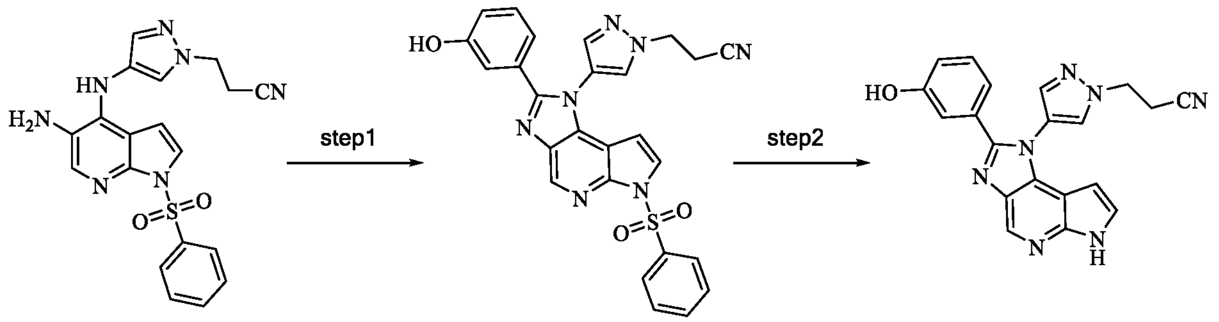


Intermediate-10

**82-1**

**KDN82**

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3g, 0.7mmol) in 10 mL of DMF, add $Na_2S_2O_5$ (0.7g, 3.5mmol) followed by adding 3-hydroxybenzaldehyde (0.2g, 1.0mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline and dried over anhydrous sodium sulfate to yield 3-(4-(2-(3-hydroxyphenyl)-6-(phenylsulfonyl)imidazo[4,5-d]pyrrolo[2,3-b]pyridin-1(6H)-yl)-1H-pyrazol-1-yl)propanenitrile as a yellow solid (0.3g, 79%). HRMS (ESI): m/z $[M+H]^+$. $C_{26}H_{20}N_7O_3S$ calculated value 510.1343, measured value 510.1351.

Step 2: Dissolve 3-(4-(2-(3-hydroxyphenyl)-6-(phenylsulfonyl)imidazo[4,5-d]pyrrolo[2,3-b]pyridin-1(6H)-yl)-1H-pyrazol-1-yl)propanenitrile (0.3g, 0.6mmol) in a mixture of 5 mL of tetrahydrofuran and 5 mL of methanol, add 5 mL of 1 M sodium hydroxide and then stir at room temperature for 5 hours. The reaction was monitored completion by TLC for 5 hours. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS82:** 3-(4-(2-(3-Hydroxyphenyl)imidazo[4,5-d]pyrrolo[2,3-b]pyridin-1(6H)-yl)-1H-pyrazol-1-yl)propanenitrile (0.1g, 46%). $^1$HNMR (300 MHz, *DMSO-d$_6$*): $\delta$ = 11.79 (s, 1H), 8.83 (s, 1H), 8.06 (s, 1H), 7.94 (s, 1H), 7.84 (d, *J*= 9.0 Hz, 1H), 7.59 (s, 1H), 7.34 (t, *J* = 9.0 Hz, 1H), 7.04 (s, 1H), 6.91 (d, *J* = 9.0 Hz, 1H), 6.89 (s, 1H), 5.35 (s, 1H), 5.03 (t, *J* = 7.5 Hz, 2H), 3.29 (t, *J* = 7.5 Hz, 2H) ppm; $^{13}$CNMR (75 MHz, *DMSO-d$_6$*) $\delta$ 157.59, 149.56, 143.43, 142.19, 132.08, 130.75, 130.61, 129.77, 120.06, 127.18, 120.74, 120.15, 117.74, 115.97, 115.61, 112.95, 100.59, 98.65, 50.65, 18.66 ppm; HRMS (ESI): m /z $[M+H]^+$. $C_{20}H_{16}N_7O$ calculated value 370.1411, measured value 370.1419.

Example 83

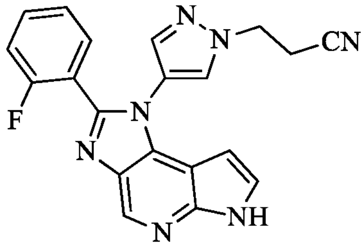

**LXS83**

3-(4-(2-(2-Fluorophenyl)imidazo[4,5-d]pyrrolo[2,3-b]pyridin-1(6H)-yl)-1H-pyrazol-1-yl)propanenitrile

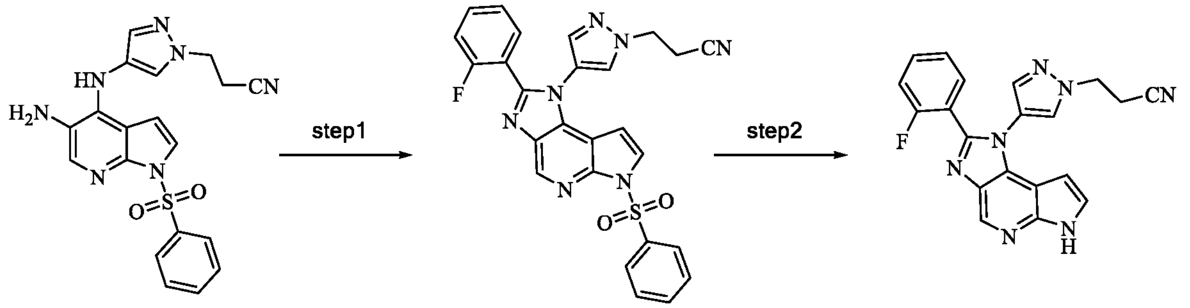


**Intermediate-10** **83-1** **KDN83**

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3g, 0.7mmol) in 10 mL of DMF, add $Na_2S_2O_5$ (0.7g, 3.5mmol) followed by adding 2-fluorobenzaldehyde (0.2g, 1.0mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline and dried over anhydrous sodium sulfate to yield 3-(4-(2-(2-fluorophenyl)-6-(phenylsulfonyl)imidazo[4,5-d]pyrrolo[2,3-b]pyridin-1(6H)-yl)-1H-pyrazol-1-yl)propanenitrile as a yellow solid (0.3g, 80%). HRMS (ESI): m/z $[M+H]^+$. $C_{26}H_{19}FN_7O_2S$ calculated value 512.1299, measured value 512.1301.

Step 2: Dissolve 3-(4-(2-(2-fluorophenyl)-6-(phenylsulfonyl)imidazo[4,5-d]pyrrolo[2,3-b]pyridin-1(6H)-yl)-1H-pyrazol-1-yl)propanenitrile (0.3g, 0.6mmol) in a mixture of 5 mL of tetrahydrofuran and 5 mL of methanol, add 5 mL of 1 M sodium hydroxide and then stir at room temperature for 5 hours. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS83:** 3-(4-(2-(2-Fluorophenyl)imidazo[4,5-d]pyrrolo[2,3-b]pyridin-1(6H)-yl)-1H-pyrazol-1-yl)propanenitrile (0.1g, 46%). 46%). $^1$HNMR (300 MHz, *DMSO-d$_6$*): $\delta$ = 12.07 (s, 1H), 8.78 (s, 1H), 8.05 (s, 1H), 7.96 (s, 1H), 7.71-7.77 (m, 2H), 7.56 (s, 1H), 7.49 (d, *J*= 7.5 Hz, 1H), 7.28 (t, *J*= 7.5 Hz, 1H), 6.88 (s, 1H), 5.04 (t, *J* = 9.0 Hz, 2H), 3.21 (t, *J* = 9.0 Hz, 2H) ppm; $^{13}$CNMR (75 MHz, *DMSO-d$_6$*) $\delta$ 158.39, 148.63, 143.39, 142.17, 130.77, 130.38, 129.76, 129.16, 129.06, 127.17, 124.88, 123.59, 120.74, 117.74, 115.69, 114.74, 101.76, 100.43, 51.98, 22.51 ppm; HRMS (ESI): m/z $[M+H]^+$. $C_{20}H_{15}FN_7$ 372.1367, measured value 372.1377.

Example 84

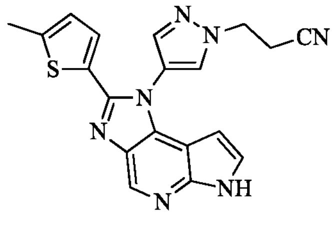

**LXS84**

3-(4-(2-(5-Methylthiophen-2-yl)imidazo[4,5-d]pyrrolo[2,3-b]pyridin-1(6H)-yl)-1H-pyrazol-1-yl)propanenitrile

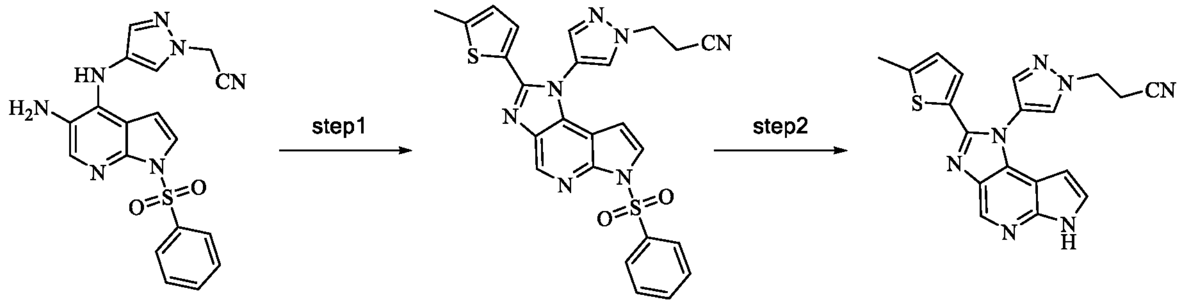


Intermediate-11 84-1 KDN84

Step 1: Dissolve 3-(4-((5-amino-1-(phenylsulfonyl)-1H-pyrrolo[2,3-b]pyridin-4-yl)amino)-1H-pyrazol-1-yl)propanenitrile (0.3g, 0.7mmol) in 10 mL of DMF, add $Na_2S_2O_5$ (0.7g, 3.5mmol) followed by adding 5-methyl-2-thiophenecarboxaldehyde (0.2g, 1.0mmol). Stir for 12h at 90 °C. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline and dried over anhydrous sodium sulfate to yield 3-(4-(2-(5-methylthiophen-2-yl)-6-(phenylsulfonyl)imidazo[4,5-d]pyrrolo[2,3-b]pyridin-1(6H)-yl)-1H-pyrazol-1-yl)propanenitrile as a yellow solid (0.3g, 80%). HRMS (ESI): m/z $[M+H]^+$. $C_{25}H_{20}N_7O_2S_2$ calculated value 514.1114, measured value 514.1119.

Step 2: Dissolve 3-(4-(2-(5-methylthiophen-2-yl)-6-(phenylsulfonyl)imidazo[4,5-d]pyrrolo[2,3-b]pyridin-1(6H)-yl)-1H-pyrazol-1-yl)propanenitrile (0.3g, 0.6mmol) in a solvent mixture of 5 mL tetrahydrofuran and 5 mL methanol. After the addition of 5 mL of 1M sodium hydroxide, the mixture was then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS84:** 3-(4-(2-(5-methylthiophen-2-yl)imidazo[4,5-d]pyrrolo[2,3-b]pyridin-1(6H)-yl)-1H-pyrazol-1-yl)propanenitrile (0.1g, 46%). $^1$HNMR (300 MHz, *DMSO-d$_6$*): $\delta$ = 11.93 (s, 1H), 8.83 (s, 1H), 8.06 (s, 1H), 7.99 (s, 1H), 7.56 (s, 1H), 7.51 (d, $J$ = 7.5 Hz, 1H), 6.89 (s, 1H), 6.83 (d, $J$ = 7.5 Hz, 1H), 5.10 (t, $J$ = 6.0 Hz, 2H), 3.26 (t, $J$ = 6.0 Hz, 2H), 2.36 (s, 3H) ppm; $^{13}$CNMR (75 MHz, *DMSO-d$_6$)* $\delta$ 151.63, 142.17, 141.63, 141.30, 134.38, 130.74, 129.74, 129.06, 127.59, 127.50, 127.14, 120.79, 117.78, 115.62, 101.86, 99.38, 51.96, 15.93, 15.22 ppm; HRMS (ESI): m/z $[M+H]^+$. $C_{19}H_{16}N_7S$ calculated value 374.1182, measured value 374.1189.

Example 85

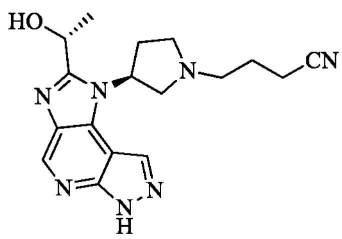

**LXS85**

4-((S)-3-(2-((R)-1-Hydroxyethyl)imidazo[4,5-d]pyrazolo[3,4-b]pyridin-1(6H)-yl)pyrrolidin-1-yl)butanenitrile

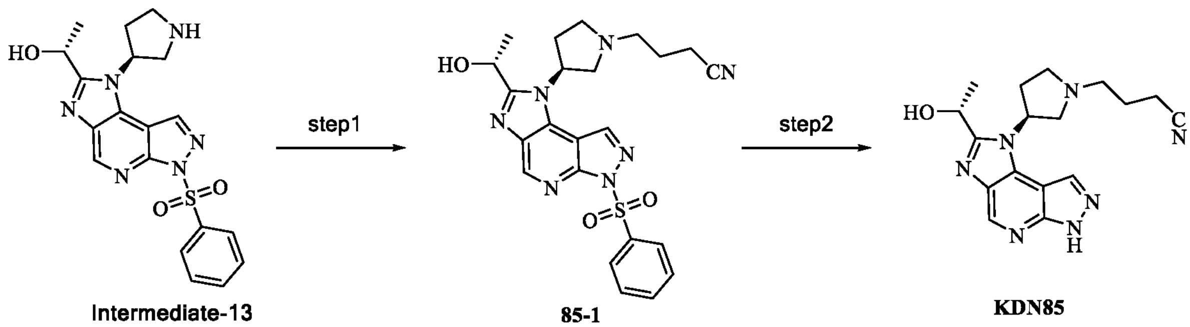


Intermediate-13 85-1 KDN85

Step 1: Dissolve (R)-1-(6-(phenylsulfonyl)-1-((S)-pyrrolidin-3-yl)-1,6-dihydroimidazo[4,5-d]pyrazolo[3,4-b]pyridin-2-yl) ethanol (0.3g, 0.7mmol) in 10 mL of tetrahydrofuran, add DIPEA (0.2g, 1.4mmol) followed by the slow dropwise addition of 4-bromobutyronitrile (0.2g, 1.1mmol). Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield 4-((S)-3-(2-((R)-1-hydroxyethyl)-6-(phenylsulfonyl)imidazo[4,5-d]pyrazolo[3,4-b]pyridin-1(6H)-yl)pyrrolidin-1-yl)butyronitrile as a pale yellow oil (0.2g, 57%). The product can be used directly in the next step without further purification. HRMS (ESI): m/z $[M+H]^+$. $C_{23}H_{26}N_7O_3S$ calculated value 480.1812, measured value 480.1825.

Step 2: 4-((S)-3-(2-((R)-1-Hydroxyethyl)-6-(phenylsulfonyl)imidazo[4,5-d]pyrazolo[3,4-b]pyridin-1(6H)-yl)pyrrolidin-1-yl)butyronitrile (0.2g, 0.4mmol) was dissolved in a solvent mixture of 5 mL tetrahydrofuran and 5 mL methanol. After the addition of 5 mL of 1 M sodium hydroxide, stir the mixture for 5 h at room temperature and monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS85:** 4-((S)-3-(2-((R)-1-hydroxyethyl)imidazo[4,5-d]pyrazolo[3,4-b]pyridin-1(6H)-yl)pyrrolidin-1-yl)butanenitrile (0.1g, 71%). $^1$HNMR (300 MHz, *DMSO-d$_6$*): $\delta$ = 12.54 (s, 1H), 8.89 (s, 1H), 7.55 (s, 1H), 4.65-4.70 (m, 1H), 3.64-3.81 (m, 1H), 2.56-2.83 (m, 2H), 2.43 (t, *J* = 9.0 Hz, 2H), 2.23-2.31 (m, 2H), 1.90-2.15 (m, 2H), 1.87 (t, *J* = 9.0 Hz, 2H), 1.66-1.76 (m, 2H), 1.41 (d, *J*= 6.0 Hz, 3H) ppm; $^{13}$CNMR (75 MHz, *DMSO-d$_6$)* $\delta$ 154.79, 148.53, 140.85, 140.53, 132.46, 125.49, 119.39, 104.75, 63.68, 58.52, 58.41, 55.92, 55.51, 27.61, 22.85, 15.94, 15.11 ppm; HRMS (ESI): m/z $[M+H]^+$.$C_{17}H_{22}N_7O$ calculated values 340.1880, measured value 340.1888.

Example 86

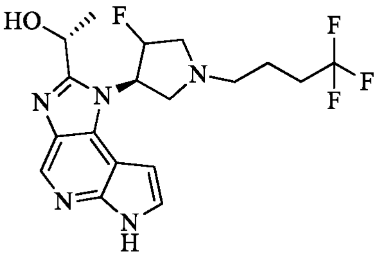

**LXS86**

(1R)-1-(1-((3R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol

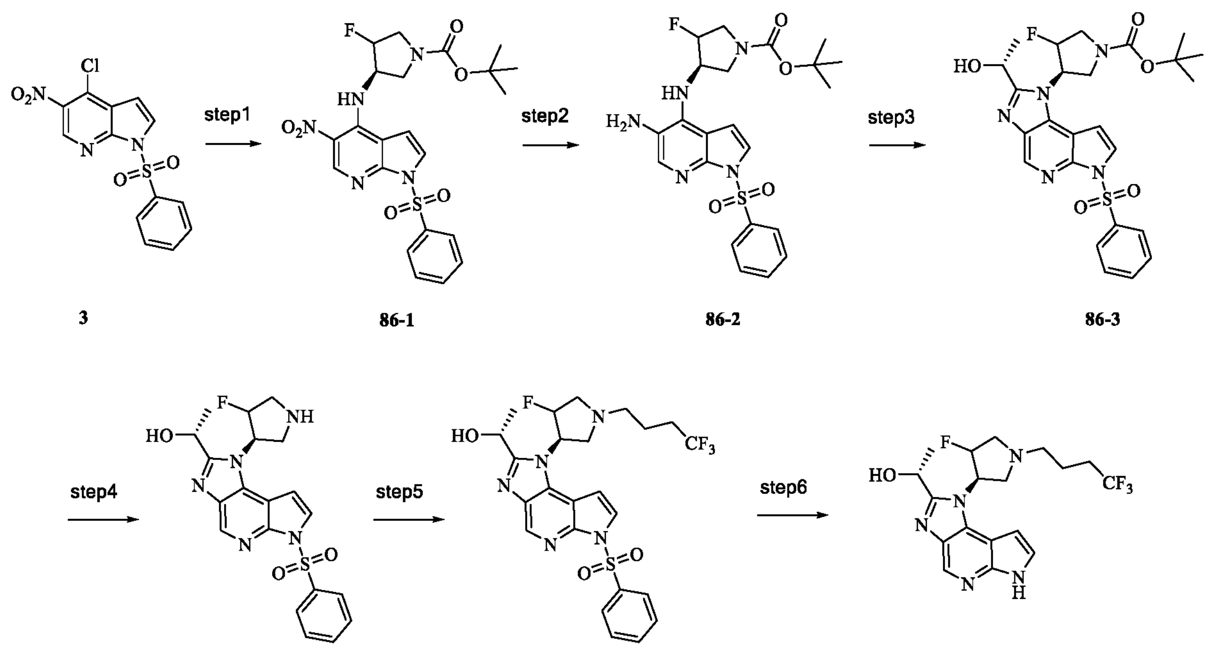


3 86-1 86-2 86-3

86-4 86-5 KDN86

Step 1: 4-Chloro-5-nitro-1-(phenylsulfonyl)-1H-pyrrolo[2,3-b]pyridine (1.0g, 3.0 mmol) was dissolved in 10 mL of tetrahydrofuran. Then, DIPEA (0.8g, 6.0mmol) and (3R) tert-butyl 3-amino-4-fluoropyrrolidine-1-carboxylate (0.9g, 4.5mmol) were added and stirred for 4h while maintaining reflux at an elevated temperature. The reaction was monitored for completion by TLC. Concentrate under vacuum to obtain a yellow oily liquid. Triturate and cure with an appropriate amount of methanol to yield (4R)-tert-butyl 3-fluoro-4-((5-nitro-1-(phenylsulfonyl)-1H-pyrrolo[2,3-b]pyridin-4-yl)amino)pyrrolidine-1-carboxylate as a yellow solid (1.2g, 80%). The product can be used directly in the next step without further purification. HRMS (ESI): m/z $[M+H]^+$. $C_{22}H_{25}FN_5O_6S$ calculated value 506.1504, measured value 506.1509.

Step 2: Dissolve (4R)-tert-butyl 3-fluoro-4-((5-nitro-1-(phenylsulfonyl)-1H-pyrrolo[2,3-b]pyridin-4-yl)amino)pyrrolidine-1-carboxylate (1.2g, 2.4mmol) in 20 mL of methanol, add palladium carbon (0.1g, 10%) and then use hydrogen to replace the air in the reaction flask more than three times. The reaction was carried out in hydrogen atmosphere and stirred at room temperature for 12 h. The reaction was monitored for completion by TLC. After filtration, the filtrate was collected and concentrated under vacuum to yield (4R)-tert-butyl 3-fluoro-4-((5-amino-1-(phenylsulfonyl)-1H-pyrrolo[2,3-b]pyridin-4-yl)amino)pyrrolidine-1-carboxylate as a light pink foamy solid (1.1g, 97%). The product can be used directly in the next step without further purification. HRMS (ESI): m/z $[M+H]^+$. $C_{22}H_{27}FN_5O_4S$ calculated value 476.1762, measured value 476.1770.

Step 3: Dissolve triethyloxonium tetrafluoroborate (1.2g, 6.3mmol) and (R)-lactamideide (0.6g, 6.3mmol) in 10 mL of tetrahydrofuran, stir for 3 h at room temperature and then concentrate the mixture under vacuum to obtain an oily liquid, followed by the addition of 10 mL of ethanol to dissolve. Then, add (4R)-tert-butyl 3-fluoro-4-((5-amino-1-(phenylsulfonyl)-1H-pyrrolo[2,3-b]pyridin-4-yl)amino)pyrrolidine-1-formate (1.0 g, 2.1 mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield (4R)-tert-butyl 3-fluoro-4-(2-((R)-1-hydroxyethyl)-6-(phenylsulfonyl)imidazo[4,5-d]pyrrolo[2,3-b]pyridin-1(6H)-yl)pyrrolidine-1-carboxylate as a green oil (0.5g, 45%). HRMS (ESI): m/z $[M+H]^+$. $C_{25}H_{29}FN_5O_5S$ calculated value 530.1868, measured value 530.1870.

Step 4: Dissolve (4R)-tert-butyl 3-fluoro-4-(2-((R)-1-hydroxyethyl)-6-(phenylsulfonyl)imidazo[4,5-d]pyrrolo[2,3-b]pyridin-1 (6H)-yl)pyrrolidine-1-carboxylate (0.5g, 0.9 mmol) in 10 mL of dichloromethane, slowly add trifluoroacetic acid (1.0g, 9.0mmol) and stir at room temperature for 12 h. Concentrate under vacuum to yield (1R)-1-(1-(((3R)-4-fluoropyrrolidin-3-yl)-6-(phenylsulfonyl)-1,6-dihydroimidazo[4,5-d]pyrrolo[2,3-b]pyridin-2-yl)ethanol as a light brown oil (0.4g, 99%). The product can be used directly in the next step without further purification. HRMS (ESI): m/z $[M+H]^+$. $C_{20}H_{21}FN_5O_3S$ calculated value 430.1344, measured value 430.1350.

Step 5: Dissolve (1R)-1-(1-(((3R)-4-fluoropyrrolidin-3-yl)-6-(phenylsulfonyl)-1,6-dihydroimidazo[4,5-d]pyrrolo[2,3-b]pyridin-2-yl)ethanol (0.3g, 0.7mmol) in 10 mL of tetrahydrofuran, add DIPEA (0.2g, 1.4mmol) and then add 4,4,4-trifluoro-1-iodobutane (0.3g, 1.1mmol) dropwise. Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (1R)-1-(1-(((3R)-4-fluoro-1-(4,4,4-trifluorobutyl)pyrrolidin-3-yl)-6-(phenylsulfonyl)-1,6-dihydroimidazo[4,5-d]pyrrolo[2,3-b]pyridin-2-yl) ethanol as a pale yellow oil (0.2 g, 53%). 53%). The product can be used directly in the next step without further purification. HRMS (ESI): m/z $[M+H]^+$. $C_{24}H_{26}F_4N_5O_3S$ calculated value 540.1687, measured value 540.1689.

Step 6: (1R)-1-(1-(((3R)-4-fluoro-1-(4,4,4-trifluorobutyl)pyrrolidin-3-yl)-6-(phenylsulfonyl)-1,6-dihydroimidazo[4,5-d]pyrrolo[2,3-b]pyridin-2-yl)ethanol (0.2g, 0.4 mmol) was dissolved in a solvent mixture of 5 mL tetrahydrofuran and 5 mL methanol. After adding 5 mL of 1 M sodium hydroxide, the reaction was stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS86:** (1R)-1-(1-((3R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol (0.1 g, 68%). $^1$HNMR (300 MHz, *DMSO-$d_6$*): $\delta$ = 11.94 (s, 1H), 8.68 (s, 1H), 7.54 (s, 1H), 6.77 (s, 1H), 4.68-4.72 (m, 1H), 3.91-4.06 (m , 1H), 3.30-3.41 (m, 1H), 2.56-2.81 (m, 2H), 2.54 (t, *J* = 4.5 Hz, 2H), 2.29-2.51 (m, 2H), 1.64-1.81 (m, 2H), 1.46 (d, *J*= 9.0 Hz, 3H), 1.36-1.42 (m, 2H) ppm; $^{13}$ C NMR (75 MHz, *DMSO-$d_6$*) $\delta$ 151.89, 148.53, 142.19, 129.06, 127.18, 126.84, 120.77, 115.69, 98.56, 91.20, 63.91, 61.77, 56.33, 54.88, 51.67, 37.71, 22.86, 11.67 ppm; HRMS (ESI): m/z $[M+H]^+$. $C_{18}H_{22}F_4N_5O$ calculated value 400.1755, measured value 400.1770.

Example 87

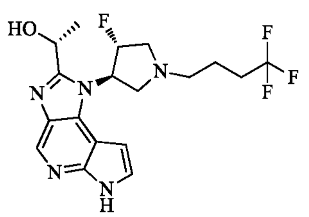

**LXS87**

(R)-1-(1-((3R,4R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-olUsing (3R,4R)-tert-butyl 3-amino-4-fluoropyrrolidine-1-carboxylate instead of (3R)-tert-butyl 3-amino-4-fluoropyrrolidine-1-carboxylate, **LXS 87** was prepared in a similar preparation way described in Example 86. The crude product was purified using a silica gel column (petroleum ether: ethyl acetate = 1:1) to yield **LXS87:** (R)-1-(1-((3R,4R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-olpyridin-2-yl) ethanol (0.1 g, 60%). $^1$HNMR (300 MHz, *DMSO-$d_6$*): $\delta$ = 11.99 (s, 1H), 8.78 (s, 1H), 7.58 (s, 1H), 6.75 (s, 1H), 4.64-4.71 (m, 1H), 3.90-4.05 (m, 1H), 3.31-3.55 (m, 1H), 2.53-2.87 (m, 2H), 2.50 (t, *J*= 6.0 Hz, 2H), 2.23-2.47 (m, 2H), 1.73- 1.88

(m, 2H), 1.41 (d, $J$ = 6.0 Hz, 3H), 1.33-1.44 (m, 2H) ppm; $^{13}$CNMR (75 MHz, *DMSO-$d_6$)* $\delta$ 148.97, 148.53, 142.19, 129.00, 127.65, 126.88, 120.73, 115.69, 97.63, 91.28, 63.63, 61.79, 56.39, 54.85, 51.63, 37.77, 22.81, 11.65 ppm; HRMS (ESI): m/z $[M+H]^+$. $C_{18}H_{22}F_4N_5O$ calculated value 400.1755, measured value 400.1768.

Example 88

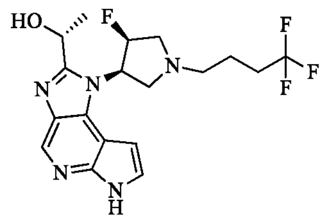

**LXS88**

(R)-1-(1-((3R,4S)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol

Using (3R,4R)-tert-butyl 3-amino-4-fluoropyrrolidine-1-carboxylate instead of (3R)-tert-butyl 3-amino-4-fluoropyrrolidine-1-carboxylate, **LXS 88** was prepared in a similar preparation way described in Example 86. The crude product was purified using a silica gel column (petroleum ether: ethyl acetate = 1:1) to yield **LXS88:** (R)-1-(1-((3R,4S)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol (0.1g, 50%). $^1$HNMR (300 MHz, *DMSO-$d_6$* ): $\delta$ = 12.13 (s, 1H), 8.81 (s, 1H), 7.60 (s, 1H), 6.57 (s, 1H), 4.58-4.70 (m, 1H), 3.90-4.05 (m, 1H), 3.33-3.42 (m, 1H), 2.51-2.86 (m, 2H), 2.42 (t, $J$= 9.0 Hz, 2H), 2.30-2.41 (m, 2H), 1.66-1.82 (m, 2H), 1.49 (d, $J$= 9.0 Hz, 3H), 1.39-1.46 (m, 2H) ppm; $^{13}$CNMR (75 MHz, *DMSO-$d_6$)* $\delta$ 151.45, 158.55, 142.19, 129.05, 127.16, 126.86, 120.74, 115.64, 98.56, 91.55, 63.65, 61.75, 56.38, 54.85, 51.68, 37.77, 22.89, 11.56 ppm; HRMS (ESI): m/z $[M+H]^+$. $C_{18}H_{22}F_4N_5O$ calculated value 400.1755, measured value 400.1763.

Example 89

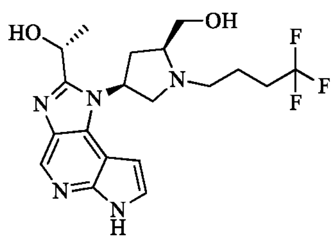

**LXS89**

(R)-1-(1-((3S,5S)-5-(Hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-olUsing tert-butyl (2S,4S)-4-amino-2-(hydroxymethyl)pyrrolidine-1-carboxylate instead of tert-butyl (3R)-3-amino-4-fluoropyrrolidine-1-carboxylate, **LXS 89** was prepared in a similar preparation way described in Example 86. The crude product was purified using a silica gel column (petroleum ether: ethyl acetate = 1:1) to yield **LXS89:** (R)-1-(1-((3S,SS)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol (0.1 g, 51%). $^1$HNMR (300 MHz, *DMSO-$d_6$*): $\delta$ = 11.98 (s, 1H), 8.78 (s, 1H), 7.59 (s , 1H), 6.88 (s, 1H), 4.68-4.74 (m, 1H), 3.79-3.85 (m, 1H), 3.34 (d, $J$ = 6.0 Hz, 2H), 2.59-2.82 (m, 2H), 2.43 (t, $J$ = 4.5 Hz, 2H), 2.33-2.41 (m, 1H), 1.87-2.12 (m, 2H), 1.64-1.81 (m, 2H), 1.47 (d, $J$ = 6.0 Hz, 3H), 1.36-1.40 (m, 2H) ppm; $^{13}$CNMR (75 MHz, *DMSO-$d_6$ )* $\delta$ 148.94, 148.57, 142.19, 129.04, 127.11, 126.88, 120.75, 115.63, 98.67, 68.74, 63.67, 62.55, 56.71, 56.21, 54.18, 37.79, 28.31, 22.84, 10.49 ppm; HRMS (ESI): m/z $[M+H]^+$. $C_{19}H_{25}F_3N_5O_2$ calculated value 412.1955, measured value 412.1961.

Example 90

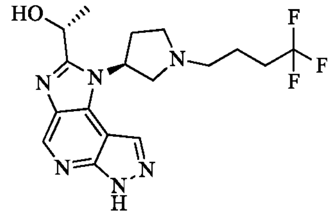

**LXS90**

(R)-1-(1-((S)-1-(4,4,4-trifluorobutyl)pyrrolidin-3-yl)-1,6-dihydroimidazo[4,5-d]pyrazolo[3,4-b]pyri din-2-yl)ethan-1-ol

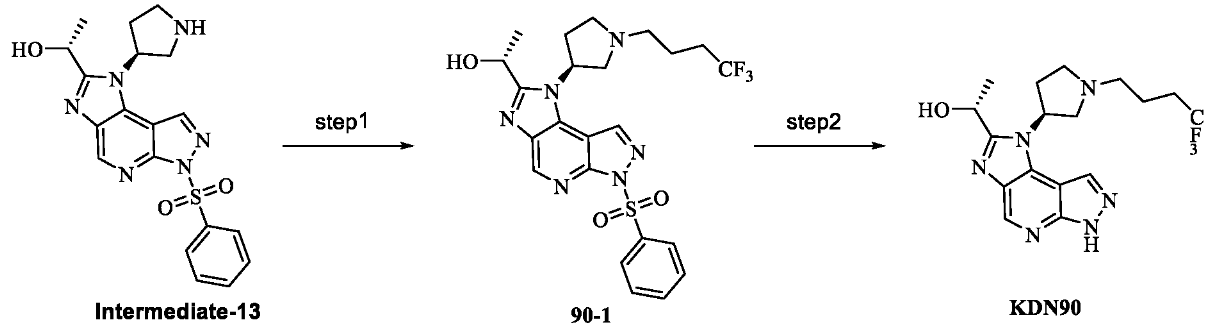


**Intermediate-13** **90-1** **KDN90**

Step 1: Dissolve (R)-1-(6-(phenylsulfonyl)-1-((S)-pyrrolidin-3-yl)-1,6-dihydroimidazo[4,5-d]pyrazolo[3,4-b]pyridin-2-yl)ethanol (0.3g, 0.7mmol) in 10 mL of tetrahydrofuran, add DIPEA (0.2g, 1.4mmol) followed by the slow dropwise addition of 4,4,4-trifluoro- 1-iodobutane (0.3g, 1.1mmol). Raise the temperature to maintain reflux and stir for 3 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (R)-1-(6-(phenylsulfonyl)-1-((S)-1-(4,4,4-trifluorobutyl)pyrrolidin-3-yl)-1,6-dihydroimidazo[4,5-d] pyrazolo[3,4-b]pyridin-2-yl) ethanol as a pale yellow oil (0.2g, 52%). The product can be used directly in the next step without further purification. HRMS (ESI): m/z $[M+H]^+$. $C_{23}H_{26}F_3N_6O_3S$ calculated value 523.1734, measured value 523.1740.

Step 2: (R)-1-(6-(Phenylsulfonyl)-1-((S)-1-(4,4,4-trifluorobutyl)pyrrolidin-3-yl)-1,6-dihydroimidazo[4,5-d] pyrazolo[3,4-b]pyridin-2-yl) ethanol (0.2g, 0.4mmol) was dissolved in a solvent mixture of 5 mL tetrahydrofuran and 5 mL methanol, and add 5 mL of 1M sodium hydroxide and then stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS90:** (R)-1-(1-((S)-1-(4,4,4-trifluorobutyl)pyrrolidin-3-yl)-1,6-dihydroimidazo[4,5-d]pyrazolo[3,4-b]pyridin-2-yl)ethan-1-ol (0.1 g, 68%). $^1$HNMR (300 MHz, *DMSO-$d_6$*): $\delta$ = 13.54 (s, 1H), 8.82 (s, 1H), 7.58 (s, 1H), 4.65-4.73 (m, 1H), 3.64-3.79 (m, 2H), 2.53-2.81 (m, 2H), 2.41 (t, $J$ = 6.0 Hz, 2H), 2.20-2.33 (m, 2H), 1.91-2.14 (m, 2H), 1.85 (m, 2H), 1.41 (d, $J$ = 9.0 Hz, 3H), 1.27-1.37 (m, 2H) ppm; $^{13}$CNMR (75 MHz, *DMSO-$d_6$*) $\delta$154.75, 148.52, 140.88, 140.51, 132.48, 126.84, 125.47, 105.37, 63.68, 58.51, 58.43, 56.38, 55.91, 38.88, 27.91, 22.86, 9.56 ppm; HRMS (ESI): m/z $[M+H]^+$. $C_{17}H_{22}F_3N_6O$ calculated value 383.1802, measured value 383.1800.

Example 91

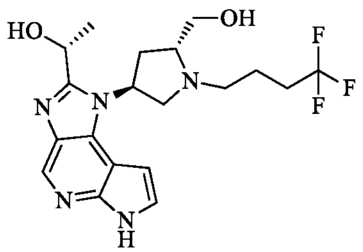

**LXS91**

(R)-1-(1-((3S,SR)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol**LXS91** was prepared by replacing tert-butyl (3R)-3-amino-4-fluoropyrrolidine-1-carboxylate with tert-butyl (2R,4S)-4-amino-2-(hydroxymethyl)pyrrolidine-1-carboxylate, using a preparation method similar to Example 86, and the crude product was purified using a silica gel column (petroleum ether: ethyl acetate = 1:1) to yield **LXS91:** (R)-1-(1-((3 S,5R)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol (0.1 g, 43%).$^1$ HNMR (300 MHz, *DMSO-$d_6$*): $\delta$ = 11.68 (s, 1H), 8.73 (s, 1H), 7.61 ( s, 1H), 6.89 (s, 1H), 4.65-4.71 (m, 1H), 3.76-3.89 (m, 1H), 3.35 (d, *J* = 6.0 Hz, 2H), 2.64-2.81 (m, 2H), 2.45 (t, *J* = 7.5 Hz, 2H), 2.37-2.39 (m, 1H), 1.89-2.15 (m , 2H), 1.60-1.84 (m, 2H), 1.43 (d, *J* = 9.0 Hz, 3H), 1.33-1.41 (m, 2H) ppm;$^{13}$ C NMR (75 MHz, *DMSO-$d_6$*) $\delta$ 151.87, 148.54, 142.19, 129.05, 127.18, 126.84, 120.77, 115.69, 98.56, 68.74, 63.69, 62.57, 56.71, 56.38, 54.18, 38.96, 28.54, 22.85, 13.74 ppm; HRMS (ESI): m/z $[M+H]^+$. $C_{19}H_{25}F_3N_5O_2$ calculated value 412.1955, measured value 412.1963.

Example 92

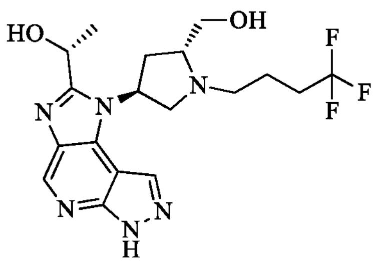

**LXS92**

(R)-1-(1-((3S,5R)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol**LXS92** was prepared by replacing tert-butyl (3R)-3-amino-4-fluoropyrrolidine-1-carboxylate with tert-butyl (2R,4S)-4-amino-2-(hydroxymethyl)pyrrolidine-1-carboxylate, using a preparation method similar to Example 86, and the crude product was purified using a silica gel column (petroleum ether: ethyl acetate = 1:1) to yield **LXS92:** (R)-1-(1-((3S,5R)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol (0.1g, 33%). $^1$HNMR (300 MHz, *DMSO-$d_6$*): $\delta$ = 13.78 (s, 1H), 8.87 (s, 1H), 7.54 ( s, 1H), 4.69-4.72 (m, 1H), 3.83-3.89 (m, 1H), 3.37 (d, *J* = 6.0 Hz, 2H), 2.61-2.80 (m, 2H), 2.47 (t, *J* = 9.0 Hz, 2H), 2.36-2.43 (m, 1H), 1.88-2.15 (m, 2H), 1.58-1.79 (m, 2H), 1.44 (d, *J* = 10.5 Hz, 3H), 1.38-1.43 (m, 2H) ppm; $^{13}$CNMR (75 MHz, *DMSO-$d_6$*) $\delta$ 155.75, 148.56, 140.83, 140.51, 132.48, 126.84, 125.42, 104.74, 68.75, 63.61, 62.51, 56.39, 56.29, 54.17, 38.67, 28.56, 22.87, 11.65 ppm; HRMS (ESI): m/z $[M+H]^+$. $C_{18}H_{24}F_3N_6O_2$ calculated value 413.1907, measured value 413.1914.

Example 93

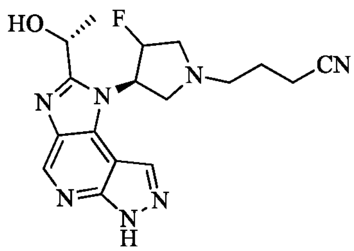

**LXS93**

4-((4R)-3-Fluoro-4-(2-((R)-1-hydroxyethyl)imidazo[4,5-d]pyrazolo[3,4-b]pyridin-1(6H)-yl)pyrrolidin-1-yl)butanenitrile

**LXS93** was prepared in a preparation way similar to Example 86 and the crude product was purified using a silica gel column (petroleum ether: ethyl acetate = 1:1) to yield LXS93: 4-((4R)-3-Fluoro-4-(2-((R)-1-hydroxyethyl)imidazo[4,5-d]pyrazolo[3,4-b]pyridin-1(6H)-yl)pyrrolidin-1-yl)butanenitrile (0.1g. 31%). $^1$HNMR (300 MHz, *DMSO-d$_6$*): $\delta$ = 13.73 (s, 1H), 8.89 (s, 1H), 7.61 (s, 1H), 4.57-4.71 (m, 1H), 3.91-4.08 (m, 1H), 3.33-3.45 (m, 1H), 2.57-2.81 (m, 2H), 2.47 (t, *J* = 6.0 Hz, 2H), 2.40-2.43 (m, 2H), 1.90 (t, *J* = 9.0 Hz, 2H), 1.70-1.76 (m, 2H), 1.43 (d, *J* = 7.5 Hz, 3H) ppm; $^{13}$CNMR (75 MHz, *DMSO-d$_6$*) $\delta$ 154.76, 148.54, 141.65, 140.51, 132.78, 125.47, 119.38, 104.78, 91.38, 64.89, 61.33, 55.59, 54.86, 51.69, 22.85, 17.56, 16.45 ppm; HRMS (ESI): m/z $[M+H]^+$. $C_{17}H_{21}FN_7O$ calculated value 358.1786, measured value 358.1789.

Example 94

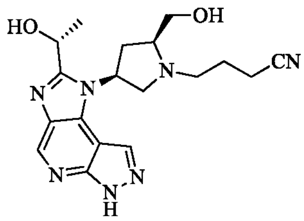

**LXS94**

4-((2S,4S)-4-(2-((R)-1-Hydroxyethyl)imidazo[4,5-d]pyrazolo[3,4-b]pyridin-1(6H)-yl)-2-(hydroxymethyl)pyrrolidin-1-yl)butanenitrile

**LXS94** was prepared using a preparation method similar to Example 86 by replacing tert-butyl (3R)-3-amino-4-fluoropyrrolidine-1-carboxylate with tert-butyl (2S,4S)-4-amino-2-(hydroxymethyl)pyrrolidine-1-carboxylate, and the crude product was purified using a silica gel column (petroleum ether: ethyl acetate = 1:1) to yield **LXS94:** 4-((2S,4S)-4-(2- ((R)-1-Hydroxyethyl)imidazo[4,5-d]pyrazolo[3,4-b]pyridin-1(6H)-yl)-2 - (hydroxymethyl)pyrrolidin-1-yl)butanenitrile (0.1g, 27%). $^1$HNMR (300 MHz, *DMSO-d$_6$*): $\delta$= 13.68 (s, 1H), 8.89 (s, 1H), 7.58 (s, 1H), 4.63-4.71 (m, 1H), 3.85-3.91 (m, 1H), 3.39 (d, *J* = 9.0 Hz, 2H), 2.66-2.81 (m, 2H), 2.39 (t, *J* = 6.0 Hz, 2H), 2.38-2.45 (m, 1H), 1.79-2.23 (m, 2H), 1.56-1.72 (m, 2H), 1.41 (d, *J* = 10.5 Hz, 3H), 1.33-1.39 (m, 2H) ppm; $^{13}$CNMR (75 MHz, *DMSO-d$_6$ )* $\delta$ 155.78, 149.65, 140.89, 140.32, 132.89, 124.23, 119.38, 104.73, 69.65, 63.65, 62.56, 57.76, 56.21, 53.39, 29.65, 22.89, 18.56, 14.65 ppm; HRMS (ESI): m/z $[M+H]^+$. $C_{18}H_{24}N_7O_2$ calculated value 370.1986, measured value 370.1990.

Example 95

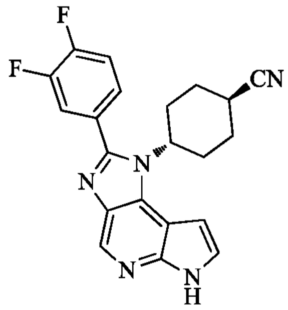

LXS95

trans-4-(2-(3,4-Difluorophenyl)imidazo[4,5-d]pyrrolo[2,3-b]pyridin-1(6H)-yl)cyclohexanecarbonitrile

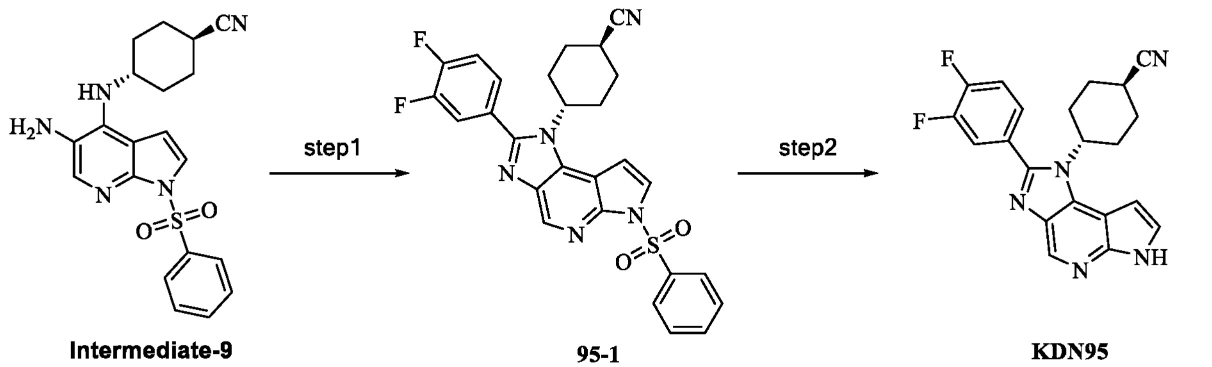


Intermediate-9 95-1 KDN95

Step 1: Dissolve trans-4-((5-amino-1-(phenylsulfonyl)-1H-pyrrolo[2,3-b]pyridin-4-yl)amino)cyclohexanecarbonitrile (0.3g, 0.8mmol) in 10 mL of DMF, add $Na_2S_2O_5$ (0.8g, 4 mmol) followed by the addition of 3,4-difluorobenzaldehyde (0.2g, 1.6mmol) dropwise. Stir for 12h at 90 °C. The reaction was monitored for completion by TLC. Saturated sodium bicarbonate was added until the reaction solution was weakly basic, and the organic phase was separated. The aqueous phase was extracted twice with dichloromethane. The organic phases were combined and washed with saturated saline, dried over anhydrous sodium sulfate and concentrated under vacuum to yield trans-4-(2-(3,4-difluorophenyl)-6-(phenylsulfonyl)imidazo[4,5-d]pyrrolo[2,3-b]pyridin-1(6H)-yl)cyclohexanecarbonitrile as a yellow oil (0.3g, 76%). HRMS (ESI): m/z $[M+H]^+$. $C_{27}H_{22}F_2N_5O_2S$ calculated value 518.1457, measured value 518.1461.

Step 2: Dissolve trans-4-(2-(3,4-difluorophenyl)-6-(phenylsulfonyl)imidazo[4,5-d]pyrrolo[2,3-b]pyridin-1(6H)-yl)cyclohexanecarbonitrile (0.3g, 0.6mmol) in a mixture of 5 mL of tetrahydrofuran and 5 mL of methanol. After adding 5 mL of 1 M sodium hydroxide, stir at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum. The residue was purified using a silica gel column (petroleum ether: ethyl acetate = 1:1) to yield **LXS95:** trans-4-(2-(3,4-difluorophenyl)imidazo[4,5-d]pyrrolo[2,3-b]pyridin-1(6H)-yl)cyclohexanecarbonitrile (0.1g, 46%). $^1$HNMR (300 MHz, *DMSO-$d_6$*): $\delta$ = 12.02 (s, 1H), 8.66 (s, 1H), 7.77-7.84 (m, 1H), 7.56-7.73 (m, 1H), 7.55 (d, *J* = 3.0 Hz, 2H), 6.88 (s, 1H), 4.41-4.49 (m , 1H), 3.15 (t, *J* = 12.0 Hz, 1H), 2.32-2.44 (m, 2H), 2.23 (d, *J* = 12.0 Hz, 2H), 2.04 (d, *J* = 12.0 Hz, 2H), 1.73-1.85 (m, 2H) ppm; $^{13}$CNMR (75 MHz, *DMSO-$d_6$*) $\delta$ 153.78, 150.05, 149.64, 148.69, 142.19, 129.05, 127.83, 127.16, 124.74, 122.75, 120.75, 117.52, 115.63, 115.18, 97.58, 65.94, 31.89, 28.78, 22.63 ppm; HRMS (ESI): m/z $[M+H]^+$. $C_{21}H_{18}F_2N_5$ calculated value 378.1525, measured value 378.1529.

Example 96

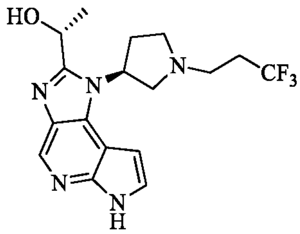

LXS96

(R)-1-(1 -((S)- 1 -(3,3,3 -Trifluoropropyl)pyrrolidin-3 -yl)imidazo[4,5-d]pyrrolo[2,3 - b]pyridin-2-yl)ethanol

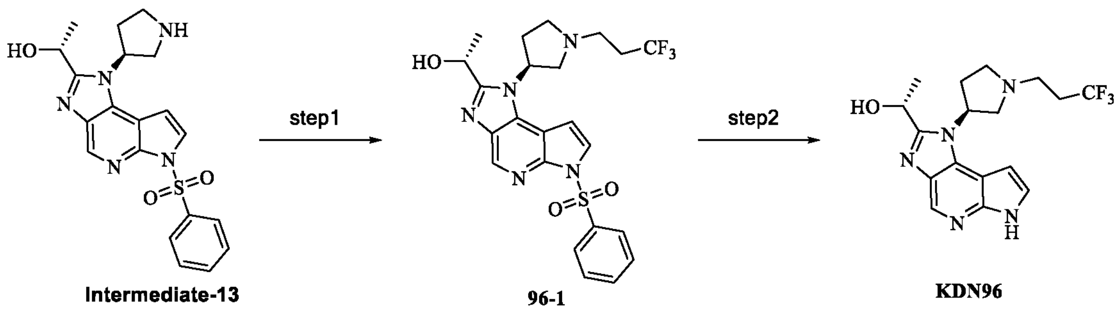


Intermediate-13 96-1 KDN96

Step 1: Dissolve (R)-1-(6-(phenylsulfonyl)-1-((S)-pyrrolidin-3-yl)imidazo[4,5-d]pyrrolo[3,4-b]pyridin-2-yl)ethanol (0.3g, 0.7mmol) in 10 mL of tetrahydrofuran, add DIPEA (0.2g, 1.4mmol) followed by the slow dropwise addition of 4,4,4-trifluoro-1-iodopropane (0.2g, 1.1mmol). Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (R)-1-(6-(phenylsulfonyl)-1-((S)-1-(3,3,3-trifluoropropyl)pyrrolidin-3-yl)imidazo[4,5-d]pyrrolo[2,3-b]pyridin-2-yl)ethanol as a pale yellow oil (0.2 g, 54%). The product can be used directly in the next step without further purification. HRMS (ESI): m/z $[M+H]^+$. $C_{23}H_{25}F_3N_5O_3S$ calculated value 508.1625, measured value 508.1630.

Step 2: Dissolve (R)-1-(6-(phenylsulfonyl)-1-((S)-1-(3,3,3-trifluoropropyl)pyrrolidin-3-yl)imidazo[4,5-d] pyrrolo[2,3-b]pyridin-2-yl)ethanol (0.2g, 0.4mmol) in a solvent mixture of 5 mL tetrahydrofuran and 5 mL methanol. After the addition of 5 mL of 1 M sodium hydroxide, stir the mixture for 5 h at room temperature and monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS96:** (R)-1-(1-((S)-1-(3,3,3-trifluoropropyl)pyrrolidin-3-yl)imidazo[4,5-d]pyrrolo[2,3-b]pyridin-2-yl)ethanol (0.1g, 69%). $^1$HNMR (300 MHz, *DMSO-d$_6$*): $\delta$ = 12.09 (s, 1H), 8.87 (s, 1H), 7.53 (s, 1H), 6.83 (s, 1H), 4.60-4.75 (m, 1H), 3.73-3.78 (m, 1H), 2.56-2.87 (m, 2H), 2,42 (t, *J* = 4.5 Hz, 2H), 2.15-2.32 (m, 2H), 1.90-2.11 (m, 2H), 1.41 (d, *J* = 6.0 Hz, 3H), 1.35-1.40 (m, 2H)ppm; $^{13}$CNMR (75 MHz, *DMSO-d$_6$*) $\delta$ 148.67, 148.51, 142.18, 129.02, 127.11, 124.88, 120.74, 115.65, 99.35, 63.64, 58.94, 58.41, 55.95, 40.25, 38.45, 27.69, 22.81 ppm; HRMS (ESI): m/z $[M+H]^+$. $C_{17}H_{21}F_3N_5O$ calculated value 368.1693, measured value 368.1670.

Example 97

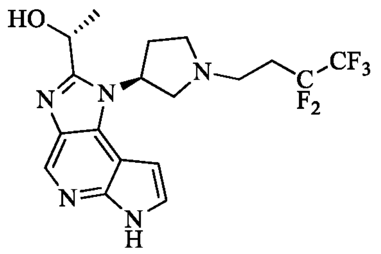

LXS97

(R)-1-(1-((S)-1-(3,3,4,4,4-Pentafluorobutyl)pyrrolidin-3-yl)imidazo[4,5-d]pyrrolo[2,3-b]pyridin-2-yl)ethanol

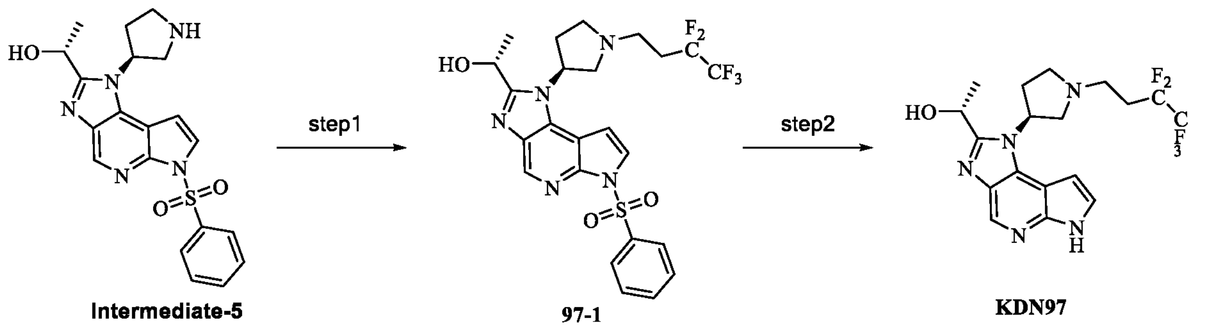


Intermediate-5 97-1 KDN97

Step 1: Dissolve (R)-1-(6-(phenylsulfonyl)-1-((S)-pyrrolidin-3-yl)imidazo[4,5-d]pyrrolo[3,4-b]pyridin-2-yl)ethanol (0.3g, 0.7mmol) in 10 mL of tetrahydrofuran, add DIPEA (0.2g, 1.4mmol) and then slowly add 1,1,1,2,2-pentafluoro-4-iodobutane (0.3g, 1.1mmol) dropwise. Then, raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield (R)-1-(1-((S)-1-(3,3,4,4,4-pentafluorobutyl)pyrrolidin-3-yl)-6-(phenylsulfonyl)imidazo[4,5-d]pyrrolo[2,3-b]pyridin-2-yl) ethanol as a pale yellow oil (0.2g, 49%). The product can be used directly in the next step without further purification. HRMS (ESI): m/z $[M+H]^+$. $C_{24}H_{25}F_5N_5O_3S$ calculated value 558.1593, measured value 558.1602.

Step 2: Dissolve (R)-1-(1-((S)-1-(3,3,4,4,4-pentafluorobutyl)pyrrolidin-3-yl)-6-(phenylsulfonyl)imidazo[4,5-d]pyrrolo[2,3-b]pyridin-2-yl)ethanol (0.2g, 0.36mmol) in a mixture of 5 mL tetrahydrofuran and 5 mL methanol, and 5 mL of 1M sodium hydroxide was added and the mixture was then stirred at room temperature for 5 h. The reaction was monitored for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum, and the residue was purified using a silica gel column (petroleum ether: ethyl acetate = 1:1) to yield **LXS97:** (R)-1-(1-((S)-1-(3,3,4,4,4-pentafluorobutyl)pyrrolidin-3-yl)imidazo[4,5-d]pyrrolo[2,3-b]pyridin-2-yl ) ethanol (0.1 g, 67%). $^1$HNMR (300 MHz, *DMSO-$d_6$*): $\delta$ = 12.85 (s, 1H), 8.77 (s, 1H), 7.56 (s, 1H), 6.89 (s, 1H), 4.60-4.75 (m, 1H), 3.73-3.78 (m, 1H), 2.56-2.87 (m, 2H), 2 ,47 (t, $J$ = 4.5 Hz, 2H), 2.15-2.32 (m, 2H), 1.90-2.11 (m, 2H), 1.45 (d, $J$= 6.0 Hz, 3H), 1.34-1.40 (m, 2H) ppm; $^{13}$CNMR (75 MHz, *DMSO-$d_6$*) $\delta$148.67, 148.51, 142.18, 131.88, 129.02, 127.11, 120.93, 119.74, 114.65, 97.35, 68.64, 53.94, 51.41, 50.95, 41.55, 37.40, 22.68, 21.40 ppm; HRMS (ESI): m/z $[M+H]^+$. $C_{18}H_{21}F_5N_5O$ calculated value 418.1661, measured value 418.1666.

Example 98

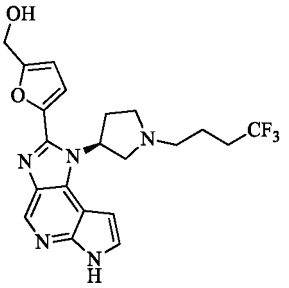

**LXS98**

(S)-(5-(1-(1-(4,4,4-trifluorobutyl)pyrrolidin-3-yl)-1,6-dihydroimidazo[4,5-d]pyrrolo[2,3-b]pyridin-2-yl)furan-2-yl)methanol

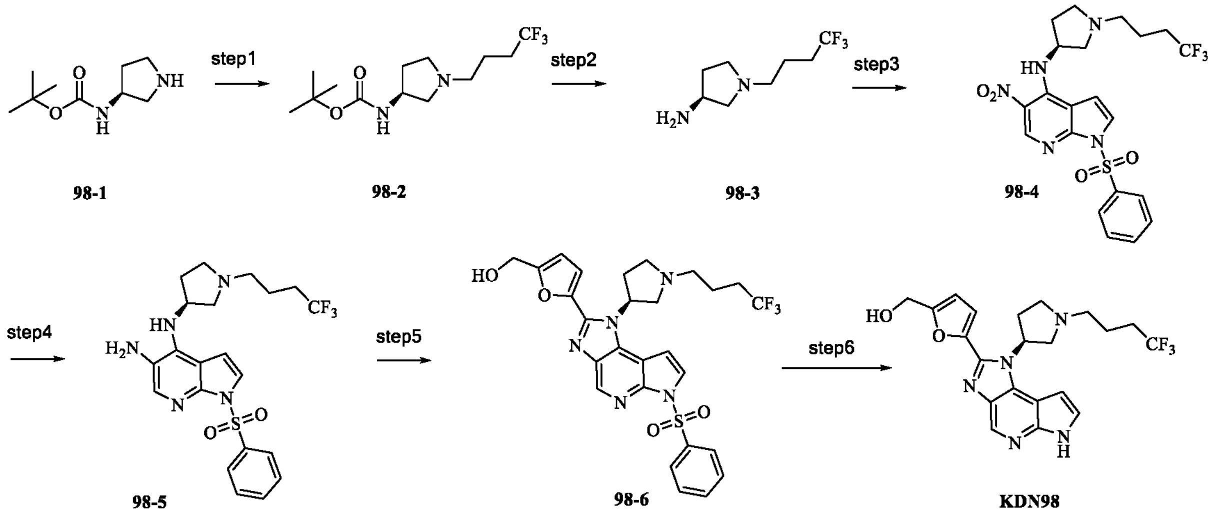


98-1 98-2 98-3 98-4 98-5 98-6 KDN98

Step 1: Dissolve 98-1 (0.5g, 2.7mmol) in 10 mL of DMF, add $K_2CO_3$ (0.7g, 5.4mmol) followed by the slow dropwise addition of 4,4,4-trifluoro-1-iodobutane (0.7g, 3.0mmol), and then stir at room temperature for 12 h. Monitor the reaction for completion by MS. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield **98-2** as a white oil (0.7g, 88%). The product was used directly in the next step without further purification. HRMS (ESI): m/z $[M+H]^+$. $C_{13}H_{24}F_3N_2O_2$ calculated value 297.1784, measured value 297.1788.

Step 2: Dissolve 98-2 (0.7g, 2.4mmol) in 10 mL of dichloromethane, add trifluoroacetic acid (2.7g, 24.0mmol) and stir at room temperature for 12 h. Monitor the reaction for completion by MS. The product was concentrated under vacuum to obtain **98-3** as a light yellow oil (0.4g, 99%). The product can be used directly in the next step without further purification. HRMS (ESI): m/z $[M+H]^+$. $C_8H_{16}F_3N_2$ calculated value 197.1260, measured value 197.1266.

Step 3: Dissolve **98-3** (0.4g, 2.0mmol) in 10 mL of tetrahydrofuran, add DIPEA (0.5g, 4.0mmol) followed by the addition of 4-chloro-5-nitro-1-(phenylsulfonyl)-1H-pyrrolo[2,3-b]pyridine (0.6g, 1.8mmol). Raise the temperature to maintain reflux and stir for 3 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield **98-4** as a yellow oil (0.6g, 68%). The product was used directly in the next step without further purification. HRMS (ESI): m/z $[M+H]^+$. $C_{21}H_{23}F_3N_5O_4S$ calculated value 498.1417, measured value 498.1420.

Step 4: Dissolve 98-4 (0.6g, 1.2mmol) in 10 mL of methanol, add palladium carbon (0.1g, 10%) and then use hydrogen to replace the air in the reaction flask more than three times. Keep the reaction in hydrogen atmosphere, stir for 12 h at room temperature, and monitor the reaction for completion by TLC. After filtration, the filtrate was collected and concentrated under vacuum to obtain **98-5** as a white foamy solid (0.5g, 89%). The product can be used directly in the next step without further purification. HRMS (ESI): m/z $[M+H]^+$. $C_{21}H_{25}F_3N_5O_2S$ calculated value 468.1676, measured

value 468.1680.

Step 5: Dissolve **98-5** (0.5g, 1.8mmol) in 10 mL ofDMF, add $Na_2S_2O_5$ (0.7g, 3.6mmol) followed by the addition of 5-hydroxymethyl furfural (0.4g, 3.2mmol) dropwise, raise the temperature to 90°C and stir for 12 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried over anhydrous sodium sulfate and concentrated under vacuum to yield **98-6** as a yellow oil (0.3g, 49%). HRMS (ESI): m/z $[M+H]^+$. $C_{27}H_{27}F_3N_5O_4S$ calculated value 574.1730, measured value 574.1739.

Step 6: Dissolve **98-6** (0.2g, 0.3mmol) in a solvent mixture of 5 mL tetrahydrofuran and 5 mL methanol, add 5 mL of 1M sodium hydroxide and then stir at room temperature for 5 h. Monitor the reaction for completion by TLC. Saturated sodium bicarbonate was added until the reaction solution was weakly basic, and the organic phase was separated. The aqueous phase was extracted twice using dichloromethane. The organic phases were combined and washed with saturated saline, dried with anhydrous sodium sulfate and concentrated under vacuum, and the residue was purified using a silica gel column (petroleum ether: ethyl acetate = 1:1) to yield **LXS98:** (S)-(5-(1-(1-(4,4,4-trifluorobutyl)pyrrolidin-3-yl)-1,6-dihydroimidazo[4,5-d]pyrrolo[2,3-b]pyridin-2-yl)furan-2-yl)methanol (0.1g, 66%). $^1$HNMR (300 MHz, *DMSO-$d_6$*): $\delta$ = 12.98 (s, 1H), 8.87 (s, 1H), 7.51 (s, 1H), 7.02 (d, *J* = 6.0 Hz, 1H), 6.84 (s, 1H), 6.59 (d, *J* = 6.0 Hz, 1H), 4.39 (s, 1H), 3.79-3.88 (m, 1H), 2.53-2.81 (m, 2H), 2.43 (t, *J = 6.0 Hz,* 2H), 2.20-2.30 (m, 2H), 1.90-2.15 (m, 2H), 1.81 (d, *J* = 6.0 Hz, 2H), 1.36 (m, 2H) ppm; $^{13}$CNMR (75 MHz , *DMSO-$d_6$)* $\delta$ 153.84, 151.12, 148.62, 144.95, 142.19, 129.08, 127.15, 126.84, 120.74, 115.64, 107.95, 104.08, 99.35, 57.85, 57.34, 56.38, 55.97, 55.84, 37.75, 27.08, 10.18 ppm; HRMS (ESI): m/z $[M+H]^+$. $C_{21}H_{23}F_3N_5O_2$ calculated value 434.1798, measured value 434.1788.

Example 99

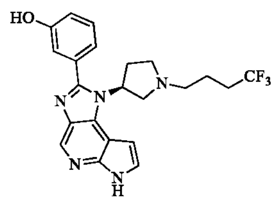

**LXS99**

(S)-3-(1-(1-(4,4,4-trifluorobutyl)pyrrolidin-3-yl)-1,6-dihydroimidazo[4,5-d]pyrrolo[2,3-b]pyridin-2-yl)phenol

**LXS99** was prepared using the raw material of 3-hydroxybenzaldehyde in a similar preparation way as described in Example 98, and the crude product was purified using a silica gel column (petroleum ether: ethyl acetate = 1:1) to yield **LXS99:** (S)-3-(1-(1-(4,4,4-trifluorobutyl)pyrrolidin-3-yl)-1,6-dihydroimidazo[4,5-d]pyrrolo[2,3-b]pyridin-2-yl)phenol (0.1g, 54%). $^1$HNMR (300 MHz, *DMSO-$d_6$*): $\delta$ = 12.17 (s, 1H), 8.89 (s, 1H), 7.84 (t, *J* = 3.0 Hz, 1H), 7.58 (s, 1H), 7.34 (t, *J* = 7.5 Hz, 1H), 7.04 (s, 1H), 6.90-7.02 (m , 1H), 6.91 (d, *J* = 3.0 Hz, 1H), 6.08 (d, *J* = 7.5 Hz, 1H), 3.79-3.81 (m, 2H), 2.50-2.80 (m, 2H), 2.45 (t, *J* = 6.0 Hz, 2H), 2.21-2.37 (m, 2H), 1.93-2.19 (m, 2H), 1.83 (d, *J* = 6.0 Hz, 2H), 1.34 (m, 2H) ppm; $^{13}$CNMR (75 MHz, *DMSO-$d_6$)* $\delta$ 157.54, 153.74, 148.62, 142.18, 132.08, 130.68, 129.08, 127.10, 126.85, 120.77, 120.65, 115.95, 115.64, 112.97, 99.34, 58.46, 58.21, 56.37, 55.94, 37.77, 27.62, 10.18 ppm; HRMS (ESI): m/z $[M+H]^+$. $C_{22}H_{23}F_3N_5O$ calculated value 430.1849, measured value 430.1933.

Example 100

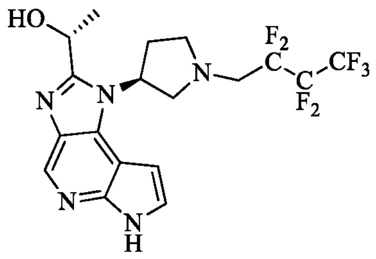

(R)-1-(1-((S)-1-(2,2,3,3,4,4,4-Heptafluorobutyl)pyrrolidin-3-yl)imidazo[4,5-d]pyrrolo[2,3-b]pyridin-2-yl)ethanol

**LXS100** was prepared using a preparation method similar to Example 97 with the raw material of 2,2,3,3,4,4,4-heptafluoroiodobutane, and the crude product was purified using a silica gel column (petroleum ether: ethyl acetate = 1:1) to yield **LXS100:** (R)-1-(1-((S)-1-(2,2,3,3,4,4,4-Heptafluorobutyl)pyrrolidin-3-yl)imidazo[4,5-d]pyrrolo[2,3-b]pyridin-2-yl)ethanol (0.1 g, 43%). $^1$HNMR (300 MHz, *DMSO-d$_6$* ): $\delta$ = 12.35 (s, 1H), 8.87 (s, 1H), 7.54 (s, 1H), 6.63 (s, 1H), 4.60-4.70 (m, 1H), 3.73-3.79 (m, 1H), 2.56-2.81 (m, 2H), 2,68 (s, 2H), 2.20-2.30 (m, 2H), 1.90-2.15 (m, 2H), 1.49 (d, *J* = 6.0 Hz, 3H) ppm;$^{13}$ C NMR (75 MHz, *DMSO-d$_6$*) $\delta$ 148.79, 148.59, 129.16, 127.19, 120.88, 120.71, 118.74, 115.65, 104.35, 99.65, 63.64, 58.94, 58.41, 55.91, 51.95, 27.55, 22.40 ppm; HRMS (ESI): m/z [M+H]$^+$. $C_{18}H_{19}F_7N_5O$ calculated value 454.1472, measured value 454.1488.

Example 101

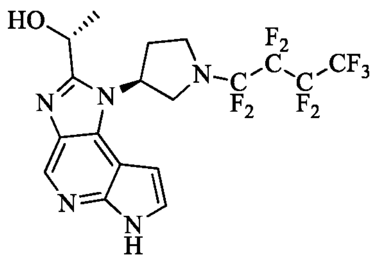

(R)-1-(1-((S)-1-(Perfluoro-butyl)pyrrolidin-3 -yl)-1,6-dihydroimidazo[4,5-d]pyrrolo[2,3-b]pyridin-2-yl)ethanol**LXS101** was prepared using a similar preparation method as in Example 97, with the raw material of perfluorobutane. The crude product was purified using a silica gel column (petroleum ether: ethyl acetate = 1:1) to yield **LXS101:** (R)-1-(1-((S)-1-(Perfluoro-butyl)pyrrolidin-3-yl)-1,6-dihydroimidazo[4, 5-d]pyrrolo[2,3-b]pyridin-2-yl)ethanol (0.1g. 64%). $^1$HNMR (300 MHz, *DMSO-d$_6$*): $\delta$ = 12.45 (s, 1H), 8.91 (s, 1H), 7.39 (s, 1H), 6.68 (s, 1H), 4.60-4.74 (m, 1H), 3.73-3.82 (m, 1H), 2.54-2.88 (m, 2H), 1.90-2.30 ( m, 4H), 1.51 (d, *J* = 6.0 Hz, 3H) ppm; $^{13}$CNMR (75 MHz, *DMSO-d$_6$*) $\delta$ 156.61, 148.61, 148.51, 142.18, 129.01, 127.34, 120.78, 118.20, 115.28, 107.24, 99.33, 63.88, 58.99, 44.84, 42.38, 27.65, 22.81 ppm; HRMS (ESI): m/z [M+H]$^+$. $C_{18}H_{17}F_9N_5O$ calculated value 490.1284, measured value 490.1288.

Example 102

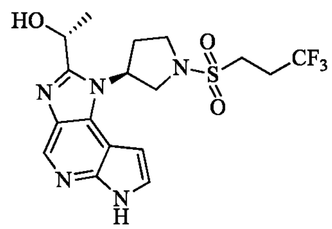

(R)-1-(1-((S)-1-((3,3,3-Trifluoropropyl)sulfonyl)pyrrolidin-3-yl)imidazo[4,5-d]pyrrolo[2,3-b]pyridin-2-yl)ethanol

(i) **LXS102** was prepared using a similar preparation method as in Example 97 using the raw material of trifluoromethylpropylsulfonyl chloride, and the crude product was purified using a silica gel column (petroleum ether: ethyl acetate = 1:1) to yield **LXS102:** (R)-1-(1-((S)-1-((3,3,3-trifluoropropyl)sulfonyl)pyrrolidin-3-yl)imidazo[4,5-d]pyrrolo[2,3-b]pyridin-2-yl) ethanol (0.2 g, 69%). $^{1}$HNMR (300 MHz, *DMSO-$d_6$* ): $\delta$ = 12.71 (s, 1H), 8.56 (s, 1H), 7.38 (s, 1H), 6.74 (s, 1H), 4.68-4.78 (m, 1H), 3.73-3.84 (m, 1H), 3.20 (d, *J* = 4.5 Hz, 2H), 3.11 (t, *J* = 6.0 Hz, 2H), 2.70-2.84 (m, 2H), 2.37 (t, *J* = 6.0 Hz, 2H), 1.91-2.15 (m, 2H), 1.43 (d, *J* = 6.0 Hz, 3H) ppm; $^{13}$CNMR (75 MHz, *DMSO-$d_6$*) $\delta$ 148.66, 148.51, 142.15, 129.05, 127.10, 125.47, 120.71, 115.64, 99.34, 63.64, 57.58, 56.14, 50.04, 40.08, 29.87, 26.27, 22.87 ppm; HRMS (ESI): m/z $[M+H]^+$. $C_{17}H_{21}F_3N_5O_3S$ calculated value 432.1312, measured value 432.1318.

Example 103

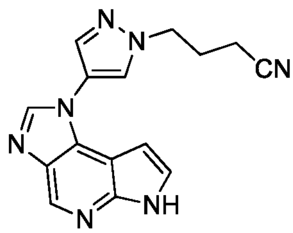

4-(4-(Imidazo[4,5-d]pyrrolo[2,3-b]pyridin-1(6H)-yl)-1H-pyrazol-1-yl)butanenitrile

**LXS103** was prepared using a preparation method similar to that used for the preparation of Intermediate 10 and Example 79 using the raw material of 4-bromopropyl cyanide, and the crude product was purified using a silica gel column (petroleum ether: ethyl acetate = 1:1) to yield **LXS103:** 4-(4-(imidazo[4,5-d]pyrrolo[2,3-b]pyridin-1(6H)-yl)-1H-pyrazol-1-yl)butanenitrile (0.1 g. 57%).$^1$ HNMR (300 MHz, *DMSO-$d_6$ ): $\delta$* = 12.13 (s, 1H), 8.88 (s, 1H), 8.06 (s, 1H), 7.94 (s, 1H), 7.55 (s, 1H), 7.15 (s, 1H), 6.68 (s, 1H), 4.46 (t, *J* = 6.0 Hz, 2H), 2.10- 2.21 (m, 2H), 1.87 (t, *J* = 6.0 Hz, 2H) ppm;$^{13}$ C NMR (75 MHz, *DMSO-$d_6$ )* $\delta$ 148.77, 142.11, 134.58, 130.77, 129.71, 129.08, 127.88, 120.74, 119.34, 115.67, 100.57, 99.37, 52.38, 22.74, 14.97 ppm; HRMS (ESI): m/z $[M+H]^+$. $C_{15}H_{14}N_7$ calculated value 292.1305, measured value 292.1316.

Example 104

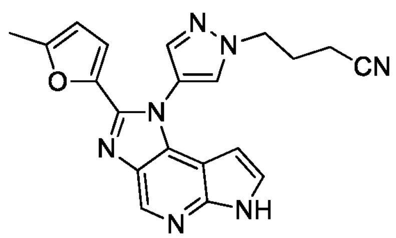

4-(4-(2-(5-methylfuran-2-yl)imidazo[4,5-d]pyrrolo[2,3-b]pyridin-1(6H)-yl)-1H-pyrazol-1-yl)butanenitrile

**LXS104** was prepared using a similar preparation method as in Example 103 using the raw material of 5-methylfuran-2-formaldehyde, and the crude product was purified using a silica gel column (petroleum ether: ethyl acetate = 1:1) to yield **LXS104:** 4-(4-(2-(5-methylfuran-2-yl)imidazo[4,5-d]pyrrolo[2,3-b]pyridin-1(6H)-yl)-1H-pyrazol-1-yl)butanenitrile (0.2 g, 67%).$^1$ HNMR (300 MHz, *DMSO-$d_6$*): $\delta$ = 12.33 (s, 1H), 8.85 (s, 1H), 8.11 (s, 1H), 7.98 (s, 1H), 7.55 (s, 1H), 6.95 (d, *J* = 6.0 Hz, 1H), 6.68 (s, 1H), 6.08 (d, *J* = 6.0 Hz, 1H), 4.38 (t, *J* = 6.0 Hz, 2H), 2.30 (s, 3H), 2.14-2.25 (m, 2H), 1.91 (t, *J* = 4.5 Hz, 2H) ppm;$^{13}$ C NMR (75 MHz, *DMSO-$d_6$*) $\delta$ 152.21, 151.47, 148.65, 142.18, 141.28, 130.75, 129.70, 129.08, 127.18, 120.78, 119.58, 115.68, 107.88, 107.67, 100.51, 99.34, 52.38, 22.74, 14.95, 13.77 ppm; HRMS (ESI): m/z $[M+H]^+$. $C_{20}H_{18}N_7O$ calculated value 372.1567, measured value 352.1588.

Example 105

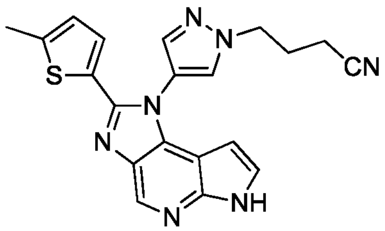

4-(4-(2-(5-Methylthiophen-2-yl)imidazo[4,5-d]pyrrolo[2,3-b]pyridin-1(6H)-yl)-1H-pyrazol-1-yl butanenitrile

**LXS105** was prepared using a preparation method similar to Example 103 using the raw material of 5-methylthiophene-2-formaldehyde, and the crude product was purified using a silica gel column (petroleum ether: ethyl acetate = 1:1) to yield **LXS105:** 4-(4-(2-(5-methylthiophen-2-yl)imidazo[4,5-d]pyrrolo[2,3-b]pyridin-1(6H)-yl)-1H-pyrazol-1-yl butanenitrile (0.1 g, 45%).$^1$ HNMR (300 MHz, *DMSO-$d_6$):* $\delta$ = 12.28 (s, 1H), 8.68 (s, 1H), 8.06 (s, 1H), 7.94 (s, 1H), 7.63 (s, 1H), 7.49 (d, *J* = 6.0 Hz, 1H), 6.83 (d, *J* = 6.0 Hz, 1H), 6.61 (s, 1H), 4.46 (t, *J* = 6.0 Hz, 2H), 2.36 (s, 3H), 2.11-2.23 (m, 2H), 1.87 (t, *J* = 4.5 Hz, 2H) ppm;$^{13}$ C NMR (75 MHz, *DMSO-$d_6$* ) $\delta$ 148.66, 142.18, 141.68, 141.24, 134.38, 130.75, 129.75, 129.04, 127.54, 127.45, 120.77, 119.35, 115.67, 100.57, 99.35, 52.38, 22.75, 15.29, 14.95 ppm; HRMS (ESI): m/z $[M+H]^+$. $C_{20}H_{18}N_7S$ calculated value 388.1339, measured value 388.1345.

Example 106

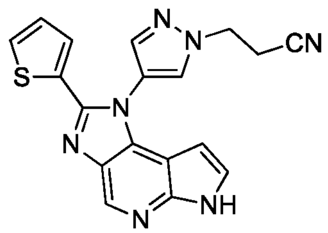

(4-(2-(Thiophen-2-yl)imidazo[4,5-d]pyrrolo[2,3-b]pyridin-1(6H)-yl)-1H-pyrazol-1-yl)propanenitrile

**LXS106** was prepared using a similar preparation method as in Example 79 using the raw material of thiophene-2formaldehyde, and the crude product was purified using a silica gel column (petroleum ether: ethyl acetate = 1:1) to yield **LXS106:** 3-(4-(2-(Thiophen-2-yl)imidazo[4,5-d]pyrrolo[2,3-b]pyridin-1(6H)-yl)-1H-pyrazol-1-yl)propanenitrile ( 0.1 g, 64%).$^1$ HNMR (300 MHz, *DMSO-$d_6$):* $\delta$ = 12.34 (s, 1H), 8.84 (s, 1H), 8.34 (s, 1H), 7.98 (s, 1H), 7.85 (d, *J*= 4.5 Hz, 1H), 7.69 (d, *J* = 4.5 Hz, 1H), 7.63 (s, 1H), 7.19 (m, 1H), 6.56 (s, 1H), 5.08 (t, *J* = 6.0 Hz, 2H), 3.20 (t, *J*= 6.0 Hz, 2H) ppm;$^{13}$ C NMR (75 MHz, *DMSO-$d_6$*) $\delta$ 148.68, 143.98, 142.18, 141.28, 130.74, 129.72, 129.10, 129.00, 128.61, 128.07, 127.15, 120.75, 117.77, 115.65, 100.58, 99.38, 49.25, 15.92 ppm; HRMS (ESI): m/z $[M+H]^+$. $C_{18}H_{14}N_7S$ calculated value 360.1026, measured value 360.1038.

Example 107

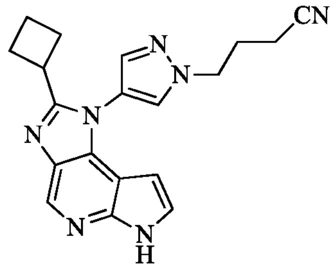

4-(4-(2-Cyclobutylimidazo[4,5-d]pyrrolo[2,3-b]pyridin-1(6H)-yl)-1H-pyrazol-1-yl)butanenitrile

**LXS107** was prepared using a similar preparation method as in Example 103 using the raw material of cyclobutanecarboxaldehyde, and the crude product was purified using a silica gel column (petroleum ether: ethyl acetate = 1:1) to yield **LXS107:** 4-(4-(2-cyclobutylimidazo[4,5-d]pyrrolo[2,3-b]pyridin-1(6H)-yl)-1H-pyrazol-1-yl)butanenitrile (0.1 g. 57%).$^1$ HNMR (300 MHz, *DMSO-$d_6$*): $\delta$ = 12.34 (s, 1H), 8.89 (s, 1H), 8.06 (s, 1H), 7.96 (s, 1H), 7.63 (s, 1H), 6.56 (s, 1H), 4.46 (t, $J$ = 6.0 Hz, 2H), 3.19-3.24 (m, 1H), 2.15-2.38 (m, 4H), 2.10-2.14 (m, 2H), 1.91-2.01 (m, 2H), 1.77-1.87 (m, 2H) ppm;$^{13}$ C NMR (75 MHz, *DMSO-$d_6$)* $\delta$ 148.60, 144.89, 142.18, 130.77, 129.71, 129.08, 127.18, 120.75, 119.37, 115.67, 100.59, 99.38, 52.38, 32.69, 26.28, 22.78, 18.93, 14.92 ppm; HRMS (ESI): m/z $[M+H]^+$. $C_{19}H_{20}N_7$ calculated value 346.1775, measured value 346.1778.

Example 108

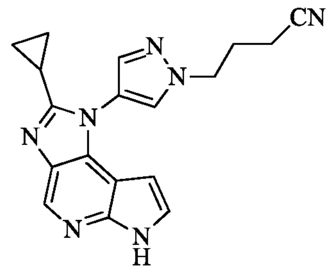

4-(4-(2-Cyclopropylimidazo[4,5-d]pyrrolo[2,3-b]pyridin-1(6H)-yl)-1H-pyrazol-1-yl)butanenitrile

**LXS108** was prepared using a similar preparation method as in Example 103 using the raw material of cyclopropylformaldehyde, and the crude product was purified using a silica gel column (petroleum ether: ethyl acetate = 1:1) to yield **LXS108:** 4-(4-(2-cyclopropylimidazo[4,5-d]pyrrolo[2,3-b]pyridin-1(6H)-yl)-1H-pyrazol-1-yl)butanenitrile (0.1 g. 62%).$^1$ HNMR (300 MHz, *DMSO-$d_6$*): $\delta$ = 12.11 (s, 1H), 8.69 (s, 1H), 8.11 (s, 1H), 7.94 (s, 1H), 7.58 (s, 1H), 6.41 (s, 1H), 4.51 (t, $J$ = 6.0 Hz, 2H), 2.10-2.18 (m, 2H), 1.77-1.87 (m, 2H), 1.35-1.52 (m, 1H), 0.99-1.24 (m, 4H) ppm;$^{13}$ C NMR (75 MHz, *DMSO-$d_6$)* $\delta$ 148.68, 144.51, 142.98, 130.77, 129.75, 129.05, 127.15, 120.77, 119.35, 115.68, 100.54, 99.35, 52.38, 22.74, 14.95, 8.59, 3.48 ppm; HRMS (ESI): m/z $[M+H]^+$. $C_{18}H_{18}N_7$ calculated value 332.1618, measured value 332.1624.

**In vitro activity test of JAK1 and JAK2 kinases**

**Experimental materials**

**1. Reagents and consumables**

| Reagent name | Supplier | Item No. | Lot number |
|---|---|---|---|
| JAK1 | Carna | 08-114 | 11CBS-0144M |

(continued)

| Reagent name | Supplier | Item No. | Lot number |
|---|---|---|---|
| JAK2 | Carna | 08-045 | 10CBS-0289R |
| Kinase substrate22 | GL | 112393 | P190917-CL112393 |
| Kinase JAK1 peptide | GL | 758318 | P191104-TL758318 |
| DMSO | Sigma | D8418-1L | SHBG3288V |
| 384-well plate | Corning | 3573 | 12619003 |

**2. Experimental apparatus**

Centrifuge (manufacturer: Eppendorf, model: 5430)
ELISA Instrument (Manufacturer: Perkin Elmer, Model: Caliper EZ Reader II)
Echo 550 (Manufacturer: Labcyte, Model: Echo 550)

**Experimental method**

**1. Kinase reaction process**

(1) Prepare 1 ×Kinase buffer.
(2) Compound concentration gradient preparation: The test compound was tested at 1000 nM, 3-fold dilution, 10 concentrations, two-well assay, and configured to a 100-fold final concentration in a 384-well plate. 250 nl of the test compound was then transferred to the 384 reaction plate using Echo550. 250 nl of 100% DMSO was added to each of the negative and positive control wells.
(3) Prepare 2.5 times the final concentration of kinase solution with 1 ×Kinase buffer.
(4) Add 10 μL of 2.5 times the final concentration of kinase solution to the compound wells and 10 μL of 1 ×Kinase buffer to the negative control wells.
(5) Centrifuge at 1000 rpm for 30 seconds, mix well with shaking, and incubate at room temperature for 10 minutes.
(6) Prepare a 25/15-fold final concentration of mixture of ATP and Kinase substrate with 1×Kinase buffer.
(7) Add 15 μL of a mixture of 25/15 times the final concentration of ATP and substrate to start the reaction.
(8) Centrifuge the 384-well plate at 1000 rpm for 30 seconds, mix well with shaking, and incubate at room temperature for an appropriate amount of time.
(9) Stop the kinase reaction by adding 30 μL of termination solution, centrifuge at 1000 rpm for 30 seconds, and mix well with shaking.
(10) Read the conversion rate with Caliper EZ Reader.

**2. Data analysis**

Calculation formula

$$\% \text{ Inhibition} = \frac{\text{Conversion\%_max - Conversion\%_sample}}{\text{Conversion\%_max - Conversion\%_min}} \times 100$$

where: Conversion%_sample is the conversion rate reading of the sample; Conversion%_min: the average value of the negative control wells, representing the conversion reading of the wells without enzyme activity; Conversion%_max: the average value of the positive control wells, representing the conversion reading of the wells without compound inhibition.

Fitting the dose-effect curve

The log value of concentration was used as the X-axis and the percentage inhibition as the Y-axis, and the function of the analytical software GraphPad Prism 5: log(inhibitor) vs. response -Variable slope, was used to fit dose-effect curves to obtain the IC50 values of each compound on enzyme activity.

The formula is Y=Bottom + (Top-Bottom)/(1+10^((LogIC50-X)*HillSlope))

The IC50 data are specified in **Table 1.**

Table 1

| Example of implementation | Compound | **JAK1** | **JAK2** |
|---|---|---|---|
| 1 | **LXS01** | C | C |
| 2 | **LXS02** | C | C |
| 3 | **LXS03** | C | C |
| 4 | **LXS04** | B | C |
| 5 | **LXS05** | B | C |
| 6 | **LXS06** | B | C |
| 7 | **LXS07** | C | C |
| 8 | **LXS08** | C | C |
| 9 | **LXS09** | C | D |
| 10 | **LXS10** | C | D |
| 11 | **LXS11** | B | C |
| 12 | **LXS12** | B | C |
| 13 | **LXS13** | C | B |
| 14 | **LXS14** | C | B |
| 15 | **LXS15** | C | B |
| 16 | **LXS16** | B | B |
| 17 | **LXS17** | B | B |
| 18 | **LXS18** | B | B |
| 19 | **LXS19** | B | B |
| 20 | **LXS20** | C | C |
| 21 | **LXS21** | C | C |
| 22 | **LXS22** | B | B |
| 23 | **LXS23** | C | C |
| 24 | **LXS24** | C | C |
| 25 | **LXS25** | C | C |
| 26 | **LXS26** | B | B |
| 27 | **LXS27** | B | B |
| 28 | **LXS28** | B | B |
| 29 | **LXS29** | B | B |
| 30 | **LXS30** | C | C |
| 31 | **LXS31** | C | C |
| 32 | **LXS32** | B | A |

(continued)

| Example of implementation | Compound | **JAK1** | **JAK2** |
|---|---|---|---|
| 33 | **LXS33** | B | B |
| 34 | **LXS34** | C | C |
| 35 | **LXS35** | B | A |
| 36 | **LXS36** | B | B |
| 37 | **LXS37** | C | C |
| 38 | **LXS38** | B | B |
| 39 | **LXS39** | B | C |
| 40 | **LXS40** | B | B |
| 41 | **LXS41** | C | C |
| 42 | **LXS42** | C | C |
| 43 | **LXS43** | B | C |
| 44 | **LXS44** | B | B |
| 45 | **LXS45** | B | B |
| 46 | **LXS46** | B | A |
| 47 | **LXS47** | C | C |
| 48 | **LXS48** | B | C |
| 49 | **LXS49** | B | C |
| 50 | **LXS50** | C | A |
| 51 | **LXS51** | B | B |
| 52 | **LXS52** | C | B |
| 53 | **LXS53** | B | B |
| 54 | **LXS54** | C | C |
| 55 | **LXS55** | C | C |
| 56 | **LXS56** | B | C |
| 57 | **LXS57** | C | C |
| 58 | **LXS58** | B | B |
| 59 | **LXS59** | B | C |
| 60 | **LXS60** | B | B |
| 61 | **LXS61** | C | C |
| 62 | **LXS62** | C | A |
| 63 | **LXS63** | C | A |
| 64 | **LXS64** | C | A |
| 65 | **LXS65** | A | C |
| 66 | **LXS66** | B | B |
| 67 | **LXS67** | B | B |
| 68 | **LXS68** | A | C |
| 69 | **LXS69** | B | B |
| 70 | **LXS70** | B | B |

(continued)

| Example of implementation | Compound | **JAK1** | **JAK2** |
|---|---|---|---|
| 71 | **LXS71** | A | C |
| 72 | **LXS72** | B | B |
| 73 | **LXS73** | A | C |
| 74 | **LXS74** | A | C |
| 75 | **LXS75** | A | C |
| 76 | **LXS76** | A | C |
| 77 | **LXS77** | C | A |
| 78 | **LXS78** | B | B |
| 79 | **LXS79** | C | A |
| 80 | **LXS80** | B | B |
| 81 | **LXS81** | B | B |
| 82 | **LXS82** | B | B |
| 83 | **LXS83** | B | B |
| 84 | **LXS84** | B | B |
| 85 | **LXS85** | A | C |
| 86 | **LXS86** | A | C |
| 87 | **LXS87** | A | C |
| 88 | **LXS88** | A | C |
| 89 | **LXS89** | B | C |
| 90 | **LXS90** | B | C |
| 91 | **LXS91** | A | C |
| 92 | **LXS92** | B | D |
| 93 | **LXS93** | A | C |
| 94 | **LXS94** | B | C |
| 95 | **LXS95** | C | B |
| 96 | **LXS96** | A | C |
| 97 | **LXS97** | A | C |
| 98 | **LXS98** | B | B |
| 99 | **LXS99** | B | B |
| 100 | **LXS100** | A | C |
| 101 | **LXS101** | A | C |
| 102 | **LXS102** | A | C |
| 103 | **LXS103** | B | B |
| 104 | **LXS104** | B | B |
| 105 | **LXS105** | C | B |
| 106 | **LXS106** | C | B |
| 107 | **LXS107** | C | B |

(continued)

| Example of implementation | Compound | **JAK1** | **JAK2** |
|---|---|---|---|
| 108 | **LXS108** | C | B |
| [0757] A<10nM; 10nM <B<100nM; 100nM <C<1000nM; D>1000nM. | | | |

## Claims

1. A compound shown in formula **I:**

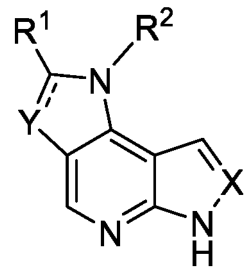

**I**

or a tautomer, stereoisomer, racemate or isotopic derivative thereof, or a pharmaceutically acceptable salt of any of the foregoing, or a crystal form or solvate of any of the foregoing;

wherein X is CH or N;
Y is NH or N; when Y is N, the $\overline{---}$ connected to Y is a double bond; when Y is NH, the $\overline{---}$ connected to Y is a single bond;
$R^1$ and $R^2$ are defined as (i), (ii) or (iii) as follows:

(i) $R^1$ is

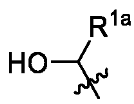

,

and $R^2$ is

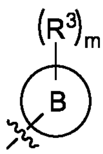

or

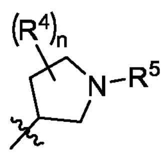

;

ring B is a benzene ring or a 5-6-membered heteroaromatic ring;

$R^{1a}$ is a $C_{1-3}$ alkyl group;
each $R^3$ is independently a halogen, a cyano group, a $C_{1-4}$ alkyl group, a $C_{1-4}$ haloalkyl group, a -O-$C_{1-4}$ alkyl group, or a -O-$C_{1-4}$ haloalkyl group;
each $R^4$ is independently a halogen, a hydroxyl group, a $C_{1-4}$ alkyl group, a $C_{1-4}$ haloalkyl group, a -O-$C_{1-4}$ alkyl group, a -O-$C_{1-4}$ haloalkyl group or a $C_{1-4}$ hydroxyalkyl group;
alternatively, two $R^4$ located on the same carbon atom or on different carbon atoms are interconnected to form $-CH_2-$ or $-(CH_2)_2-$;
$R^5$ is $-S(O)_2R^{5a}$, $-C(O)R^{5b}$, $-C(O)NR^{5c}R^{5d}$, $-C(O)OR^{5e}$, $-C(O)NHR^{5k}$ or $-L^1-R^{5f}$;

$R^{5a}$ is a $C_{2-6}$ alkyl group, a $C_{2-6}$ haloalkyl group, $-L^1-R^{5f}$, an unsubstituted or substituted 6-10-membered aryl group or an unsubstituted or substituted 5-10-membered heteroaryl group, said substituted 6-10-membered aryl group and substituted 5-10-membered heteroaryl group are defined as structures where 1, 2, 3 or 4 hydrogen atoms in the 6-10-membered aryl group and the 5-10-membered heteroaryl group are independently substituted by $R^{5g}$;

$R^{5b}$, $R^{5c}$, $R^{5d}$, $R^{5e}$ and $R^{5k}$ are each independently a $C_{1-6}$ alkyl group, a $C_{1-6}$ haloalkyl group, $-L^1-R^{5f}$, an unsubstituted or substituted 6-10-membered aryl group, or an unsubstituted or substituted 5-10-membered heteroaryl group, said substituted 6 -10-membered aryl group and substituted 5-10-membered heteroaryl group are defined as structures where 1, 2, 3 or 4 hydrogen atoms in the 6-10-membered aryl group and the 5-10-membered heteroaryl group are independently substituted by $R^{5h}$;

each $R^{5g}$ and $R^{5h}$ is independently a halogen, a cyano group, a $C_{1-4}$ alkyl group, a $C_{1-4}$ haloalkyl group, a $-O-C_{1-4}$ alkyl group, a $-O-C_{1-4}$ haloalkyl group, or a phenyl group;

each $L^1$ is independently $-[C(R^aR^b)]_{1-5}-$, $-[C(R^aR^b)]_{1-2}-C(O)-[C(R^aR^b)]_{1-2}-$, $-[C(R^aR^b)]_{1-2}-C(O)NH-[C(R^aR^b)]_{1-2}-$, $-[C(R^aR^b)]_{1-2}-NHC(O)-[C(R^aR^b)]_{1-2}-$, $-[C(R^aR^b)]_{1-2}-S(O)_2-[C(R^aR^b)]_{1-2}-$, $-[C(R^aR^b)]_{1-2}-NHS(O)_2-[C(R^aR^b)]_{1-2}-$ or $-[C(R^aR^b)]_{1-2}-S(O)_2NH-[C(R^aR^b)]_{1-2}$;

each $R^{5f}$ is independently H, F, $CHF_2$, $CH_2F$, $CF_3$ or CN;

each $R^a$ is independently H, a halogen or a $C_{1-3}$ alkyl group;

each $R^b$ is independently H, a halogen or a $C_{1-3}$ alkyl group;

alternatively, $R^a$ and $R^b$, along with the carbon atom connected to them, form a cyclopropyl group;

m is 0, 1, 2, 3 or 4;

n is 0, 1, 2, 3 or 4;

(ii) $R^1$ is

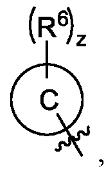

,

and $R^2$ is

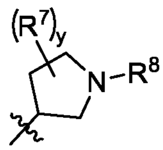

or

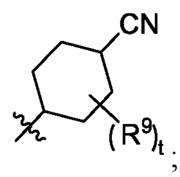

;

ring C is a benzene ring or a 5-6-membered heteroaromatic ring;

each $R^6$ is independently a halogen, a hydroxyl group, an amino group, a cyano group, a $C_{1-4}$ alkyl group, a $C_{1-4}$ haloalkyl group, a $-O-C_{1-4}$ alkyl group, a $-O-C_{1-4}$ haloalkyl group, a $-S-C_{1-4}$ alkyl group, a $-S(O)_2-C_{1-4}$ alkyl group or a $C_{1-4}$ hydroxyalkyl group;

each $R^7$ is independently $R^4$;

alternatively, two $R^7$ located on the same carbon atom or on different carbon atoms are interconnected to form $-CH_2-$ or $-(CH_2)_2-$;

$R^8$ is $R^5$, $-S(O)_2R^{8a}$, $-C(O)R^{8b}$, $-C(O)NR^{8c}R^{8d}$, $-C(O)OR^{8e}$ or $-C(O)NHR^{8k}$;

$R^{8a}$, $R^{8b}$, $R^{8c}$, $R^{8d}$, $R^{8e}$ and $R^{8k}$ are each independently a methyl group, $-CF_3$, a $C_{2-6}$ alkenyl group or a $C_{3-6}$ cycloalkyl group;

each $R^9$ is independently $R^4$;

alternatively, two $R^9$ located on the same carbon atom or on different carbon atoms are interconnected to form $-CH_2-$ or $-(CH_2)_2-$;

z is 0, 1, 2, 3 or 4;

y is 0, 1, 2, 3 or 4;

t is 0, 1, 2, 3 or 4;

(iii) $R^1$ is H, $CF_3$ or

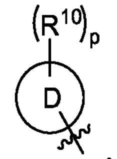

,

and $R^2$ is

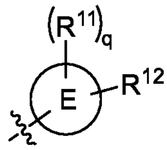

;

ring D is a $C_{3-6}$ cycloalkyl group, benzene ring or 5-6-membered heteroaromatic ring;
ring E is a benzene ring or a 5-6-membered heteroaromatic ring;
each $R^{10}$ and $R^{11}$ is independently a halogen, a hydroxyl group, an amino group, a cyano group, a $C_{1-4}$ alkyl group, a $C_{1-4}$ haloalkyl group, a -O-$C_{1-4}$ alkyl group, a -O-$C_{1-4}$ haloalkyl group, a -S-$C_{1-4}$ alkyl group, a -$S(O)_2$-$C_{1-4}$ alkyl group or a $C_{1-4}$ hydroxyalkyl group;
$R^{12}$ is a cyano group, -$L^3$-$R^{12f}$ or

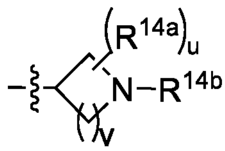

;

each $R^{14a}$ is independently a halogen, a cyano group, a $C_{1-4}$ alkyl group, a cyano-substituted $C_{1-4}$ alkyl group, a $C_{1-4}$ haloalkyl group, a -O-$C_{1-4}$ alkyl group, a -O-$C_{1-4}$ haloalkyl group, or a $C_{1-4}$ hydroxyalkyl group.
$R^{14b}$ is $R^5$ or a -$S(O)_2$ -$C_{1-4}$ alkyl group;
$L^3$ is -$[C(R^eR^f)]_{1-5}$-, -C(O)-, -C(O)NH-, -NHC(O)-, -$S(O)_2$-, -$NHS(O)_2$-, -$S(O)_2NH$-, -$[C(R^eR^f)]_{1-2}$-C(O)-$[C(R^eR^f)]_{1-2}$-, -$[C(R^eR^f)]_{1-2}$-C(O)NH-$[C(R^eR^f)]_{1-2}$-, -$[C(R^eR^f)]_{1-2}$-NHC(O)-$[C(R^eR^f)]_{1-2}$-, -$[C(R^eR^f)]_{1-2}$-$S(O)_2$-$[C(R^eR^f)]_{1-2}$-, -$[C(R^eR^f)]_{1-2}$-$NHS(O)_2$-$[C(R^eR^f)]_{1-2}$- or - $[C(R^eR^f)]_{1-2}$-$S(O)_2NH$-$[C(R^eR^f)]_{1-2}$-;
$R^{12f}$ is H, F, $CHF_2$, $CH_2F$, $CF_3$ or CN;
each $R^e$ and $R^f$ is independently H, a halogen, a $C_{1-3}$ alkyl group, or a $C_{3-6}$ cycloalkyl group; alternatively, $R^e$ and $R^f$ along with the carbon atom connected to them, form a cyclopropyl group;
p is 0, 1, 2, 3 or 4;
q is 0, 1, 2, 3 or 4;
v is 0, 1, 2 or 3;
u is 0, 1 or 2; and
the heteroatom in the above heteroaryl group is independently N, O or S, and the number of heteroatoms in the above heteroaryl group is independently 1, 2, 3 or 4.

**2.** The compound as claimed in claim 1, or a tautomer, stereoisomer, racemate or isotopic derivative thereof, or a pharmaceutically acceptable salt of any of the foregoing, or a crystalline or solvated form of any of the foregoing, which is **characterized by**: $R^{1a}$ is a methyl group;

and/or, ring B is a benzene ring;
and/or, the $C_{2-6}$ alkyl group in the definition of $R^{5a}$ is an ethyl group, a n-propyl group or a n-butyl group;
and/or, the halogen in the definition of $R^4$ is fluorine;
and/or, the $C_{1-4}$ alkyl group in the definition of $R^4$ is a methyl group;

and/or, the $C_{1-4}$ haloalkyl group in the definition of $R^4$ is a $C_{1-4}$ fluoroalkyl group;
and/or, the -O-$C_{1-4}$ alkyl group in the definition of $R^4$ is -O-CHs;
and/or, the -O-$C_{1-4}$ haloalkyl group in the definition of $R^4$ is a -O-$C_{1-4}$ fluoroalkyl group;
and/or, the $C_{1-4}$ hydroxyalkyl group in the definition of $R^4$ is -$CH_2$OH;
and/or, "unsubstituted or substituted 6-10-membered aryl group" in the definition of $R^{5a}$ is "unsubstituted or substituted phenyl group", or "unsubstituted or substituted naphthyl group";
and/or, "unsubstituted or substituted 5-10-membered heteroaryl group" in the definition of $R^{5a}$ is "unsubstituted or substituted 5- or 6-membered heteroaryl group";
and/or, the halogen in the definitions of $R^{5g}$ and $R^{5h}$ is fluorine;
and/or, the $C_{1-4}$ alkyl group in the definitions of $R^{5g}$ and $R^{5h}$ is a methyl group;
and/or, the -O-$C_{1-4}$ haloalkyl group in the definitions of $R^{5g}$ and $R^{5h}$ is -$OCF_3$;
and/or, the $C_{1-4}$ haloalkyl group in the definitions of $R^{5g}$ and $R^{5h}$ is a $C_{1-4}$ fluoroalkyl group;
and/or, the -O-$C_{1-4}$ alkyl group in the definitions of $R^{5g}$ and $R^{5h}$ is -O-CHs;
and/or, "unsubstituted or substituted 6-10-membered aryl group" in the definition of $R^{5b}$ is "unsubstituted or substituted phenyl group", or "unsubstituted or substituted naphthyl group";
and/or, the halogen in the definition of $R^a$ is fluorine;
and/or, the $C_{1-3}$ alkyl group in the definition of $R^a$ is a methyl or ethyl group;
and/or, the halogen in the definition of $R^b$ is fluorine;
and/or, the $C_{1-3}$ alkyl group in the definition of $R^b$ is a methyl or ethyl group;
and/or, each $L^1$ is independently $-[C(R^aR^b)]_{1-5}-$ or $-[C(R^aR^b)]_{1-2}-C(O)NH-[C(R^aR^b)]_{1-2}-$;
and/or, m is 1;
and/or, n is 0, 1 or 2;
and/or, the 5-6-membered heteroaromatic ring in the definition of ring C is an imidazole, thiazole, furan, thiophene or pyridine;
and/or, the halogen in the definition of $R^6$ is fluorine;
and/or, the $C_{1-4}$ alkyl group in the definition of $R^6$ is a methyl group;
and/or, the $C_{1-4}$ haloalkyl group in the definition of $R^6$ is a $C_{1-4}$ fluoroalkyl group;
and/or, the -O-$C_{1-4}$ alkyl group in the definition of $R^6$ is -O-CHs;
and/or, the -O-$C_{1-4}$ haloalkyl group in the definition of $R^6$ is a -O-$C_{1-4}$ fluoroalkyl group;
and/or, the -S-$C_{1-4}$ alkyl group in the definition of $R^6$ is -S-$CH_3$;
and/or, the -$S(O)_2$-$C_{1-4}$ alkyl group in the definition of $R^6$ is -$S(O)_2$-$CH_3$;
and/or, the $C_{1-4}$ hydroxyalkyl group in the definition of $R^6$ is -$CH_2$-OH;
and/or, the $C_{2-6}$ alkenyl group in the definitions of $R^{8a}$, $R^{8b}$, $R^{8c}$, $R^{8d}$, $R^{8e}$ and $R^{8k}$ is a vinyl group;
and/or, the $C_{3-6}$ cycloalkyl group in the definitions of $R^{8a}$, $R^{8b}$, $R^{8c}$, $R^{8d}$, $R^{8e}$ and $R^{8k}$ is a cyclopropyl group;
and/or,

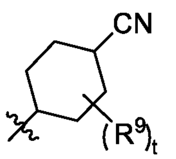

is *cis* or *trans* conformation;
and/or, t is 0;
and/or, y is 0;
and/or, z is 0, 1 or 2;
and/or, the $C_{3-6}$ cycloalkyl group in the definition of ring D is a cyclopropyl or cyclobutyl group;
and/or, the 5-6-membered heteroaromatic ring in the definition of ring D is a furan or a thiophene;
and/or, the 5-6-membered heteroaromatic ring in the definition of ring E is pyrazole;
and/or, the halogens in the definitions of $R^{10}$ and $R^{11}$ are fluorine or chlorine;
and/or, the $C_{1-4}$ alkyl group in the definitions of $R^{10}$ and $R^{11}$ is a methyl group;
and/or, the $C_{1-4}$ haloalkyl group in the definitions of $R^{10}$ and $R^{11}$ is a $C_{1-4}$ fluoroalkyl group;
and/or, the -O-$C_{1-4}$ alkyl group in the definitions of $R^{10}$ and $R^{11}$ is -O-CHs;
and/or, the -O-$C_{1-4}$ haloalkyl group in the definitions of $R^{10}$ and $R^{11}$ is a -O-$C_{1-4}$ fluoroalkyl group;
and/or, the -S-$C_{1-4}$ alkyl group in the definitions of $R^{10}$ and $R^{11}$ is -S-$CH_3$;
and/or, the -$S(O)_2$-$C_{1-4}$ alkyl group in the definitions of $R^{10}$ and $R^{11}$ is -$S(O)_2$-$CH_3$;
and/or, the $C_{1-4}$ hydroxyalkyl group in the definitions of $R^{10}$ and $R^{11}$ is -$CH_2$-OH;
and/or, q is 0;

and/or, p is 0, 1 or 2;
and/or, the $-S(O)_2-C_{1-4}$ alkyl group in the definition of $R^{14b}$ is $-S(O)_2-CH_2CH_3$, $-S(O)_2-CH_2CH_2CH_3$ or $-S(O)_2-CH_2CH_2CH_2CH_3$;
and/or, the halogen in the definitions of $R^e$ and $R^f$ is fluorine;
and/or, the $C_{1-3}$ alkyl group in the definitions of $R^e$ and $R^f$ is a methyl or ethyl group;
and/or, the $C_{3-6}$ cycloalkyl group in the definitions of $R^e$ and $R^f$ is a cyclopropyl group, a cyclobutyl group or a cyclopentyl group.

**3.** The compound as claimed in claim 1 or 2, or a tautomer, stereoisomer, racemate or isotopic derivative thereof, or a pharmaceutically acceptable salt of any of the foregoing, or a crystalline or solvated form of any of the foregoing, which is **characterized by**:

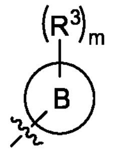

is

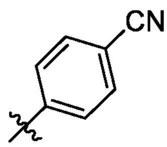

;

and/or, each $R^4$ is independently a halogen or a $C_{1-4}$ hydroxyalkyl group, or, two $R^4$ located at the same carbon atom or at different carbon atoms are interconnected to form $-CH_2-$ or $-(CH_2)_2-$; and/or, "unsubstituted or substituted 6-10-membered aryl group" in the definition of $R^{5a}$ is "unsubstituted phenyl group", or "phenyl group independently substituted with 1, 2, 3 or 4 of the following functional groups: a halogen, a $C_{1-4}$ alkyl group, a $-O-C_{1-4}$ haloalkyl group, or a phenyl group";

and/or, "unsubstituted or substituted 5-10-membered heteroaryl group" in the definition of $R^{5a}$ is "unsubstituted or substituted thiophene group";
and/or, "unsubstituted or substituted 6-10-membered aryl group" in the definition of $R^{5b}$ is "unsubstituted phenyl group", or "phenyl group independently substituted with 1, 2, 3 or 4 of the following functional groups: a halogen or a cyano group";
and/or, $-L^1-R^{5f}$ in the definition of $R^{5b}$ is $-C(R^aR^b)-R^{5f}$;
and/or, the $C_{1-4}$ haloalkyl group in the definitions of $R^{5g}$ and $R^{5h}$ is $-CF_3$;
and/or, each $R^a$ is independently H, fluorine, a methyl group or an ethyl group; each $R^b$ is independently H, fluorine, a methyl group or an ethyl group, or, $R^a$ and $R^b$ along with the carbon atom connected to them, form a cyclopropyl group;
and/or, when $R^2$ is

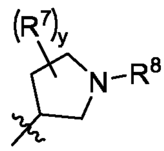

,

ring C is a benzene ring, imidazole, furan or pyridine;
and/or, when $R^2$ is

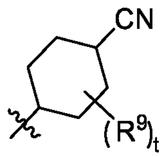,

ring C is a benzene ring, thiazole, furan or thiophene;
and/or, $R^8$ is $R^5$, $-S(O)_2R^{8a}$ or $-C(O)R^{8b}$;
and/or, each $R^6$ is independently fluorine, a hydroxyl group, an amino group, a cyano group, a methyl group, $CF_3$, -O-CHs, $-S-CH_3$, $-S(O)_2-CH_3$ or $-CH_2-OH$;
and/or, $R^{12}$ is a cyano group, $-C(R^eR^f)-R^{12f}$, $-[C(R^eR^f)_2]-R^{12f}$, $-[C(R^eR^f)_3]-R^{12f}$ or

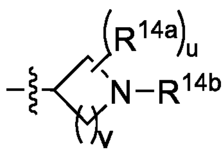;

and/or, $R^{14a}$ is a cyano-substituted $C_{1-4}$ alkyl group;
and/or, v is 0;
and/or, u is 1.

**4.** The compound as claimed in at least one of claims 1-3, or a tautomer, stereoisomer, racemate or isotopic derivative thereof, or a pharmaceutically acceptable salt of any of the foregoing, or a crystalline or solvated form of any of the foregoing, which is **characterized by**: each $R^{5g}$ is independently a halogen, a $C_{1-4}$ alkyl group, a $-O-C_{1-4}$ haloalkyl group or a phenyl group;

and/or, "unsubstituted or substituted 6-10-membered aryl group" in the definition of $R^{5a}$ is

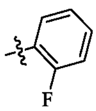, 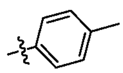, 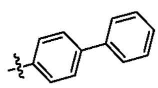, 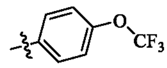 or 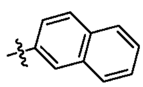;

and/or, "unsubstituted or substituted 5-10-membered heteroaryl group" in the definition of $R^{5a}$ is

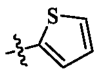;

and/or, "unsubstituted or substituted 6-10-membered aryl group" in the definition of $R^{5b}$ is

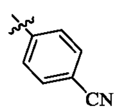 or 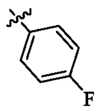;

and/or, $-L^1-R^{5f}$ in the definition of $R^{5b}$ is

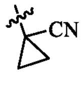;

and/or, the $C_{1-6}$ alkyl group in the definition of $R^{5e}$ is a tert-butyl group;
and/or, $-L^1-R^{5f}$ in the definition of $R^5$ is $-[C(R^aR^b)]_{1-5}-R^{5f}$ or $-[C(R^aR^b)]_{1-2}-C(O)NH-[C(R^aR^b)]_{1-2}-$, preferably $-C(R^aR^b)-R^{5f}$, $-[C(R^aR^b)]_2-R^{5f}$, $-[C(R^aR^b)]_3-R^{5f}$, $-[C(R^aR^b)]_4-R^{5f}$ or $-[C(R^aR^b)]_5-R^{5f}$;

and/or, $R^8$ is $-S(O)_2C(R^aR^b)-C_{1-6}$ alkyl, $-S(O)_2-CF_3$, $-S(O)_2C(R^aR^b)-R^{5f}$, $-S(O)_2[C(R^aR^b)_2]-R^{5f}$, $-S(O)_2[C(R^aR^b)_3]-R^{5f}$, $-C(R^aR^b)-R^{5f}$, $-[C(R^aR^b)_2]-R^{5f}$, $-[C(R^aR^b)_3]-R^{5f}$,

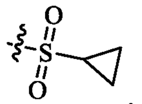, 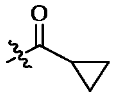,

$C(O)CH=CH_2$ or $-C(O)NHC(R^aR^b)-R^{5f}$;
and/or,

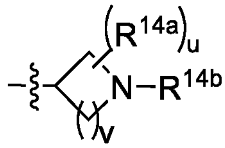

is

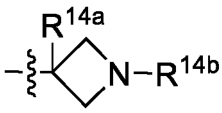;

and/or, $R^{14a}$ is $-CH_2CN$;
and/or, $R^{14b}$ is a $-S(O)_2-C_{1-4}$ alkyl group;

**5.** The compound as claimed in any one of claims 1-4, or a tautomer, stereoisomer, racemate or isotopic derivative thereof, or a pharmaceutically acceptable salt of any of the foregoing, or a crystalline or solvated form of any of the foregoing, which is **characterized by**: $R^{5a}$ is a $C_{2-6}$ alkyl group, an unsubstituted or substituted 6-10-membered aryl group or an unsubstituted or substituted 5-10-membered heteroaryl group;

and/or, $R^{5b}$ is $-L^1-R^{5f}$, or an unsubstituted or substituted phenyl group;
and/or, $R^{5e}$ is a $C_{1-6}$ alkyl group;
and/or, $R^{5c}$, $R^{5d}$ and $R^{5k}$ are each independently $-L^1-R^{5f}$,
and/or, each $R^{5f}$ is independently F, $CF_3$ or CN;
and/or, $R^8$ is $-S(O)_2(CH_2)_2-CH_3$, $-S(O)_2-CF_3$, $-C(O)NHCH_2-CF_3$, $-C(O)CH=CH_2$,

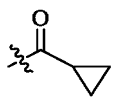,

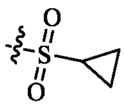,

$-CH_2-CN$, $-(CH_2)_2-CN$ or $-(CH_2)_3-CF_3$;
and/or,

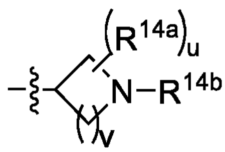

is

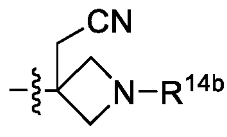
;

and/or, $R^{14b}$ is a $-S(O)_2-C_{1-4}$ alkyl group;
and/or,

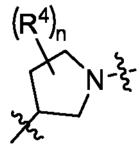

is

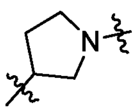, 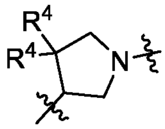, 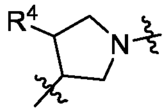 or 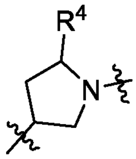;

and/or, ring C is a benzene ring,

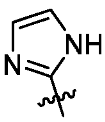, 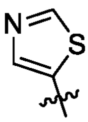, 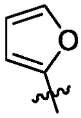, 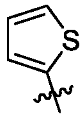 or 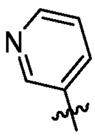.

**6.** The compound as claimed in at least one of claims 1-5, or a tautomer, stereoisomer, racemate or isotopic derivative thereof, or a pharmaceutically acceptable salt of any of the foregoing, or a crystalline or solvated form of any of the foregoing, which is **characterized by**: $-C(O)NHR^{5k}$ in the definition of $R^5$ is $-C(O)NHCH_2-CF_3$;

and/or, $-S(O)_2R^{5a}$ in the definition of $R^5$ is $-S(O)_2(CH_2)_2-CF_3$, $-S(O)_2(CH_2)_2-CH_3$, $-S(O)_2CH_2-CH_3$, $-S(O)_2(CH_2)_3-CH_3$, $-S(O)_2(CH_2)_2-CF_3$,

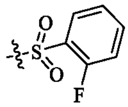, 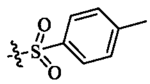, 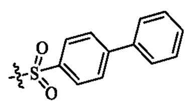,

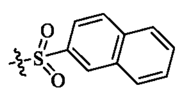, 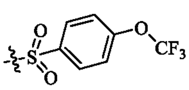 or 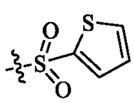,

preferably $-S(O)_2(CH_2)_2-CF_3$, $-S(O)_2(CH_2)_2-CH_3$, $-S(O)_2CH_2-CH_3$, $-S(O)_2(CH_2)_3-CH_3$, $-S(O)_2(CH_2)_2-CF_3$,

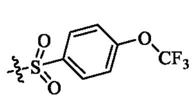 or 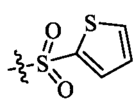;

and/or, $-C(O)R^{5b}$ in the definition of $R^5$ is

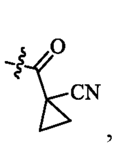, 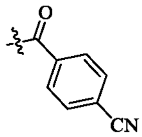 or 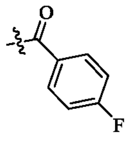;

and/or, $-C(O)OR^{5e}$ in the definition of $R^5$ is $-C(O)OC(CH_3)_2-CH_3$;

and/or, $-L^1-R^{5f}$ in the definition of $R^5$ is $-C(R^aR^b)-CN$, $-[C(R^aR^b)]_2-CN$, $-[C(R^aR^b)]_3-CN$, $-[C(R^aR^b)]_4-CN$, $-[C(R^aR^b)]-CF_3$, $-[C(R^aR^b)]_2-CF_3$, $-[C(R^aR^b)]_3-CF_3$, $-C(R^aR^b)C(O)NH-C(R^aR^b)CF_3$ or $-[C(R^aR)]_5-F$, preferably $-C(R^aR^b)-CN$, $-[C(R^aR^b)]_2-CN$, $-[C(R^aR^b)]_3-CN$, $-[C(R^aR^b)]_4-CN$, $-[C(R^aR^b)]-CF_3$, $-[C(R^aR^b)]_2-CF_3$, $-[C(R^aR^b)]_3-CF_3$, or $-[C(R^aR^b)]_5-F$.

**7.** The compound as claimed in any one of claims 1-6, or a tautomer, stereoisomer, racemate or isotopic derivative thereof, or a pharmaceutically acceptable salt of any of the foregoing, or a crystalline or solvated form of any of the foregoing, which is **characterized by**: $R^5$ is $-CH_2-CN$, $-(CH_2)_2-CN$, $-(CH_2)_3-CN$, $-(CH_2)_4-CN$, $-(CH_2)_2-CF_3$, $-CH_2(CF_2)_2-CF_3$, $-(CF_2)_3-CF_3$, $-(CH_2)_2CF_2-CF_3$, $-(CH_2)_3-CF_3$, $-CH(CH_2CH_3)C(O)NH-CH_2CF_3$, $-CH_2C(O)NHCH_2-CF_3$, $-CH(CH_3)C(O)NHCH_2-CF_3$, $-(CH_2)_5-F$,

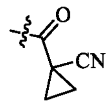,

$-S(O)_2(CH_2)_2-CF_3$, $-C(O)NHCH_2-CF_3$, $-S(O)_2(CH_2)_2-CH_3$, $-S(O)_2CH_2-CH_3$, $-S(O)_2(CH_2)_3-CH_3$, $-S(O)_2(CH_2)_2-CF_3$,

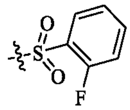,

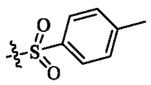, 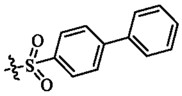, 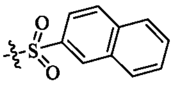, 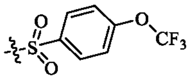, 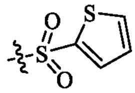,

$-C(O)OC(CH_3)_2-CH_3$,

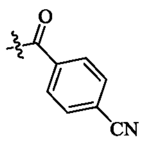 or 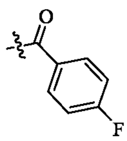;

preferably $-CH_2-CN$, $-(CH_2)_2-CN$, $-(CH_2)_3-CN$, $-(CH_2)_4-CN$, $-(CH_2)_2-CF_3$, $-CH_2(CF_2)_2-CF_3$, $-(CF_2)_3-CF_3$, $-(CH_2)_2CF_2-CF_3$, $-(CH_2)_3-CF_3$, $-CH(CH_2CH_3)C(O)NH-CH_2CF_3$, $-CH_2C(O)NHCH_2-CF_3$, $-CH(CH_3)C(O)NHCH_2-CF_3$, $-(CH_2)_5-F$, $-S(O)_2(CH_2)_2-CF_3$, $-S(O)_2CH_2-CH_3$, $-S(O)_2(CH_2)_3-CH_3$, $-S(O)_2(CH_2)_2-CF_3$,

 or ;

and/or,

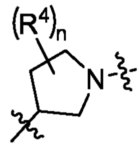

is

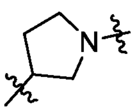, 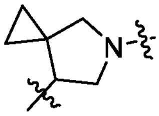, 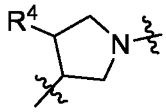 or 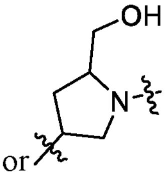;

and/or,

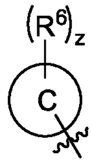

is

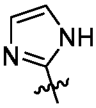, 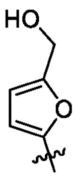, 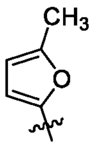, 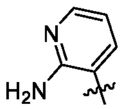, 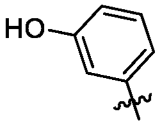, 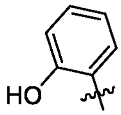, 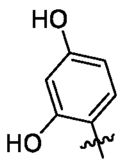,

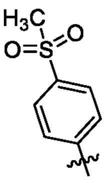, 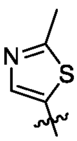, 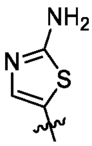, 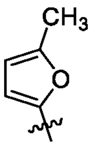, 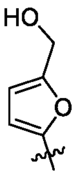, 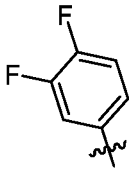, 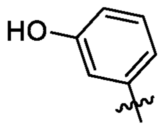, 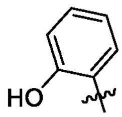, 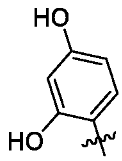,

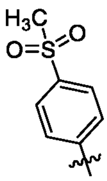, 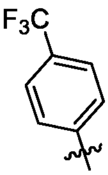, 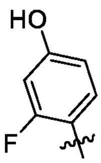, 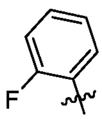, 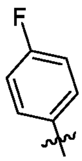, 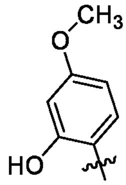, 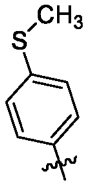 or 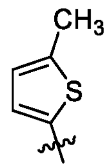;

and/or, $R^8$ is $-S(O)_2C(R^aR^b)-CH_3$, $-S(O)_2-CF_3$, $-C(O)NHC(R^aR^b)-CF_3$, $-C(O)CH=CH_2$,

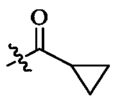, 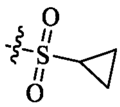,

$-C(R^aR^b)-CN$, $-[C(R^aR^b)_2]-CN$ or $-[C(R^aR^b)_3]-CF_3$;
and/or,

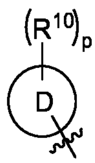

is

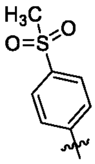, 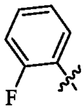, 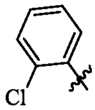, 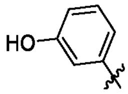, 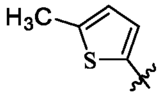, 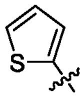, 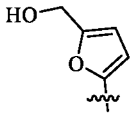,

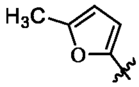, 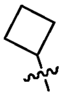, 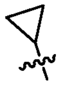 or 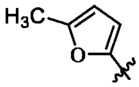;

and/or, $R^{12}$ is $-CN$, $-CH_2-CN$, $-(CH_2)_2-CN$, $-(CH_2)_3-CN$,

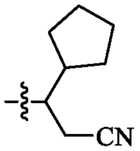, 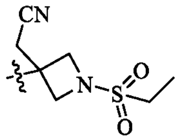,

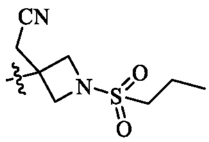 or 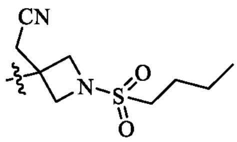,

preferably $-CH_2-CN$, $-(CH_2)_2-CN$, $-(CH_2)_3-CN$;
and/or,

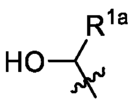

is

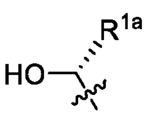 or 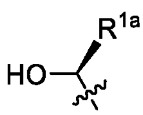;

and/or,

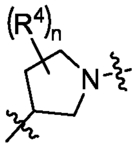

is

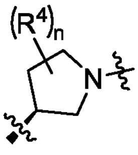 or 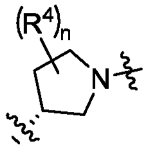 ;

and/or,

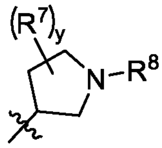

is

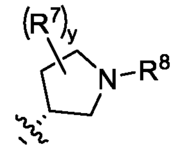 or 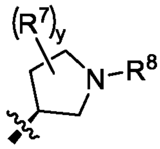 .

**8.** The compound as claimed in claim 1, or a tautomer, stereoisomer, racemate or isotopic derivative thereof, or a pharmaceutically acceptable salt of any of the foregoing, or a crystalline or solvated form of any of the foregoing, which is **characterized by**: said compound has the following formulae **I-1a, I-1b, I-1c, I-1d, I-2, I-3C, I-3D, I-3E, I-3F, I-3G, I-4E, I-4F, I-4J, I-4L, I-4M, I-5, I-6, I-7A, I-7B, I-7C, I-7D, I-7E, I-7F, I-7G** or the structure shown in **I-7H;**

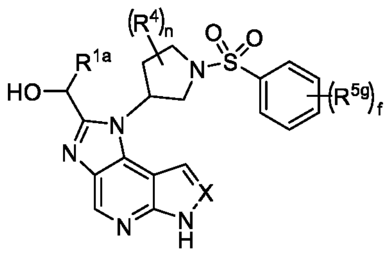

**I-1a**

,

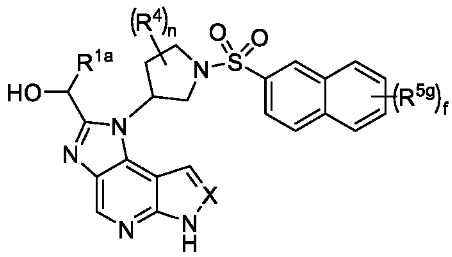

**I-1b**

,

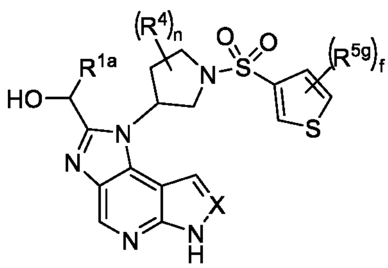

**I-1c**

,

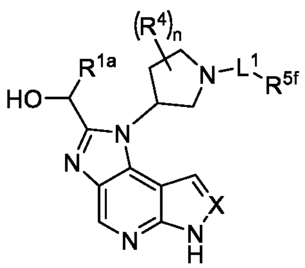

**I-1d**

,

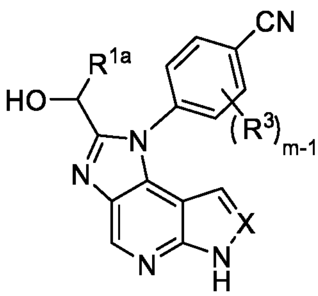

**I-2**

,

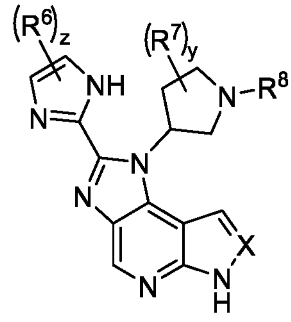

**I-3C** ,

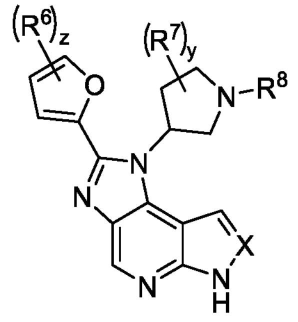

**I-3D** ,

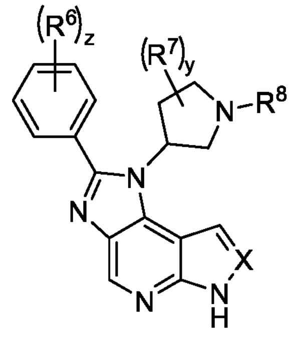

**I-3E** ,

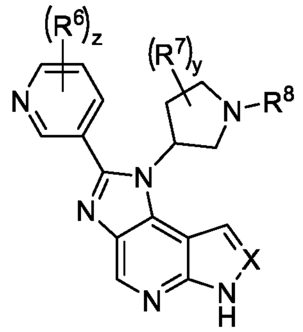

**I-3F** ,

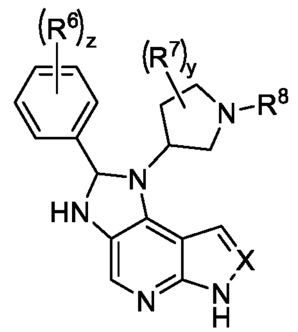

**I-3G** ,

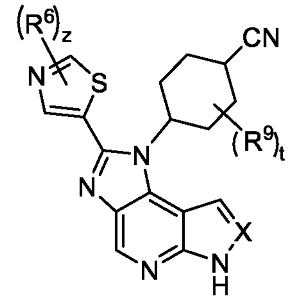

**I-4E** ,

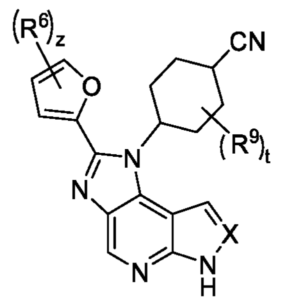

**I-4F** ,

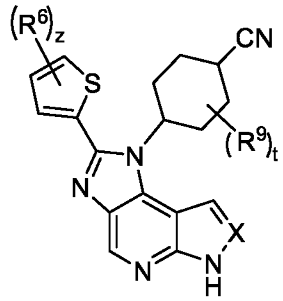

**I-4J** ,

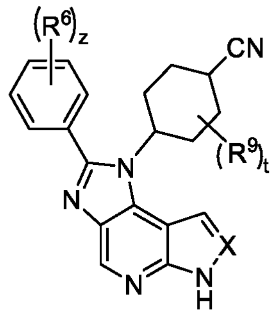

**I-4L** ,

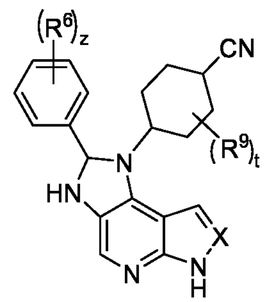

**I-4M** ,

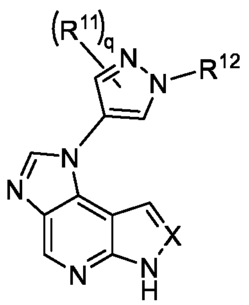

**I-5** ,

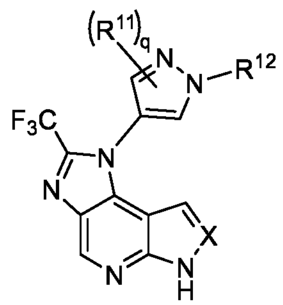

**I-6** ,

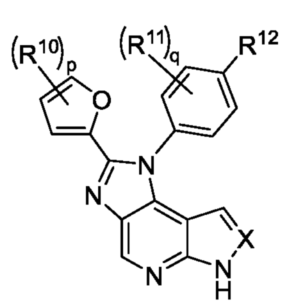

**I-7A** ,

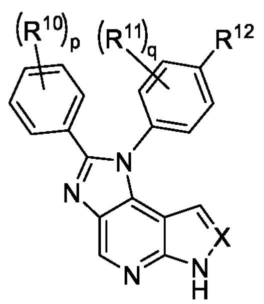

**I-7B** ,

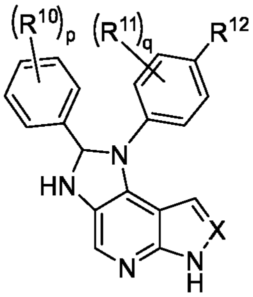

**I-7C** ,

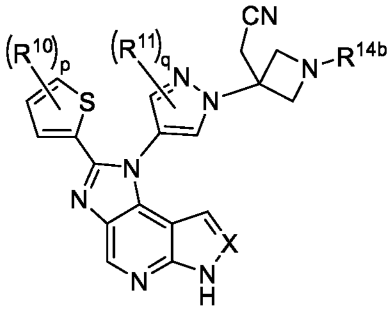

**I-7G** ,

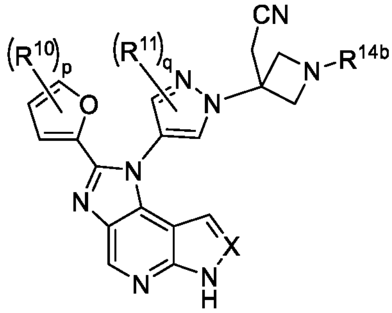

**I-7H** ;

wherein X, $R^{1a}$, $R^4$, $R^{5g}$, n, $L^1$, $R^{5f}$, $R^6$, $R^7$, $R^8$, y, $R^9$, z, t, $R^{10}$, $R^{11}$, $R^{12}$, $R^{14b}$, p, and q are defined as described in at least one of claims 1-7;
each f is independently 0, 1, 2, 3 or 4.

**9.** The compound as claimed in claim 1, or a tautomer, stereoisomer, racemate or isotopic derivative thereof, or a pharmaceutically acceptable salt of any of the foregoing, or a crystalline or solvated form of any of the foregoing, **characterized by** that said compound has any of the following structures:

| Chemical name | Structure |
|---|---|
| Tert-butyl 3-(2-((R)-1-hydroxyethyl)imidazo[4,5-d]pyrrolo[2,3-b]pyridin-1 (6H)-yl)pyrrolidine-1-carboxylate | 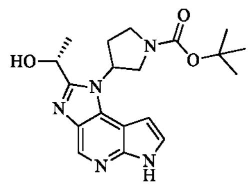 |
| 4-(3 -(2-((R)-1-Hydroxy ethyl)imidazo[4,5- d]pyrrolo[2,3-b]pyridin-1(6H)-yl) pyrrolidin-1-carbonyl)benzonitrile | 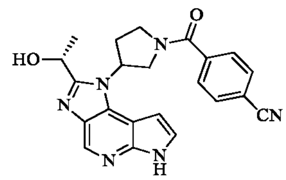 |
| (4-Fluorophenyl)(3-(2-((R)-1-Hydroxy ethyl)imidazo[4,5-d]pyrrolo[2,3-b] pyridin-1(6H)-yl)pyrrolidin-1- yl)methanone | 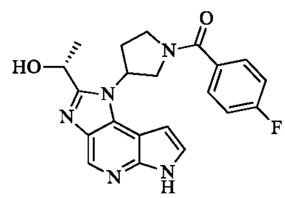 |
| (1R)-1-(1-(1-(ethylsulfonyl)pyrrolidin-3-yl)-1,6-dihydroimidazo[4,5-d] pyrrolo[2,3-b]pyridin-2-yl)ethan-1-ol | 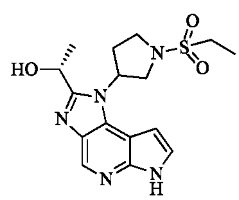 |
| (1R)-1-(1-(1-(propylsulfonyl)pyrrolidin-3-yl)-1,6-dihydroimidazo[4,5-d] pyrrolo[2,3-b]pyridin-2-yl)ethan-1-ol | 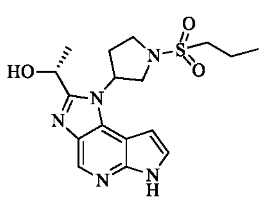 |

(continued)

| Chemical name | Structure |
|---|---|
| (1R)-1-(1-(1-(butylsulfonyl)pyrrolidin-3-yl)-1,6-dihydroimidazo[4,5-d] pyrrolo[2,3-b]pyridin-2-yl)ethan-1-ol | 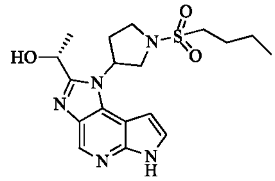 |
| (1R)-1-(1-(1-((2-fluorophenyl)sulfonyl)pyrrolidin-3-yl)-1,6-dihydroimidazo [4,5-d]pyrrolo[2,3-b]pyridin-2-yl)ethan-1-ol | 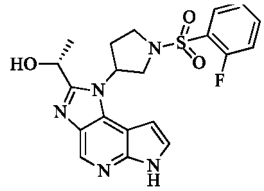 |
| (1R)-1-(1-(1-tosylpyrrolidin-3-yl)-1,6-dihydroimidazo[4,5-d]pyrrolo[2,3-b] pyridin-2-yl)ethan-1-ol | 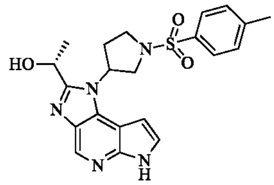 |
| (1R)-1-(1-(1-([1,1'-biphenyl]-4-ylsulfonyl)pyrrolidin-3-yl)-1,6-dihydroimidazo[4,5-d]pyrrolo[2,3-b]pyridin-2-yl)ethan-1-ol | 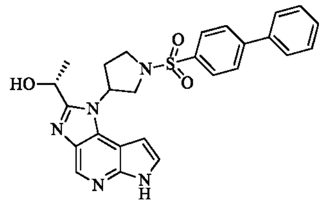 |
| (1R)-1-(1-(1-(naphthalen-2-ylsulfonyl)pyrrolidin-3-yl)-1,6-dihydroimidazo [4,5-d]pyrrolo[2,3-b]pyridin-2-yl)ethan-1-ol | 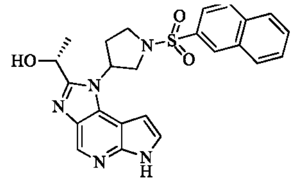 |
| (1R)-1-(1-(1-((4-(trifluoromethoxy)phenyl)sulfonyl)pyrrolid in-3-yl)-1,6-dihydroimidazo[4,5-d]pyrrolo[2,3-b]pyridin-2-yl)ethan-1-ol | 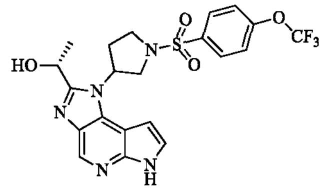 |
| (1R)-1-(1-(1-(thiophen-2-ylsulfonyl)pyrrolidin-3-yl)-1,6-dihydroimidazo [4,5-d]pyrrolo[2,3-b]pyridin-2-yl)ethan-1-ol | 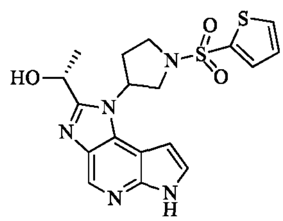 |

(continued)

| Chemical name | Structure |
|---|---|
| 2-(1-(Ethylsulfonyl)-3-(4-(2-(5-(hydroxymethyl)furan-2-yl)imidazo[4, 5-d] pyrrolo[2,3-b]pyridin-1(6H)-yl)-1H-pyrazol-1-yl)azetidin-3-yl)acetonitrile | 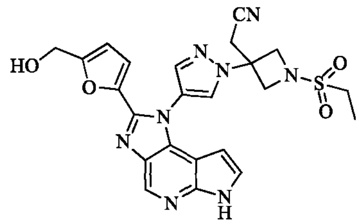 |
| 2-(1-(Propylsulfonyl)-3-(4-(2-(5-(hydroxymethyl)furan-2-yl)imidazo[4,5-d] pyrrolo[2,3-b]pyridin-1(6H)-yl)-1H-pyrazol-1-yl)azetidin-3-yl)acetonitrile | 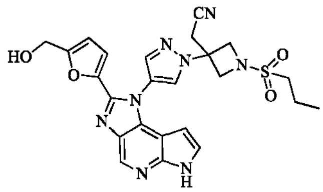 |
| 2-(1-(Butylsulfonyl)-3-(4-(2-(5-(hydroxymethyl)furan-2-yl)imidazo[4,5-d] pyrrolo[2,3-b]pyridin-1(6H)-yl)-1H-pyrazol-1-yl)azetidin-3-yl)acetonitrile | 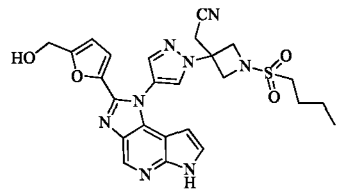 |
| (5-(1-(1-(thiophen-2-ylsulfonyl)pyrrolidin-3-yl)-1,6-dihydroimidazo[4,5-d] pyrrolo[2,3-b]pyridin-2-yl)furan-2-yl)methanol | 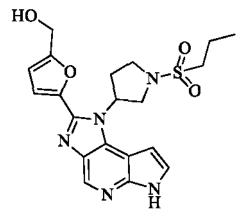 |
| Cyclopropyl(3-(2-(5-(hydroxymethyl)furan-2-yl)imidazo[4,5-d]pyrrolo[2,3-b]pyridin-1(6H)-yl)pyrrolidin-1-yl) methanone | 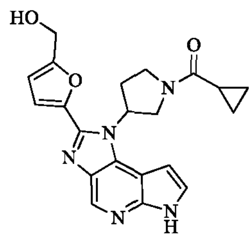 |
| 2-(1H-imidazol-2-yl)-1-(1-(propylsulfonyl)pyrrolidin-3-yl)-1,6-dihydroimidazo[4,5-d]pyrrolo[2,3-b]pyridine | 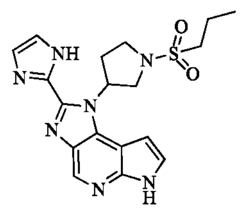 |
| 2-(1H-imidazol-2-yl)-1-(1-((trifluoromethyl)sulfonyl)pyrrolidin-3-yl)-1,6-dihydroimidazo[4,5-d]pyrrolo[2,3-b]pyridine | 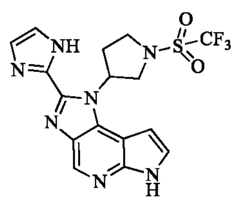 |

(continued)

| Chemical name | Structure |
|---|---|
| (R)-3-(2-((R)-1-Hydroxyethyl)imidazo[4,5-d]pyrrolo[2,3-b]pyridin-1 (6H)-yl)-N-(2,2,2-trifluoroethyl)pyrrolidine-1-carboxamide | 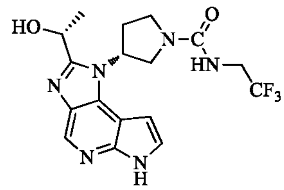 |
| (R)-1-(1-((R)-1-(Propylsulfonyl)pyrrolidin-3-yl)-1,6-dihydroimidazo[4,5-d] pyrrolo[2,3-b]pyridin-2-yl)ethanol | 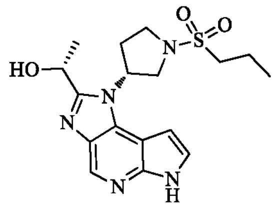 |
| trans-4-(2-(5-(Hydroxymethyl)furan-2-yl)imidazo[4,5-d]pyrrolo[2,3-b] pyridin-1(6H)-yl)cyclohexanecarbonitrile | 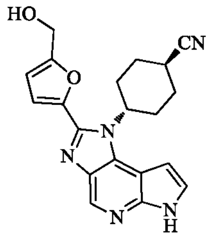 |
| (R)-3-(2-(5-(Hydroxymethyl)furan-2-yl)imidazo[4,5-d]pyrrolo[2,3-b] pyridin-1(6H)-yl)-N-(2,2,2-trifluoroethyl)pyrrolidine-1-carboxamide | 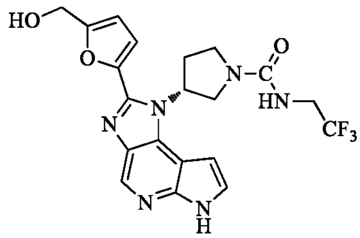 |
| (R)-3-(2-(1H-imidazol-2-yl)imidazo[4,5-d]pyrrolo[2,3-b]pyridin-1 (6H)-yl)-N-(2,2,2-trifluoroethyl)pyrrolidine-1-carboxamide | 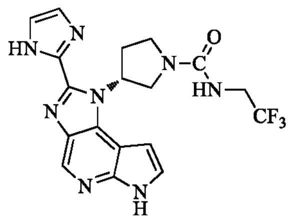 |
| (R)-1-(3-(2-(1H-imidazol-2-yl)imidazo[4, 5-d]pyrrolo[2,3 -b]pyridin-1 (6H)-yl)pyrrolidin-1-yl)prop-2-en-1-one | 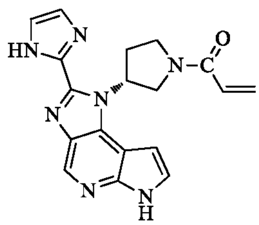 |
| trans-4-(2-(2-Methylthiazol-5-yl)imidazo[4,5-d]pyrrolo[2,3-b]pyridin-1 (6H)-yl)cyclohexanecarbonitrile | 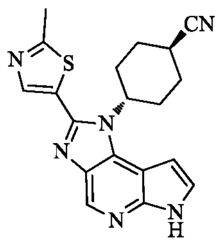 |

(continued)

| Chemical name | Structure |
|---|---|
| trans-4-(2-(2-Aminothiazol-5-yl)imidazo[4,5-d]pyrrolo[2,3-b]pyridin-1 (6H)-yl)cyclohexanecarbonitrile | 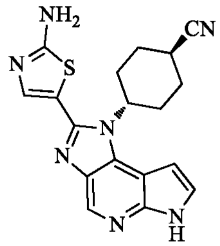 |
| trans-4-(2-(2-Methylthiophen-5-yl)imidazo[4,5-d]pyrrolo[2,3-b]pyridin-1 (6H)-yl)cyclohexanecarbonitrile | 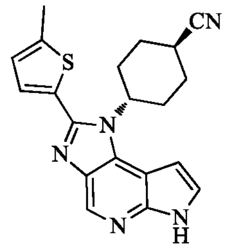 |
| trans-4-(2-(2-Methylfuran-5-yl)imidazo[4,5-d]pyrrolo[2,3-b]pyridin-1 (6H)-yl)cyclohexanecarbonitrile | 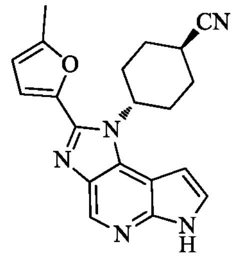 |
| 2-(1-(1-(propylsulfonyl)pyrrolidin-3-yl)-1,6-dihydroimidazo[4,5-d]pyrrolo [2,3-b]pyridin-2-yl)phenol | 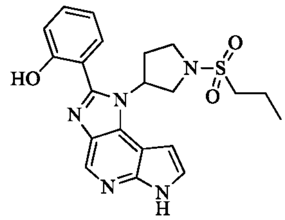 |
| 4-(1-(1-(Propylsulfonyl)pyrrolidin-3-yl)-1, 6-dihydroimidazo[4,5-d]pyrrolo [2,3-b]pyridin-2-yl)benzene-1,3-diol | 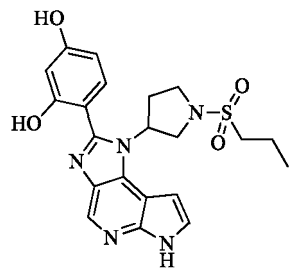 |
| 3-(1-(1-(propylsulfonyl)pyrrolidin-3-yl)-1,6-dihydroimidazo[4,5-d]pyrrolo [2,3-b]pyridin-2-yl)phenol | 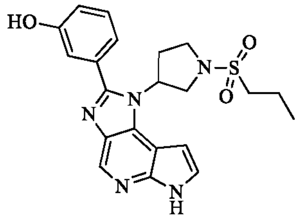 |

(continued)

| Chemical name | Structure |
|---|---|
| 1-(3-(2-(2,4-Dihydroxyphenyl)imidazo[4,5-d]pyrrolo[2,3-b]pyridin-1 (6H)-yl)pyrrolidin-1-yl)prop-2-en-1-one | 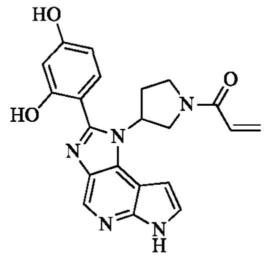 |
| 3-(2-(3-Hydroxyphenyl)imidazo[4,5-d]pyrrolo[2,3-b]pyridin-1 (6H)-yl)-N-(2,2,2-trifluoroethyl)pyrrolidine-1-carboxamide | 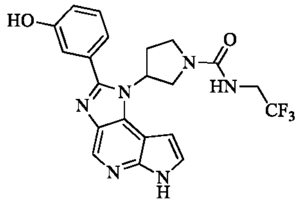 |
| trans-4-(2-(3-Hydroxyphenyl)imidazo[4,5-d]pyrrolo[2,3-b]pyridin-1(6H)-yl) cyclohexanecarbonitrile | 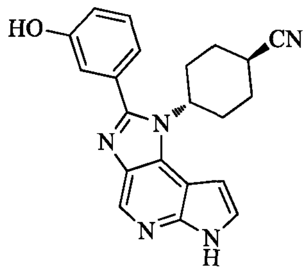 |
| trans-4-(2-(2,4-Dihydroxyphenyl)imidazo[4,5-d]pyrrolo[2,3-b]pyridin-1 (6H)-yl)cyclohexanecarbonitrile | 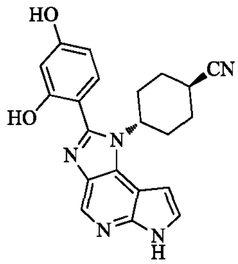 |
| trans-4-(2-(4-(Trifluoromethyl)phenyl)imidazo[4,5-d]pyrrolo[2,3-b]pyridin-1(6H)-yl)cyclohexanecarbonitrile | 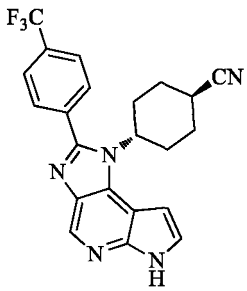 |
| trans-4-(2-(2-Hydroxyphenyl)imidazo[4,5-d]pyrrolo[2,3-b]pyridin-1(6H)-yl) cyclohexanecarbonitrile | 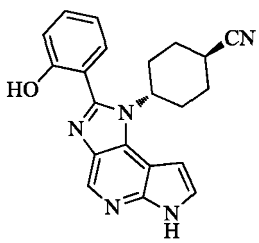 |

(continued)

| Chemical name | Structure |
|---|---|
| trans-4-(2-(2-Fluoro-4-hydroxyphenyl)imidazo[4,5-d]pyrrolo[2,3-b]pyridin-1(6H)-yl)cyclohexanecarbonitrile | 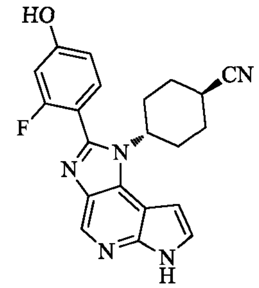 |
| trans-4-(2-(2-Fluorophenyl)imidazo[4,5-d]pyrrolo[2,3-b]pyridin-1(6H)-yl) cyclohexanecarbonitrile | 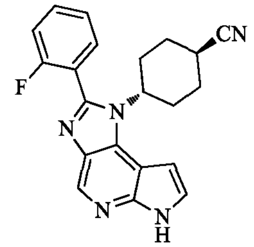 |
| trans-4-(2-(2-Hydroxy-4-methoxyphenyl)imidazo[4,5-d]pyrrolo[2,3-b] pyridin-1(6H)-yl)cyclohexanecarbonitrile | 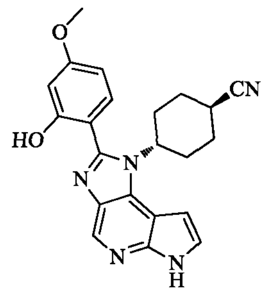 |
| trans-4-(2-(4-(Methylthio)phenyl)imidazo[4,5-d]pyrrolo[2,3-b]pyridin-1 (6H)-yl)cyclohexanecarbonitrile | 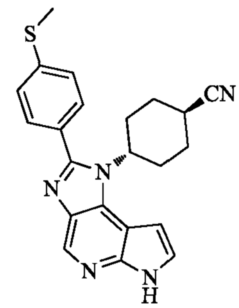 |
| trans-4-(2-(4-Fluorophenyl)imidazo[4,5-d]pyrrolo[2,3-b]pyridin-1(6H)-yl) cyclohexanecarbonitrile | 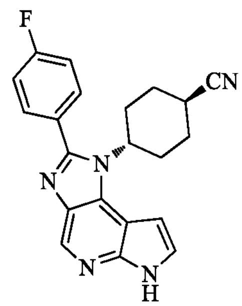 |

(continued)

| Chemical name | Structure |
|---|---|
| trans-4-(2-(4-(Methylsulfonyl)phenyl)-2,3-dihydroimidazo[4,5-d]pyrrolo[2,3-b]pyridin-1(6H)-yl)cyclohexanecarbonitrile | 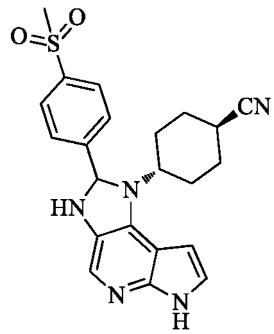 |
| (5-(1-(1-(cyclopropylsulfonyl)pyrrolidin-3-yl)-1,6-dihydroimidazo[4,5-d]pyrrolo[2,3-b]pyridin-2-yl)furan-2-yl)methanol | 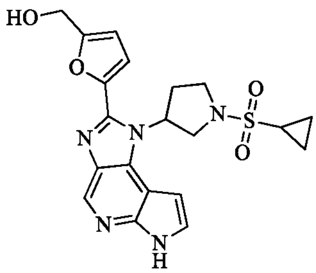 |
| 3-(4-(2-(5-(Hydroxymethyl)furan-2-yl)imidazo[4,5-d]pyrrolo[2,3-b]pyridin-1(6H)-yl)-1H-pyrazol-1-yl)propanenitrile | 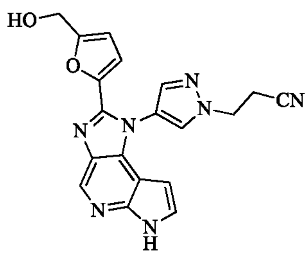 |
| 3-(1-(1-(propylsulfonyl)pyrrolidin-3-yl)-1,6-dihydroimidazo[4,5-d]pyrrolo[2,3-b]pyridin-2-yl)pyridin-2-amine | 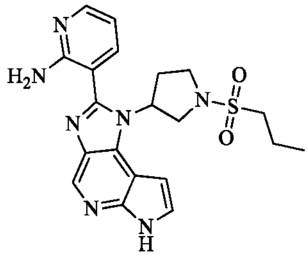 |
| 2-(3-(2-((R)-1-Hydroxyethyl)imidazo[4,5-d]pyrrolo[2,3-b]pyridin-1(6H)-yl)pyrrolidin-1-yl)acetonitrile | 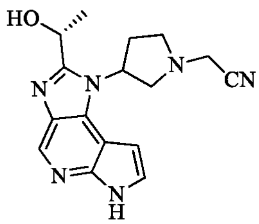 |
| 3-(3-(2-((R)-1-Hydroxyethyl)imidazo[4,5-d]pyrrolo[2,3-b]pyridin-1(6H)-yl)pyrrolidin-1-yl)propanenitrile | 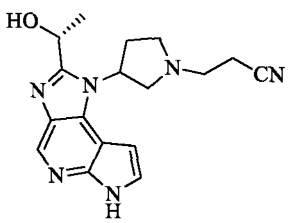 |

(continued)

| Chemical name | Structure |
|---|---|
| 3-(4-(2-(5-methylfuran-2-yl)imidazo[4,5-d]pyrrolo[2,3-b]pyridin-1(6H)-yl)-1H-pyrazol-1-yl)propanenitrile | 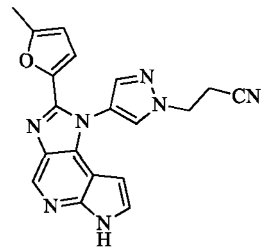 |
| 3-(3-(2-(5-(Hydroxymethyl)furan-2-yl)imidazo[4,5-d]pyrrolo[2,3-b]pyridin-1(6H)-yl)pyrrolidin-din-1-yl)propanenitrile | 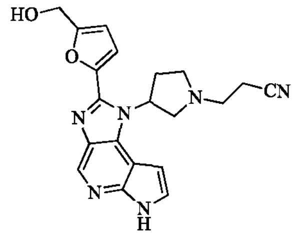 |
| 2-(3-(2-(5-(hydroxymethyl)furan-2-yl)imidazo[4,5-d]pyrrolo[2,3-b]pyridin-1 (6H)-yl)pyrrolidin-1-yl)acetonitrile | 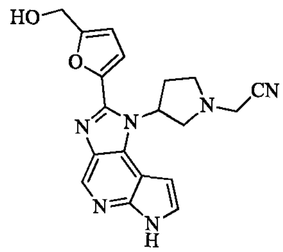 |
| 2-(3-(2-(3-Hydroxyphenyl)imidazo[4,5-d]pyrrolo[2,3-b]pyridin-1(6H)-yl) pyrrolidin-1-yl)acetonitrile | 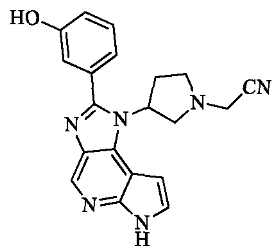 |
| (R)-4-(2-(1-Hydroxyethyl)imidazo[4,5-d]pyrrolo[2,3-b]pyridin-1(6H)-yl) benzonitrile | 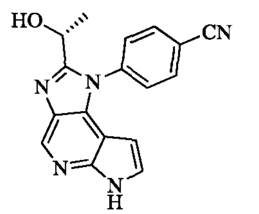 |
| 4-(2-(5-(Hydroxymethyl)furan-2-yl)imidazo[4,5-d]pyrrolo[2,3-b]pyridin-1 (6H)-yl)benzonitrile | 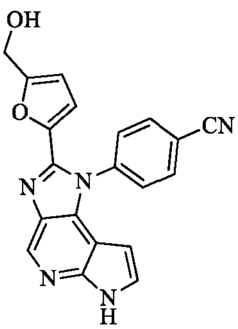 |

(continued)

| Chemical name | Structure |
|---|---|
| 4-(2-(3-Hydroxyphenyl)imidazo[4,5-d]pyrrolo[2,3-b]pyridin-1(6H)-yl) benzonitrile | 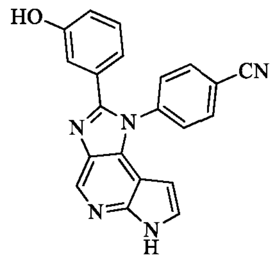 |
| 4-(2-(4-(Methylsulfonyl)phenyl)-2,3-dihydroimidazo[4,5-d]pyrrolo[2,3-b] pyridin-1(6H)-yl)benzonitrile | 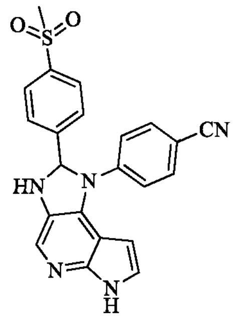 |
| 3-((R)-3-(2-((R)-1-Hydroxyethyl)imidazo[4,5-d]pyrrolo[2,3-b]pyridin-1 (6H)-yl)pyrrolidin-1-yl)propanenitrile | 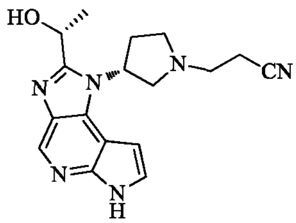 |
| 3-((S)-3-(2-((R)-1-hydroxyethyl)imidazo[4,5-d]pyrrolo[2,3-b]pyridin-1 (6H)-yl)pyrrolidin-1-yl)propanenitrile | 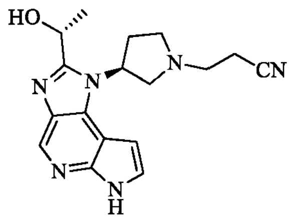 |
| 3-(3-(2-(4-(Methylsulfonyl)phenyl)-2,3- dihydroimidazo[4,5-d]pyrrolo[2,3-b]pyridin-1(6H)-yl)pyrrolidin-1-yl)propanenitrile | 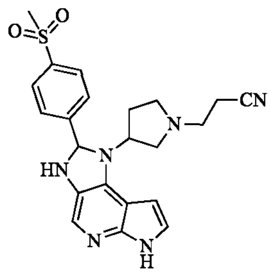 |
| 1-(3-(2-((R)-1-Hydroxyethyl)imidazo[4,5-d]pyrrolo[2,3-b]pyridin-1(6H)-yl) pyrrolidin-1-carbonyl)cyclopropanecarbonitrile | 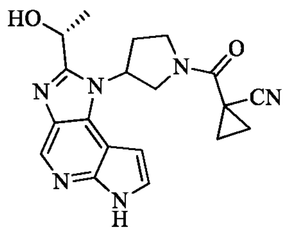 |

(continued)

| Chemical name | Structure |
|---|---|
| 2-(1-(Ethylsulfonyl)-3-(4-(2-(5-methylthien-2-yl)imidazo[4,5-d]pyrrolo[2,3-b]pyridin-1(6H)-yl)-1H-pyrazol-1-yl)azetidin-3-yl)acetonitrile | 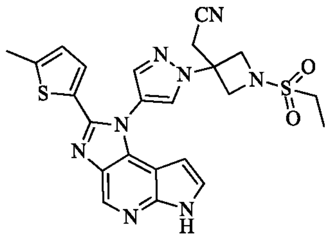 |
| 2-(1-(Ethylsulfonyl)-3-(4-(2-(thiophen-2-yl)imidazo[4,5-d]pyrrolo[2,3-b]pyridin-1(6H)-yl)-1H-pyrazol-1-yl)azetidin-3-yl)acetonitrile | 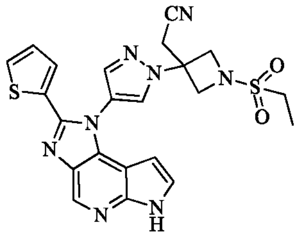 |
| 3-(4-(2-(Trifluoromethyl)imidazo[4,5-d]pyrrolo[2,3-b]pyridin-1(6H)-yl)-1H-pyrazol-1-yl)propanenitrile | 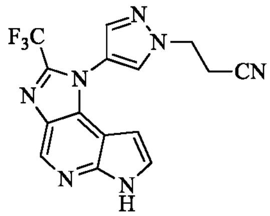 |
| 4-((S)-3-(2-((R)-1-Hydroxyethyl)imidazo[4,5-d]pyrrolo[2,3-b]pyridin-1(6H)-yl)pyrrolidin-1-yl)butanenitrile | 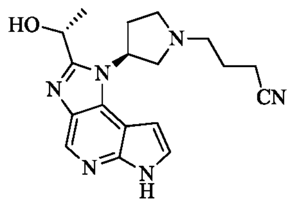 |
| 3-Cyclopentyl-3-(4-(2-(trifluoromethyl)imidazo[4,5-d]pyrrolo[2,3-b]pyridin-1(6H)-yl)-1H-pyrazol-1-yl)propanenitrile | 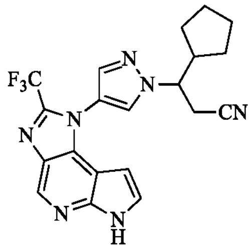 |
| 3-(4-(2-(4-(Methylsulfonyl)phenyl)-2,3- dihydroimidazo[4,5-d]pyrrolo[2,3-b]pyridin-1(6H)-yl)-1H-pyrazol-1-yl)propanenitrile | 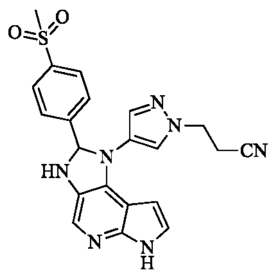 |

(continued)

| Chemical name | Structure |
|---|---|
| 5-((S)-3-(2-((R)-1-Hydroxyethyl)imidazo[4,5-d]pyrrolo[2,3-b]pyridin-1 (6H)-yl)pyrrolidin-1- yl)pentanonitrile | 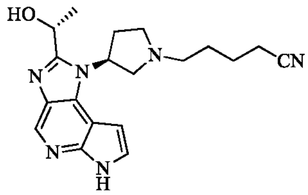 |
| 4-((S)-7-(2-((R)-1-Hydroxyethyl)imidazo[4,5-d]pyrrolo[2,3-b]pyridin-1 (6H)-yl)-5-azaspiro[2.4]hept-5-yl)butanenitrile | 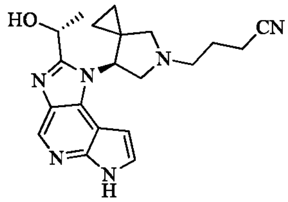 |
| (R)-1-(1-((S)-5-(4,4,4-Trifluorobutyl)-5-azaspiro[2.4]hept-7-yl)-1,6-dihydroimidazo[4,5-d]pyrrolo[2,3-b]pyridin-2-yl)ethanol | 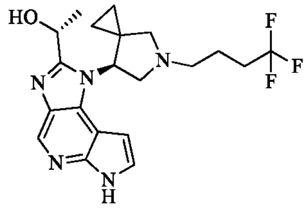 |
| (R)-1-(1-((S)-1-(4,4,4-Trifluorobutyl)pyrrolidin-3-yl)-1,6-dihydroimidazo [4,5-d]pyrrolo[2,3-b]pyridin-2-yl)ethanol | 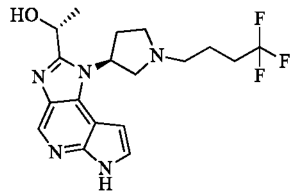 |
| 5-((S)-7-(2-((R)-1-Hydroxyethyl)imidazo[4,5-d]pyrrolo[2,3-b]pyridin-1 (6H)-yl)-5-azaspiro[2.4]hept-5-yl)pentanonitrile | 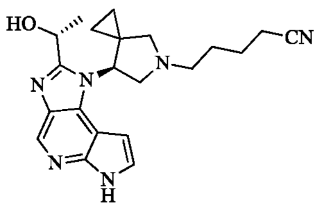 |
| (R)-1-(1-((S)-1-(5-Fluoropentyl)pyrrolidin-3-yl)-1,6-dihydroimidazo[4,5-d] pyrrolo[2,3-b]pyridin-2-yl)ethanol | 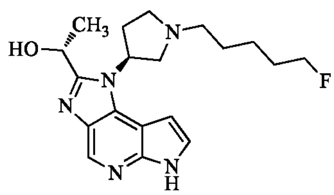 |
| 2-((S)-3-(2-((R)-1-Hydroxyethyl)imidazo[4,5-d]pyrrolo[2,3-b]pyridin-1 (6H)-yl)pyrrolidin-1-yl)-N-(2,2,2-trifluoroethyl)acetamide | 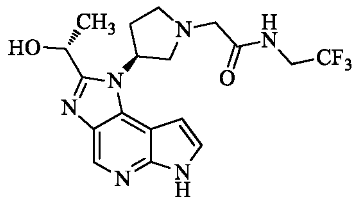 |

(continued)

| Chemical name | Structure |
|---|---|
| 2-((S)-3-(2-((R)-1-Hydroxyethyl)imidazo[4,5-d]pyrrolo[2,3-b]pyridin-1 (6H)-yl)pyrrolidin-1-yl)-N-(2,2,2-trifluoroethyl)propanamide | 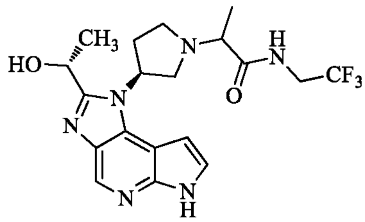 |
| 2-((S)-3-(2-((R)-1-Hydroxyethyl)imidazo[4,5-d]pyrrolo[2,3-b]pyridin-1 (6H)-yl)pyrrolidin-1-yl)-N-(2,2,2-trifluoroethyl)butanamide | 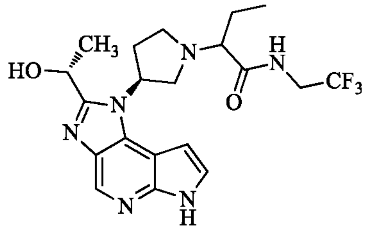 |
| 2-(1-(ethylsulfonyl)-3-(4-(2-(5-methylfuran-2-yl)imidazo[4,5-d]pyrrolo[2,3-b]pyridin-1(6H)-yl)-1H-pyrazol-1-yl)azetidin-3-yl)acetonitrile | 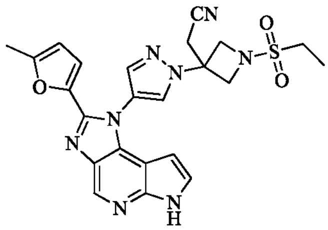 |
| 2-(4-(2-(Trifluoromethyl)imidazo[4,5-d]pyrrolo[2,3-b]pyridin-1(6H)-yl)-1H-pyrazol-1-yl)acetonitrile | 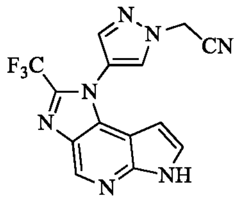 |
| 3-(4-(Imidazo[4,5-d]pyrrolo[2,3-b]pyridin-1(6H)-yl)-1H-pyrazol-1-yl) propanenitrile | 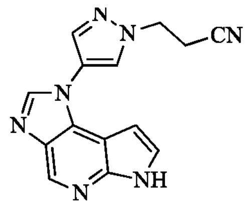 |
| 3-(4-(2-(2-Chlorophenyl)imidazo[4,5-d]pyrrolo[2,3-b]pyridin-1(6H)-yl)-1H-pyrazol-1-yl)propanenitrile | 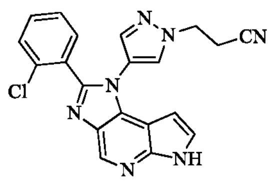 |
| 2-(4-(2-(5-(Hydroxymethyl)furan-2-yl)imidazo[4,5-d]pyrrolo[2,3-b]pyridin-1(6H)-yl)-1H-pyrazol-1-yl)acetonitrile | 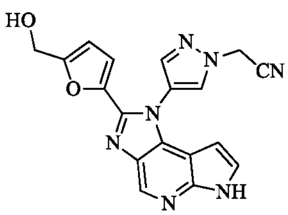 |

(continued)

| Chemical name | Structure |
|---|---|
| 3-(4-(2-(3-Hydroxyphenyl)imidazo[4,5-d]pyrrolo[2,3-b]pyridin-1(6H)-yl)-1H-pyrazol-1-yl)propanenitrile | 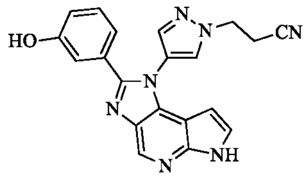 |
| 3-(4-(2-(2-Fluorophenyl)imidazo[4,5-d]pyrrolo[2,3-b]pyridin-1(6H)-yl)-1H-pyrazol-1-yl)propanenitrile | 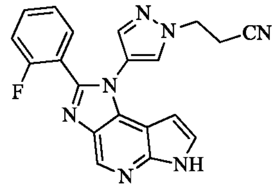 |
| 3-(4-(2-(5-methylthiophen-2-yl)imidazo[4,5-d]pyrrolo[2,3-b]pyridin-1 (6H)-yl)-1H-pyrazol-1-yl)propanenitrile | 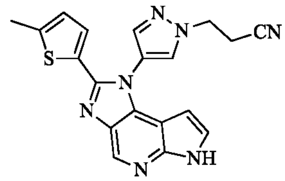 |
| 4-((S)-3-(2-((R)-1-Hydroxyethyl)imidazo[4,5-d]pyrazolo[3,4-b]pyridin-1 (6H)-yl)pyrrolidin-1- yl)butanenitrile | 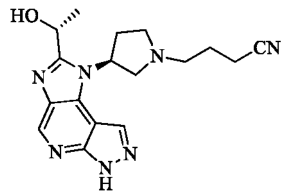 |
| (1R)-1-(1-((3R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol | 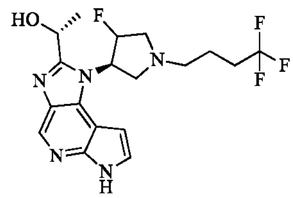 |
| (R)-1-(1-((3R,4R)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol | 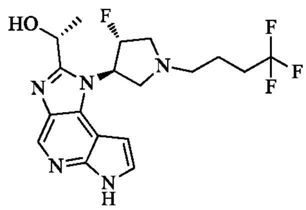 |
| (R)-1-(1-((3R,4S)-4-fluoro-1-(4,4,4-trifluorobutyl)pyrrolidin-3-yl)-1,6-dihydroimidazo[4,5-d]pyrrolo[2,3-b]pyridin-2-yl)ethan-1-ol | 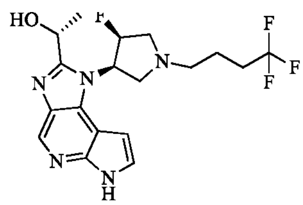 |

(continued)

| Chemical name | Structure |
|---|---|
| (R)-1-(1-((3S,5S)-5-(hydroxymethyl)-1- (4,4,4-trifluorobutyl)pyrrolidin-3-yl)-1,6-dihydroimidazo[4,5-d]pyrrolo[2,3-b]pyridin-2-yl)ethan-1-ol | 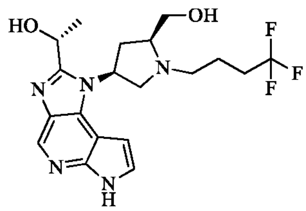 |
| (R)-1-(1-((S)-1-(4,4,4-Trifluorobutyl)pyrrolidin-3-yl)-1,6-dihydroimidazo [4,5-d]pyrazolo[3,4-b]pyridin-2-yl)ethanol | 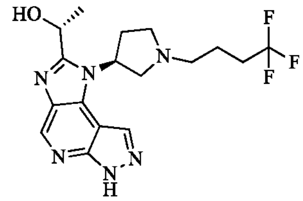 |
| (R)-1-(1-((3S,5R)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol | 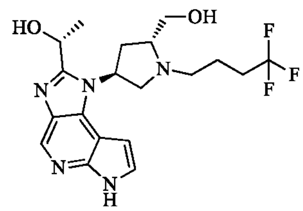 |
| (R)-1-(1-((3S,5R)-5-(hydroxymethyl)-1-(4,4,4-trifluorobutyl)pyrrolidin-3-yl)-1,6-dihydroimidazo[4,5-d]pyrrolo[2,3-b]pyridin-2-yl)ethan-1-ol | 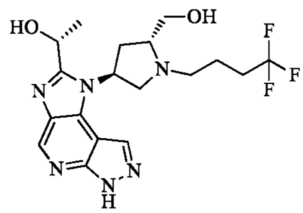 |
| 4-((4R)-3-Fluoro-4-(2-((R)-1-hydroxyethyl)imidazo[4,5-d]pyrazolo[3,4-b] pyridin-1(6H)-yl)pyrrolidin-1-yl)butanenitrile | 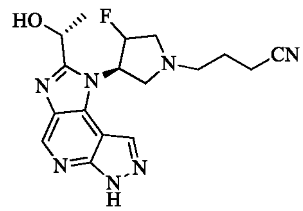 |
| 4-((2S,4S)-4-(2-((R)-1-Hydroxyethyl)imidazo[4,5-d]pyrazolo[3,4-b] pyridin-1(6H)-yl)-2-(hydroxymethyl)pyrrolidin-1-yl)butanenitrile | 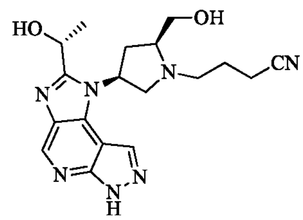 |

(continued)

| Chemical name | Structure |
|---|---|
| trans-4-(2-(3,4-Difluorophenyl)imidazo[4,5-d]pyrrolo[2,3- b]pyridin-1 (6H)-yl)cyclohexanecarbonitrile | 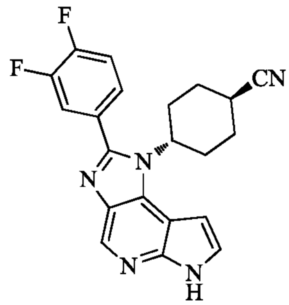 |
| (R)-1-(1-((S)-1-(3,3,3-Trifluoropropyl)pyrrolidin-3-yl)-1,6-dihydroimidazo [4,5-d]pyrrolo[2,3-b]pyridin-2-yl)ethanol | 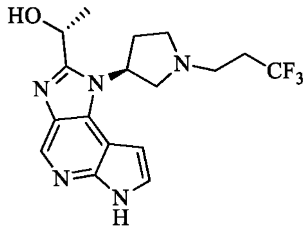 |
| (R)-1-(1-((S)-1-(3,3,4,4,4-Pentafluorobutyl)pyrrolidin-3-yl)-1,6-dihydroimidazo[4,5-d]pyrrolo[2,3-b]pyridin-2-yl)ethanol | 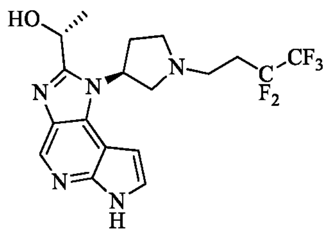 |
| (S)-(5-(1-(1-(4,4,4-trifluorobutyl)pyrrolidin-3-yl)-1,6-dihydroimidazo[4,5-d] pyrrolo[2,3-b]pyridin-2-yl)furan-2-yl)methanol | 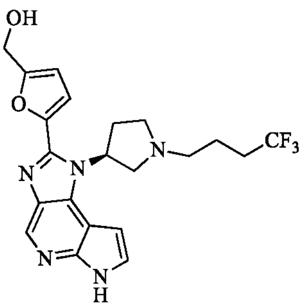 |
| (S)-3-(1-(1-(4,4,4-trifluorobutyl)pyrrolidin-3-yl)-1,6-dihydroimidazo[4,5-d] pyrrolo[2,3-b]pyridin-2-yl)phenol | 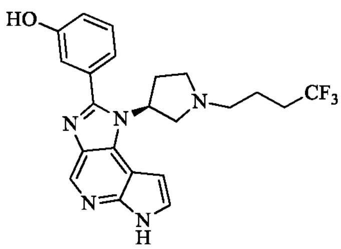 |
| (R)-1-(1-((S)-1-(2,2,3,3,4,4,4-heptafluorobutyl)pyrrolidin-3-yl)-1,6-dihydroimidazo[4,5-d]pyrrolo[2,3-b]pyridin-2-yl)ethanol | 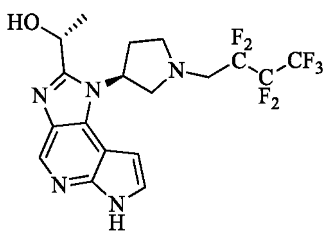 |

(continued)

| Chemical name | Structure |
|---|---|
| (R)-1-(1-((S)-1-(Perfluorobutyl)pyrrolidin-3-yl)-1,6-dihydroimidazo[4,5-d]pyrrolo[2,3-b]pyridin-2-yl)ethanol | 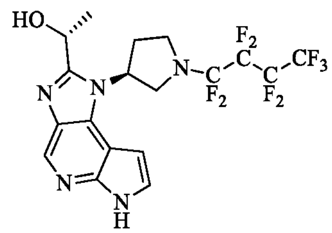 |
| (R)-1-(1-((S)-1-((3,3,3-Trifluoropropyl)sulfonyl)pyrrolidin-3-yl)-1,6-dihydroimidazo[4,5-d]pyrrolo[2,3-b]pyridin-2-yl)ethanol | 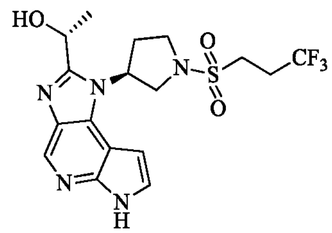 |
| 4-(4-(Imidazo[4,5-d]pyrrolo[2,3-b]pyridin-1(6H)-yl)-1H-pyrazol-1-yl)butanenitrile | 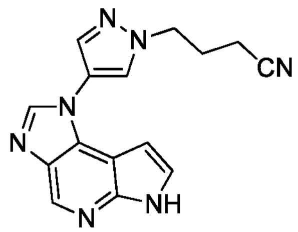 |
| 4-(4-(2-(5-methylfuran-2-yl)imidazo[4,5-d]pyrrolo[2,3-b]pyridin-1(6H)-yl)-1H-pyrazol-1-yl)butanenitrile | 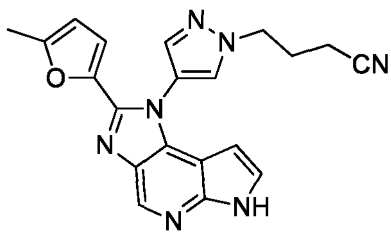 |
| 4-(4-(2-(5-Methylthiophen-2-yl)imidazo[4,5-d]pyrrolo[2,3-b]pyridin-1(6H)-yl)-1H-pyrazol-1-yl butanenitrile | 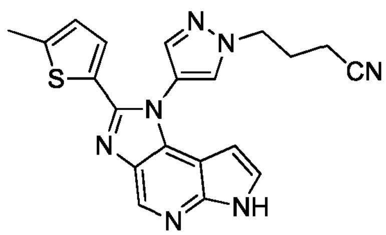 |
| 3-(4-(2-(Thiophen-2-yl)imidazo[4,5-d]pyrrolo[2,3-b]pyridin-1(6H)-yl)-1H-pyrazol-1-yl)propanenitrile | 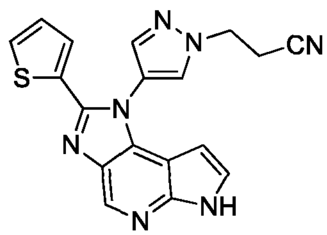 |

(continued)

| Chemical name | Structure |
|---|---|
| 4-(4-(2-Cyclobutylimidazo[4,5-d]pyrrolo[2,3-b]pyridin-1(6H)-yl)-1H-pyrazol-1-yl)butanenitrile | 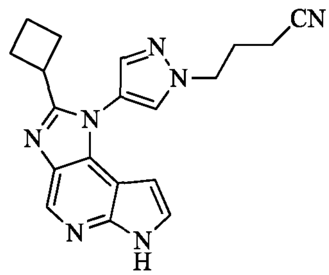 |
| 4-(4-(2-Cyclopropylimidazo[4,5-d]pyrrolo[2,3-b]pyridin-1(6H)-yl)-1H-pyrazol-1-yl)butanenitrile | 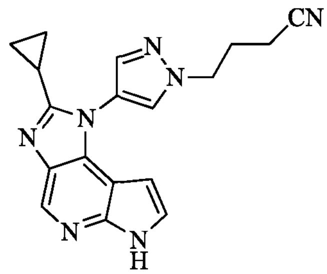 |

**10.** A method of preparing a compound as shown in Formula **I,** which includes the following step: reacting a compound as shown in Formula **II** with a base in an organic solvent to obtain said compound as shown in Formula **I**;

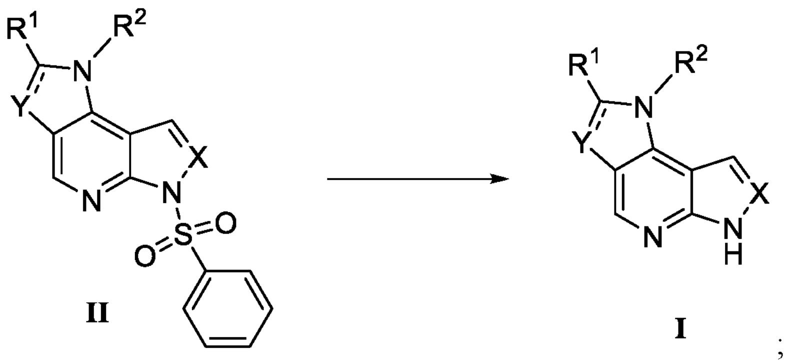

II

I

;

wherein X, Y, - - -, $R^1$ and $R^2$ are defined as described in at least one of claims 1-9.

**11.** A compound as shown in Formula **II**:

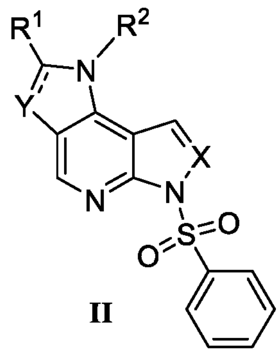

II

;

wherein X, Y, - - -, $R^1$ and $R^2$ are defined as described in at least one of claims 1-9.

**12.** A pharmaceutical composition comprising

(i) the compound of formula **I** as claimed in at least one of claims 1-9, or a tautomer, stereoisomer, racemate or isotopic derivative thereof, or a pharmaceutically acceptable salt of any of the foregoing, or a crystalline or solvated form of any of the foregoing; and
(ii) a pharmaceutically acceptable vehicle.

**13.** A method for inhibiting Janus kinase in vivo, in vitro, or in vitro, comprising: contacting the compound of formula **I** as claimed in at least one of claims 1-9, or a tautomer, stereoisomer, racemate or isotopic derivative thereof, or a pharmaceutically acceptable salt of any of the foregoing, or a crystalline or solvated form of any of the foregoing, or the pharmaceutical composition as claimed in claim 12 with Janus kinase.

**14.** A method for treating diseases associated with Janus kinase in a subject in need thereof, comprising: administering a therapeutically effective amount of the compound of formula **I** as claimed in at least one of claims 1-9, or a tautomer, stereoisomer, racemate or isotopic derivative thereof, or a pharmaceutically acceptable salt of any of the foregoing, or a crystalline or solvated form of any of the foregoing, or the pharmaceutical composition as claimed in claim 12 to the subject; and
the mentioned diseases associated with Janus kinase can be autoimmune diseases or cancer.

## INTERNATIONAL SEARCH REPORT

International application No.
**PCT/CN2021/140062**

### A. CLASSIFICATION OF SUBJECT MATTER

C07D 471/14(2006.01)i; A61K 31/437(2006.01)i; A61P 35/00(2006.01)i; A61P 35/02(2006.01)i; A61P 37/06(2006.01)i; A61P 17/06(2006.01)i; A61P 19/02(2006.01)i; A61P 1/00(2006.01)i; A61P 25/28(2006.01)i; A61P 7/00(2006.01)i; A61P 29/00(2006.01)i

According to International Patent Classification (IPC) or to both national classification and IPC

### B. FIELDS SEARCHED

Minimum documentation searched (classification system followed by classification symbols)

C07D471/-; A61K31/-; A61P35/-; A61P37/-; A61P17/-; A61P19/-; A61P1/-; A61P25/-; A61P7/-; A61P29/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方, WANFANG; STN: 咪唑, 吡啶, 吡咯, 吡唑, 苯磺酰基, Janus激酶, JAK激酶, 癌, 肿瘤, 自身免疫性疾病, 李志裕, 卞金磊, 景甜, 徐鹏飞, 沈沛, 王举波, 邱志霞, 徐熙童, 中国药科大学, +imidazo+, +pyridin+, +pyrrolo+, +pyrazol+, +phenylsulfonyl+, China Pharmaceutical University, Janus kinase, JAK, cancer, tumor, neoplasm, autoimmune disease, 式I结构, the structure of formula I, 式II结构, the structure of formula II, CAS号152478-56-3, CAS Number 152478-56-3, CAS号152478-57-4, CAS Number 152478-57-4

### C. DOCUMENTS CONSIDERED TO BE RELEVANT

| Category* | Citation of document, with indication, where appropriate, of the relevant passages | Relevant to claim No. |
|---|---|---|
| E | CN 114149428 A (CHINA PHARMACEUTICAL UNIVERSITY) 08 March 2022 (2022-03-08)<br>claims 1-14 | 1-13 |
| X | XU, Pengfei et al. "Discovery of Imidazopyrrolopyridines Derivatives as Novel and Selective Inhibitors of JAK2"<br>*European Journal of Medicinal Chemistry,* Vol. vol. 218, 26 March 2021 (2021-03-26), ISSN: 0223-5234,<br>Table 1-3, Table 5, Scheme 1, Scheme 2, sections 4.2.5 and 4.2.6 | 1-13 |
| X | CN 102712640 A (F. HOFFMANN-LA ROCHE LTD.) 03 October 2012 (2012-10-03)<br>claims 1-21, description, pages 518 and 522, table, compounds 893, 930 and 931, page 567, page 568, page 211, embodiment 158, table 4, page 80, reaction process 14, pages 104-106 | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 August 2022** | **07 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/140062**

**C. DOCUMENTS CONSIDERED TO BE RELEVANT**

| Category* | Citation of document, with indication, where appropriate, of the relevant passages | Relevant to claim No. |
|---|---|---|
| A | CN 113396150 A (LYNK PHARMACEUTICALS CO., LTD.) 14 September 2021 (2021-09-14)<br>entire document | 1-13 |
| A | WO 2021104488 A1 (CHIA TAI TIANQING PHARMACEUTICAL GROUP CO., LTD. et al.) 03 June 2021 (2021-06-03)<br>entire document | 1-13 |
| A | WO 2013007765 A1 (F. HOFFMANN-LA ROCHE AG) 17 January 2013 (2013-01-17)<br>entire document | 1-13 |

Form PCT/ISA/210 (second sheet) (January 2015)

**INTERNATIONAL SEARCH REPORT**

International application No.
**PCT/CN2021/140062**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13(部分),14**
because they relate to subject matter not required to be searched by this Authority, namely:

[1] The method for the in vivo inhibition of Janus kinases of claim 13 and the method for treating diseases related to Janus kinases in a subject of claim 14 belong to the scope of treatment methods for living human or animal bodies, and belong to the cases set out in PCT Rule 39.1(iv) for which an international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/140062**

| Patent document cited in search report | Publication date (day/month/year) | Patent family member(s) | Publication date (day/month/year) |
|---|---|---|---|
| CN 114149428 A | 08 March 2022 | None | |
| CN 102712640 A | 03 October 2012 | WO 2011086053 A1 | 21 July 2011 |
| | | BR 112016017269 A2 | 08 August 2017 |
| | | JP 2013517220 A | 16 May 2013 |
| | | US 2011201593 A1 | 18 August 2011 |
| | | MX 2012008049 A | 01 August 2012 |
| | | RU 2012132278 A | 20 February 2014 |
| | | BR 112012017269 A2 | 03 May 2016 |
| | | AR 079984 A1 | 07 March 2012 |
| | | TW 201129565 A | 01 September 2011 |
| | | EP 2523957 A1 | 21 November 2012 |
| | | CA 2781578 A1 | 21 July 2011 |
| | | KR 20140015162 A | 06 February 2014 |
| CN 113396150 A | 14 September 2021 | US 2022009927 A1 | 13 January 2022 |
| | | CA 3115897 A1 | 07 May 2020 |
| | | WO 2020088659 A1 | 07 May 2020 |
| | | JP 2022506908 A | 17 January 2022 |
| | | EP 3856742 A1 | 04 August 2021 |
| | | TW 202035408 A | 01 October 2020 |
| | | KR 20210090173 A | 19 July 2021 |
| | | SG 11202103694U A | 28 May 2021 |
| | | IL 282786 A | 30 June 2021 |
| | | AU 2019372677 A1 | 13 May 2021 |
| | | BR 112021008128 A2 | 03 August 2021 |
| WO 2021104488 A1 | 03 June 2021 | AU 2020390378 A1 | 09 June 2022 |
| | | CA 3162572 A1 | 03 June 2021 |
| | | TW 202200579 A | 01 January 2022 |
| WO 2013007765 A1 | 17 January 2013 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BERGE et al.** Pharmaceutical Salts. *Journal of Pharmaceutical Science,* 1977, vol. 66, 1-19
- Handbook of Pharmaceutical Salts: Properties, Selection, and Use. Wiley-VCH, 2002